(19) **Europäisches Patentamt / European Patent Office / Office européen des brevets**

(11) **EP 3 875 605 B1**

(12) **EUROPEAN PATENT SPECIFICATION**

(45) Date of publication and mention
of the grant of the patent:
**08.05.2024 Bulletin 2024/19**

(21) Application number: **21155139.5**

(22) Date of filing: **09.05.2016**

(51) International Patent Classification (IPC):
***C12Q 1/6883*** *(2018.01)*

(52) Cooperative Patent Classification (CPC):
**C12Q 1/6883;** C12Q 2600/158; C12Q 2600/178

(54) **METHOD FOR DIAGNOSIS AND PROGNOSIS OF CHRONIC HEART FAILURE**

DIAGNOSE- UND PROGNOSEVERFAHREN FÜR CHRONISCHE HERZINSUFFIZIENZ

PROCÉDÉ DE DIAGNOSTIC ET DE PRONOSTIC D'INSUFFISANCE CARDIAQUE CHRONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.05.2015 SG 10201503644Q**

(43) Date of publication of application:
**08.09.2021 Bulletin 2021/36**

(62) Document number(s) of the earlier application(s) in
accordance with Art. 76 EPC:
**16793096.5 / 3 294 910**

(73) Proprietors:
• **Agency for Science, Technology and Research**
**Singapore 138632 (SG)**
• **National University Hospital (Singapore) Pte Ltd**
**Singapore 119074 (SG)**
• **National University of Singapore**
**Singapore 119077 (SG)**

(72) Inventors:
• **ZOU, Ruiyang**
**138668 Singapore (SG)**
• **ZHOU, Lihan**
**138668 Singapore (SG)**
• **TOO, Heng-Phon**
**138668 Singapore (SG)**
• **RICHARDS, Arthur Mark**
**119228 Singapore (SG)**
• **WONG, Lee Lee**
**320113 Singapore (SG)**
• **LAM, Su Ping Carolyn**
**119228 Singapore (SG)**

(74) Representative: **Mewburn Ellis LLP**
**Aurora Building**
**Counterslip**
**Bristol BS1 6BX (GB)**

(56) References cited:
**WO-A2-2011/133036 WO-A2-2014/187884
DE-A1-102012 101 557**

• **RAFFAELE MARFELLA ET AL: "Circulating
microRNA changes in heart failure patients
treated with cardiac resynchronization therapy:
responders vs. non-responders", EUROPEAN
JOURNAL OF HEART FAILURE, vol. 15, no. 11,
27 November 2013 (2013-11-27), pages
1277-1288, XP055331263, NL ISSN: 1388-9842,
DOI: 10.1093/eurjhf/hft088**
• **Affymetrix: "GeneChip miRNA 4.0 Array", , 3
December 2013 (2013-12-03), XP055733360,
Retrieved from the Internet:
URL:https://www.carrerasresearch.org/en/ge
nechip-mirna-4-0-array_38720.pdf [retrieved on
2020-09-23]**
• **TIJSEN ANKE J ET AL: "MiR423-5p as a
circulating biomarker for heart failure",
CIRCULATION RESEARCH, AMERICAN HEART
ASSOCIATION, INC, US, vol. 106, no. 6, 2 April
2010 (2010-04-02) , pages 1035-1039,
XP002586118, ISSN: 1524-4571, DOI:
10.1161/CIRCRESAHA.110.218297**

Remarks:
The complete document including Reference
Table(s) and the Sequence Listing(s) can be
downloaded from the EPO website

**Description**

**FIELD OF THE INVENTION**

The present disclosure relates generally to the field of molecular biology. In particular, the present disclosure relates to the use of biomarkers for the detection and diagnosis of heart failure.

**BACKGROUND OF THE INVENTION**

Cardiovascular disease including heart failure is a major health problem accounting for about 30% of human deaths worldwide [1]. Heart failure is also the leading cause of hospitalization in adults over the age of 65 years globally [2]. Adults at middle age have a 20% risk of developing heart failure in their life time. Despite treatment advances, morbidity and mortality (~50% at 5 years) for heart failure remain high and consume about 2% of health care budgets in many economies [3-6]. The prevalence of heart failure will increase due to the aging of the population, increasing prevalence of major risk factors such as diabetes, obesity and increased initial survival in acute myocardial infarction and severe hypertension.

Heart failure has been traditionally viewed as a failure of contractile function and left ventricular ejection fraction (LVEF) has been widely used to define systolic function, assess prognosis and select patients for therapeutic interventions. However, it is recognised that heart failure can occur in the presence of normal or near-normal EF: so-called "heart failure with preserved ejection fraction (HFPEF)" which accounts for a substantial proportion of clinical cases of heart failure [7-9]. Heart failure with severe dilation and/or markedly reduced EF: so- called "heart failure with reduced ejection fraction (HFREF)" is the best understood type of heart failure in terms of pathophysiology and treatment [10]. There are some epidemiological differences between patients with HFREF and those with HFPEF. The latter are generally older and more often women, are less likely to have coronary artery disease (CAD) and more likely to have underlying hypertension [7, 8, 11]. In addition, patients with HFPEF do not obtain similar clinical benefits from angiotensin converting enzyme inhibition or angiotensin receptor blockade as patients with HFREF [12, 13]. The symptoms of heart failure may develop suddenly- 'acute heart failure' leading to hospital admission, but they can also develop gradually.

Timely diagnosis, categorization of heart failure subtype - HFREF or HFPEF, and improved risk stratification are critical for the management and treatment of heart failure. Accordingly, there is a need to provide for methods of determining the risk of a subject in developing heart failure. There is also a need to provide for methods of categorizing heart failure subtypes.

WO2014/187884A2 relates to a method for prognosis of heart failure based on the determination of expression profiles of at least one miRNAs representative for prognosis of heart failure compared to a reference. DE102012101557A1 relates to the use of certain microRNAs and / or genes both individually and in combination as markers for identifying individual non-ischemic cardiomyopathies or storage diseases of the heart, and their use in diagnostic systems. MarΣella et al 2013 "Circulating microRNA changes in heart failure patients treated with cardiac resynchronization therapy: responders vs. non-responders" investigated the effects of long-term cardiac resynchronization therapy on changes of circulating miRNAs potentially involved in structural alterations of the failing heart. The Affymetrix "GeneChip miRNA 4.0 Array" data sheet describes a microarray chip that provides a complete measurement of small non-coding RNA transcripts involved in gene regulation. WO2011/133036A2 provides means and methods for identifying and/or treating a patient suffering from or at risk of suffering from cardiovascular disease by measuring mi RNA present in a sample of said patient.

**SUMMARY OF THE INVENTION**

The invention is defined in the appended claims. In one aspect, there is provided a method of determining whether a subject suffers from heart failure or is at risk of developing heart failure. In some examples, the method includes the steps of a) measuring the level of at least one miRNA from a list of miRNAs "increased" or at least one from a list of miRNAs "reduced" as listed in Table 25, or Table 20, or Table 21, or Table 22, in a sample obtained from the subject. In some examples, the method further includes the step of b) determining whether the level of at least one miRNA from a list of miRNAs is different as compared to a control, wherein altered levels of the miRNA indicates that the subject has heart failure or is at a risk of developing heart failure.

In another aspect, there is provided a method of determining whether a subject suffers from a heart failure. In some examples, the heart failure is selected from the group consisting of a heart failure with reduced left ventricular ejection fraction (HFREF) and a heart failure with preserved left ventricular ejection fraction (HFPEF). In some examples, the method includes the steps of a) detecting the level of at least one miRNA as listed in Table 9 in a sample obtained from the subject. In some examples, the method further includes the step of determining whether the levels of the at least one miRNA indicates that the subject has, or is at a risk of, developing heart failure with reduced left ventricular ejection fraction (HFREF) or heart failure with preserved left ventricular ejection fraction (HFPEF).

In yet another aspect, there is provided a method for determining the risk of a heart failure patient having an altered risk of death. In some examples, the method includes the steps of a) detecting the levels of at least one miRNA as listed in Table 14 in a sample obtained from the subject. In some examples, the method also includes the step of b) measuring the levels of at least one miRNAs listed in Table 14. In some examples, the method also includes the step of c) determining whether the levels of at least one miRNAs listed in Table 14 is different as compared to the levels of the miRNAs of a control population, wherein altered levels of the miRNA indicates that the subject is likely to have an altered risk of death (altered observed (all-cause) survival rate) compared to the control population.

In yet another aspect, there is provided a method for determining the risk of a heart failure patient having an altered risk of disease progression to hospitalization or death. In some examples, the method comprises the step of a) detecting the levels of at least one miRNA as listed in Table 15 in a sample obtained from the subject. In some examples, the method includes the step of b) measuring the levels of at least one miRNAs listed in Table 15. In some examples, the method further includes the step of c) determining whether the levels of at least one miRNAs listed in Table 15 is different as compared to the levels of the miRNAs of a control population, wherein altered levels of the miRNA indicates that the subject is likely to have an altered risk of disease progression to hospitalization or death (altered event free survival rate) compared to the control population.

In yet another aspect, there is provided a method of determining the risk of developing heart failure in a subject or determining whether a subject suffers from heart failure. In some examples, the method includes the step of: (a) detecting the presence of miRNA in a sample obtained from the subject. In some examples, the method further includes the step of (b) measuring the levels of at least three miRNAs listed in Table 16 or Table 23 in the sample. In some examples, the method further includes the step of (c) using a score based on the levels of the miRNAs measured in step (a) to predict the likelihood of the subject to develop or to have heart failure.

In yet another aspect, there is provided a method of determining the risk of developing heart failure in a subject or determining whether a subject suffers from heart failure. In some examples, the method includes the steps of: (a) detecting the presence of miRNA in a sample obtained from the subject. In some examples, the method also includes the step of (b) measuring the levels of at least three miRNAs listed in Table 17 in the sample. In some examples, the method also includes the step of (c) using a score based on the levels of the miRNAs measured in step (a) to predict the likelihood of the subject to develop or to have heart failure.

In yet another aspect, there is provided a method of determining the likelihood of a subject to be suffering from a heart failure with reduced left ventricular ejection fraction (HFREF) or a heart failure with preserved left ventricular ejection fraction (HFPEF). In some examples, the method includes the step of: (a) detecting the presence of miRNA in a sample obtained from the subject. In some examples, the method includes the step of (b) measuring the levels of at least three miRNA listed in Table 18 in the sample. In some examples, the method includes (c) using a score based on the levels of the miRNAs measured in step (a) to predict the likelihood of the subject to be suffering from, a heart failure with reduced left ventricular ejection fraction (HFREF) or a heart failure with preserved left ventricular ejection fraction (HFPEF).

In yet another aspect, there is provided a method of determining the likelihood of a subject having a heart failure with reduced left ventricular ejection fraction (HFREF) or a heart failure with preserved left ventricular ejection fraction (HFPEF). In some examples, the method includes the step of: (a) detecting the presence of miRNA in a sample obtained from the subject. In some examples, the method includes the step of (b) measuring the levels of at least three miRNAs listed in Table 19 or Table 24 in the sample. In some examples, the method also includes the step of (c) using a score based on the levels of the miRNAs measured in step (a) to predict the likelihood of the subject to be suffering from, a heart failure with reduced left ventricular ejection fraction (HFREF) or a heart failure with preserved left ventricular ejection fraction (HFPEF).

## BRIEF DESCRIPTION OF THE DRAWINGS

The disclosure will be better understood with reference to the detailed description when considered in conjunction with the non-limiting examples and the accompanying drawings, in which:

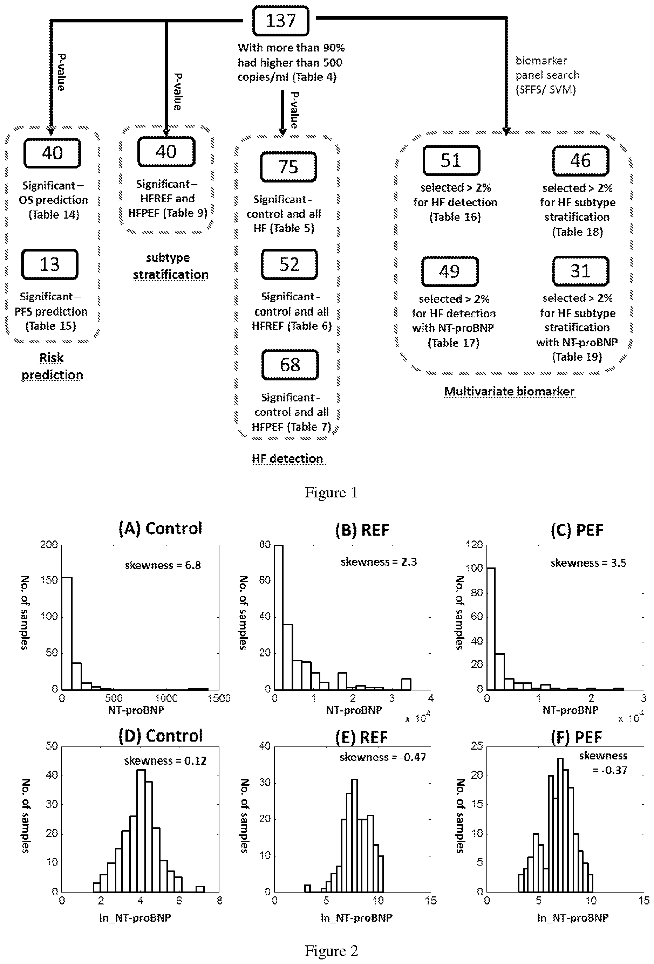

**Figure 1** shows a schematic diagram showing a summary of the number of miRNAs identified from studies described herein.

**Figure 2** shows histogram and skewness diagrams of N-terminal prohormone of brain natriuretic peptide (NT-proBNP) and natural logarithm of the N-terminal prohormone of brain natriuretic peptide level (ln_NT-proBNP). Distribution of NT-proBNP level (A-C) and ln_NT-proBNP level (the natural logarithm of NT-proBNP, D-F) for the control subjects (A, D), heart failure with reduced left ventricular ejection fraction subjects (HFREF) (B, E) and heart failure with preserved left ventricular ejection fraction subjects (HFPEF) (C, F). The skewness of each graph was calculated and is displayed. Figure 2 shows that the N-terminal prohormone of brain natriuretic peptide (NT-proBNP) in all groups was positively skewed. In contrast, the natural logarithm of the N-terminal prohormone of brain natriuretic peptide (ln_NT-proBNP) level has less skewness. Therefore, the natural logarithm of the N-terminal prohormone of brain natriuretic peptide level was used for all analysis involving NT-proBNP.

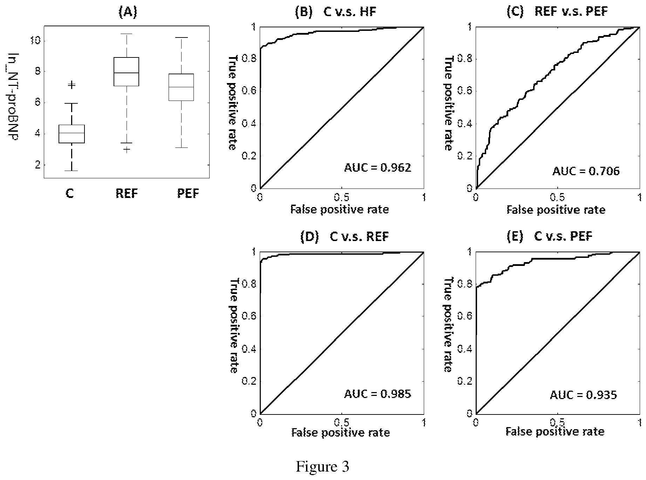

**Figure 3** shows the results of the analysis of the performance of natural logarithm of the N-terminal prohormone of brain natriuretic peptide (ln_NT-proBNP) as a biomarker for heart failure. In particular, (A) shows a boxplot representation of ln_NT-proBNP (the natural logarithm of NT-proBNP) levels. Each boxplot presents the 25th, 50th, and 75th percentiles in the distribution. (B-D) show the receiver operating characteristic curves of ln_NT-proBNP for the of control vs heart failure (HFREF and HFPEF, B), HFREF vs heart HFPEF (C), control vs HFREF (D) and control vs HFPEF (E). AUC: area under the receiver operating characteristic curve, C: control (healthy), HF: heart failure, HFREF: heart failure with reduced left ventricular ejection fraction subjects, HFPEF: heart failure with preserved left ventricular ejection fraction subjects. Figure 3A shows the loss of NT-proBNP test performance is more pronounced in HFPEF. Figure 3B-D shows the natural logarithm of the N-terminal prohormone of brain natriuretic peptide (ln_NT-proBNP) performed better in detecting HFREF than HFPEF.

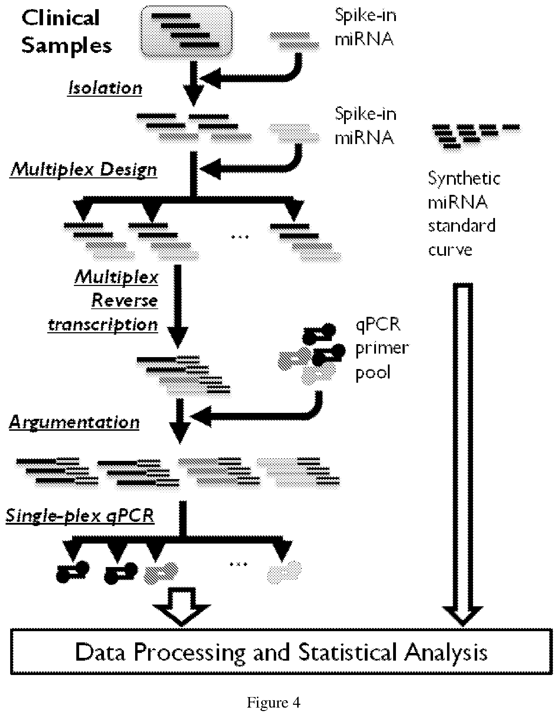

**Figure 4** shows an exemplary workflow of a high-throughput miRNA RT-qPCR measurement. The steps shown in Figure 4 includes isolation, multiplex groups, multiplex RT, augmentation, single-plex PCR and synthetic miRNA standard curve. Details of each steps are as follows: Isolation refers to the step of isolating and purifying the miRNA from plasma samples; Spike-in miRNA refers to the non-natural synthetic miRNAs mimics (small single-stranded RNA with length range from 22-24 bases) that were added into the samples to monitor the efficiencies at each step including isolation, reverse transcription, augmentation and qPCR; Multiplex Design refers to the miRNA assays that were deliberately divided into a number of multiplex groups (45-65 miRNA per group) *in silico* to minimize non-specific amplifications and primer-primer interaction during the RT and augmentation processes; Multiplex reverse transcription refers to the various pools of reverse transcription primers that were combined and added to different multiplex groups to generate cDNA; Augmentation refers to a pool of PCR primers were combined and added to the each cDNA pool generated from a certain multiplex group and the optimized touch down PCR was carried out to enhance the amount of all cDNAs in the group simultaneously; Single-plex qPCR refers to the augmented cDNA pools that were distributed in to various wells in the 384 well plates and single-plex qPCR reactions were then carried out; and Synthetic miRNA standard curve refers to Synthetic miRNA stand curves that were measured together with the samples for the interpolation of absolute copy numbers in all the measurements.

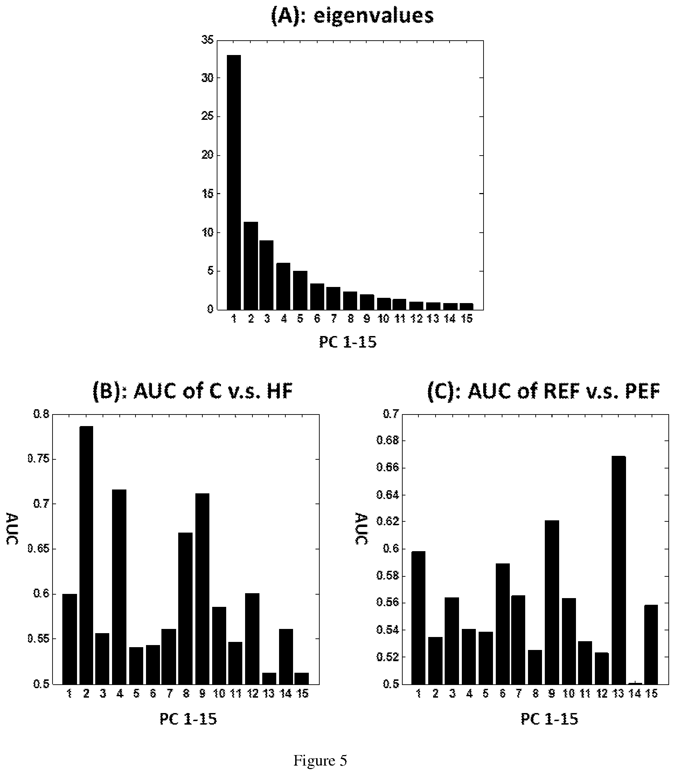

**Figure 5** shows bar graph results of principal component analysis. Principal component analysis was performed for all 137 reliably detected mature miRNA (Table 4) based on the log2 scale expression levels (copy/mL). (A): the eigenvalues for the topped 15 principal components. (B), the classification efficiencies (AUC) of the topped 15 principal components on separating control (C) and heart failure (HF). (C), the classification efficiencies (AUC) of the topped 15 principal components on separating HFREF (heart failure with reduced ejection fraction) and HFPEF (heart failure with preserved ejection fraction). AUC: area under the receiver operating characteristic curve. Figure 5 shows a multivariate assay may be required to capture the information in multiple dimensions for the classification of HFREF and HFPEF.

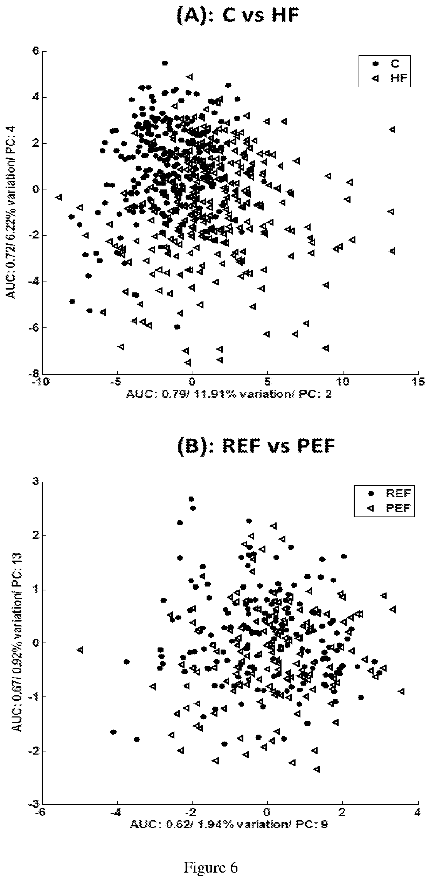

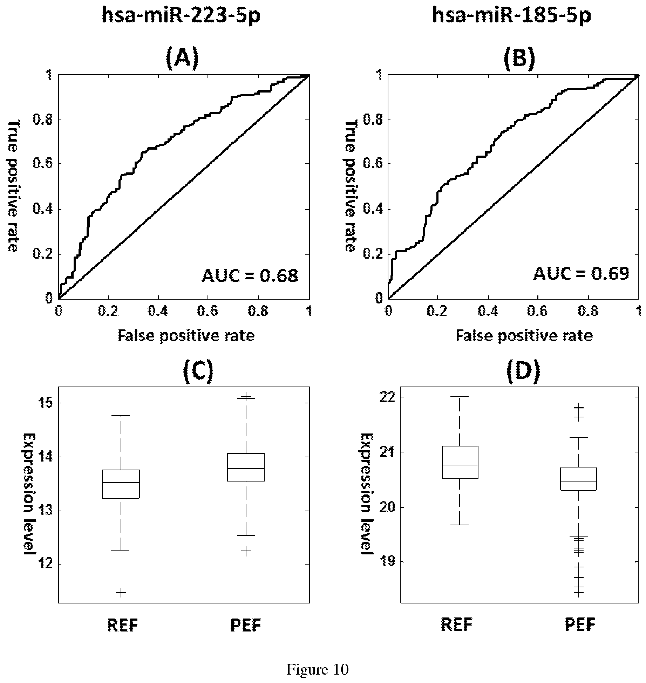

**Figure 6** shows a scatter plot of the top (AUC) principal components in heart failure subjects as compared to control. In particular, the top (AUC) principal components used for discrimination between control (C, black cycle) and heart failure (HF, white triangle) subjects are shown in A. The top (AUC) two principal components for distinguishing HFREF (heart failure with reduced ejection fraction, black cycle) from HFPEF (heart failure with preserved ejection fraction, white triangle) subjects are shown in B. AUC: area under the receiver operating characteristic curve. PC: principal component number based on Figure 10. Variation: the percentage of the variations represented by the principal components calculated by eigenvalues. Figure 6 shows it is possible to separate the control, HFREF and HFPEF subjects based on their miRNA profiles.

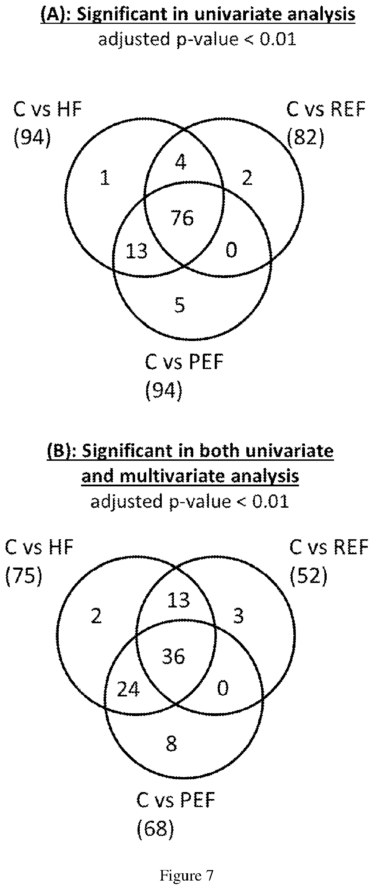

**Figure 7** shows Venn diagrams showing the overlap of biomarkers that could be used for the detection of heart failure. The comparisons between control (healthy) and various groups of heart failure patients (HF, HFREF and HFPEF) were carried out by univariate analysis (t-test) and multivariate analysis (logistic regression) incorporating age and AF (Atrial Fibrillation or Flutter), hypertension and diabetes. For the three comparisons: C vs HF (HFREF and HFPEF), C vs HFREF and C vs HFPEF, the numbers and overlaps of miRNAs with p-values (after false discovery rate correction) lower than 0.01 for in univariate analysis (A) and multivariate analysis (B) are shown. HF: heart failure, HFPEF: heart failure with preserved ejection fraction, HFREF: heart failure with reduced ejection fraction, C: control (healthy). Figure 7 shows many of the miRNAs were found to differ between control and only one of the two heart failure subtypes, thus demonstrate genuine differences between the two subtypes in terms of miRNA expression.

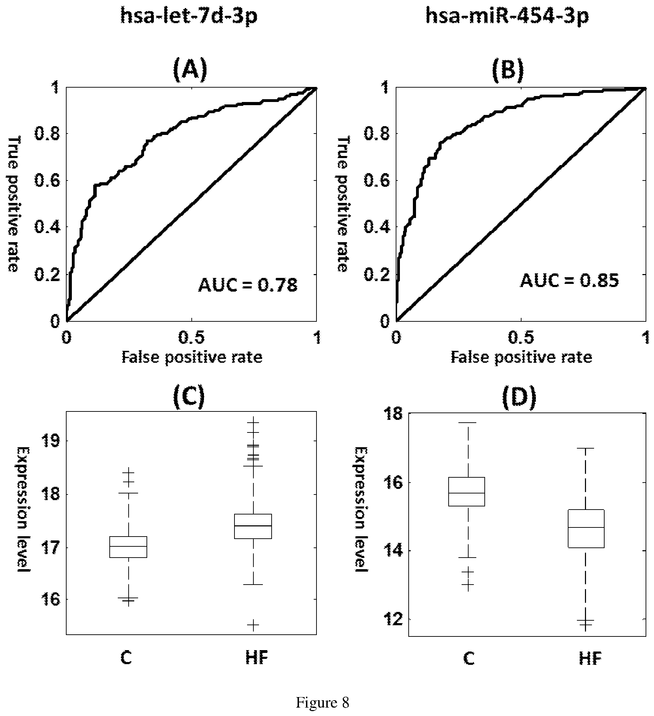

**Figure 8** shows boxplot and receiver operative characteristics curves of the top up-regulated and down-regulated miRNAs between healthy control and heart failure patients. The boxplot and receiver operating characteristic (ROC) curves of top (based on AUC) up-regulated (A: ROC curve, C: boxplot) and down-regulated (B: ROC curve, D: boxplot) miRNAs in all heart failure patients compared to the control (healthy) subjects. The expression levels (copy/ml) of miRNAs were presented in log2 scale. The boxplot presented the 25th, 50th, and 75th percentiles in the distribution of the expression levels. C: control (healthy), HF: heart failure. AUC: area under the receiver operating characteristic curve. Figure 8 shows combination of multiple miRNAs may enhance the performance of heart failure diagnosis.

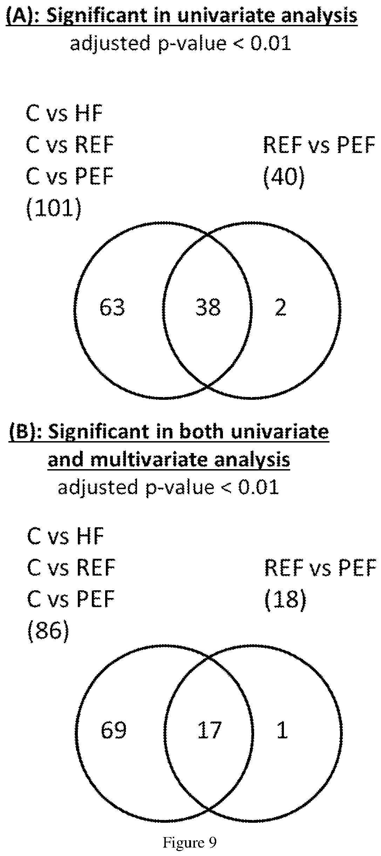

**Figure 9** shows Venn diagrams showing the overlap of biomarkers for the detection of heart failure and the categorization of heart failure subtypes. Comparisons between HFREF and HFPEF were carried out by univariate analysis (t-test) and multivariate analysis (logistic regression) incorporating age, gender, BMI (Body Mass Index) and AF (Atrial Fibrillation or Flutter), hypertension (p-value, ln_BNP). The miRNAs with p-values (after false discovery rate correction) lower than 0.01 in univariate analysis (A) and multivariate analysis (B) were compared to the miRNAs for the detection of heart failure (either C vs HF or C vs HFREF or C vs HFPEF, Figure 5). HF: heart failure, HFPEF: heart failure with preserved ejection fraction, HFREF: heart failure with reduced ejection fraction, C: control (healthy) subject.

**Figure 10** shows boxplots and receiver operating characteristics (ROC) curve of top up-regulated and down-regulated miRNAs in HFPEF patients compared to that of HFREF patients. The boxplot and receiver operating characteristic (ROC) curves of topped (based on AUC) up-regulated (A: ROC curve, C: boxplot) and down-regulated (B: ROC curve, D: boxplot) miRNAs in HFPEF patients compared to that of HFREF patients. The expression levels (copy/ml) of miRNAs were presented in log2 scale. The boxplot presented the 25th, 50th, and 75th percentiles in the distribution of the expression levels. HFPEF: heart failure with preserved ejection fraction, HFREF: heart failure with reduced ejection fraction, AUC: area under the receiver operating characteristic curve. Figure 10 shows that combining the multiple miRNAs in a multivariate index assay may provide more diagnostic power for subtype categorization.

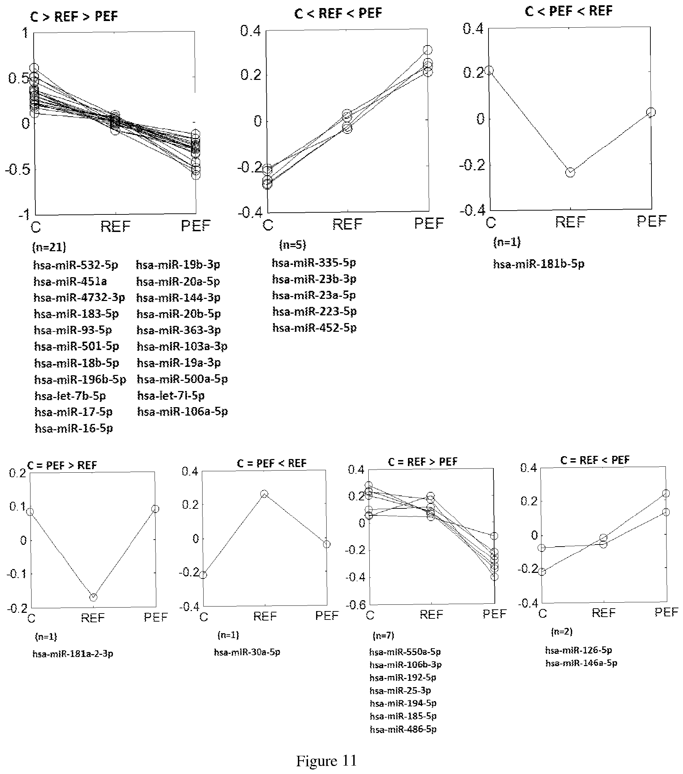

**Figure 11** shows line graphs of the overlapped miRNAs for the detection of heart failure and for the categorization of heart failure subtypes. The 38 overlapped miRNAs between control, heart failure (HFREF or HFPEF) and HFREF, HFPEF (Figure 7, A) were separated into 7 groups based on the changes. The two groups were defined as equal if the p-value (t-test) of the miRNA after false discovery test was higher than 0.01. The expression levels were based on the log2 scale and were standardized to zero mean for each miRNA. HFPEF: heart failure with preserved ejection fraction, HFREF: heart failure with reduced ejection fraction, C: control (healthy). Figure 11 shows that unlike the LVEF and NT-proBNP, HFPEF had more distinct miRNA profiles than the HFREF subtype compared to the healthy control. Figure 11 demonstrates miRNA could complement NT-proBNP to provide better discrimination of HFPEF.

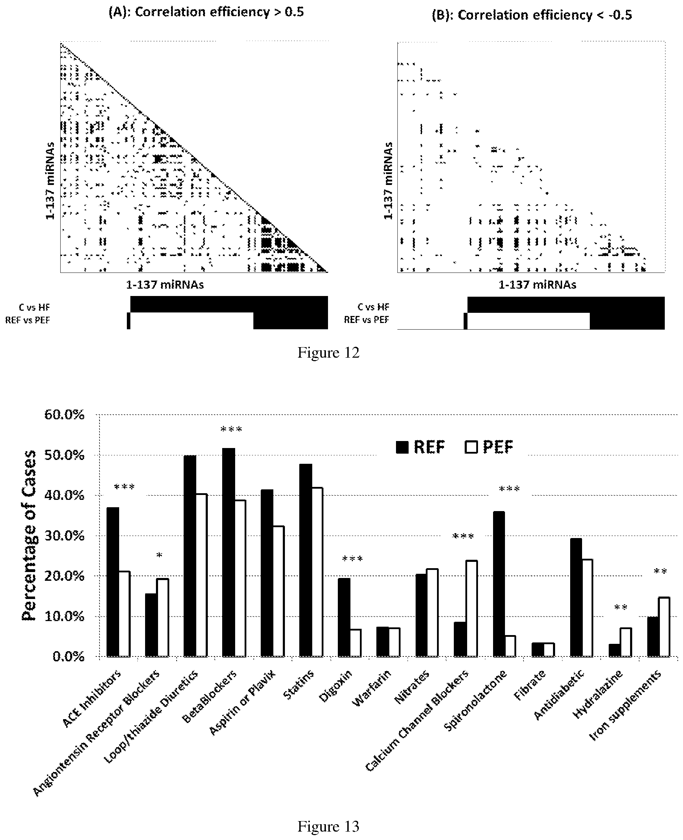

**Figure 12** shows the scatter plot of the correlation analysis between all reliably detected miRNAs. Based on the log2 scale expression levels (copy/mL), Pearson's linear correlation coefficients were calculated between all 137 reliable detected miRNA targets (Table 4). Each dot represents a pair of miRNAs where the correlation coefficient is higher than 0.5 (A, positively correlated) or below -0.5 (B, negatively correlated). The differentially expressed miRNAs for C vs HF and HFREF vs HFPEF are indicated as black in the horizontal dimension. HF: heart failure, HFPEF: heart failure with preserved ejection fraction, HFREF: heart failure with reduced ejection fraction, C: control (healthy). Figure 12 demonstrates that many pairs of miRNAs were regulated similarly among all subjects.

**Figure 13** shows bar graph representing the pharmacotherapy for HFREF and HFPEF. The numbers of cases for various anti-HF drug treatments are summarized for the 327 subjects included in the prognosis analysis, divided into HFREF and HFPEF subtypes. The Chi-square test was applied to compare the two subtypes for each treatment. *: p-value < 0.05, **: p-value < 0.01, * * *: p-value < 0.001. Figure 13 is a summary of treatments according to the current clinical practice and was included among clinical variables for the analysis of prognostic markers.

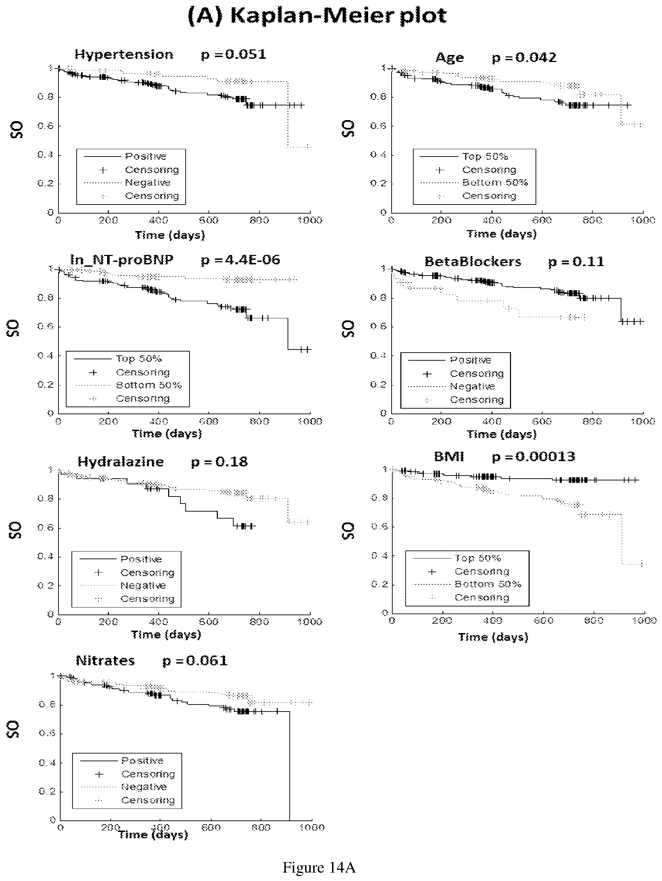

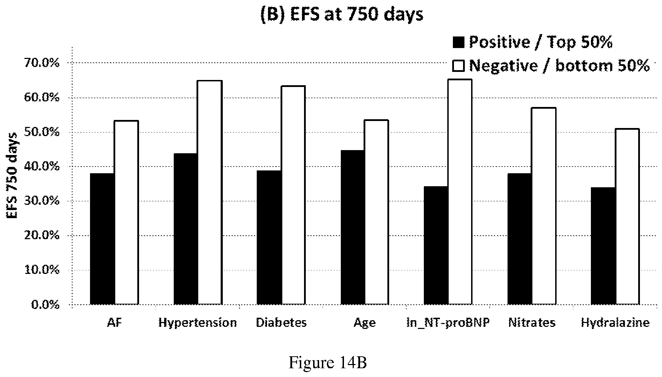

**Figure 14** shows the survival analyses of subjects. In particular, (A) shows the Kaplan-Meier plots of clinical variables significantly predictive of observed survival (Table 14) based on univariate analysis (p-values < 0.05). For the categorical variables, the positive group (black) and negative groups (gray) were compared. For normally distributed variables, subjects with supra-median (black) and infra-median (gray) values were compared. The log-rank test was performed to test the between the two groups for each variable and the p-values were shown above each plot. (B) shows a bar graph representing the percentage of observed survival (OS) at 750 days after treatment.

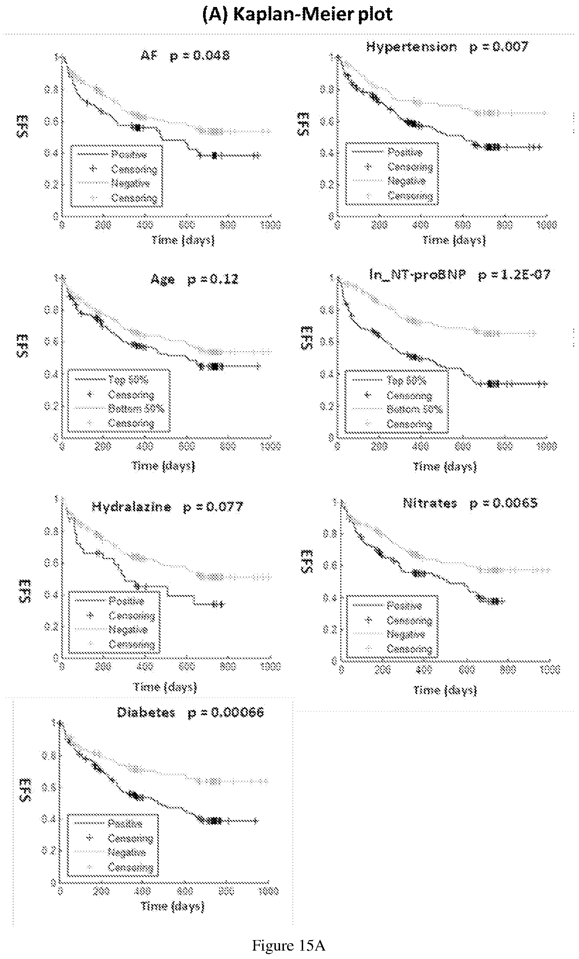

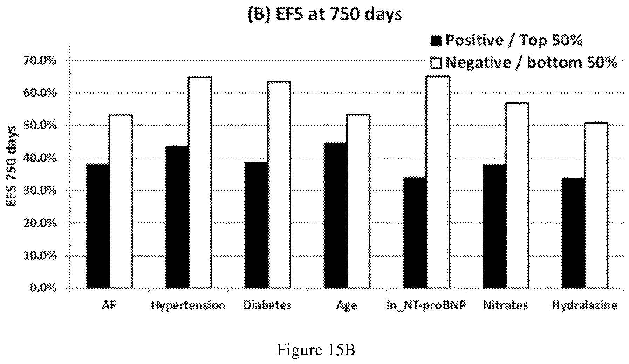

**Figure 15** shows the survival analysis for event free survival. In particular, (A) shows Kaplan-Meier plots of clinical variables significantly predictive of for event free survival (Table 14) based on univariate analysis (p-values < 0.05). For the categorical variables, the positive group (black) and negative groups (gray) were compared. For normally distributed variables, subjects with supra-median (black) and infra-median (gray) values were compared. The log-rank test was performed to test the between the two groups for each variable and the p-values were shown above each plot. (B), shows bar graph representing the percentage of event free survival (EFS) at 750 days after treatment.

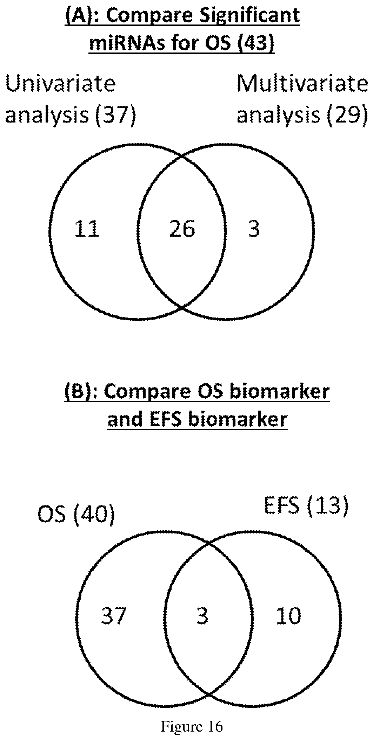

**Figure 16** shows Venn diagrams of the comparison between biomarkers for observed survival (OS) and event free survival (EFS). In particular, (A) shows the comparison between the miRNAs significantly prognostic for OS identified by univariate analysis and multivariate analysis with CoxPH model. (B) shows the comparison between the significant miRNAs for the prognosis of OS and for the prognosis of EFS. The miRNAs were either identified by univariate analysis or multivariate analysis with CoxPH model. Figure 16 demonstrates differing mechanisms for death and recurrent decompensated heart failure.

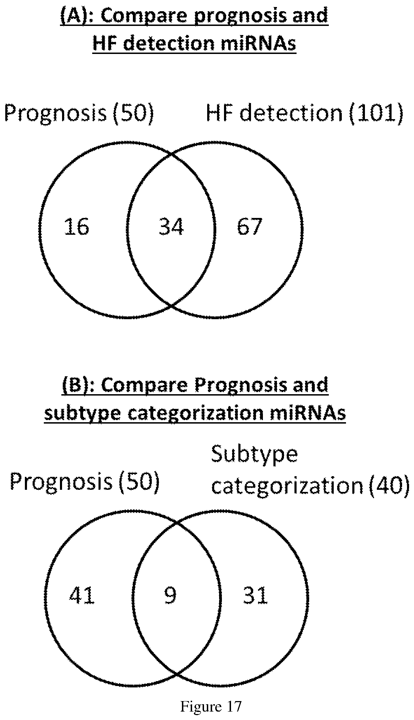

**Figure 17** shows Venn diagrams of the comparison between biomarkers for observed survival (OS) and event free survival (EFS). In particular, (A) shows the comparison between the miRNAs significantly prognostic by CoxPH model (either for OS or for EFS) and for detection of HF (either subtype). All the miRNAs were either identified by univariate analysis or multivariate analysis. (B) shows the comparison between the significant miRNAs for the prognosis identify by CoxPH model (either for OS or for EPS) and for categorization of two HF subtypes. All the miRNAs were either identified by univariate analysis or multivariate analysis. Figure 17 shows a large portion of the prognostic markers were not found in the other two lists indicating that a separate set of miRNA may be used or combined to form an assay for the prognosis.

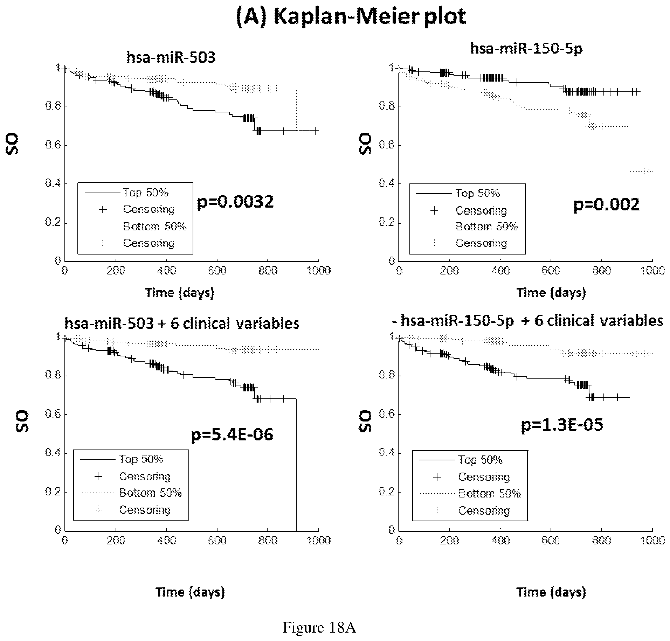

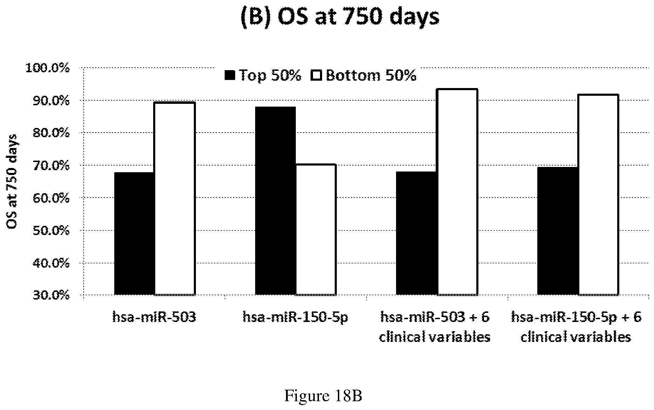

**Figure 18** shows the analysis of miRNA with maximum and minimum hazard ratio for observed survival (OS). In (A), miRNA with the maximum hazard ratio (hsa-miR-503) and minimum hazard ratio (hsa-miR-150-5p) for observed survival (OS) were used to construct the univariate CoxPH model or the multivariate CoxPH model including six additional clinical variables: gender, hypertension, BMI, ln_NT-proBNP, BetaBlockers and Warfarin for observed survival (OS). All the level of normal variables including BMI, ln _NT-proBNP and the miRNA expression level (log2 scale) were scaled to have one standard deviation. Based on the value of the explanation score according to the on CoxPH model, the top 50% of the subjects (black) and the bottom 50% of the subjects (gray) were compared. The log-rank test was performed to test the between the two groups and the p-values were shown (B), the observed survival (OS)at 750 days after treatment.

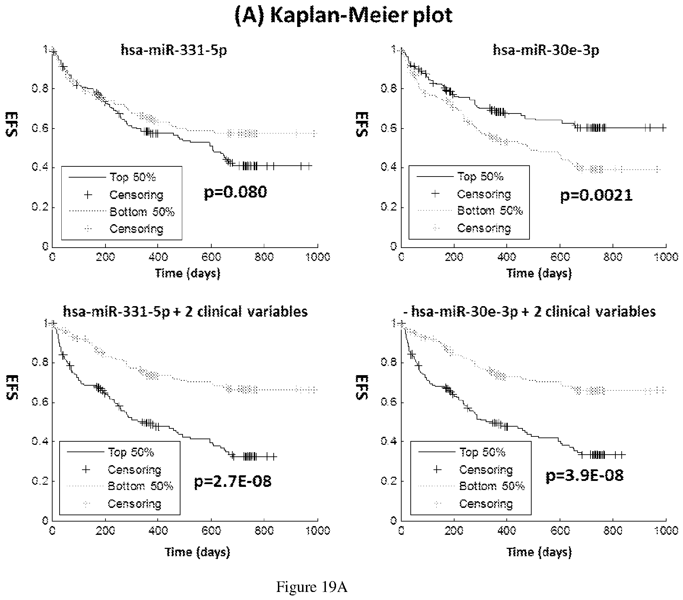

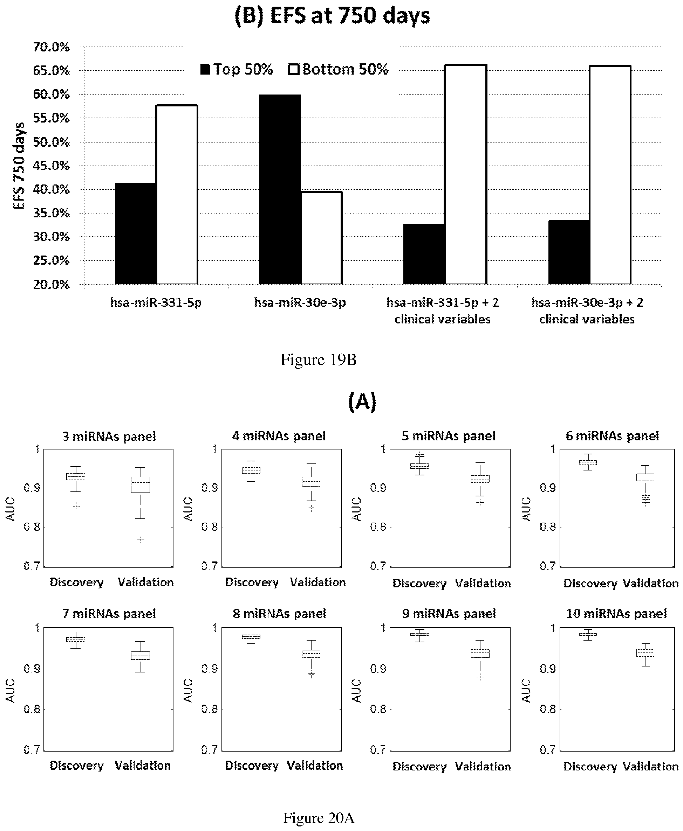

**Figure 19** shows the analysis of miRNA with maximum and minimum hazard ratio for EFS. In (A), the miRNA with the maximum hazard ratio (hsa-miR-331-5p) and minimum hazard ratio (hsa-miR-191-5p) for EFS were used to construct the univariate CoxPH model or the multivariate CoxPH model including 2 additional clinical variables: diabetes condition and ln_NT-proBNP for EFS. All the level of normal variables including diabetes condition ln_NT-proBNP and the miRNA expression level (log2 scale) were scaled to have one standard deviation. Based on the value of the explanation score according to the on CoxPH model, the top 50% of the subjects (black) and the bottom 50% of the subjects (gray) were compared. The log-rank test was performed to test the between the two groups and the p-values were shown (B), the EFS at 750 days after treatment.

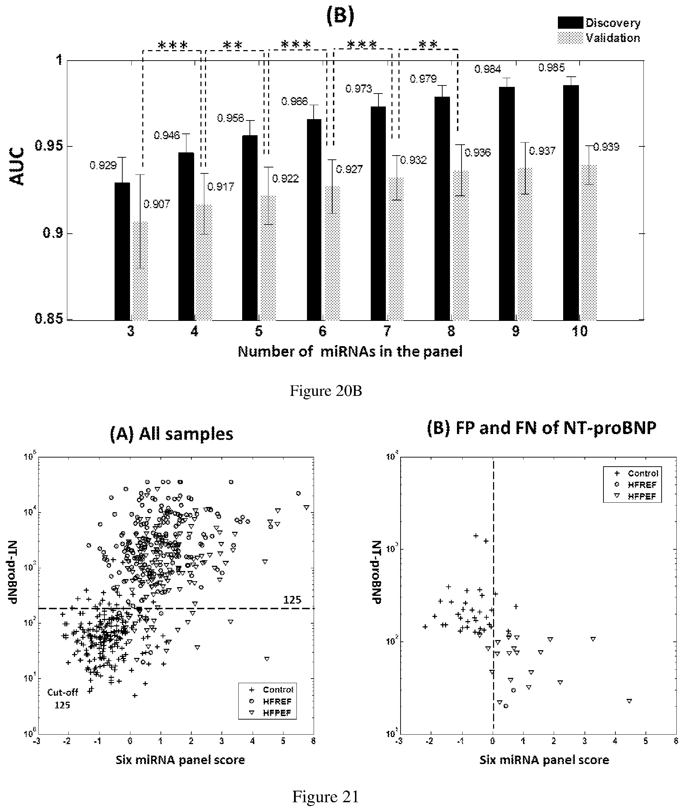

**Figure 20** shows the representative results that generates multivariate biomarker panels for heart failure detection. In (A), the boxplots show the diagnostic power (AUC) of multivariate biomarker panels (number of miRNAs = 3-10) in the discovery and validation phases for heart failure detection during the two fold cross validation *in silico.* The boxplot presents the 25th, 50th, and 75th percentiles in the AUC for the classification of healthy and heart failure patients. The quantitative representation of the result for the discovery set (black) and validation set (gray) are shown in (B). The error bar represents the standard deviation of the AUC. In order to test the significance of the AUC improvement in the validation set when more miRNAs were included in the panel, the right-tailed t-test was carried to compare all the adjacent gray bars. *: p-value < 0.05; **: p-value < 0.01; ***: p-value < 0.001.

**Figure 21** shows the comparison between multivariate miRNA score and NT-proBNP on HF detection using 2 dimensional plot. (A) shows 2 dimensional plot of the NT-proBNP level (y-axis) and one of the six-miRNA panel score (x-axis) for all subjects. The threshold for NT-proBNP (125) is indicated by the dashed line. The false positive and false negative subjects by NT-proBNP were boxed. (B) shows 2 dimensional plot of the NT-proBNP level (y-axis) and the six-miRNA panel score (x-axis) for false positive and false negative subjects as classified by NT-proBNP using the 125pg/ml threshold. The threshold miRNA score (0) is indicated by the dashed line. Control subjects are indicated by crosses; HFREF subjects by filled circles and HFPEF subjects by empty triangles. Figure 21 validated the hypothesis that miRNA biomarkers carry different information from that of N-terminal prohormone of brain natriuretic peptide (NT-proBNP).

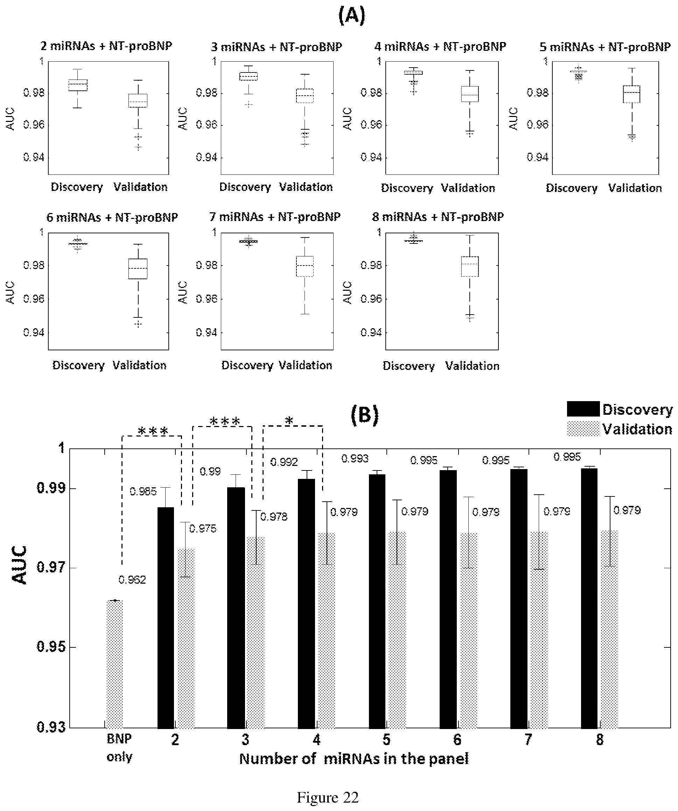

**Figure 22** shows the analysis of multivariate biomarker panels for heart failure detection combining miRNAs with NT-proBNP. (A) show a series of boxplots of the diagnostic power (AUC) of multivariate biomarker panels (ln _NT-proBNP plus 2-8 miRNAs) in the discovery and validation phases for HF detection during the two fold cross validation *in silico.* The boxplot presented the 25th, 50th, and 75th percentiles in the AUC for the classification of healthy and HF patients. (B) shows the quantitative representation the result for discovery set (black) and validation set (gray) as well as the ln-NT-proBNP itself (the first column). The error bar represented the standard deviation of the AUC. In order to test the significance of the AUC improvement in the validation set when more miRNAs were included in the panel, the right-tailed t-test was carried to compare all the adjacent gray bars. *: p-value < 0.05; **: p-value < 0.01; ***: p-value < 0.001. Thus, Figure 22 shows significantly improved classification efficiency when miRNA is combined with N-terminal prohormone of brain natriuretic peptide (NT-proBNP).

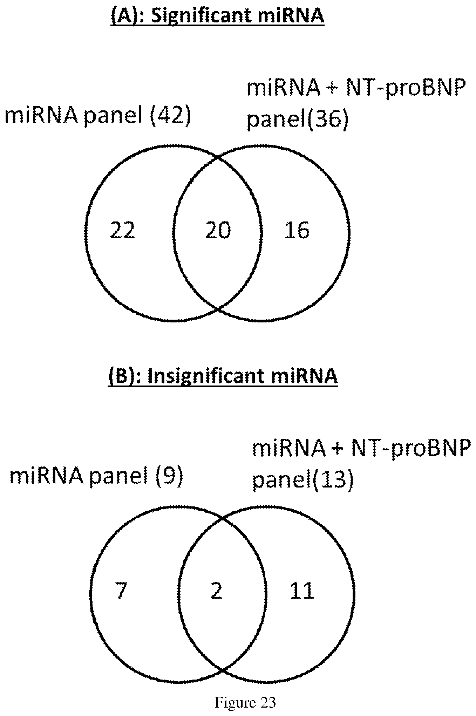

**Figure 23** shows Venn diagram of the overlap of miRNAs selected for multivariate HF detection panels with or without the addition of N-terminal prohormone of brain natriuretic peptide (NT-proBNP). Comparison between biomarkers selected for HF detection using miRNA along (Table 16) or using miRNA together with NT-proBNP (Table 17) during the multivariate biomarker search process. The significant miRNAs (A) and insignificant miRNAs (B) were compared separately. Figure 23 shows when using NT-proBNP, a different list of miRNAs may be used.

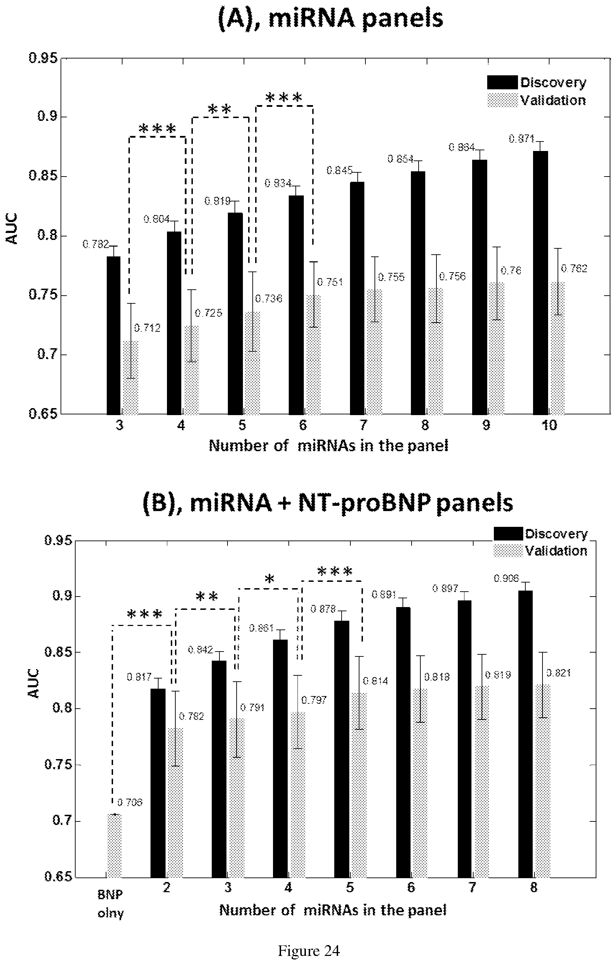

**Figure 24** shows the representative results that generates multi-miRNA panels for heart failure subtype stratification with and without the addition of NT-proBNP. (A) shows multivariate miRNA biomarker panel search (3-10 miRNAs) for heart failure subtype categorization The AUC result for discovery set (black bars) and validation set (gray bars) are shown. (B) shows multivariate miRNA and NT-proBNP biomarker panel search (ln_NT-proBNP plus 2-8 miRNAs) for heart failure subtype categorization. The AUC result for discovery set (black bars) and validation set (gray bars) as well as the ln_NT-proBNP itself (the first column) are shown. The error bar represents the standard deviation of the AUC. The right-tailed t-test was carried to compare all the adjacent gray bars. *: p-value < 0.05; **: p-value < 0.01; ***: p-value < 0.001. Figure 24 shows even clearer classifications may be achieved when both miRNA and NT-proBNP are used.

## BRIEF DESCRIPTION OF TABLES

The disclosure will be better understood with reference to the detailed description when considered in conjunction with the non-limiting examples and the accompanying tables, in which:

**Table 1** is a summary of reported serum/ plasma miRNA biomarkers for heart failure. The studies that measured the cell-free serum/plasma miRNAs or the whole blood were included in the table. Only miRNAs validated with qPCR are shown. Up-regulated: miRNAs that had a higher level in HF patients than in the control (healthy) subject. Down-regulated: miRNAs that had a lower level in HF patients than in the control (healthy) subject. The numbers in "Study design" indicated the number of samples used in the study. PBMC: Peripheral blood mononuclear cells, AMI: acute myocardial infarction, HF: heart failure, HF: heart failure, HFPEF: heart failure with preserved left ventricular ejection fraction, HFREF: heart failure with reduced left ventricular ejection fraction, BNP: brain natriuretic peptide, C: control (healthy subjects).

**Table 2** is a table listing the clinical information of the subjects included in the study. The clinical information of the 546 subjects included in the study. All the plasma samples were stored at - 80 °C prior to use. N.A.: not available, C: control (healthy subjects), PEF: heart failure with preserved left ventricular ejection fraction, REF: heart failure with reduced left ventricular ejection fraction

**Table 3** is a table listing the characteristics of the healthy subjects and heart failure patients. The Ejection Fraction (left ventricular ejection fraction), ln_NT-proBNP, Age, Body Mass Index are shown as arithmetic mean $\pm$ standard deviation and the NT-proBNP is shown as geometric mean. The percentage next to the variable name indicates the percentage of subjects with known value for the variable. HF: heart failure, HFPEF: heart failure with preserved ejection fraction, HFREF: heart failure with reduced ejection fraction, C: control (healthy) subject. For the comparisons of the variables between control and heart failure (C vs HF) and between HFPEF and HFREF (HFREF vs HFPEF), t-test was used for normal variables and chi-squared test were used for categorical variables.

**Table 4** is a table listing the sequences of 137 reliably detected mature miRNA. The 137 mature miRNA were reliably detected in the plasma samples. The definition of "reliably detected" was that at least 90% of the plasma samples had a concentration higher than 500 copies per ml. The miRNAs were named according to the miRBase V18 release.

**Table 5** is a table listing miRNAs that are differentially expressed between control and all heart failure subjects. Comparisons between control (healthy) and all heart failure subjects (both HFREF and HFPEF) were carried out by univariate analyses (p-value, t-test) and multivariate analyses with adjustment for age and AF (Atrial Fibrillation or Flutter), hypertension, diabetes (p-value, Logistic regression). The enhancements by miRNAs to the diagnostic performance of ln_NT-proBNP for heart failure were tested with logistic regression with adjustment for age and AF (Atrial Fibrillation or Flutter), hypertension and diabetes (p-value, ln_BNP). All the p-values were adjusted for false discovery rate correction using Bonferroni method. Only those miRNAs had p-values lower than 0.01 for both the "p-value, t-test" test and "p-value, Logistic regression" test were shown. Fold change: the miRNA expression level in heart failure subjects divided by that in the control subjects.

**Table 6** is a table listing miRNAs that are differentially expressed between control and HFREF subjects. Comparisons between control (healthy) and HFREF subjects (heart failure with reduced left ventricular ejection fraction) were carried out by univariate analyses (p-value, t-test) and multivariate analyses with adjustment for age, AF (Atrial Fibrillation or Flutter), hypertension and diabetes (p-value, Logistic regression). The enhancements by miRNAs of the discrimination of HFREF by ln _NT-proBNP were tested by logistic regression with adjustment for age and AF (Atrial Fibrillation or Flutter), hypertension and diabetes (p-value, ln_BNP). All p-values were adjusted for false discovery rate correction using the Bonferroni method. Only those miRNAs with p-values < 0.01 for both the "p-value, t-test" test and "p-value, Logistic regression" test were shown. Fold change: the miRNA expression level in HFREF subjects divided by that in the control subjects.

**Table 7** is a table listing miRNAs that are differentially expressed between control and HFPEF subjects. Comparisons between control (healthy) and HFPEF subjects (heart failure with preserved left ventricular ejection fraction) were carried out by univariate analyses (p-value, t-test) and multivariate analyses with adjustment for age and AF (Atrial Fibrillation or Flutter), hypertension and diabetes (p-value, Logistic regression). The enhancements by miRNAs of the discrimination by ln _NT-proBNP of HFPEF diagnosis were tested with logistic regression with adjustment for age, AF (Atrial Fibrillation or Flutter), hypertension and diabetes (p-value, ln_BNP). All the p-values were adjusted for false discovery rate correction using the Bonferroni method. Only those miRNAs with p-values < 0.01 for both the "p-value, t-test" test and "p-value, Logistic regression" test were shown. Fold change: the miRNA expression level in HFPEF subjects divided by that in the control subjects.

Table 8 is a table listing the comparison between the current study and previously published reports. The miRNAs not listed in Table 4 (expression levels ≥500 copies/ml) were indicated as N.A. (not available) which may not be included in the study or were below detection limit. Up: the miRNA had a higher expression level in heart failure patients compared to that of control (healthy) subjects. Down: the miRNA had a lower expression level in heart failure patients compared to that of control (healthy) subjects. Those miRNAs with p-values after false discovery rate correction lower than 0.01 were indicated as No Change. For hsa-miR-210, there were contradictions for the direction of changes in various literature reports (indicated Up & Down).

Table 9 is a table listing miRNAs that are differentially expressed between HFREF and HFPEF subjects. Comparisons between HFREF (heart failure with reduced left ventricular ejection fraction) and HFPEF subjects (heart failure with preserved left ventricular ejection fraction) were carried out by univariate analyses (p-value, t-test) and multivariate analyses with adjustment for age, gender, BMI (Body Mass Index) and AF (Atrial Fibrillation or Flutter)and hypertension (p-value, Logistic regression). The enhancements by miRNAs to the ability of ln _NT-proBNP to discriminate between HFREF and HFPEF categorization were tested with logistic regression with adjustment for age, gender, BMI (Body Mass Index),AF (Atrial Fibrillation or Flutter)and hypertension (p-value, ln_BNP). All the p-values were adjusted for false discovery rate correction using the Bonferroni method. Only those miRNAs with p-values < 0.01 for the "p-value, t-test" test were shown. Fold change: the miRNA expression level in HFPEF subjects divided by that in the HFREF subjects.

Table 10 is a table listing the clinical information of the subjects included in the prognosis study. The clinical information of the 327 subjects included in the prognosis study. All subjects were followed-up for two years after recruitment to the SHOP cohort study. 49 patients passed away during follow up.

Table 11 is a table listing the treatments of subjects included in the prognosis study. Drug treatment of the 327 subjects included in the prognosis study; Name of the medicine, Me1: ACE Inhibitors, Me2: Angiotensin 2 Receptor Blockers, Me3: Loop/thiazide Diuretics, Me4: Beta Blockers, Me5: Aspirin or Plavix, Me6: Statins, Me7: Digoxin, Me8: Warfarin, Me9: Nitrates Calcium, Me10: Channel Blockers, Me11: Spironolactone, Me12: Fibrate, Me13: Antidiabetic, Me14: Hydralazine, Me15: Iron supplements.

Table 12 is a table listing the analysis of clinical variables for observed survival. The clinical parameters included in analyses on observed survival using Cox proportional hazard model included drug treatments and other variables. The level of age, BMI, LVEF and ln _NT-proBNP were scaled to have one standard deviation. In the multivariate analysis, all variables were included. The cells for those variables with p-value less than 0.05 are indicated in gray. ln(HR): natural logarithm of hazard ratio (a positive value indicated a higher chance of death with the higher value of the variable), SE: standard error.

Table 13 is a table listing the analysis of clinical variables for Event free survival. The clinical parameters for analysis of Event free survival used Cox proportional hazards models with the level of age, BMI, LVEF and ln_NT-proBNP scaled to have one standard deviation. Drug treatments were also included. In the multivariate analysis, all variables were included. The cells for those variables with p-value < 0.05 were indicated gray. ln(HR): natural logarithm of hazard ratio (a positive value indicated a higher chance of death with the higher value of the variable), SE: standard error.

Table 14 is a table listing miRNAs that are significantly predictive of observed survival. Each of the miRNAs was analyzed for association with observed survival using Cox proportional hazard model with univariate and multivariate analyses which included additional clinical variables: gender, hypertension, BMI, ln_NT-proBNP, BetaBlockers and Warfarin. All the normally distributed variables including ln_NT-proBNP, BMI and miRNA expression level (log2 scale) were scaled to have one standard deviation. Those p-values < 0.05 are indicated as gray cells. ln(HR): natural logarithm of hazard ratio (a positive value indicated a higher chance of death with the higher value of the variable), SE: standard error.

Table 15 is a table listing miRNAs significantly predictive of event free survival. Each of the miRNA was analyzed for associations with event free survival using Cox proportional hazard model with univariate and multivariate analyses which included additional clinical variables: diabetes and ln_NT-proBNP. All the normally distributed variables including ln _NT-proBNP and miRNA expression level (log2 scale) were scaled to have one standard deviation. Those p-values < 0.05 are indicated as gray cells. ln(HR): natural logarithm of hazard ratio (a positive value indicated a higher chance of death with the higher value of the variable), SE: standard error.

Table 16 is a table listing miRNAs identified in multivariate panel search process for heart failure detection. The miRNAs selected for the assembly of biomarker panels with 6, 7, 8, 9, and 10 miRNAs for heart failure detection are listed. Prevalence was defined by the counts of the miRNA in all panels divided by the total number of panels. The panels with the top 10% and bottom 10% AUC were excluded to avoid counting of falsely discovered biomarkers due to fitting of inaccurate data from subpopulations generated by the randomization process in cross-validation analysis. Only the miRNAs used in more than 2% of the panels were listed. The changes of the miRNAs in various subtypes of heart failure were defined based on Table 5-7.

**Table 17** is a table listing the miRNAs that are identified in multivariate panel search process for heart failure (HF) detection in conjunction with NT-proBNP. The miRNAs selected for the assembly of biomarker panels with ln_NT-proBNP and 3, 4, 5, 6, 7 and 8 miRNAs for heart failure detection are listed. Prevalence was defined by the counts of the miRNA in all panels divided by the total number of panels. The panels with the top 10% and bottom 10% AUC were excluded to avoid counting of falsely discovered biomarkers due to fitting of inaccurate data from subpopulations generated by the randomization process in cross-validation analysis. Only the miRNAs used in more than 2% of the panels were listed. The significances of the miRNAs additional to ln_NT-proBNP in discriminating various subtypes of heart failure were determined based on the logistic regression using the selected miRNA and ln_NT-proBNP as predictive variables where the p-values for the significant miRNAs after FDR correction were < 0.01.

**Table 18** is a table listing the miRNAs that are identified in multivariate panel search process for HF subtype categorization. The miRNAs selected for the assembly of biomarker panels with 6, 7, 8, 9, and 10 miRNAs for heart failure (HF) subtype categorization are listed. Prevalence was defined by the counts of the miRNA in all panels divided by the total number of panels. The panels with the top 10% and bottom 10% AUC were excluded to avoid counting of falsely discovered biomarkers due to fitting of inaccurate data from subpopulations generated by the randomization process in cross-validation analysis. Only the miRNAs used in more than 2% of the panels were listed. The changes of the miRNAs in between the HFREF and HFPEF subtypes were defined based on Table 9.

**Table 19** is a table listing the miRNAs identified in multivariate panel search process for HF subtype categorization in conjunction with NT-proBNP. The miRNAs selected for the assembly of biomarker panels with ln_NT-proBNP and 5, 6, 7 and 8 miRNAs for HF subtype categorization are listed. Prevalence was defined by the counts of the miRNA in all panels divided by the total number of panels. The panels with the top 10% and bottom 10% AUC were excluded to avoid counting of falsely discovered biomarkers due to fitting of inaccurate data from subpopulations generated by the randomization process in cross-validation analysis. Only the miRNAs used in more than 2% of the panels were listed. The significances of the miRNAs additional to ln_NT-proBNP were determined based on the logistic regression using the selected miRNA and ln_NT-proBNP as predictive variables where the p-values for the significant miRNAs after FDR correction were < 0.01.

**Table 20** is a table listing miRNAs identified for heart failure (HF) detection. Comparisons between control (healthy) and all heart failure subjects (both HFREF and HFPEF) were carried out by univariate analyses (p-value, t-test) and multivariate analyses with adjustment for age and AF (Atrial Fibrillation or Flutter), hypertension, diabetes (p-value, Logistic regression). The enhancements by miRNAs to the diagnostic performance of ln_NT-proBNP for heart failure were tested with logistic regression with adjustment for age and AF (Atrial Fibrillation or Flutter), hypertension and diabetes (p-value, ln_BNP). All the p-values were adjusted for false discovery rate correction using Bonferroni method. Only those miRNAs had p-values lower than 0.01 for both the "p-value, t-test" test and "p-value, Logistic regression" test were shown. Fold change: the miRNA expression level in HF subjects divided by that in the control subjects. Table 20 corresponds to Table 5 with the exception that the miRNAs listed in Table 20 are not part of the miRNAs known in the art (i.e. as listed in Table 1 and Table 8).

**Table 21** is a table listing the miRNAs identified for HFREF detection. Comparisons between control (healthy) and HFREF subjects (heart failure with reduced left ventricular ejection fraction) were carried out by univariate analyses (p-value, t-test) and multivariate analyses with adjustment for age, AF (Atrial Fibrillation or Flutter), hypertension and diabetes (p-value, Logistic regression). The enhancements by miRNAs of the discrimination of HFREF by ln_NT-proBNP were tested by logistic regression with adjustment for age and AF (Atrial Fibrillation or Flutter), hypertension and diabetes (p-value, ln_BNP). All p-values were adjusted for false discovery rate correction using the Bonferroni method. Only those miRNAs with p-values < 0.01 for both the "p-value, t-test" test and "p-value, Logistic regression" test were shown. Fold change: the miRNA expression level in HFREF subjects divided by that in the control subjects. Table 21 corresponds to Table 6 with the exception that the miRNAs listed in Table 21 are not part of the miRNAs known in the art (i.e. as listed in Table 1 and Table 8).

**Table 22** is a table listing the miRNAs identified for HFPEF detection. Comparisons between control (healthy) and HFPEF subjects (heart failure with preserved left ventricular ejection fraction) were carried out by univariate analyses (p-value, t-test) and multivariate analyses with adjustment for age and AF (Atrial Fibrillation or Flutter), hypertension and diabetes (p-value, Logistic regression). The enhancements by miRNAs of the discrimination by ln_NT-proBNP of HFPEF diagnosis were tested with logistic regression with adjustment for age, AF (Atrial Fibrillation or Flutter), hypertension and diabetes (p-value, ln_BNP). All the p-values were adjusted for false discovery rate correction using the Bonferroni method. Only those miRNAs with p-values < 0.01 for both the "p-value, t-test" test and "p-value, Logistic regression" test were shown. Fold change: the miRNA expression level in HFPEF subjects divided by that in the control subjects. Table 22 corresponds to Table 7 with the exception that the miRNAs listed in Table 22 are not part of the miRNAs known in the art (i.e. as listed in Table 1 and Table 8).

**Table 23** is a table listing frequently selected miRNAs for heart failure detection in multivariate panel search process. The miRNAs selected for the assembly of biomarker panels with 6, 7, 8, 9, and 10 miRNAs for heart failure detection are listed. Prevalence was defined by the counts of the miRNA in all panels divided by the total number of panels. The panels with the top 10% and bottom 10% AUC were excluded to avoid counting of falsely discovered biomarkers due to fitting of inaccurate data from subpopulations generated by the randomization process in cross-validation analysis. Only the miRNAs used in more than 2% of the panels were listed. The changes of the miRNAs in various subtypes of heart failure HF were defined based on Table 20-22. Table 23 corresponds to Table 16 with the exception that the miRNAs listed in Table 23 are not part of the miRNAs known in the art (i.e. as listed in Table 1 and Table 8).

**Table 24** is a table listing frequently selected miRNAs for HF detection in multivariate panel search process in conjunction with NT-proBNP. The miRNAs selected for the assembly of biomarker panels with ln_NT-proBNP and 3, 4, 5, 6, 7 and 8 miRNAs for HF detection are listed. Prevalence was defined by the counts of the miRNA in all panels divided by the total number of panels. The panels with the top 10% and bottom 10% AUC were excluded to avoid counting of falsely discovered biomarkers due to fitting of inaccurate data from subpopulations generated by the randomization process in cross-validation analysis. Only the miRNAs used in more than 2% of the panels were listed. The significances of the miRNAs additional to ln_NT-proBNP in discriminating various subtypes of HF were determined based on the logistic regression using the selected miRNA and ln_NT-proBNP as predictive variables where the p-values for the significant miRNAs after FDR correction were < 0.01. Table 24 corresponds to Table 17 with the exception that the miRNAs listed in Table 24 are not part of the miRNAs known in the art (i.e. as listed in Table 1 and Table 8).

**Table 25** is a table listing microRNAs that may be used specifically for heart failure detection. To the best of the inventors' knowledge, these miRNAs are only associated with heart failure. miRNAs listed in Table 25 are not part of miRNAs known in the art (i.e. as listed in Table 1 and Table 8).

**Table 26** is a table listing exemplary biomarker panels for heart failure detection. Based on the biomarkers provided, an example of the formula, cutoffs and performance of the panel are provided in the table.

**Table 27** is a table listing exemplary biomarker panels for heart failure subtype detection. Based on the biomarkers provided, an example of the formula, cutoffs and performance of the panel are provided in the table.

## DETAILED DESCRIPTION OF THE PRESENT INVENTION

Timely diagnosis, accurate categorization of heart failure subtype, including, but not limited to heart failure with reduced left ventricular ejection fraction (HFREF), heart failure with preserved left ventricular ejection fraction (HFPEF), and the like, and improved risk stratification are important for the management and treatment of heart failure. An attractive approach is the use of circulating biomarkers [14]. The established circulating biomarkers in heart failure are the cardiac natriuretic peptides, B type natriuretic peptide (BNP) and its co-secreted congener, N-terminal prohormone brain natriuretic peptide (NT-proBNP). Both have proven diagnostic utility in acute heart failure and are independently related to prognosis at all stages of heart failure leading to their inclusion in all major international guidelines for the diagnosis and management of heart failure [14, 15]. However, confounders including age, renal function, obesity and atrial fibrillation do impair their diagnostic performance [16, 17]. In asymptomatic left ventricular dysfunction, early symptomatic heart failure and treated heart failure, the discriminating power of B peptides is markedly diminished with half of all stable HFREF cases exhibiting BNP below 100 pg/ml and 20% with NT-proBNP below values employed to rule out heart failure in the acutely symptomatic state [18]. This loss of test performance is even more pronounced in the cases of HFPEF [19]. B peptides reflect cardiac ventricular transmural distending pressures and myocyte stretch which (being dependent on chamber diameter as well as intra-ventricular pressures and wall thickness) is far less elevated in HFPEF with normal or reduced ventricular lumen volume and thickened ventricular walls, compared with HFREF with typically dilated ventricles and eccentric remodeling [20]. Therefore there is an unmet need for biomarkers that complement or replace B type peptides in screening for heart failure in its early or partly treated state and in monitoring status in the chronic phase of heart failure. This is particularly true for HFPEF with B peptides level lower than HFREF and often normal [21]. Currently, the categorization of heart failure subtype is dependent on imaging and imaging interpretation by a cardiologist. There is no biomarker based test available for this purpose. Therefore, a minimally invasive method to improve the diagnosis of heart failure as well as categorization into HF subtype is desirable.

MicroRNAs (miRNAs) are small non-coding RNAs that play central roles in the regulation of gene expression dysregulation of microRNAs is implicated in the pathogenesis of various diseases [22-26]. Since their discovery in 1993 [27], miRNAs have been estimated to regulate more than 60% of all human genes [28], with many miRNAs identified as key players in critical cellular functions such as proliferation [29] and apoptosis [30]. The discovery of miRNAs in human serum and plasma has raised the possibility of using circulating miRNA as biomarkers for diagnosis, prognosis, and treatment decisions for many diseases [31-35]. An integrated multidimensional method for the diagnosis of HF using miRNA or miRNA in conjunction with BNP/NT-proBNP may improve the diagnosis. Combining genomic marker(s), such as miRNAs, and protein marker(s), such as BNP/NT-proBNP may strengthen diagnostic power in HF compared to sole use of BNP/NT-proBNP. Recently, various attempts had been made to identify circulating cell-free miRNA biomarkers in serum or plasma to distinguish HF patients from healthy subjects [36-47] (Table 1).

**Table 1. Summary of reported serum/ plasma miRNA biomarkers for heart failure**

| Publication | Study design | Up-regulated | Down-regulated | Discovery Study design | Validation Study design |
|---|---|---|---|---|---|
| Vogel et al [1] | Predict HFREF | miR-200b*, miR-622, miR-1228* | - | whole blood, 53 HFREF/ 39 C, microarray | serum, 14 REF/ 8 C, qPCR |
| Endo et al [2] | Outcome as change of BNP in 3 weeks | - | miR-210 | Start with miR-210 only | Plasma, 39 NYHA II heart failure, qPCR |
| Zhang et al [3] | Predict the development of HF after AMI | - | miR-1 | Start with miR-1 only | Plasma, 49 AMI patients with various EF, qPCR |
| Fukushima et al [4] | Predicts HF | - | miR-126 | Start with three miRNAs | Plasma, 10 HF/ 17 C |
| Corsten et al [5] | Predicts acute HF | miR-499 | - | Start with six miRNAs | Plasma, 33 HF/ 34 C |
| Matsumoto et al [6] | Predict the development of HF after AMI | miR-192, miR-194, miR-34a, | - | Serum, 7 HF/ 7 C, Taqman, qPCR array | Serum, 21 HF/ 65 C, qPCR of 14 miRNAs (additional 2 not based on discovery) |
| Goren et al [7] | Predict HFREF | miR-423-5p, miR-320a, miR-22, miR-92b, miR-17*, miR-532-3p, miR-92a, miR-30a, miR-21, miR-29c, miR-101 | miR-199b-5p, miR-33a, miR-27b, miR-331-3p, miR-744, miR-28-5p, miR-574-3p, miR-223, miR-142-3p, miR-27a, miR-191, miR-335, miR-24, miR-151-5p | Serum, pooled samples, 2 HF/ 2 C, qPCR 370 miRNAs | Serum 30 HF/ 30 C, qPCR 186 miRNAs |
| Xiao et al [8] | Predict chronic HF | miR-142-3p miR-29b | miR-107, miR-139, miR-142-5p, miR-107, miR-125b, miR-497, | PBMC 15 HC/9 C, qPCR 159 miRNAs | PBMC 34 HC/ 19 C, qPCR 12 miRNAs |
| Tijsen et al [9] | Predict acute HF | miR-423-5p, miR-18b*, miR-129-5p, miR-1254, miR-675, miR-622 | - | Plasma, HF 12/C 12, microarray | Plasma, HF 30/C 39, qPCR 16 miRNAs |
| Zhao et al [10] | Predict chronic HF | miR-210, miR-30a | - | Serum, pooled samples HF 1/C 1, qPCR 27 miRNAs | Serum, HF 22/C 18, qPCR 9 miRNAs |

(continued)

| Publication | Study design | Up-regulated | Down-regulated | Discovery Study design | Validation Study design |
|---|---|---|---|---|---|
| Goren et al [11] | Predict chronic HF - PEF | - | - | - | Serum, HF 41/C 35, qPCR: miR-150 |
| Ellis et al [12] | Predict chronic HF - HFPEF/REF | miR-185, | miR-103, miR-142-3p, miR-30b, miR-342-3p, miR-150, miR-199a-3p, miR-23a, miR-27b, miR-324-5p | Plasma, HF 32/C 29, qPCR array | Plasma, HF 44/C 106, qPCR, 17 miRNAs |

These studies reported a set of miRNAs differentially regulated in heart failure subjects. However, there is a lack of concordance between these published works. Among 67 reported miRNAs, only three were found up-regulated in more than one report. In particular, hsa-miR-210 was reported as up-regulated in HF in one report and down-regulated in another (Table 1). The lack of agreement between studies could be due to a number of reasons including the use of small sample sizes or the variability in the sample selection such as stage of disease and, importantly, the controls used [32, 48]. Pre-analytical process including experimental design and workflow are critical in biomarkers identification and validation. Most studies to date have used a high-throughput array platform to screen a limited number of samples. This approach lacks sensitivity and reproducibility. It has yielded a small set of targets (less than 10 miRNAs) identified for further validation. Most of the studies have yet to be corroborated in larger patient groups. Another approach which has been adopted widely is based on screening of reported candidate miRNAs using quantitative real time polymerase chain reaction (qPCR). Evaluation of different technologies based on array versus qPCR platforms showed there are substantial differences in the performance of these platforms for miRNAs measurement. This could contribute to the observed inconsistency between studies [49]. Thus far, there is no consensus on the specific circulating serum/plasma miRNAs that might be used as heart failure biomarkers. None of the miRNA profiles previously reported was useful for categorization into heart failure subtypes. Hence, there is a need to build a robust pre-designated technology platform for heart failure biomarkers discovery and validation, and to ensure the reproducibility of the results.

In this disclosure, panels of circulating miRNAs were identified as potential heart failure biomarkers. These multivariate index assays are defined by Food and Drug Agency (FDA) guidelines, quoted as below: "combines the values of multiple variables using an interpretation function to yield a single, patient-specific result (e.g., a "classification," "score," "index," etc.), that is intended for use in the diagnosis of disease or other conditions, or in the cure, mitigation, treatment or prevention of disease, and provides a result whose derivation is non-transparent and cannot be independently derived or verified by the end user." Thus, highly reliable qPCR-based quantitative data following the MIQE (Minimum Information for publication of Quantitative Real-Time PCR Experiments) guidelines is a pre-requisite and the use of the state-of-the art mathematical and biostatistics tools are essential to determine the inter-relationship of these multiple variables simultaneously.

There are a variety of miRNA measurement methods including hybridization-based (microarray, northern blotting, bioluminescent), sequencing-based and qPCR-based [50]. Due to the small size of miRNA's (about 22 nucleotides), the most robust technology that provides precise, reproducible and accurate quantitative result with the greatest dynamic range is the qPCR-based platform [51]; currently, it is a gold standard commonly used to validate the results from other technologies, such as sequencing and microarray data. A variation of this method is digital PCR [52], an emerging technology based on similar principles but yet to gain widespread acceptance and use.

In this study, 203 miRNAs were profiled by qPCR in the plasma of 338 chronic heart failure patients (180 HFREF and 158 HFPEF) and 208 non-heart failure subjects (control group). This is a larger cohort of miRNA screening in heart failure than any reported in the literature to date. A summary of the number of miRNAs identified for various proposed approaches used in this study is depicted in Figure 1.

The inventors of the present disclosure have established a well-designed workflow with multi-layered technical and sample controls. This is to ensure the reliability of the assay and minimize the possible cross-over of contaminants and technical noise. For heart failure diagnosis biomarkers discovery, 203 miRNAs were screened and the inventors detected 137 miRNAs expressed across all the plasma samples. Of which, 75 miRNAs were identified to be significantly altered between heart failure (HFREF and/or HFPEF) and controls. A list of 52 miRNAs was able to distinguish HFREF from controls and 68 were found to be significantly differentially expressed between HFPEF and controls. Accordingly, the present inventors found a group of miRNAs that were able to distinguish HFREF from HFPEF. The present inventors have also found a group of miRNAs that are dysregulated in heart failure compared to controls.

Thus, in one aspect, there is provided a method of determining whether a subject suffers from heart failure or is at risk of developing heart failure. In some examples, the method comprises the steps of a) measuring the level of at least one miRNA from a list of miRNAs "increased" (above control) or at least one from a list of miRNAs "reduced" (below control) as listed in Table 25, or Table 20, or Table 21, or Table 22, in a sample obtained from the subject. In some examples, the method further comprises b) determining whether the level of miRNA is different as compared to a control, wherein altered levels of the miRNA indicates that the subject has heart failure or is at a risk of developing heart failure.

**Table 20. miRNAs for heart failure detection**

| Increased (n=33) | | | | | |
|---|---|---|---|---|---|
| Name | p-value, t-test | p-value, Logistic regression | p-value, ln_BNP | Fold change | AUC |
| hsa-let-7d-3p | 8.9E-23 | 4.1E-09 | 3.8E-05 | 1.32 | 0.78 |
| hsa-miR-197-3p | 8.9E-23 | 2.7E-08 | 7.9E-05 | 1.27 | 0.77 |
| hsa-miR-24-3p | 2.8E-22 | 5.5E-10 | 6.7E-05 | 1.30 | 0.76 |
| hsa-miR-221-3p | 5.4E-19 | 4.9E-09 | 6.2E-05 | 1.35 | 0.73 |
| hsa-miR-503 | 1.1E-17 | 1.2E-07 | 9.7E-04 | 1.69 | 0.73 |
| hsa-miR-130b-3p | 1.2E-14 | 3.9E-07 | 1.3E-03 | 1.27 | 0.72 |
| hsa-miR-23b-3p | 1.1E-13 | 2.6E-06 | 8.9E-04 | 1.31 | 0.71 |
| hsa-miR-21-3p | 2.4E-14 | 8.0E-06 | >0.01 | 1.25 | 0.70 |
| hsa-miR-223-5p | 4.4E-13 | 1.6E-06 | 9.2E-04 | 1.23 | 0.70 |
| hsa-miR-34b-3p | 9.5E-14 | 4.6E-04 | >0.01 | 1.84 | 0.69 |
| hsa-miR-148a-3p | 2.0E-12 | 2.3E-06 | 1.8E-03 | 1.28 | 0.68 |
| hsa-miR-23a-5p | 6.2E-11 | 4.3E-04 | >0.01 | 1.25 | 0.67 |
| hsa-miR-335-5p | 1.3E-10 | 2.5E-06 | 2.3E-04 | 1.33 | 0.67 |
| hsa-miR-124-5p | 3.8E-09 | 9.8E-04 | >0.01 | 1.54 | 0.66 |
| hsa-miR-382-5p | 6.0E-10 | 1.7E-05 | 7.6E-03 | 1.56 | 0.66 |
| hsa-miR-134 | 6.4E-10 | 2.9E-05 | 6.7E-03 | 1.57 | 0.66 |
| hsa-let-7e-3p | 7.6E-07 | 1.1E-03 | >0.01 | 1.33 | 0.65 |
| hsa-miR-598 | 4.9E-08 | 4.8E-05 | >0.01 | 1.20 | 0.65 |
| hsa-miR-627 | 2.8E-08 | 5.5E-04 | >0.01 | 1.31 | 0.65 |
| hsa-miR-199a-3p | 1.3E-05 | 4.1E-03 | >0.01 | 1.27 | 0.64 |
| hsa-miR-27b-3p | 1.6E-06 | 8.7E-04 | 3.8E-04 | 1.20 | 0.64 |
| hsa-miR-146b-5p | 6.3E-07 | 8.7E-04 | 3.4E-04 | 1.25 | 0.64 |
| hsa-miR-146a-5p | 3.1E-06 | 4.3E-03 | 9.7E-04 | 1.25 | 0.64 |
| hsa-miR-331-5p | 2.7E-07 | 2.7E-03 | >0.01 | 1.13 | 0.64 |
| hsa-miR-654-3p | 7.4E-08 | 2.0E-03 | >0.01 | 1.44 | 0.63 |
| hsa-miR-375 | 1.1E-05 | 7.9E-03 | >0.01 | 1.43 | 0.63 |
| hsa-miR-132-3p | 9.8E-07 | 7.4E-04 | >0.01 | 1.12 | 0.63 |
| hsa-miR-27a-3p | 2.0E-05 | 2.4E-03 | 4.9E-03 | 1.16 | 0.63 |
| hsa-miR-128 | 5.9E-06 | 8.6E-04 | >0.01 | 1.11 | 0.63 |
| hsa-miR-299-3p | 2.9E-06 | 3.3E-03 | >0.01 | 1.43 | 0.62 |
| hsa-miR-424-5p | 4.0E-07 | 1.3E-03 | >0.01 | 1.25 | 0.62 |

(continued)

| Increased (n=33) | | | | | |
|---|---|---|---|---|---|
| Name | p-value, t-test | p-value, Logistic regression | p-value, ln_BNP | Fold change | AUC |
| hsa-miR-154-5p | 5.9E-06 | 1.0E-03 | >0.01 | 1.41 | 0.62 |
| hsa-miR-377-3p | 1.3E-05 | 3.9E-03 | >0.01 | 1.37 | 0.60 |
| Reduced n=(34) | | | | | |
| Name | p-value, t-test | p-value, Logistic regression | p-value, BNP | Fold change | AUC |
| hsa-miR-454-3p | 3.3E-43 | 3.0E-14 | 5.6E-06 | 0.47 | 0.85 |
| hsa-miR-30c-5p | 8.9E-23 | 1.9E-10 | 3.2E-04 | 0.65 | 0.75 |
| hsa-miR-17-5p | 2.4E-19 | 1.4E-06 | 3.4E-04 | 0.73 | 0.74 |
| hsa-miR-196b-5p | 2.2E-15 | 7.8E-06 | 2.0E-04 | 0.79 | 0.73 |
| hsa-miR-500a-5p | 5.4E-19 | 1.1E-07 | 3.8E-04 | 0.68 | 0.73 |
| hsa-miR-106a-5p | 1.1E-16 | 1.3E-06 | 4.7E-05 | 0.76 | 0.72 |
| hsa-miR-20a-5p | 2.6E-17 | 1.4E-06 | 7.9E-05 | 0.74 | 0.72 |
| hsa-miR-451a | 5.4E-19 | 9.8E-08 | 7.9E-05 | 0.54 | 0.72 |
| hsa-miR-29b-3p | 1.5E-16 | 4.6E-08 | 6.7E-05 | 0.76 | 0.71 |
| hsa-miR-374b-5p | 2.4E-16 | 1.1E-07 | 1.8E-03 | 0.69 | 0.71 |
| hsa-miR-20b-5p | 1.5E-16 | 2.3E-06 | 8.1E-05 | 0.60 | 0.71 |
| hsa-miR-501-5p | 2.2E-14 | 3.3E-06 | 1.2E-04 | 0.71 | 0.70 |
| hsa-miR-18b-5p | 4.4E-13 | 3.9E-05 | 4.7E-05 | 0.78 | 0.69 |
| hsa-miR-23c | 3.1E-12 | 1.2E-06 | >0.01 | 0.68 | 0.69 |
| hsa-miR-551b-3p | 3.0E-12 | 3.2E-05 | >0.01 | 0.65 | 0.69 |
| hsa-miR-26a-5p | 4.7E-13 | 3.9E-05 | >0.01 | 0.74 | 0.69 |
| hsa-miR-183-5p | 1.8E-12 | 2.8E-05 | 3.8E-04 | 0.59 | 0.68 |
| hsa-miR-16-5p | 4.2E-12 | 1.9E-05 | 8.4E-04 | 0.71 | 0.68 |
| hsa-miR-532-5p | 1.2E-11 | 8.0E-06 | 4.9E-04 | 0.77 | 0.67 |
| hsa-miR-363-3p | 3.9E-11 | 1.7E-04 | 2.7E-03 | 0.70 | 0.67 |
| hsa-miR-374c-5p | 4.5E-10 | 3.7E-04 | >0.01 | 0.71 | 0.67 |
| hsa-let-7b-5p | 3.5E-11 | 3.8E-04 | >0.01 | 0.80 | 0.66 |
| hsa-miR-15a-5p | 3.8E-09 | 9.8E-04 | 4.7E-03 | 0.82 | 0.66 |
| hsa-miR-144-3p | 3.9E-11 | 9.4E-05 | 3.8E-04 | 0.63 | 0.66 |
| hsa-miR-93-5p | 1.3E-09 | 3.8E-04 | 1.3E-03 | 0.82 | 0.66 |
| hsa-miR-181b-Sp | 3.1E-09 | 1.2E-07 | >0.01 | 0.80 | 0.66 |
| hsa-miR-19b-3p | 2.3E-09 | 3.4E-05 | 8.3E-05 | 0.80 | 0.65 |
| hsa-miR-4732-3p | 2.4E-08 | 4.7E-04 | 3.5E-03 | 0.70 | 0.64 |
| hsa-miR-484 | 5.9E-07 | 9.9E-03 | >0.01 | 0.89 | 0.64 |
| hsa-miR-25-3p | 3.3E-07 | 4.4E-03 | 8.8E-03 | 0.79 | 0.63 |
| hsa-miR-192-5p | 8.9E-06 | 9.9E-04 | >0.01 | 0.76 | 0.63 |
| hsa-miR-205-5p | 3.2E-05 | 2.0E-03 | >0.01 | 0.75 | 0.62 |
| hsa-miR-19a-3p | 2.2E-06 | 1.1E-03 | 6.9E-04 | 0.84 | 0.61 |

(continued)

| Name | p-value, t-test | p-value, Logistic regression | p-value, BNP | Fold change | AUC |
|---|---|---|---|---|---|
| hsa-miR-32-5p | 7.5E-06 | 8.3E-03 | >0.01 | 0.88 | 0.61 |

**Table 21. miRNAs identified for HFREF detection**

| Increased (n=21) | | | | | |
|---|---|---|---|---|---|
| Name | p-value, t-test | p-value, Logistic regression | p-value, ln_BNP | Fold change | AUC |
| hsa-let-7d-3p | 5.0E-15 | 1.2E-06 | >0.01 | 1.26 | 0.75 |
| hsa-miR-24-3p | 5.9E-15 | 5.1E-07 | >0.01 | 1.27 | 0.74 |
| hsa-miR-503 | 5.0E-15 | 1.9E-06 | >0.01 | 1.74 | 0.74 |
| hsa-miR-197-3p | 6.6E-14 | 6.7E-05 | >0.01 | 1.22 | 0.73 |
| hsa-miR-130b-3p | 1.6E-10 | 1.1E-05 | >0.01 | 1.23 | 0.71 |
| hsa-miR-221-3p | 4.4E-12 | 1.2E-06 | >0.01 | 1.31 | 0.71 |
| hsa-miR-34b-3p | 2.4E-12 | 7.2E-04 | >0.01 | 1.90 | 0.70 |
| hsa-miR-21-3p | 1.1E-09 | 3.6E-04 | >0.01 | 1.22 | 0.69 |
| hsa-miR-132-3p | 7.4E-09 | 6.8E-04 | >0.01 | 1.16 | 0.68 |
| hsa-miR-331-5p | 2.7E-09 | 2.2E-03 | >0.01 | 1.18 | 0.68 |
| hsa-miR-124-5p | 3.3E-09 | 5.6E-04 | >0.01 | 1.62 | 0.67 |
| hsa-miR-148a-3p | 1.2E-08 | 2.6E-04 | >0.01 | 1.26 | 0.66 |
| hsa-miR-23b-3p | 3.2E-07 | 2.0E-04 | >0.01 | 1.22 | 0.66 |
| hsa-miR-375 | 1.0E-06 | 3.8E-03 | >0.01 | 1.55 | 0.65 |
| hsa-miR-134 | 2.4E-06 | 4.2E-04 | >0.01 | 1.48 | 0.64 |
| hsa-miR-627 | 1.4E-05 | 2.2E-03 | >0.01 | 1.28 | 0.64 |
| hsa-miR-382-5p | 6.2E-06 | 6.4E-04 | >0.01 | 1.45 | 0.63 |
| hsa-miR-598 | 6.1E-05 | 2.6E-04 | >0.01 | 1.16 | 0.63 |
| hsa-miR-23a-5p | 1.4E-05 | 3.3E-03 | >0.01 | 1.17 | 0.63 |
| hsa-miR-223-5p | 3.7E-04 | 9.3E-03 | >0.01 | 1.12 | 0.62 |
| hsa-miR-335-5p | 1.6E-04 | 2.6E-04 | >0.01 | 1.19 | 0.61 |
| Reduced (n=23) | | | | | |
| Name | p-value, t-test | p-value, Logistic regression | p-value, ln_BNP | Fold change | AUC |
| hsa-miR-454-3p | 2.9E-30 | 2.0E-11 | >0.01 | 0.48 | 0.83 |
| hsa-miR-30c-5p | 3.7E-19 | 1.1E-08 | >0.01 | 0.64 | 0.76 |
| hsa-miR-374b-5p | 1.1E-15 | 5.7E-07 | >0.01 | 0.66 | 0.73 |
| hsa-miR-23c | 1.3E-13 | 2.7E-07 | >0.01 | 0.63 | 0.72 |
| hsa-miR-551b-3p | 6.9E-11 | 1.5E-04 | >0.01 | 0.63 | 0.70 |
| hsa-miR-17-5p | 1.5E-11 | 2.6E-04 | >0.01 | 0.80 | 0.70 |
| hsa-miR-26a-5p | 6.9E-11 | 6.7E-05 | >0.01 | 0.73 | 0.70 |
| hsa-miR-181b-5p | 1.9E-11 | 1.4E-06 | >0.01 | 0.73 | 0.70 |
| hsa-miR-500a-5p | 2.1E-10 | 6.7E-05 | >0.01 | 0.73 | 0.69 |

(continued)

| Name | p-value, t-test | p-value, Logistic regression | p-value, ln_BNP | Fold change | AUC |
|---|---|---|---|---|---|
| hsa-miR-196b-5p | 1.0E-07 | 1.7E-03 | >0.01 | 0.84 | 0.68 |
| hsa-miR-374c-5p | 1.4E-08 | 6.3E-04 | >0.01 | 0.70 | 0.68 |
| hsa-miR-451a | 2.1E-10 | 1.1E-04 | >0.01 | 0.62 | 0.68 |
| hsa-miR-29b-3p | 2.8E-09 | 8.8E-05 | >0.01 | 0.80 | 0.67 |
| hsa-miR-20a-5p | 1.7E-08 | 7.8E-04 | >0.01 | 0.81 | 0.67 |
| hsa-miR-106a-5p | 3.0E-07 | 4.7E-03 | >0.01 | 0.85 | 0.65 |
| hsa-miR-181a-2-3p | 1.1E-04 | 5.9E-03 | >0.01 | 0.84 | 0.65 |
| hsa-miR-501-5p | 4.3E-06 | 4.3E-03 | >0.01 | 0.80 | 0.64 |
| hsa-miR-20b-5p | 2.0E-06 | 4.0E-03 | >0.01 | 0.75 | 0.63 |
| hsa-miR-183-5p | 5.8E-06 | 1.4E-03 | >0.01 | 0.69 | 0.63 |
| hsa-miR-16-5p | 1.6E-05 | 3.8E-03 | >0.01 | 0.79 | 0.63 |
| hsa-miR-125a-5p | 7.9E-05 | 6.4E-04 | >0.01 | 0.81 | 0.62 |
| hsa-miR-205-5p | 3.3E-04 | 5.9E-03 | >0.01 | 0.76 | 0.61 |
| hsa-miR-532-5p | 2.3E-04 | 9.3E-03 | >0.01 | 0.85 | 0.61 |

**Table 22. miRNAs identified for HFPEF detection**

| Increased (n=30) | | | | | |
|---|---|---|---|---|---|
| Name | p-value, t-test | p-value, Logistic regression | p-value, ln_BNP | Fold change | AUC |
| hsa-let-7d-3p | 4.40E-22 | 5.90E-07 | 4.10E-05 | 1.39 | 0.81 |
| hsa-miR-197-3p | 4.10E-24 | 2.10E-07 | 6.40E-05 | 1.34 | 0.81 |
| hsa-miR-223-5p | 6.80E-21 | 2.10E-07 | 7.80E-05 | 1.37 | 0.79 |
| hsa-miR-24-3p | 5.80E-19 | 2.10E-07 | 4.10E-05 | 1.33 | 0.78 |
| hsa-miR-221-3p | 5.90E-17 | 5.90E-07 | 4.10E-05 | 1.4 | 0.76 |
| hsa-miR-23b-3p | 1.60E-16 | 1.40E-05 | 2.60E-04 | 1.41 | 0.76 |
| hsa-miR-130b-3p | 2.20E-14 | 9.00E-05 | 9.10E-04 | 1.32 | 0.74 |
| hsa-miR-335-5p | 2.40E-14 | 5.30E-06 | 4.10E-05 | 1.5 | 0.73 |
| hsa-miR-21-3p | 8.50E-13 | 8.20E-05 | 3.60E-03 | 1.29 | 0.72 |
| hsa-miR-23a-5p | 1.90E-12 | 1.40E-03 | 3.60E-03 | 1.34 | 0.72 |
| hsa-miR-503 | 1.40E-11 | 3.80E-05 | 5.80E-04 | 1.64 | 0.72 |
| hsa-miR-148a-3p | 4.40E-11 | 1.10E-04 | 2.20E-03 | 1.3 | 0.7 |
| hsa-miR-146a-5p | 3.10E-08 | 2.50E-04 | 1.50E-04 | 1.37 | 0.69 |
| hsa-miR-199a-3p | 2.70E-07 | 8.40E-04 | 2.20E-03 | 1.38 | 0.69 |
| hsa-let-7e-3p | 4.00E-08 | 1.30E-03 | 8.50E-03 | 1.43 | 0.68 |
| hsa-miR-134 | 3.90E-09 | 2.90E-04 | 1.60E-03 | 1.67 | 0.68 |
| hsa-miR-382-5p | 1.00E-09 | 1.00E-04 | 1.10E-03 | 1.69 | 0.68 |
| hsa-miR-128 | 2.60E-07 | 2.90E-04 | 2.50E-03 | 1.15 | 0.67 |
| hsa-miR-146b-5p | 9.90E-08 | 1.60E-04 | 6.40E-05 | 1.33 | 0.67 |

(continued)

| Increased (n=30) | | | | | |
|---|---|---|---|---|---|
| **Name** | **p-value, t-test** | **p-value, Logistic regression** | **p-value, ln_BNP** | **Fold change** | **AUC** |
| hsa-miR-27a-3p | 1.60E-06 | 3.40E-04 | 5.80E-04 | 1.21 | 0.67 |
| hsa-miR-27b-3p | 5.10E-07 | 6.70E-04 | 1.50E-04 | 1.26 | 0.67 |
| hsa-miR-598 | 3.10E-08 | 1.20E-03 | >0.01 | 1.25 | 0.67 |
| hsa-miR-101-3p | 2.70E-08 | 5.10E-04 | 6.70E-03 | 0.74 | 0.67 |
| hsa-miR-55 1b-3p | 3.30E-08 | 8.90E-03 | >0.01 | 0.67 | 0.67 |
| hsa-miR-627 | 4.20E-07 | 8.70E-03 | >0.01 | 1.36 | 0.66 |
| hsa-miR-185-5p | 1.70E-09 | 1.80E-03 | 4.60E-03 | 0.8 | 0.66 |
| hsa-miR-299-3p | 2.30E-07 | 2.30E-03 | >0.01 | 1.59 | 0.65 |
| hsa-miR-425-3p | 1.30E-04 | 9.30E-04 | 1.60E-03 | 1.15 | 0.65 |
| hsa-miR-154-5p | 1.10E-06 | 9.10E-04 | 3.60E-03 | 1.55 | 0.64 |
| hsa-miR-377-3p | 2.40E-06 | 8.30E-03 | >0.01 | 1.52 | 0.64 |
| **Reduced (n=33)** | | | | | |
| **Name** | **p-value, t-test** | **p-value, Logistic regression** | **p-value, ln_BNP** | **Fold change** | **AUC** |
| hsa-miR-454-3p | 8.9E-36 | 5.9E-07 | 4.1E-05 | 0.45 | 0.87 |
| hsa-miR-106a-5p | 3.4E-22 | 5.9E-07 | 4.1E-05 | 0.68 | 0.80 |
| hsa-miR-17-5p | 4.0E-21 | 2.5E-05 | 2.9E-04 | 0.67 | 0.80 |
| hsa-miR-20b-5p | 4.1E-24 | 5.0E-07 | 4.1E-05 | 0.47 | 0.79 |
| hsa-miR-20a-5p | 7.0E-21 | 2.1E-06 | 6.4E-05 | 0.66 | 0.79 |
| hsa-miR-196b-5p | 5.6E-18 | 2.8E-05 | 1.8E-04 | 0.75 | 0.78 |
| hsa-miR-451a | 3.3E-21 | 5.9E-07 | 7.0E-05 | 0.46 | 0.78 |
| hsa-miR-18b-5p | 3.0E-17 | 8.2E-05 | 9.2E-05 | 0.69 | 0.77 |
| hsa-miR-500a-5p | 2.1E-19 | 7.0E-06 | 1.6E-04 | 0.62 | 0.77 |
| hsa-miR-29b-3p | 1.0E-18 | 1.3E-06 | 6.2E-05 | 0.72 | 0.76 |
| hsa-miR-501-5p | 5.2E-19 | 2.4E-06 | 4.5E-05 | 0.62 | 0.76 |
| hsa-miR-532-5p | 1.3E-16 | 1.2E-06 | 7.0E-05 | 0.69 | 0.75 |
| hsa-let-7b-5p | 1.1E-16 | 3.8E-05 | 9.0E-04 | 0.71 | 0.74 |
| hsa-miR-30c-5p | 1.3E-15 | 1.0E-04 | 2.1E-03 | 0.67 | 0.74 |
| hsa-miR-183-5p | 2.9E-15 | 3.8E-05 | 3.8E-04 | 0.50 | 0.74 |
| hsa-miR-144-3p | 1.9E-16 | 3.6E-06 | 1.5E-04 | 0.51 | 0.73 |
| hsa-miR-93-5p | 4.6E-15 | 2.5E-05 | 5.0E-04 | 0.74 | 0.73 |
| hsa-miR-16-5p | 6.5E-15 | 4.1E-06 | 2.6E-04 | 0.62 | 0.73 |
| hsa-miR-363-3p | 1.5E-13 | 4.5E-05 | 1.3E-03 | 0.62 | 0.73 |
| hsa-miR-25-3p | 3.3E-12 | 8.2E-05 | 2.5E-03 | 0.68 | 0.71 |
| hsa-miR-4732-3p | 1.1E-13 | 1.1E-05 | 9.1E-04 | 0.57 | 0.71 |
| hsa-miR-192-5p | 2.2E-09 | 4.1E-04 | >0.01 | 0.65 | 0.70 |
| hsa-miR-19b-3p | 1.5E-11 | 1.6E-05 | 1.0E-04 | 0.74 | 0.70 |
| hsa-miR-15a-5p | 3.3E-08 | 3.0E-03 | 3.6E-03 | 0.80 | 0.69 |

(continued)

| Name | p-value, t-test | p-value, Logistic regression | p-value, ln_BNP | Fold change | AUC |
|---|---|---|---|---|---|
| hsa-miR-486-5p | 2.2E-10 | 3.4E-04 | >0.01 | 0.64 | 0.69 |
| hsa-miR-374b-5p | 3.4E-10 | 5.1E-03 | >0.01 | 0.72 | 0.68 |
| hsa-miR-484 | 4.0E-08 | 9.9E-03 | >0.01 | 0.86 | 0.67 |
| hsa-miR-194-5p | 1.0E-06 | 4.6E-03 | >0.01 | 0.71 | 0.67 |
| hsa-miR-101-3p | 2.7E-08 | 5.1E-04 | 6.7E-03 | 0.74 | 0.67 |
| hsa-miR-551b-3p | 3.3E-08 | 8.9E-03 | >0.01 | 0.67 | 0.67 |
| hsa-miR-185-5p | 1.7E-09 | 1.8E-03 | 4.6E-03 | 0.80 | 0.66 |
| hsa-miR-19a-3p | 3.3E-08 | 1.9E-04 | 9.1E-04 | 0.79 | 0.66 |
| hsa-miR-550a-5p | 3.5E-05 | 3.0E-03 | 5.7E-03 | 0.81 | 0.62 |

**Table 25. Specific novel microRNAs for Heart failure detection**

| Increased (n=6), AUC> 0.7 | | | | | |
|---|---|---|---|---|---|
| Name | p-value, t-test | p-value, Logistic regression | p-value, ln_BNP | Fold change | AUC |
| hsa-let-7d-3p | 8.90E-23 | 4.10E-09 | 3.80E-05 | 1.32 | 0.78 |
| hsa-miR-197-3p | 8.90E-23 | 2.70E-08 | 7.90E-05 | 1.27 | 0.77 |
| hsa-miR-221-3p | 5.40E-19 | 4.90E-09 | 6.20E-05 | 1.35 | 0.73 |
| hsa-miR-503 | 1.10E-17 | 1.20E-07 | 9.70E-04 | 1.69 | 0.73 |
| hsa-miR-130b-3p | 1.20E-14 | 3.90E-07 | 1.30E-03 | 1.27 | 0.72 |
| hsa-miR-23b-3p | 1.10E-13 | 2.60E-06 | 8.90E-04 | 1.31 | 0.71 |

| Reduced n=(6), AUC >0.7 | | | | | |
|---|---|---|---|---|---|
| Name | p-value, t-test | p-value, Logistic regression | p-value, ln_BNP | Fold change | AUC |
| hsa-miR-30c-5p | 8.90E-23 | 1.90E-10 | 3.20E-04 | 0.65 | 0.75 |
| hsa-miR-17-5p | 2.40E-19 | 1.40E-06 | 3.40E-04 | 0.73 | 0.74 |
| hsa-miR-196b-5p | 2.20E-15 | 7.80E-06 | 2.00E-04 | 0.79 | 0.73 |
| hsa-miR-106a-5p | 1.10E-16 | 1.30E-06 | 4.70E-05 | 0.76 | 0.72 |
| hsa-miR-20a-5p | 2.60E-17 | 1.40E-06 | 7.90E-05 | 0.74 | 0.72 |
| hsa-miR-451a | 5.40E-19 | 9.80E-08 | 7.90E-05 | 0.54 | 0.72 |

As used herein throughout the disclosure, the term "miRNA" refers to microRNA, small non-coding RNA molecules, and are found in plants, animals and some viruses. miRNA are known to have functions in RNA silencing and post-transcriptional regulation of gene expression. These highly conserved RNAs regulate the expression of genes by binding to the 3'-untranslated regions (3'-UTR) of specific mRNAs. For example, each miRNA is thought to regulate multiple genes, and since hundreds of miRNA genes are predicted to be present in higher eukaryotes. miRNA may be at least 10 nucleotides and of not more than 35 nucleotides covalently linked together. In some examples, the miRNA may be molecules of 10 to 33 nucleotides, or of 15 to 30 nucleotides in length, or 17 to 27 nucleotides, or 18 to 26 nucleotides in length. In some examples, the miRNA may be molecules of 10, or 11, or 12, or 13, or 14, or 15, or 16, or 17, or 18, or 19, or 20, or 21, or 22, or 23, or 24, or 25, or 26, or 27, or 28, or 29, or 30, or 31, or 32, or 33, or 34, or 35 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are non- coding RNAs). As used herein throughout the disclosure, the miRNA measured may be at least 90%, 95%, 97.5%, 98%, or 99% sequence identity to the miRNAs as listed in any one of the tables provided in the present disclosure. Thus, in some examples, the measure miRNA has at least 90%, 95%, 97.5%, 98%, or 99% sequence identity to the miRNAs as listed in any one of, Table 9, Table 14, Table 15, Table 16, Table 17, Table 18, Table 19, Table 20, Table 21, Table 22, Table 23, Table 24 or Table 25. As used herein, the term "sequence identity" "sequence identity" refers to a relationship between two or more polypeptide sequences or two or more polynucleotide sequences, namely a reference sequence and a given sequence to be compared with the reference sequence. Sequence identity is determined by comparing the given sequence to the reference sequence after the sequences have been optimally aligned to produce the highest degree of sequence similarity, as determined by the match between strings of such sequences. Upon such alignment, sequence identity is ascertained on a position-by-position basis, e.g., the sequences are "identical" at a particular position if at that position, the nucleotides or amino acid residues are identical. The total number of such position identities is then divided by the total number of nucleotides or residues in the reference sequence to give % sequence identity. Sequence identity can be readily calculated by methods known to the person skilled in the art.

As used herein throughout the disclosure, the term "heart failure", or "HF", refers to a complex clinical syndrome in which the pumping function of the heart becomes insufficient (ventricular dysfunction) to meet the needs of the vital system and tissues of the body. The severity of heart failure may range from non-severe (mild), which manifest in the subject having no limitation of physical activity, to increasing severity, which manifest in the subject unable to carry on any physical activity without discomfort. Heart failure is a progressive and chronic disease, worsening over time. In extreme cases, heart failure may lead to the need for a heart transplant. In some examples, the subject may be determined to be at risk of developing heart failure if the subject may have further heart failure, such as deterioration into recurrent acute decompensated heart failure or death among those with known chronic heart failure.

As used herein throughout the disclosure, the terms "subject" and "patient" are to be used interchangeably to refer to individual or mammal suspected to be affected by heart failure. The patient may be predicted (or determined, or diagnosed) to be affected by heart failure, i.e. diseased, or may be predicted to be not affected by heart failure, i.e. healthy. The subject may also be determined to be affected by a specific form of heart failure. In some examples, the heart failure patient may be a subject who has had primary diagnosis of heart failure and/or being treated 3-5 days when symptomatically improved, with resolution of bedside physical signs of heart failure and considered fit to discharge. Thus, the subject may further be determined to develop heart failure or a specific form of heart failure. It should be noted that a subject that is determined as being healthy, i.e. not suffering from heart failure or from a specific form of heart failure, may possibly suffer from another disease not tested/known. As used herein, the subject of the present disclosure may be any mammal, including both a human and another mammal, e.g. an animal such as a dog, cat, rabbit, mouse, rat, or monkey. In some examples, the subject may be human. Therefore, the miRNA from a subject may be a human miRNA or a miRNA from another mammal, e.g. an animal miRNA such as a mouse, monkey or rat miRNA, or the miRNAs comprised in a set may be human miRNAs or miRNAs from another mammal, e.g. animal miRNAs such as mouse, monkey or rat miRNAs. As illustrated by Table 2 in the Experimental Section, the subject of the present disclosure may be of Asian descent or ethnicity. In some examples, the subject may include, but is not limited to any Asian ethnicity, including, Chinese, Indian, Malay, and the like.

On the other hand, the term "control" or "control subject", as used in the context of the present disclosure, may refer to (a sample obtained from) subject known to be affected with heart failure (positive control, e.g good prognosis, poor prognosis), i.e. diseased, and/or a subject with heart failure subtype HFPEF, and/or heart failure subtype HFREF, and/or a subject known to be not affected with heart failure (negative control), i.e. healthy. It may also refer to (a sample obtained from) a subject known to be effected by another disease/condition. It should be noted that a control subject that is known to be healthy, i.e. not suffering from heart failure, may possibly suffer from another disease not tested/known. Thus, in some examples, the control may be a non-heart failure subject (or sometimes referred to as a normal subject). The control subject may be any mammal, including both a human and another mammal, e.g. an animal such as a rabbit, mouse, rat, or monkey. In some examples, the control is human. In some examples, the control may be (samples obtained from) an individual subject or a cohort of subjects.

It would be appreciated by the person skilled in the art that the methods as described herein are not to be used to replace the physician's role in diagnosing the condition in a subject. As would be appreciated, clinical diagnosis of heart failure in a subject would require the physician's analysis of other symptoms and/or other information that may be available to the physicians. The methods as described herein are meant to provide support or additional information for the physicians to make the final diagnosis of the patient/subject.

As used herein throughout the disclosure, the term "sample" refers to a bodily fluid or extracellular fluid. In some examples, the bodily fluid may include, but is not limited to, cellular and non-cellular components of amniotic fluid, breast milk, bronchial lavage, cerebrospinal fluid, colostrum, interstitial fluid, peritoneal fluids, pleural fluid, saliva, seminal fluid, urine, tears, whole blood, including plasma, red blood cells, white blood cells, serum, and the like. In some examples, the bodily fluid may be blood, serum plasma, and/or plasma.

In some examples, an increase in the level of miRNAs as listed as "increased" in Table 20 or Table 25, as compared to the control, indicates the subject to have heart failure or is at a risk of developing heart failure.

In some examples, a reduction in the level of miRNAs as listed as "reduced" in Table 20 or Table 25 as compared to the control, indicates the subject to have heart failure or is at a risk of developing heart failure.

As used herein the term "miRNA level" or "level of miRNA" as used in the context of the present disclosure, represents the determination of the miRNA expression level (or miRNA expression profile) or a measure that correlates with the miRNA expression level in a sample. The miRNA expression level may be generated by any convenient means known in the art, such as, but are not limited to, nucleic acid hybridization (e.g. to a microarray), nucleic acid amplification (PCR, RT-PCR, qRT-PCR, high-throughput RT- PCR), ELISA for quantitation, next generation sequencing (e.g. ABI SOLID, Illumina Genome Analyzer, Roche/454 GS FLX), flow cytometry (e.g. LUMINEX) and the like, that allow the analysis of miRNA expression levels and comparison between samples of a subject (e.g. potentially diseased) and a control subject (e.g. reference sample(s)). The sample material measured by the aforementioned means may be a raw or treated sample or total RNA, labeled total RNA, amplified total RNA, cDNA, labeled cDNA, amplified cDNA, miRNA, labeled miRNA, amplified miRNA or any derivatives that may be generated from the aforementioned RNA/DNA species. By determining the miRNA expression level, each miRNA is represented by a numerical value. The higher the value of an individual miRNA, the higher is the (expression) level of said miRNA, or the lower the value of an individual miRNA, the lower is the (expression) level of said miRNA. When a higher value of an individual miRNA is detected over and beyond the control, the miRNA expression is referred to as "increased" or "upregulated". On the other hand, when a lower value of an individual miRNA is detected that is below the control, the miRNA expression is then referred to as "decreased" or "downregulated".

The "miRNA (expression) level", as used herein, represents the expression level/expression profile/expression data of a single miRNA or a collection of expression levels of at least two miRNAs, or least 3, or least 4, or least 5, or least 6, or least 7, or least 8, or least 9, or least 10, or least 11, or least 12, or least 13, or least 14, or least 15, or least 16, or least 17, or least 18, or least 19, or least 20, or least 21, or least 22, or least 23, or least 24, or least 25, or least 26, or least 27, or least 28, or least 29, or least 30, or least 31, or least 32, or least 33, or least 34, or least 35, or more, or up to all known miRNAs.

In some examples, the method of determining whether a subject suffers or is at risk of suffering heart failure may include measuring the change in levels of at least two, at least three, at least four, at least five, at least six, at least seven, at least eight, at least nine, at least 10, at least 11, at least two to at least 20, at least 10 to at least 50, at least 40 to at least 66, or all miRNA as listed in Table 20. In some examples, the method of determining whether a subject suffers or is at risk of suffering heart failure may include measuring the change in levels of at least two, at least three, at least four, at least five, at least six, at least seven, at least eight, at least nine, at least 10, at least 11, or all miRNA as listed in Table 25.

In some examples, in the method as described herein, an increase in the level of miRNAs as listed as "increased" in Table 21, as compared to the control, may indicate the subject to have heart failure with reduced left ventricular ejection fraction (HFREF) or may be at a risk of developing heart failure with reduced left ventricular ejection fraction (HFREF). In some examples, in the method as described herein, a reduction in the level of miRNAs as listed as "reduced" in Table 21 as compared to the control, may indicate the subject to have heart failure with reduced left ventricular ejection fraction (HFREF) or may be at a risk of developing heart failure with reduced left ventricular ejection fraction (HFREF). In some examples, the method of determining whether a subject suffers or is at risk of suffering HFREF may include measuring the change in levels of at least two, or at least three, or at least four, or at least five, or at least six, or at least seven, or at least eight, or at least nine, or at least 10, or at least 11, or at least two to at least 20, or at least 10 to at least 43, or at least 15 to at least 43, or at least 30 to at least 43, or at least 40, or all miRNA as listed in Table 21.

As used herein, the term "heart failure with reduced left ventricular ejection fraction (HFREF)" or "heart failure with preserved left ventricular ejection fraction (HFPEF)" refers to the same term as commonly used in the art. For example, the term HFREF may also be referred to as systolic heart failure. In HFREF, the heart muscle does not contract effectively and less oxygen-rich blood is pumped out to the body. In contrast, the term "heart failure with preserved left ventricular ejection fraction (HFPEF)" refers to a diastolic heart failure. In HFPEF, the heart muscle contracts normally but the ventricles do not relax as they should during ventricular filling or when the ventricles relax).

In some examples, in the method as described herein, an increase in the level of miRNAs as listed as "increased" in Table 22, as compared to the control, may indicate the subject to have heart failure with preserved left ventricular ejection fraction (HFPEF) or may be at a risk of developing heart failure with preserved left ventricular ejection fraction (HFPEF). In some examples, in the method as described herein, a reduction in the level of miRNAs as listed as "reduced" in Table 22 as compared to the control, may indicate the subject to have heart failure with preserved left ventricular ejection fraction (HFPEF) or may be at a risk of developing heart failure with preserved left ventricular ejection fraction (HFPEF). In some examples, the method of determining whether a subject may suffer or may be at risk of suffering HFPEF may include measuring the change in levels of at least two, or at least three, or at least four, or at least five, or at least six, or at least seven, or at least eight, or at least nine, or at least 10, or at least 11, or at least two to at least 20, or at least 10 to at least 50, or at least 20 to at least 55, or at least 30 to at least 60, or at least 35 to at least 60, or at least 40 to at least 60, or at least 40 to at least 62, or all miRNA as listed in Table 22.

In another aspect, there is provided a method of determining whether a subject suffers from a heart failure selected from the group consisting of a heart failure with reduced left ventricular ejection fraction (HFREF) and a heart failure with preserved left ventricular ejection fraction (HFPEF), the method comprising the steps of a) detecting (or measuring) the levels of at least one miRNA as listed in Table 9 in a sample obtained from the subject and b) determining whether it is different as compared to a control, wherein altered levels of the miRNA may indicate that the subject has, or may be at a risk of, developing heart failure with reduced left ventricular ejection fraction (HFREF) or heart failure with preserved left ventricular ejection fraction (HFPEF).

Table 9. miRNAs differentially expressed between HFREF and HFPEF subjects

| Up-regulated (n=10) | | | | | |
|---|---|---|---|---|---|
| Name | p-value, t-test | p-value, Logistic regression | p-value, ln_BNP | Fold change | AUC |
| hsa-miR-223-5p | 3.5E-07 | 2.4E-04 | 7.3E-05 | 1.22 | 0.68 |
| hsa-miR-335-5p | 3.4E-04 | 2.9E-03 | 1.9E-03 | 1.26 | 0.63 |
| hsa-miR-452-5p | 2.4E-03 | >0.17 | >0.04 | 1.26 | 0.62 |
| hsa-miR-23b-3p | 7.1E-03 | >0.12 | 5.3E-03 | 1.16 | 0.62 |
| hsa-miR-181b-5p | 1.2E-03 | >0.01 | >0.01 | 1.20 | 0.62 |
| hsa-miR-146a-5p | 8.5E-03 | >0.16 | >0.03 | 1.20 | 0.62 |
| hsa-miR-181a-2-3p | 3.8E-04 | 2.9E-03 | 9.2E-03 | 1.20 | 0.61 |
| hsa-miR-199b-5p | 1.3E-03 | 3.3E-03 | 1.9E-03 | 1.24 | 0.61 |
| hsa-miR-126-5p | 5.7E-03 | >0.02 | >0.01 | 1.14 | 0.61 |
| hsa-miR-23a-5p | 6.4E-03 | >0.15 | >0.02 | 1.14 | 0.60 |
| Down-regulated (n=30) | | | | | |
| Name | p-value, t-test | p-value, Logistic regression | p-value, ln_BNP | Fold change | AUC |
| hsa-miR-185-5p | 1.9E-08 | 5.2E-05 | 4.2E-05 | 0.79 | 0.69 |
| hsa-miR-20b-5p | 3.2E-07 | 1.0E-03 | 1.5E-04 | 0.63 | 0.68 |
| hsa-miR-550a-5p | 3.5E-07 | 2.7E-04 | 1.1E-03 | 0.73 | 0.68 |
| hsa-miR-106a-5p | 9.6E-07 | 2.9E-03 | 4.5E-04 | 0.80 | 0.67 |
| hsa-miR-486-5p | 2.7E-06 | 9.5E-04 | 3.3E-04 | 0.67 | 0.66 |
| hsa-let-7b-5p | 6.3E-06 | 2.9E-03 | 3.3E-04 | 0.81 | 0.66 |
| hsa-miR-93-5p | 2.0E-06 | 1.0E-03 | 3.3E-04 | 0.81 | 0.65 |
| hsa-miR-20a-5p | 2.3E-05 | 9.3E-03 | 7.8E-04 | 0.81 | 0.65 |
| hsa-miR-25-3p | 2.4E-05 | 2.9E-03 | 8.7E-04 | 0.76 | 0.65 |
| hsa-miR-18b-5p | 9.9E-06 | 9.4E-03 | 1.9E-03 | 0.80 | 0.65 |
| hsa-miR-532-5p | 3.7E-05 | 4.0E-03 | 1.5E-03 | 0.80 | 0.65 |
| hsa-miR-501-5p | 4.7E-05 | 2.1E-03 | 7.5E-04 | 0.77 | 0.64 |
| hsa-miR-4732-3p | 8.4E-05 | 2.9E-03 | 1.5E-03 | 0.69 | 0.64 |

(continued)

| Down-regulated (n=30) | | | | | |
|---|---|---|---|---|---|
| Name | p-value, t-test | p-value, Logistic regression | p-value, In_BNP | Fold change | AUC |
| hsa-miR-144-3p | 1.1E-04 | 2.9E-03 | 7.5E-04 | 0.68 | 0.63 |
| hsa-miR-192-5p | 2.3E-03 | >0.08 | >0.04 | 0.75 | 0.63 |
| hsa-miR-17-5p | 2.7E-04 | >0.21 | 5.7E-03 | 0.83 | 0.62 |
| hsa-miR-363-3p | 1.2E-03 | >0.06 | >0.02 | 0.79 | 0.62 |
| hsa-miR-103a-3p | 1.2E-03 | >0.07 | >0.03 | 0.87 | 0.62 |
| hsa-miR-16-5p | 9.8E-04 | >0.22 | 3.2E-03 | 0.79 | 0.62 |
| hsa-miR-194-5p | 5.3E-03 | >0.18 | >0.05 | 0.78 | 0.62 |
| hsa-miR-183-5p | 1.4E-03 | >0.14 | >0.01 | 0.71 | 0.62 |
| hsa-miR-451a | 1.7E-03 | >0.13 | 3.2E-03 | 0.73 | 0.61 |
| hsa-miR-19b-3p | 1.7E-03 | >0.04 | 7.0E-03 | 0.85 | 0.61 |
| hsa-miR-30a-5p | 1.8E-03 | >0.20 | >0.07 | 0.81 | 0.60 |
| hsa-miR-106b-3p | 6.4E-03 | >0.03 | >0.01 | 0.90 | 0.60 |
| hsa-miR-19a-3p | 7.3E-03 | >0.09 | >0.05 | 0.87 | 0.60 |
| hsa-let-7i-5p | 1.2E-03 | >0.11 | >0.07 | 0.90 | 0.59 |
| hsa-miR-196b-5p | 7.7E-03 | >0.19 | >0.06 | 0.90 | 0.59 |
| hsa-miR-500a-5p | 8.5E-03 | >0.10 | >0.06 | 0.86 | 0.58 |
| hsa-miR-122-5p | 8.7E-03 | >0.05 | >0.01 | 0.68 | 0.58 |

In some examples, in the method as described herein, an increase in the level of miRNAs as listed as "increased" in Table 9, as compared to the control, may indicate the subject has heart failure with reduced left ventricular ejection fraction (HFREF) or heart failure with preserved left ventricular ejection fraction (HFPEF). In some examples, in the method as described herein, a reduction in the level of miRNAs as listed as "reduced" in Table 9 as compared to the control, may indicate the subject has developing heart failure with reduced left ventricular ejection fraction (HFREF) or heart failure with preserved left ventricular ejection fraction (HFPEF).

In some examples of the method for determining whether a subject suffers from a heart failure selected from the group consisting of a heart failure with reduced left ventricular ejection fraction (HFREF) and a heart failure with preserved left ventricular ejection fraction (HFPEF), the method may comprise measuring the change in levels of at least two, or at least three, or at least four, or at least five, or at least six, or at least seven, or at least eight, or at least nine, or at least 10, or at least 11, or at least two to at least 20, or at least 10 to at least 39, or all miRNA as listed in Table 9.

In the examples of the method for determining whether a subject suffers from a heart failure selected from the group consisting of a heart failure with reduced left ventricular ejection fraction (HFREF) and a heart failure with preserved left ventricular ejection fraction (HFPEF), the control may be a subject that has either a heart failure with reduced left ventricular ejection fraction (HFREF) or a heart failure with preserved left ventricular ejection fraction (HFPEF). In some examples, the control may be a patient with a heart failure with reduced left ventricular ejection fraction (HFREF), differential expression of miRNAs as listed in Table 9 indicates the subject to have a heart failure with preserved left ventricular ejection fraction (HFPEF). In some examples, when the control is a patient with a heart failure with preserved left ventricular ejection fraction (HFPEF), differential expression of miRNAs as listed in Table 9 indicates the subject to have a heart failure with reduced left ventricular ejection fraction (HFREF).

In addition, the inventors of the present disclosure also examined the use of these miRNAs as prognostic markers. That is, the methods of the present disclosure may be used to predict possible risk of events of death or hospitalization in the future or prospects of progress determined by diagnosing a disease. Prognosis in patients with heart failure means predicting the possibility of observed survival (survival free of death) or event free survival (survival free of hospitalization or death). As used herein, the term "observed survival", or "all-cause survival", or "all-cause mortalilty", or "all-cause of death", refers to observed survival rate of subjects in view of any causes of death. This term is in contrast to the term "event free survival (EFS)", which refers to the absence of the recurrent hospital admission for heart failure (i.e. the length of time after heart failure treatment during which recurrent admission for decompensated heart failure is avoided) and any causes of death.

The inventors of the present disclosure found that there were a number of miRNAs that were found to be good predictors for either the observed (all-cause) survival (OS) (i.e. observed survival rate due to all causes of death) or event free survival (EFS) combination of recurrent admission for heart failure (i.e. the length of time after heart failure treatment during which recurrent admission for decompensated heart failure is avoided) and all causes of death in chronic heart failure patients. Thus, the present disclosure may also be used in a method of predicting the prognosis of a subject. Thus, in another aspect of the present disclosure, there is provided a method for determining the risk of a heart failure patient having an altered risk of death (or decreased observed (all-cause) survival rate). In some examples, the method may comprise the steps of a) detecting the levels of at least one miRNA as listed in Table 14 in a sample obtained from the subject; and/or measuring the levels of at least one miRNAs listed in Table 14; and b) determining whether the levels of at least one miRNAs listed in Table 14 is different as compared to the levels of the miRNAs of a control population, wherein altered levels of the miRNA indicates that the subject is likely to have an altered risk of death (altered observed (all-cause) survival rate) compared to the control population.

**Table 14. miRNAs that may be used in predicting observed survival**

| HR > 1 OR ln(HR) > 0 (n = 26) | | | | | | | | |
|---|---|---|---|---|---|---|---|---|
| | Univariate analysis | | | | Multivariate analysis | | | |
| | HR | ln(HR) | SE of ln(HR) | p-value | HR | ln(HR) | SE of ln(HR) | p-value |
| hsa-miR-503 | 1.90 | 0.64 | 0.17 | 1.40E-04 | 1.79 | 0.58 | 0.19 | 2.80E-03 |
| hsa-miR-186-5p | 1.72 | 0.54 | 0.17 | 1.70E-03 | 1.54 | 0.43 | 0.17 | 9.50E-03 |
| hsa-miR-21-3p | 1.65 | 0.5 | 0.15 | 1.00E-03 | 1.48 | 0.39 | 0.17 | 2.70E-02 |
| hsa-miR-337-3p | 1.60 | 0.47 | 0.15 | 1.80E-03 | 1.60 | 0.47 | 0.16 | 3.10E-03 |
| hsa-miR-424-5p | 1.57 | 0.45 | 0.14 | 1.50E-03 | 1.38 | 0.32 | 0.16 | 4.20E-02 |
| hsa-miR-127-3p | 1.57 | 0.45 | 0.16 | 5.10E-03 | 1.51 | 0.41 | 0.17 | 1.40E-02 |
| hsa-miR-369-3p | 1.57 | 0.45 | 0.16 | 6.50E-03 | 1.52 | 0.42 | 0.17 | 1.60E-02 |
| hsa-miR-487b | 1.52 | 0.42 | 0.15 | 5.80E-03 | 1.72 | 0.54 | 0.17 | 1.80E-03 |
| hsa-miR-485-3p | 1.52 | 0.42 | 0.16 | 6.90E-03 | 1.57 | 0.45 | 0.17 | 6.90E-03 |
| hsa-miR-379-5p | 1.52 | 0.42 | 0.16 | 9.00E-03 | 1.62 | 0.48 | 0.17 | 4.40E-03 |
| hsa-miR-299-3p | 1.51 | 0.41 | 0.15 | 6.90E-03 | 1.39 | 0.33 | 0.16 | 3.30E-02 |
| hsa-miR-377-3p | 1.49 | 0.4 | 0.15 | 7.80E-03 | 1.51 | 0.41 | 0.15 | 6.90E-03 |
| hsa-miR-495 | 1.48 | 0.39 | 0.16 | 1.30E-02 | 1.62 | 0.48 | 0.17 | 5.10E-03 |
| hsa-miR-654-3p | 1.48 | 0.39 | 0.15 | 8.10E-03 | 1.46 | 0.38 | 0.15 | 1.00E-02 |
| hsa-miR-493-5p | 1.46 | 0.38 | 0.16 | 1.50E-02 | 1.46 | 0.38 | 0.16 | 1.70E-02 |
| hsa-miR-382-5p | 1.45 | 0.37 | 0.14 | 9.30E-03 | 1.35 | 0.3 | 0.14 | 3.60E-02 |
| hsa-miR-23a-3p | 1.45 | 0.37 | 0.17 | 3.40E-02 | 1.48 | 0.39 | 0.18 | 3.00E-02 |
| hsa-miR-154-5p | 1.43 | 0.36 | 0.14 | 1.10E-02 | 1.34 | 0.29 | 0.15 | 4.40E-02 |
| hsa-miR-134 | 1.43 | 0.36 | 0.14 | 1.00E-02 | 1.30 | 0.26 | 0.14 | 6.70E-02 |
| hsa-miR-136-5p | 1.40 | 0.34 | 0.15 | 1.80E-02 | 1.48 | 0.39 | 0.16 | 1.40E-02 |
| hsa-miR-128 | 1.39 | 0.33 | 0.15 | 2.70E-02 | 1.39 | 0.33 | 0.16 | 3.50E-02 |
| hsa-miR-200c-3p | 1.38 | 0.32 | 0.15 | 3.00E-02 | 1.38 | 0.32 | 0.16 | 4.40E-02 |
| hsa-miR-1226-3p | 1.38 | 0.32 | 0.16 | 5.50E-02 | 1.55 | 0.44 | 0.18 | 1.70E-02 |
| hsa-miR-24-3p | 1.35 | 0.3 | 0.15 | 4.50E-02 | 1.23 | 0.21 | 0.15 | 1.60E-01 |
| hsa-miR-29c-5p | 1.30 | 0.26 | 0.15 | 7.90E-02 | 1.39 | 0.33 | 0.17 | 4.80E-02 |
| hsa-miR-374b-5p | 1.19 | 0.17 | 0.15 | 2.60E-01 | 1.42 | 0.35 | 0.17 | 4.00E-02 |

(continued)

| HR < 1 OR ln(HR) < 0 (n = 14) | | | | | | | | |
| --- | --- | --- | --- | --- | --- | --- | --- | --- |
| | Univariate analysis | | | | Multivariate analysis | | | |
| | HR | ln(HR) | SE of ln(HR) | p-value | HR | ln(HR) | SE of ln(HR) | p-value |
| hsa-miR-150-5p | 0.52 | -0.66 | 0.13 | 1.30E-07 | 0.59 | -0.52 | 0.14 | 3.20E-04 |
| hsa-miR-192-5p | 0.64 | -0.44 | 0.15 | 3.80E-03 | 0.76 | -0.28 | 0.18 | 1.10E-01 |
| hsa-miR-122-5p | 0.68 | -0.39 | 0.15 | 9.90E-03 | 0.76 | -0.28 | 0.17 | 9.60E-02 |
| hsa-miR-500a-5p | 0.70 | -0.36 | 0.15 | 1.70E-02 | 0.79 | -0.23 | 0.16 | 1.50E-01 |
| hsa-miR-181a-2-3p | 0.70 | -0.35 | 0.11 | 9.70E-04 | 0.67 | -0.4 | 0.14 | 3.50E-03 |
| hsa-miR-194-5p | 0.70 | -0.35 | 0.15 | 2.10E-02 | 0.79 | -0.24 | 0.17 | 1.50E-01 |
| hsa-miR-92a-3p | 0.70 | -0.35 | 0.16 | 2.60E-02 | 0.70 | -0.36 | 0.16 | 2.20E-02 |
| hsa-miR-660-5p | 0.71 | -0.34 | 0.15 | 2.50E-02 | 0.73 | -0.32 | 0.16 | 3.80E-02 |
| hsa-miR-486-5p | 0.72 | -0.33 | 0.14 | 2.20E-02 | 0.74 | -0.3 | 0.15 | 4.30E-02 |
| hsa-miR-375 | 0.72 | -0.33 | 0.14 | 1.80E-02 | 0.77 | -0.26 | 0.14 | 6.40E-02 |
| hsa-miR-101-3p | 0.73 | -0.32 | 0.15 | 3.30E-02 | 0.81 | -0.21 | 0.16 | 1.90E-01 |
| hsa-miR-30c-5p | 0.73 | -0.31 | 0.15 | 3.50E-02 | 0.84 | -0.18 | 0.16 | 2.40E-01 |
| hsa-miR-20b-5p | 0.74 | -0.3 | 0.13 | 2.00E-02 | 0.84 | -0.17 | 0.15 | 2.50E-01 |
| hsa-miR-10b-5p | 0.74 | -0.3 | 0.14 | 3.30E-02 | 0.80 | -0.22 | 0.15 | 1.30E-01 |

As used herein, the term "hazard ratio" refers to a term commonly known in the art to relate to a rate, or an estimate of the potential for "death" or "hospital admission" per unit time at a particular instant, given that the subject has "survived" until that instant (of "death" or "hospital admission". It is used to measure the magnitude of difference between two survival curves. Hazard ratio (HR) > 1 indicates the higher risk of having short survival time and Hazard ratio (HR) < 1 indicates the higher risk of having longer survival time. As known in the art, the hazard ratio may be calculated by Cox proportional hazards (CoxPH) model.

In some examples, in the method as described herein, an increase in the level of miRNA as listed as "hazard ratio > 1" in Table 14, as compared to the control, may indicate the subject has an increased risk of death (decreased observed (all-cause) survival rate). In some examples, in the method as described herein, a reduction in the level of miRNA as listed as "hazard ratio > 1" in Table 14, as compared to the control, may indicate the subject has a decreased risk of death (increased observed (all-cause) survival rate).

In some examples, in the method as described herein, an increase in the level of miRNA as listed as "hazard ratio < 1" in Table 14, as compared to the control, may indicate the subject has a decreased risk of death (increased observed (all-cause) survival rate). In some examples, in the method as described herein, a reduction in the level of miRNA as listed as "hazard ratio < 1" in Table 14, as compared to the control, may indicate the subject has an increased risk of death (decreased observed (all-cause) survival rate).

In another aspect, there is provided a method for determining the risk of a heart failure patient having an altered risk of disease progression to hospitalization or death (decreased event free survival rate). In some examples, the method comprises the steps of a) detecting the levels of at least one miRNA as listed in Table 15 in a sample obtained from the subject; and/or measuring the levels of at least one miRNAs listed in Table 15; and b) determining whether the levels of at least one miRNAs listed in Table 15 is different as compared to the levels of the miRNAs of a control population, wherein altered levels of the miRNA indicates that the subject is likely to have an altered risk of disease progression to hospitalization or death (altered event free survival rate)compared to the control population.

**Table 15. miRNAs predictive of event free survival**

| HR >1 OR ln(HR) > 0 (n = 4) | | | | | | | | |
|---|---|---|---|---|---|---|---|---|
| | Univariate analysis | | | | Multivariate analysis | | | |
| | HR | ln(HR) | SE of ln(HR) | p-value | HR | ln(HR) | SE of ln(HR) | p-value |
| hsa-miR-331-5p | 1.27 | 0.24 | 0.08 | 0.0025 | 1.12 | 0.11 | 0.08 | 0.15 |
| hsa-miR-21-3p | 1.25 | 0.22 | 0.08 | 0.01 | 1.11 | 0.1 | 0.09 | 0.27 |
| hsa-miR-497-5p | 1.20 | 0.18 | 0.08 | 0.033 | 1.07 | 0.07 | 0.08 | 0.39 |
| hsa-miR-22-3p | 1.25 | 0.22 | 0.06 | 0.00048 | 1.06 | 0.06 | 0.07 | 0.34 |
| HR< 1 OR ln(HR) < 0 (n = 9) | | | | | | | | |
| | Univariate analysis | | | | Multivariate analysis | | | |
| | HR | ln(HR) | SE of ln(HR) | p-value | HR | ln(HR) | SE of ln(HR) | p-value |
| hsa-miR-30e-3p | 0.80 | -0.22 | 0.08 | 0.007 | 0.88 | -0.13 | 0.09 | 0.14 |
| hsa-miR-191-5p | 0.81 | -0.21 | 0.08 | 0.013 | 0.90 | -0.1 | 0.09 | 0.27 |
| hsa-miR-30b-5p | 0.82 | -0.2 | 0.08 | 0.018 | 0.97 | -0.03 | 0.09 | 0.7 |
| hsa-miR-454-3p | 0.83 | -0.19 | 0.08 | 0.018 | 0.94 | -0.06 | 0.09 | 0.47 |
| hsa-miR-150-5p | 0.83 | -0.19 | 0.08 | 0.017 | 0.89 | -0.12 | 0.09 | 0.17 |
| hsa-miR-17-5p | 0.83 | -0.19 | 0.07 | 0.0054 | 0.88 | -0.13 | 0.08 | 0.09 |
| hsa-miR-103a-3p | 0.83 | -0.19 | 0.08 | 0.017 | 0.91 | -0.09 | 0.08 | 0.29 |
| hsa-miR-374b-5p | 0.84 | -0.17 | 0.08 | 0.048 | 0.98 | -0.02 | 0.09 | 0.8 |
| hsa-miR-551b-3p | 0.85 | -0.16 | 0.08 | 0.036 | 0.96 | -0.04 | 0.08 | 0.62 |

In some examples, in the method as described herein, an increase in the level of miRNA as listed as "hazard ratio > 1" in Table 15, as compared to the control, may indicate the subject has an increased risk of disease progression to hospitalization or death (decreased event free survival rate). In some examples, in the method as described herein, a reduction in the level of miRNA as listed as "hazard ratio > 1" in Table 15, as compared to the control, may indicate the subject has a decreased risk of disease progression to hospitalization or death (increased event free survival rate).

In some examples, in the method as described herein, an increase in the level of miRNA as listed as "hazard ratio < 1" in Table 15, as compared to the control, may indicate the subject has a decreased risk of disease progression to hospitalization or death (increased event free survival rate). In some examples, in the method as described herein, a reduction in the level of miRNA as listed as "hazard ratio < 1" in Table 15, as compared to the control, may indicate the subject has an increased risk of disease progression to hospitalization or death (decreased event free survival rate).

In some examples, in the method as described herein, the control may be control population or cohort of heart failure subjects. In some examples, the control population may be a population or cohort of heart failure patients where the microRNA expression levels and risk of death or disease progression for the population can be determined. In some examples, the expression level of microRNAs for the control population may be the mean or median expression level for all subjects (including the patient in question) in the population. In some examples, if 10% of the patients in the control population died within 5 years, the risk of death within 5 years is 10% for the control population. In some examples, the control population include the heart failure patient whose risk of death or disease progression is to be determined with the microRNA expression levels.

In some examples, in the method as described herein, the heart failure patient may be a subject who has had primary diagnosis of heart failure and/or being treated 3-5 days when symptomatically improved, with resolution of bedside physical signs of heart failure and considered fit to discharge. In some examples, the patient may be a stable compensated heart failure patient, which have yet to have further deterioration into recurrent acute decompensated heart failure that require re-hospitalization or death.

In yet another aspect, there is provided a method of determining the risk of developing heart failure in a subject or determining whether a subject suffers from heart failure, comprising the steps of: (a) detecting the presence of miRNA in a sample obtained from the subject; and/or measuring the levels of at least three miRNAs listed in Table 16 or Table 23 in the sample; and (b) using a score based on the levels of the miRNAs measured in step (a) to predict the likelihood of the subject to develop or to have heart failure. In some examples, the method may further comprise measuring the levels of at least one miRNA as listed in "insignificant group" in Table 16, or Table 23 and wherein the at least one miRNA is hsa-miR-10b-5p.

As used herein throughout the disclosure and with reference to all methods as described herein, the term "score" refers to an integer or number, that can be determined mathematically, for example by using computational models a known in the art, which can include but are not limited to, SMV, as an example, and that is calculated using any one of a multitude of mathematical equations and/or algorithms known in the art for the purpose of statistical classification. Such a score is used to enumerate one outcome on a spectrum of possible outcomes. The relevance and statistical significance of such a score depends on the size and the quality of the underlying data set used to establish the results spectrum. For example, a blind sample may be input into an algorithm, which in turn calculates a score based on the information provided by the analysis of the blind sample. This results in the generation of a score for said blind sample. Based on this score, a decision can be made, for example, how likely the patient, from which the blind sample was obtained, has heart failure or not. The ends of the spectrum may be defined logically based on the data provided, or arbitrarily according to the requirement of the experimenter. In both cases the spectrum needs to be defined before a blind sample is tested. As a result, the score generated by such a blind sample, for example the number "45" may indicate that the corresponding patient has heart failure, based on a spectrum defined as a scale from 1 to 50, with "1" being defined as being heart failure-free and "50" being defined as having heart failure. Therefore, the term "score", refers to a mathematical score, which can be calculated using any one of a multitude of mathematical equations and/or algorithms known in the art for the purpose of statistical classification. Examples of such mathematical equations and/or algorithms can be, but are not limited to, a (statistical) classification algorithm selected from the group consisting of support vector machine algorithm, logistic regression algorithm, multinomial logistic regression algorithm, Fisher's linear discriminant algorithm, quadratic classifier algorithm, perceptron algorithm, k-nearest neighbours algorithm, artificial neural network algorithm, random forests algorithm, decision tree algorithm, naive Bayes algorithm, adaptive Bayes network algorithm, and ensemble learning method combining multiple learning algorithms. In another example, the classification algorithm is pre-trained using the expression level of the control. In some examples, the classification algorithm compares the expression level of the subject with that of the control and returns a mathematical score that identifies the likelihood of the subject to belong to either one of the control groups. In some examples, the classification algorithm may compare the expression level of the subject with that of the control and returns a mathematical score that identifies the likelihood of the subject to belong to either one of the control groups. Examples of algorithms that may be used in the present disclosure are provided below.

**Table 16. miRNAs for multivariate detection of heart failure**

| Significant miRNAs | | | | |
|---|---|---|---|---|
| Name | prevalence in biomarker panels | Significant for all HF | Significant for HFREF | Significant for HFPEF |
| hsa-miR-55 1b-3p | 59.7% | Yes | Yes | Yes |
| hsa-miR-24-3p | 57.3% | Yes | Yes | Yes |
| hsa-miR-576-5p | 39.7% | Yes | No | Yes |
| hsa-miR-375 | 39.7% | Yes | Yes | Yes |
| hsa-miR-451a | 37.5% | Yes | Yes | Yes |
| hsa-miR-503 | 37.0% | Yes | Yes | Yes |
| hsa-miR-374b-5p | 25.5% | Yes | Yes | Yes |
| hsa-miR-423-5p | 24.9% | Yes | Yes | Yes |
| hsa-miR-181b-5p | 24.2% | Yes | Yes | Yes |

(continued)

| Significant miRNAs | | | | |
|---|---|---|---|---|
| Name | prevalence in biomarker panels | Significant for all HF | Significant for HFREF | Significant for HFPEF |
| hsa-miR-454-3p | 24.2% | Yes | Yes | Yes |
| hsa-miR-484 | 23.0% | Yes | Yes | Yes |
| hsa-miR-191-5p | 20.2% | Yes | Yes | Yes |
| hsa-miR-1280 | 14.8% | Yes | Yes | Yes |
| hsa-miR-205-5p | 12.3% | Yes | Yes | Yes |
| hsa-miR-424-5p | 11.9% | Yes | Yes | Yes |
| hsa-miR-106a-5p | 11.8% | Yes | Yes | Yes |
| hsa-miR-532-5p | 11.1% | Yes | Yes | Yes |
| hsa-miR-197-3p | 10.9% | Yes | Yes | Yes |
| hsa-miR-598 | 10.9% | Yes | Yes | Yes |
| hsa-miR-34b-3p | 10.6% | Yes | Yes | Yes |
| hsa-miR-103a-3p | 9.9% | Yes | Yes | Yes |
| hsa-miR-30b-5p | 9.7% | Yes | Yes | Yes |
| hsa-miR-199a-3p | 9.7% | Yes | No | Yes |
| hsa-let-7b-3p | 9.6% | Yes | Yes | Yes |
| hsa-miR-374c-5p | 6.1% | Yes | Yes | Yes |
| hsa-miR-148a-3p | 5.7% | Yes | Yes | Yes |
| hsa-miR-23c | 5.2% | Yes | Yes | Yes |
| hsa-miR-132-3p | 5.0% | Yes | Yes | No |
| hsa-miR-200b-3p | 4.5% | No | Yes | No |
| hsa-miR-21-5p | 4.5% | Yes | Yes | Yes |
| hsa-miR-130b-3p | 4.2% | Yes | Yes | Yes |
| hsa-miR-221-3p | 4.0% | Yes | Yes | Yes |

(continued)

| Significant miRNAs | | | | |
|---|---|---|---|---|
| **Name** | **prevalence in biomarker panels** | **Significant for all HF** | **Significant for HFREF** | **Significant for HFPEF** |
| hsa-miR-223-5p | 3.9% | Yes | Yes | Yes |
| hsa-miR-627 | 3.7% | Yes | Yes | Yes |
| hsa-miR-550a-5p | 3.4% | No | No | Yes |
| hsa-miR-382-5p | 3.4% | Yes | Yes | Yes |
| hsa-miR-19b-3p | 3.2% | Yes | Yes | Yes |
| hsa-miR-20a-5p | 3.2% | Yes | Yes | Yes |
| hsa-miR-23b-3p | 3.0% | Yes | Yes | Yes |
| hsa-miR-30a-5p | 2.7% | Yes | Yes | No |
| hsa-miR-363-3p | 2.4% | Yes | Yes | Yes |
| hsa-miR-30c-5p | 2.4% | Yes | Yes | Yes |
| Insignificant miRNAs | | | | |
| **Name** | **prevalence in biomarker panels** | **Significant for all HF** | **Significant for HFREF** | **Significant for HFPEF** |
| hsa-miR-10b-5p | 35.0% | No | No | No |
| hsa-miR-29c-3p | 13.9% | No | No | No |
| hsa-miR-660-5p | 12.1% | No | No | No |
| hsa-miR-133a | 7.9% | No | No | No |
| hsa-miR-379-5p | 5.2% | No | No | No |
| hsa-miR-10a-5p | 4.7% | No | No | No |
| hsa-miR-92a-3p | 4.0% | No | No | No |
| hsa-miR-222-3p | 3.7% | No | No | No |
| hsa-miR-200c-3p | 3.5% | No | No | No |

**Table 23. miRNAs for heart failure detection**

| Significant miRNAs (n=35) | | | | |
|---|---|---|---|---|
| Name | prevalence in biomarker panels | Significant for all HF | Significant for HFREF | Significant for HFPEF |
| hsa-miR-55 1b-3p | 59.7% | Yes | Yes | Yes |
| hsa-miR-24-3p | 57.3% | Yes | Yes | Yes |
| hsa-miR-576-5p | 39.7% | Yes | No | Yes |
| hsa-miR-375 | 39.7% | Yes | Yes | Yes |
| hsa-miR-451a | 37.5% | Yes | Yes | Yes |
| hsa-miR-503 | 37.0% | Yes | Yes | Yes |
| hsa-miR-374b-5p | 25.5% | Yes | Yes | Yes |
| hsa-miR-181b-Sp | 24.2% | Yes | Yes | Yes |
| hsa-miR-454-3p | 24.2% | Yes | Yes | Yes |
| hsa-miR-484 | 23.0% | Yes | Yes | Yes |
| hsa-miR-205-5p | 12.3% | Yes | Yes | Yes |
| hsa-miR-424-5p | 11.9% | Yes | Yes | Yes |
| hsa-miR-106a-5p | 11.8% | Yes | Yes | Yes |
| hsa-miR-532-5p | 11.1% | Yes | Yes | Yes |
| hsa-miR-197-3p | 10.9% | Yes | Yes | Yes |
| hsa-miR-598 | 10.9% | Yes | Yes | Yes |
| hsa-miR-34b-3p | 10.6% | Yes | Yes | Yes |
| hsa-miR-199a-3p | 9.7% | Yes | No | Yes |
| hsa-let-7b-3p | 9.6% | Yes | Yes | Yes |
| hsa-miR-374c-5p | 6.1% | Yes | Yes | Yes |
| hsa-miR-148a-3p | 5.7% | Yes | Yes | Yes |
| hsa-miR-23c | 5.2% | Yes | Yes | Yes |
| hsa-miR-132-3p | 5.0% | Yes | Yes | No |
| hsa-miR-200b-3p | 4.5% | No | Yes | No |

(continued)

| Significant miRNAs (n=35) | | | | |
|---|---|---|---|---|
| Name | prevalence in biomarker panels | Significant for all HF | Significant for HFREF | Significant for HFPEF |
| hsa-miR-130b-3p | 4.2% | Yes | Yes | Yes |
| hsa-miR-221-3p | 4.0% | Yes | Yes | Yes |
| hsa-miR-223-5p | 3.9% | Yes | Yes | Yes |
| hsa-miR-627 | 3.7% | Yes | Yes | Yes |
| hsa-miR-550a-5p | 3.4% | No | No | Yes |
| hsa-miR-382-5p | 3.4% | Yes | Yes | Yes |
| hsa-miR-19b-3p | 3.2% | Yes | Yes | Yes |
| hsa-miR-20a-5p | 3.2% | Yes | Yes | Yes |
| hsa-miR-23b-3p | 3.0% | Yes | Yes | Yes |
| hsa-miR-363-3p | 2.4% | Yes | Yes | Yes |
| hsa-miR-30c-5p | 2.4% | Yes | Yes | Yes |
| Insignificant miRNAs (n=7) | | | | |
| Name | prevalence in biomarker panels | Significant for all HF | Significant for HFREF | Significant for HFPEF |
| hsa-miR-10b-5p | 35.0% | No | No | No |
| hsa-miR-660-5p | 12.1% | No | No | No |
| hsa-miR-133a | 7.9% | No | No | No |
| hsa-miR-379-5p | 5.2% | No | No | No |
| hsa-miR-10a-5p | 4.7% | No | No | No |
| hsa-miR-222-3p | 3.7% | No | No | No |
| hsa-miR-200c-3p | 3.5% | No | No | No |

In some examples, the method as disclosed herein measures the change in levels of: at least four, or at least five, or at least six, or at least seven, or at least eight, or at least nine, or at least 10, or at least 11, or at least two to at least 20, or at least 10 to at least 45, or at least 40 to at least 50, or all miRNA as listed in Table 16. In some examples, the method as disclosed herein measures at least four, or at least five, or at least six, or at least seven, or at least eight, or at least nine, or at least 10, or at least 11, or at least two to at least 20, or at least 10 to at least 41, or all miRNA as listed in Table 23.

In some examples, the method as disclosed herein measures the level of at least one (or multiple) miRNAs (in a subject's plasma sample). The measurement of at least one miRNAs may be combined to generate a score for the prediction of heart failure or the classification of HFREF and HFPEF subtypes. In some examples, the formula to generate the score may be Formula 1, which formula is as follows:

**Formula 1 -**

$$prediction\ score = B + \sum_{i=1}^{n} K_i \times \log_2 copy\_miRNA_i,$$

where $\log_2 copy\_miRNA_i$ is log transformed copy numbers (copy/ml of plasma) of individual miRNAs'; $K_i$ is the coefficients used to weight multiple miRNA targets; and $B$ is a constant value to adjust the scale of the prediction score.

Formula 1 here demonstrated the use of a linear model for the prediction of heart failure or classification of HFREF and HFPEF subtypes. The prediction score (unique for each subject) is the number to make the predictive or diagnostic decisions.

In the Experimental Section of the present disclosure, the diagnostic utility of the identified miRNAs underwent further statistical evaluation. Multivariate miRNA biomarker panels (HF panel, HFREF and HFPEF panels) were then formulated by sequence forward floating search (SFFS) [53] and support vector machine (SVM) [54] with repeated cross-validation *in silico.* The inventors of the present disclosure found some of the miRNAs in the biomarker panels consistently produced AUC values (Areas Under the Curve) of ≥0.92 for HF detection (Figure 20, B) and AUC ≥0.75 for subtype categorization (Figure 24, A) in the receiver operating characteristic (ROC) plot. The miRNA panels, when used in combination with NT-proBNP, exhibited marked improved discriminative power and better classification accuracies for both purposes (Figure 22, B and 24, B).

Thus, in another aspect, there is provided a method of determining the risk of developing heart failure in a subject or determining whether a subject suffers from heart failure, comprising the steps of: (a) detecting the presence of miRNA in a sample obtained from the subject; and/or measuring the levels of at least two miRNAs listed in Table 17 in the sample; and (b) using a score based on the levels of the miRNAs measured in step (a) to predict the likelihood of the subject to develop or to have heart failure.

**Table 17. miRNAs to be used in conjunction with NP-proBNP in detection of heart failure**

| Significant miRNAs (additional to ln_NT-proBNP) | | | | |
|---|---|---|---|---|
| Name | prevalence in biomarker panels | Significant for all HF | Significant for HFREF | Significant for HFPEF |
| hsa-miR-454-3p | 42.0% | Yes | No | Yes |
| hsa-miR-451a | 38.6% | Yes | No | Yes |
| hsa-miR-503 | 36.7% | Yes | No | Yes |
| hsa-miR-1280 | 30.9% | Yes | No | Yes |
| hsa-miR-103a-3p | 22.1% | Yes | No | Yes |
| hsa-miR-106a-5p | 19.2% | Yes | No | Yes |
| hsa-miR-375 | 16.2% | Yes | No | Yes |
| hsa-miR-148a-3p | 13.8% | Yes | No | Yes |
| hsa-miR-24-3p | 12.9% | Yes | No | Yes |
| hsa-miR-17-5p | 11.6% | Yes | No | Yes |
| hsa-miR-25-3p | 11.0% | Yes | No | Yes |
| hsa-miR-30b-5p | 10.9% | Yes | No | Yes |

(continued)

| Significant miRNAs (additional to ln_NT-proBNP) | | | | |
|---|---|---|---|---|
| Name | prevalence in biomarker panels | Significant for all HF | Significant for HFREF | Significant for HFPEF |
| hsa-miR-196b-5p | 9.8% | Yes | No | Yes |
| hsa-miR-34b-3p | 7.3% | Yes | No | Yes |
| hsa-miR-363-3p | 6.8% | Yes | No | Yes |
| hsa-miR-374b-5p | 6.7% | Yes | No | No |
| hsa-miR-193a-5p | 6.6% | Yes | No | No |
| hsa-miR-197-3p | 5.1% | Yes | No | Yes |
| hsa-miR-101-3p | 4.8% | Yes | No | Yes |
| hsa-miR-532-5p | 4.7% | Yes | No | Yes |
| hsa-miR-30c-5p | 4.3% | Yes | No | Yes |
| hsa-miR-16-5p | 4.3% | Yes | No | Yes |
| hsa-miR-144-3p | 4.2% | Yes | No | Yes |
| hsa-miR-183-5p | 4.2% | Yes | No | Yes |
| hsa-miR-20b-5p | 4.1% | Yes | No | Yes |
| hsa-miR-501-5p | 4.0% | Yes | No | Yes |
| hsa-miR-423-5p | 3.9% | Yes | No | No |
| hsa-miR-130b-3p | 3.9% | Yes | No | Yes |
| hsa-miR-20a-5p | 3.6% | Yes | No | Yes |
| hsa-miR-29b-3p | 3.2% | Yes | No | Yes |
| hsa-let-7b-5p | 3.1% | Yes | No | Yes |
| hsa-miR-500a-5p | 2.6% | Yes | No | Yes |
| hsa-miR-19b-3p | 2.3% | Yes | No | Yes |
| hsa-miR-4732-3p | 2.2% | Yes | No | Yes |

(continued)

| Significant miRNAs (additional to ln_NT-proBNP) | | | | |
|---|---|---|---|---|
| Name | prevalence in biomarker panels | Significant for all HF | Significant for HFREF | Significant for HFPEF |
| hsa-let-7d-3p | 2.2% | Yes | No | Yes |
| hsa-miR-15a-5p | 2.0% | Yes | No | Yes |
| Insignificant miRNAs (additional to ln_NT-proBNP) | | | | |
| Name | prevalence in biomarker panels | Significant for all HF | Significant for HFREF | Significant for HFPEF |
| hsa-miR-576-5p | 25.4% | No | No | No |
| hsa-miR-124-5p | 15.9% | No | No | No |
| hsa-miR-192-5p | 9.6% | No | No | No |
| hsa-miR-551b-3p | 8.1% | No | No | No |
| hsa-miR-150-5p | 7.6% | No | No | No |
| hsa-miR-191-5p | 6.6% | No | No | No |
| hsa-miR-10b-5p | 6.5% | No | No | No |
| hsa-miR-181a-2-3p | 4.2% | No | No | No |
| hsa-miR-181b-5p | 2.8% | No | No | No |
| hsa-miR-26a-5p | 2.8% | No | No | No |
| hsa-miR-205-5p | 2.6% | No | No | No |
| hsa-miR-92a-3p | 2.4% | No | No | No |
| hsa-miR-424-5p | 2.3% | No | No | No |

In some examples, the methods as described herein may further comprise the step of determining the level of Brain Natriuretic Peptide (BNP) and/or N-terminal prohormone of brain natriuretic peptide (NT-proBNP). In some examples, both NT-proBNP and BNP are good markers of prognosis and diagnosis of heart failure, such as chronic heart failure.

In some examples, the method as described herein may measure the altered levels of at least three, or at least four, or at least five, or at least six, or at least seven, or at least eight, or at least nine, or at least 10, or at least 11, or at least two to at least 20, or at least 10 to at least 45, or at least 40 to at least 48, or all miRNA as listed in Table 17.

In some examples, in the methods as disclosed herein, where BNP and/or NT-proBNP are used together with miRNA, Formula 2 can be used instead. In Formula 2, the level of BNP/NT-proBNP in the plasma sample is included into the linear model. In one example, Formula 2 is as follows:

**Formula 2 -**

$$prediction\ score = B + BNP + \sum_{i=1}^{n} K_i \times \log_2 copy\_miRNA_i,$$

wherein, $\log_2 copy\_miRNA_i$ is log transformed copy numbers (copy/ml of plasma) of individual miRNAs'; $K_i$ is the coefficients used to weight multiple miRNA targets; $B$ is a constant value to adjust the scale of the prediction score; $BNP$ is a measure positively or negatively correlated with the level of BNP and/or NT-proBNP in the sample.

Additionally, for the prediction of heart failure, the prediction score (which would be unique for each subject) is the number that indicates the likelihood of a subject having heart failure. In some examples, the outcome of the methods as described herein (i.e. prediction of likelihood or diagnosis) may be found in Formula 3. If the value is higher than a pre-set cutoff value, the subject will be diagnosed or predicted to have heart failure. If the value is lower than a pre-set cutoff value, the subject will be diagnosed or predicted to be without heart failure. Formula 3 is as follows:

**Formula 3 -**

$$outcome = \begin{cases} have\ heart\ failure,\ if\ prediction\ score > cutoff \\ no\ heart\ failure,\ if\ prediciton\ score < cutoff \end{cases}$$

In some examples, for the classification of HFREF and HFPEF subtypes, the prediction score (which is unique for each subject) may be the number that indicates the likelihood of a heart failure subject having HFPEF subtype of heart failure. In some examples, the outcome of the diagnosis may be found in Formula 4. If the value is higher than a pre-set cutoff value, the heart failure subject will be diagnosed as (or predicted to) having HFPEF subtype of heart failure. If the value is lower than a pre-set cutoff value, the heart failure subject will be diagnosed as (or predicted to) have HFREF subtype of heart failure by this test.

**Formula 4 -**

$$outcome = \begin{cases} have\ HFPEF\ subtype\ of\ heart\ failure,\ if\ prediction\ score > cutoff \\ have\ HFREF\ subtype\ of\ heart\ failure,\ if\ prediciton\ score < cutoff \end{cases}$$

In another aspect, there is provided a method of determining the likelihood of a subject to be suffering from a heart failure with reduced left ventricular ejection fraction (HFREF) or a heart failure with preserved left ventricular ejection fraction (HFPEF). In some examples, the method comprises the steps of: (a) detecting the presence of miRNA in a sample obtained from the subject; and/or measuring the levels of at least three miRNA listed in Table 18 in the sample; and (b) using a score based on the levels of the miRNAs measured in step (a) to predict the likelihood of the subject to be suffering from, a heart failure with reduced left ventricular ejection fraction (HFREF) or a heart failure with preserved left ventricular ejection fraction (HFPEF).

**Table 18. miRNA for determining heart failure subtype categorization**

| Significant miRNAs | |
| --- | --- |
| Name | prevalence in biomarker panels |
| hsa-miR-30a-5p | 94.6% |
| hsa-miR-181a-2-3p | 83.7% |
| hsa-miR-486-5p | 64.6% |
| hsa-miR-199b-5p | 55.6% |
| hsa-miR-451a | 39.3% |
| hsa-miR-144-3p | 37.7% |
| hsa-miR-20b-5p | 24.2% |
| hsa-miR-223-5p | 21.6% |
| hsa-miR-20a-5p | 16.3% |

(continued)

| Significant miRNAs | |
|---|---|
| Name | prevalence in biomarker panels |
| hsa-miR-106a-5p | 10.1% |
| hsa-miR-93-5p | 4.9% |
| hsa-miR-18b-5p | 4.4% |
| hsa-miR-103a-3p | 4.1% |
| hsa-miR-500a-5p | 4.1% |
| hsa-let-7i-5p | 3.5% |
| hsa-miR-196b-5p | 3.5% |
| hsa-miR-335-5p | 3.4% |
| hsa-miR-183-5p | 3.3% |
| hsa-miR-146a-5p | 2.6% |
| hsa-miR-25-3p | 2.4% |
| hsa-miR-17-5p | 2.4% |
| hsa-miR-185-5p | 2.3% |
| Insignificant miRNAs | |
| Name | prevalence in biomarker panels |
| hsa-miR-191-5p | 42.3% |
| hsa-miR-1275 | 32.8% |
| hsa-miR-124-5p | 31.7% |
| hsa-miR-532-3p | 28.0% |
| hsa-miR-23a-3p | 22.0% |
| hsa-miR-484 | 15.1% |
| hsa-miR-125a-5p | 11.3% |
| hsa-miR-10b-5p | 11.0% |
| hsa-miR-101-3p | 8.4% |
| hsa-miR-423-5p | 7.6% |
| hsa-miR-660-5p | 7.3% |
| hsa-miR-374b-5p | 7.1% |
| hsa-miR-193a-5p | 6.4% |
| hsa-miR-92a-3p | 5.2% |
| hsa-miR-15a-5p | 4.9% |
| hsa-miR-200c-3p | 4.1% |
| hsa-miR-497-5p | 3.8% |
| hsa-miR-425-3p | 2.9% |
| hsa-miR-32-5p | 2.7% |
| hsa-miR-139-5p | 2.7% |
| hsa-miR-503 | 2.6% |
| hsa-miR-221-3p | 2.3% |

(continued)

| Insignificant miRNAs | |
| --- | --- |
| Name | prevalence in biomarker panels |
| hsa-miR-345-5p | 1.9% |
| hsa-miR-551b-3p | 1.8% |

In some examples, the method as described herein may measure the altered levels of at least four, at least five, at least six, at least seven, at least eight, at least nine, at least 10, at least 11, at least two to at least 20, at least 10 to at least 30, at least 40 to at least 45 or all miRNA as listed in Table 18.

In some examples, the score in the method as disclosed herein may be calculated by the formulas provided herein. In some examples, the formula may be at least one of the formula, including, but is not limited to, Formula 1, and/or Formula 2. The outcome of the methods as disclosed herein may be determined by the formula, such as, but not limited to, Formula 3, Formula 4, and the like.

In yet another aspect, there is provided a method of determining the likelihood of a subject having a heart failure with reduced left ventricular ejection fraction (HFREF) or a heart failure with preserved left ventricular ejection fraction (HFPEF), comprising the steps of: (a) detecting the presence of miRNA in a sample obtained from the subject; and/or measuring the levels of at least two miRNAs listed in Table 19 or Table 24 in the sample; and (b) using a score based on the levels of the miRNAs measured in step (a) to predict the likelihood of the subject to be suffering from, a heart failure with reduced left ventricular ejection fraction (HFREF) or a heart failure with preserved left ventricular ejection fraction (HFPEF).

**Table 19. miRNAs for use in conjunction of NT-proBNP when categorizing heart failure subtype**

| Significant miRNAs (additional to ln_NT-proBNP) | |
| --- | --- |
| Name | prevalence in biomarker panels |
| hsa-miR-199b-5p | 91.5% |
| hsa-miR-30a-5p | 66.7% |
| hsa-miR-486-5p | 49.3% |
| hsa-miR-181a-2-3p | 35.5% |
| hsa-miR-20b-5p | 31.4% |
| hsa-miR-122-5p | 10.8% |
| hsa-miR-223-5p | 10.0% |
| hsa-miR-144-3p | 9.8% |
| hsa-miR-106a-5p | 8.9% |
| hsa-miR-20a-5p | 6.5% |
| hsa-miR-451a | 5.3% |
| hsa-miR-25-3p | 3.9% |
| hsa-miR-103a-3p | 3.6% |
| hsa-miR-335-5p | 2.3% |
| Insignificant miRNAs (additional to ln_NT-proBNP) | |
| Name | prevalence in biomarker panels |
| hsa-miR-191-5p | 74.9% |
| hsa-miR-186-5p | 29.1% |
| hsa-miR-1275 | 18.1% |
| hsa-miR-484 | 16.6% |
| hsa-miR-532-3p | 13.9% |

(continued)

| Insignificant miRNAs (additional to ln_NT-proBNP) | |
|---|---|
| Name | prevalence in biomarker panels |
| hsa-miR-132-3p | 11.0% |
| hsa-miR-124-5p | 10.6% |
| hsa-miR-15a-5p | 8.8% |
| hsa-miR-425-3p | 8.7% |
| hsa-miR-374b-5p | 7.4% |
| hsa-miR-23a-3p | 6.4% |
| hsa-miR-92a-3p | 5.8% |
| hsa-miR-150-5p | 4.5% |
| hsa-miR-26a-5p | 2.7% |
| hsa-miR-598 | 2.4% |
| hsa-miR-660-5p | 2.3% |
| hsa-miR-454-3p | 2.0% |

**Table 24. miRNAs for use in conjunction of NT-proBNP when categorizing heart failure subtype**

| Significant miRNAs (additional to ln_NT-proBNP) (n=32) | | | | |
|---|---|---|---|---|
| Name | prevalence in biomarker panels | Significant for all HF | Significant for HFREF | Significant for HFPEF |
| hsa-miR-454-3p | 42.0% | Yes | No | Yes |
| hsa-miR-451a | 38.6% | Yes | No | Yes |
| hsa-miR-503 | 36.7% | Yes | No | Yes |
| hsa-miR-106a-5p | 19.2% | Yes | No | Yes |
| hsa-miR-375 | 16.2% | Yes | No | Yes |
| hsa-miR-148a-3p | 13.8% | Yes | No | Yes |
| hsa-miR-24-3p | 12.9% | Yes | No | Yes |
| hsa-miR-17-5p | 11.6% | Yes | No | Yes |
| hsa-miR-25-3p | 11.0% | Yes | No | Yes |
| hsa-miR-196b-5p | 9.8% | Yes | No | Yes |
| hsa-miR-34b-3p | 7.3% | Yes | No | Yes |
| hsa-miR-363-3p | 6.8% | Yes | No | Yes |
| hsa-miR-374b-5p | 6.7% | Yes | No | No |
| hsa-miR-193a-5p | 6.6% | Yes | No | No |

(continued)

| Significant miRNAs (additional to ln_NT-proBNP) (n=32) | | | | |
|---|---|---|---|---|
| **Name** | **prevalence in biomarker panels** | **Significant for all HF** | **Significant for HFREF** | **Significant for HFPEF** |
| hsa-miR-197-3p | 5.1% | Yes | No | Yes |
| hsa-miR-101-3p | 4.8% | Yes | No | Yes |
| hsa-miR-532-5p | 4.7% | Yes | No | Yes |
| hsa-miR-30c-5p | 4.3% | Yes | No | Yes |
| hsa-miR-16-5p | 4.3% | Yes | No | Yes |
| hsa-miR-144-3p | 4.2% | Yes | No | Yes |
| hsa-miR-183-5p | 4.2% | Yes | No | Yes |
| hsa-miR-20b-5p | 4.1% | Yes | No | Yes |
| hsa-miR-501-5p | 4.0% | Yes | No | Yes |
| hsa-miR-130b-3p | 3.9% | Yes | No | Yes |
| hsa-miR-20a-5p | 3.6% | Yes | No | Yes |
| hsa-miR-29b-3p | 3.2% | Yes | No | Yes |
| hsa-let-7b-5p | 3.1% | Yes | No | Yes |
| hsa-miR-500a-5p | 2.6% | Yes | No | Yes |
| hsa-miR-19b-3p | 2.3% | Yes | No | Yes |
| hsa-miR-4732-3p | 2.2% | Yes | No | Yes |
| hsa-let-7d-3p | 2.2% | Yes | No | Yes |
| hsa-miR-15a-5p | 2.0% | Yes | No | Yes |
| **Insignificant miRNAs (additional to ln_NT-proBNP) (n=10)** | | | | |
| **Name** | **prevalence in biomarker panels** | **Significant for all HF** | **Significant for HFREF** | **Significant for HFPEF** |
| hsa-miR-576-5p | 25.4% | No | No | No |
| hsa-miR-124-5p | 15.9% | No | No | No |
| hsa-miR-192-5p | 9.6% | No | No | No |

(continued)

| Insignificant miRNAs (additional to ln_NT-proBNP) (n=10) | | | | |
|---|---|---|---|---|
| Name | prevalence in biomarker panels | Significant for all HF | Significant for HFREF | Significant for HFPEF |
| hsa-miR-55 1b-3p | 8.1% | No | No | No |
| hsa-miR-10b-5p | 6.5% | No | No | No |
| hsa-miR-181a-2-3p | 4.2% | No | No | No |
| hsa-miR-181b-Sp | 2.8% | No | No | No |
| hsa-miR-26a-5p | 2.8% | No | No | No |
| hsa-miR-205-5p | 2.6% | No | No | No |
| hsa-miR-424-5p | 2.3% | No | No | No |

As illustrated in the Experimental Section and Figure, for example Figures 22 and 24, when a method present disclosure is used with an additional step of determining NT-proBNP, the method provides a surprisingly accurate prediction. Thus, in some examples, the method may further comprise the step of determining the level of Brain Natriuretic Peptide (BNP) and/or N-terminal prohormone of brain natriuretic peptide (NT-proBNP).

In some examples, the method as described herein may measure the altered levels of at least three, or at least four, at least five, at least six, at least seven, at least eight, at least nine, at least 10, at least 11, at least two to at least 20, at least 10 to at least 30, or all miRNA as listed in Table 19 or at least four, at least five, at least six, at least seven, at least eight, at least nine, at least 10, at least 11, at least two to at least 20, at least 10 to at least 41, or all miRNA as listed in Table 24.

In some examples, the levels of at least one of the miRNAs measured in step (b), when compared to a control, is not altered in the subject. In such examples, the miRNA which levels when compared to a control is not altered in the subject is the miRNAs listed as "insignificant" in the respective tables.

In some examples, the score in the method as disclosed herein may be calculated by Formula 2.

As would be understood by the person skilled in the art, the classification algorithm, as used herein in any of the methods described in the disclosure, may be pre-trained using the expression level of the control. In examples where the classification algorithm is to be pre-trained using pre-existing clinical data, the control may be at least one selected from the group consisting of a heart failure free control (normal) and a heart failure patient. The control may include a cohort of subject(s) having heart failure and/or not having heart failure (i.e. heart failure free). Thus, in some examples of the method as disclosed herein, the control may include, but not limited to, a heart failure free control, and a heart failure patient, a HFPEF subtype heart failure patient, a HFREF subtype heart failure patient, and the like.

The present disclosure discusses the differential comparison of expression levels of miRNA in the establishment of a panel of miRNAs, based on which a determination of whether a subject is at risk of developing heart failure, or a determination whether a subject suffers from heart failure can be made. As disclosed therein, the methods as disclosed herein require the differential comparison of miRNA expression levels, usually from different groups. In one example, the comparison is made between two groups. These comparison groups can be defined as being, but are not limited to, heart failure, heart failure-free (normal). Within the heart failure groups, further subgroups, for example but not limited to, HFREF and HFPEF, can be found. Differential comparisons can also be made between these groups described herein. Thus, in some examples, the expression level of the miRNAs can be expressed as, but not limited to, concentration, log(concentration), threshold cycle/quantification cycle (Ct/Cq) number, two to the power of threshold cycle/quantification cycle (Ct/Cq) number and the like.

In any of the methods as described herein, the methods may further include, but is not limited to, the steps of obtaining a sample from the subject at different time points, monitoring the course of the heart failure, staging the heart failure, measuring the miRNA level and/or NT-proBNP level in the (sample obtained from) subject, and the like.

In some examples, based on the current cohort as described in the Experimental Section below, biomarker panels including multiple miRNAs or biomarker panels including multiple miRNAs and BNP/NT-proBNP may be developed. The prediction score calculation may be optimized by methods known in the art, for example with a linear SVM model. In some examples, the biomarker panels consisting various number of miRNAs targets may be optimized by SFFS and SVM, where the AUC was optimized for the prediction of heart failure (Table 26) or classifications of heart failure subtypes (Table 27). Exemplary formulas, cutoffs and the performance of the panel are provided in Tables.

**Table 26.** Exemplary biomarker panels for HF detection.

| Biomarker | Combination of biomarker panel | cutoff value | AUC (95% CI) | Sensitivity (95% CI) | Specificity (95% CI) | Accuracy (95% CI) |
|---|---|---|---|---|---|---|
| 3 miRNAs Panel | -0.58*miR-454-3p - 0.57*miR-551b-3p +1.31*miR-24-3p-11.47 | 0 | 0.93 (0.91 - 0.95) | 85.2% (81.9% - 88.0%) | 87.0% (83.8% - 89.7%) | 85.9% (82.6%-88.7%) |
| 4 miRNAs Panel | -0.54*miR-454-3p - 0.62*miR-551b-3p +1.39*miR-24-3p -0.54*miR-10b-5p -7.13 | 0 | 0.94 (0.93 - 0.96) | 87.3% (84.1% - 89.9%) | 88.0% (84.9% - 90.5%) | 87.5% (84.4% - 90.2%) |
| 5 miRNAs Panel | -0.95*miR-451a +0.38*miR-503 +1.12*miR-576-5p - 1.17*miR-30b-5p +0.73*let-7b-3p +21.47 | 0 | 0.95 (0.93 - 0.97) | 87.3% (84.1%-89.9%) | 90.4% (87.5% - 92.7%) | 88.5% (85.4%-91.0%) |
| 6 miRNAs Panel | -0.84*miR-451a - 0.46*miR-551b-3p +0.34*miR-503 +1.11 *miR-576-5p +1.02*miR-24-3p - 1.02*miR-374b-5p +6.61 | 0 | 0.96 (0.94 - 0.98) | 87.6% (84.4% - 90.2%) | 92.3% (89.7% - 94.4%) | 89.4% (86.4%-91.8%) |
| 7 miRNAs Panel | -0.73*miR-451a - 0.54*miR-551b-3p +0.30*miR-503 +1.06*miR-576-5p - 1.16*miR-374b-5p +0.85*miR-24-3p +0.37*miR-199a-3p +4.60 | 0 | 0.97 (0.95 - 0.98) | 87.3% (84.1%-89.9%) | 93.8% (91.3% - 95.6%) | 89.7% (86.8%-92.1%) |
| 8 miRNAs Panel | -0.84*miR-451a - 0.45*miR-551b-3p +0.35*miR-503 +1.13*miR-576-5p +0.30*miR-375 +0.94*miR-24-3p - 0.28*miR-205-5p - 0.98*miR-374b-5p +6.52 | 0 | 0.97 (0.96 - 0.98) | 91.4% (88.7% - 93.6%) | 94.2% (91.8% - 96.0%) | 92.5% (89.9%-94.5%) |
| 2 miRNAs panel + NTproBNP | -0.88*miR-103a-3p +0.88*miR-24-3p +0.43*log2(BNP) -3.48 | 0 | 0.98 (0.98 - 0.99) | 92.9% (90.3% - 94.9%) | 95.2% (93.0% - 96.8%) | 93.8% (91.3%-95.6%) |
| 3 miRNAs panel + NTproBNP | -1.09*miR-103a-3p +0.73*miR-24-3p +0.44*miR-148a-3p +0.45*log2(BNP) -2.87 | 0 | 0.99 (0.98 - 0.99) | 94.4% (92.0% - 96.1%) | 95.2% (93.0% - 96.8%) | 94.7% (92.4% - 96.4%) |
| 4 miRNAs panel + NTproBNP | +0.93*miR-24-3p - 0.98*miR-103a-3p +0.50*miR-148a-3p - 0.42*miR-181b-5p +0.42*log2(BNP) -5.06 | 0 | 0.99 (0.98 - 1.00) | 93.8% (91.3% - 95.6%) | 96.2% (94.1% - 97.6%) | 94.7% (92.4% - 96.4%) |
| 5 miRNAs panel + NTproBNP | +0.27*miR-503 +0.84*miR-576-5p - 0.92*miR-451a - 0.99*miR-30b-5p +0.69*miR-148a-3p +0.46*log2 (BNP) +14.70 | 0 | 0.99 (0.99 - 1.00) | 95.0% (92.7% - 96.6%) | 97.6% (95.8% - 98.7%) | 96.0% (93.9% - 97.4%) |

(continued)

| Biomarker | Combination of biomarker panel | cutoff value | AUC (95% CI) | Sensitivity (95% CI) | Specificity (95% CI) | Accuracy (95% CI) |
|---|---|---|---|---|---|---|
| 6 miRNAs panel + NTproBNP | -0.87*miR-451a +1.30*miR-576-5p - 1.13*miR-30b-5p +0.81*miR-148a-3p - 0.57*miR-15a-5p +0.47*miR-99b-5p +0.48*log2(BNP) +16.75 | 0 | 0.99 (0.99 - 1.00) | 96.2% (94.1% - 97.6%) | 96.6% (94.7% - 98.0%) | 96.3% (94.3% - 97.7%) |
| 7 miRNAs panel + NTproBNP | -0.99*miR-451a +0.28*miR-503 +1.16*miR-576-5p +0.83*miR-148a-3p +0.35*miR-181a-2-3p - 1.20*miR-30b-5p - 0.60*miR-15a-5p +0.53*log2(BNP) +22.38 | 0 | 1.00 (0.99 - 1.00) | 95.9% (93.7% - 97.3%) | 96.6% (94.7% - 98.0%) | 96.2% (94.1% - 97.6%) |

As used herein in Table 26, the symbol "*" refers to "×" or multiplication symbol; "-" refers to negative value; "+" refers to addition; and "log2(BNP)" refers to the log 2 value of BNP expression. The second column of Table 26 also illustrates exemplary formulas for calculating the score as used in the method used herein. In the formula, the measuring unit for microRNA is copy/ml plasma and for NT-proBNP is pg/ml plasma. As would be apparent to the person skilled in the art, the coefficients and cutoffs in the formulas would have to be adjusted in accordance with different detection system used for the measurement and/or different units used to represent the microRNA expression level and BNP level/type. The adjustment of the formula would not be beyond the skill of the average person skilled in the art.

Thus, in another aspect, there is provided a method of determining the risk of developing heart failure in a subject or determining whether a subject suffers from heart failure, comprising the steps of (a) detecting the presence of miRNAs of a selected panel as listed in Table 26 in a sample obtained from the subject; or measuring the levels of miRNAs as listed in the selected panel of Table 26 in the sample; and (b) assigning a score based on the levels of the miRNAs measured in step (a) to predict the likelihood of the subject to develop or to have heart failure. In one example, the score is calculated based on the formula as listed in Table 26. In one example, when a two miRNAs biomarker panel is required, the method may detect and measure the level of miRNAs listed in Table 26 as "2 miRNAs Panel". In some examples, when a three miRNAs biomarker panel is required, the method may detect and measure the level of miRNAs listed in Table 26 as "3 miRNAs Panel". In some examples, when a four miRNAs biomarker panel is required, the method may detect and measure the level of miRNAs listed in Table 26 as "4 miRNAs Panel". In some examples, when a five miRNAs biomarker panel is required, the method may detect and measure the level of miRNAs listed in Table 26 as "5 miRNAs Panel". In some examples, when a six miRNAs biomarker panel is required, the method may detect and measure the level of miRNAs listed in Table 26 as "6 miRNAs Panel". In some examples, when a seven miRNAs biomarker panel is required, the method may detect and measure the level of miRNAs listed in Table 26 as "7 miRNAs Panel". In some examples, when a eight miRNAs biomarker panel is required, the method may detect and measure the level of miRNAs listed in Table 26 as "8 miRNAs Panel". In some examples, the methods may be performed with an additional step of detecting and measuring the level of NTproBNP in the sample thereof.

In some examples, for the prediction of heart failure, the prediction score (which would be unique for each subject) is the number that indicates the likelihood of a subject having heart failure. In some examples, the outcome of the methods as described herein (i.e. prediction of likelihood or diagnosis) may be found in Formula 3. If the value is higher than a pre-set cutoff value, the subject will be diagnosed or predicted to have heart failure. If the value is lower than a pre-set cutoff value, the subject will be diagnosed or predicted to be without heart failure. Formula 3 is as follows:

**Formula 3 -**

$$outcome = \begin{cases} have\ heart\ failure, & if\ prediction\ score > cutoff \\ no\ heart\ failure, & if\ prediciton\ score < cutoff \end{cases}$$

Table 27. Exemplary biomarker panels for heart failure subtype classification.

| Biomarker | Combination of biomarker panel | cutoff value | AUC (95% CI) | Sensitivity (95 % CI) | Specificity (95 % CI) | Accuracy (95 % CI) |
|---|---|---|---|---|---|---|
| 3 miRNAs Panel | -0.31*miR-486-5p - 0.35*miR-30a-5p +0.37*miR-181a-2-3p +8.14 | 0 | 0.77 (0.72 - 0.82) | 69.6% (64.4% - 74.4%) | 70.6% (65.3% - 75.3%) | 70.1% (64.9% - 74.9%) |
| 4 miRNAs Panel | -0.29*miR-30a-5p +0.43*miR-181a-2-3p - 0.24*miR-1275 -0.27*miR-20b-5p +8.80 | 0 | 0.79 (0.74 - 0.84) | 78.5% (73.6% - 82.7%) | 71.7% (66.5% - 76.4%) | 74.9% (69.8% - 79.3%) |
| 5 miRNAs Panel | -0.51*miR-20b-5p - 0.23*miR-1275 +0.24*miR-451a +0.43*miR-181a-2-3p - 0.27*miR-30a-5p +6.51 | 0 | 0.80 (0.75 - 0.85) | 78.5% (73.6% - 82.7%) | 72.2%(67.1 % - 76.9%) | 75.1% (70.1 % - 79.6%) |
| 6 miRNAs Panel | -0.50*miR-486-5p - 0.35*miR-30a-5p +0.34*miR-199b-5p +0.42*miR-181a-2-3p - 0.46*miR-191-5p +0.39*miR-484 +9.98 | 0 | 0.82 (0.77 - 0.86) | 74.1% (69.0% - 78.6%) | 73.3% (68.2% - 77.9%) | 73.7% (68.6% - 78.2%) |
| 2 miRNAs panel + NTproBNP | -0.39 * miR-20b-Sp +0.34*miR-199b-5p - 0.19*log2(BNP) +5.47 | 0 | 0.83 (0.78 - 0.87) | 74.1% (69.0% - 78.6%) | 76.1% (71.1% - 80.5%) | 75.1 % (70.1% - 79.6%) |
| 3 miRNAs panel + NTproBNP | -0.34*miR-20b-5p +0.41*miR-199b-5p - 0.27*miR-30a-5p - 0.19*log2 (BNP) +8.02 | 0 | 0.84 (0.80 - 0.89) | 71.5% (66.3% - 76.2%) | 78.9% (74.1% - 83.1%) | 75.4% (70.4% - 79.9%) |
| 4 miRNAs panel + NTproBNP | -0.47 * miR-20b-Sp +0.47*miR-199b-5p - 0.52*miR-191-5p +0.50*miR-186-5p - 0.24*log2(BNP) +7.35 | 0 | 0.87 (0.83 - 0.91) | 77.2% (72.3% - 81.5%) | 78.9% (74.1% - 83.1%) | 78.1% (73.2% - 82.3%) |
| 5 miRNAs panel + NTproBNP | -0.44*miR-20b-5p +0.49*miR-199b-5p - 0.50*miR-191-5p +0.56*miR-186-5p - 0.29*miR-30a-5p - 0.24*log2(BNP) +9.79 | 0 | 0.88 (0.85 - 0.92) | 79.7% (75.0% - 83.8%) | 77.2% (72.3% - 81.5%) | 78.4% (73.6% - 82.6%) |
| 6 miRNAs panel + NTproBNP | -0.43*miR-20b-5p - 0.18*miR-1275 +0.47*miR-199b-5p - 0.49*miR-191-5p +0.54*miR-186-5p - 0.27*miR-30a-5p - 0.24*log2(BNP) +11.85 | 0 | 0.89 (0.85 - 0.92) | 80.4% (75.7% - 84.4%) | 76.7% (71.7% - 81.0%) | 78.4% (73.6% - 82.6%) |

As used herein in Table 27, the symbol "*" refers to "×" or multiplication symbol; "-" refers to negative value; "+" refers to addition; and "log2(BNP)" refers to the log 2 value of BNP expression. The second column of Table 27 also illustrates exemplary formulas for calculating the score as used in the method used herein. In the formula, the measuring unit for microRNA is copy/ml plasma and for NT-proBNP is pg/ml plasma. As would be apparent to the person skilled in the art, the coefficients and cutoffs in the formulas would have to be adjusted in accordance with different detection system used for the measurement and/or different units used to represent the microRNA expression level and BNP level/type. The adjustment of the formula would not be beyond the skill of the average person skilled in the art.

In yet another aspect, there is provided a method of determining the likelihood of a subject to be suffering from a heart failure with reduced left ventricular ejection fraction (HFREF) or a heart failure with preserved left ventricular ejection fraction (HFPEF), comprising the steps of (a) detecting the presence of miRNAs of a selected panel as listed in Table 27 in a sample obtained from the subject; or measuring the levels of miRNAs as listed in the selected panel of Table 27 in the sample; and (b) assigning a score based on the levels of the miRNAs measured in step (a) to predict the likelihood of the subject to be suffering from, a heart failure with reduced left ventricular ejection fraction (HFREF) or a heart failure with preserved left ventricular ejection fraction (HFPEF). In one example, the score is calculated based on the formula as listed in Table 27. In one example, when a two miRNAs biomarker panel is required, the method may detect and measure the level of miRNAs listed in Table 27 as "2 miRNAs Panel". In some examples, when a three miRNAs biomarker panel is required, the method may detect and measure the level of miRNAs listed in Table 27 as "3 miRNAs Panel". In some examples, when a four miRNAs biomarker panel is required, the method may detect and measure the level of miRNAs listed in Table 27 as "4 miRNAs Panel". In some examples, when a five miRNAs biomarker panel is required, the method may detect and measure the level of miRNAs listed in Table 27 as "5 miRNAs Panel". In some examples, when a six miRNAs biomarker panel is required, the method may detect and measure the level of miRNAs listed in Table 27 as "6 miRNAs Panel". In some examples, the methods may be performed with an additional step of detecting and measuring the level of NTproBNP in the sample thereof.

In some examples, for the classification of HFREF and HFPEF subtypes, the prediction score (which is unique for each subject) may be the number that indicates the likelihood of a heart failure subject having HFPEF subtype of heart failure. In some examples, the outcome of the diagnosis may be found in Formula 4. If the value is higher than a pre-set cutoff value, the heart failure subject will be diagnosed as (or predicted to) having HFPEF subtype of heart failure. If the value is lower than a pre-set cutoff value, the heart failure subject will be diagnosed as (or predicted to) have HFREF subtype of heart failure by this test.

## Formula 4 -

$$
outcome = \begin{cases} \text{have HFPEF subtype of heart failure,} & \text{if prediction score} > \text{cutoff} \\ \text{have HFREF subtype of heart failure,} & \text{if prediciton score} < \text{cutoff} \end{cases}
$$

In one example, the methods as described herein may be implemented into a device capable to (or adapted to) perform all of (or part of) the steps described in the present disclosure. Thus, in one example, the present disclosure provides for a device adapted to (or capable of) adapting to perform the methods as described herein.

In another aspect, there is provided a kit for use (or adapted to be used, or when used) in any of the methods as described herein. In one example, the kit may comprise reagents for determining the expression of the at least one gene listed in Table 9, or at least one gene listed in Table 14, or at least one gene listed in Table 15, or at least two genes listed in Table 16, or at least two genes listed in Table 17, or at least two genes listed in Table 18, or at least two genes listed in Table 19, or at least one genes listed in Table 20; or at least one gene listed in Table 21; or at least one gene listed in Table 22; or at least one gene listed in Table 23; or at least one gene listed in Table 24; or at least one gene listed in Table 25.

In some examples, the reagents may comprise a probe, primer or primer set adapted to or capable of ascertaining the expression of at least one gene listed in Table 9, or ate last one gene listed in Table 14, or at least one gene listed in Table 15, or at least two genes listed in Table 16, or at least two genes listed in Table 17, or at least two genes listed in Table 18, or at least two genes listed in Table 19, or at least one genes listed in Table 20; or at least one gene listed in Table 21; or at least one gene listed in Table 22; or at least one gene listed in Table 23; or at least one gene listed in Table 24; or at least one gene listed in Table 25.

In some examples, the kit may further comprise a reagent for determining the level of Brain Natriuretic Peptide (BNP) and/or N-terminal prohormone of brain natriuretic peptide (NT-proBNP).

In some examples, the methods as described herein may further comprise a step of treating the subject predicted to (or diagnosed as) having heart failure or heart failure subtype to at least one therapeutic agent for treating heart failure (or heart failure subtype). In some examples, the method may further comprise therapies known for alleviating and/or reducing the symptoms of heart failure. In some examples, the method as described herein may further comprise the administration of agents including, but not limited to, classes of drugs that are proven to improve prognosis in heart failure (for example, ACEI's/ARB's, angiotensin receptor blockers, Loop/thiazide diuretics, beta blockers, mineralocorticoid antagonists, aspirin or Plavix, statins, digoxin, warfarin, nitrates, calcium channel blockers, spironolactone, fibrate, antidiabetic, hydralazine, iron supplements, anticoagulant, antiplatelet and the likes).

## EXPERIMENTAL SECTION

## METHOD

**Pre-analytics (sample collection and miRNA extraction):** Plasma samples were stored frozen at -80°C prior to use. Total RNA from 200 μl of each plasma sample was isolated using the well-established TRI Reagent (Sigma-Aldrich®) following the manufacturer's protocol. Plasma contains minute amounts of RNA. To reduce the loss of RNA and monitor extraction efficiency, rationally designed isolation enhancers (MS2) and spike-in control RNAs (MiRXES™) were added to the specimen prior to isolation.

**RT-qPCR:** The isolated total RNAs and synthetic RNA standards were converted to cDNA in optimized multiplex reverse transcription reactions with a second set of spike-in control RNAs to detect the presence of inhibitors and monitor the RT-qPCR efficiency. The Improm II (Promega®) reverse transcriptase was used to perform the reverse transcription following manufacturer's instruction. The synthesized cDNA is then subjected to a multiplex augmentation step and quantified using a Sybr Green based single-plex qPCR assays (MIQE compliant) (MiRXES™). Applied Biosystems® ViiA 7 384 Real-Time PCR System or Bio-rad® CFX384 Touch Real-Time PCR Detection System was used for qPCR reactions. The overview and details of miRNA RT-qPCR measurement workflow was summarized in Figure 2.

**Data processing:** The raw Cycles to Threshold (Ct) values were processed and the absolute copy numbers of the target miRNAs in each sample were determined by intrapolation of the synthetic miRNA standard curves. The technical variations introduced during RNA isolation and the processes of RT-qPCR were normalized by the spike-in control RNAs. For the analysis of single miRNA, the biological variations were further normalized by a set of validated endogenous reference miRNAs stably expressed across all control and disease samples.

## RESULTS

### I. Characteristics of study participants

A well-designed clinical study (case-control study) was carried out to ensure the accurate identification of biomarkers for chronic heart failure (HF). A total number of 338 chronic heart failure patients (180 HFREF and 158 HFPEF) from the Singapore population were used in this study and comparisons were made with 208 non-heart failure subjects matched for race, gender and age, serving as the control group. Patients with heart failure were recruited from the Singapore Heart Failure Outcomes and Phenotypes (SHOP) study [55]. Patients were included if they presented with a primary diagnosis of acute decompensated heart failure (ADHF) or attended clinics for management of heart failure within 6 months of a known episode of ADHF . Controls without overt coronary artery disease or history of heart failure were recruited through the ongoing epidemiological Singapore Longitudinal Ageing Study (SLAS) [56]. All patients and controls underwent detailed clinical examination including comprehensive Doppler echocardiography for confirmation of the presence (or absence) of clinical heart failure. LVEF was assessed using the biplane method of disks as recommended by the American Society of Echocardiography (ASE) guidelines. Patients with validated heart failure and LVEF ≥50% were categorized as HFPEF, whereas those with LVEF ≤40% were classified as HFREF. Patients with EF between 40% and 50% were excluded. Assessments including blood plasma samples were deliberately undertaken when patients had received treatment (typically for 3 -5 days), were symptomatically improved with resolution of bedside physical signs of heart failure and were considered fit for discharge. This ensured assessment of marker performance in the treated or "chronic" phase of heart failure. Clinical characteristics and demographic information are given in Table 2. All plasma samples were stored at -80 °C prior to use.

**Table 2. Clinical information of the subjects included in the study**

| Sample Type | Gender | Race | Atrial Fibrillation or Flutter | Hypertension | Diabetes | Age | Body Mass Index |
|---|---|---|---|---|---|---|---|
| C | Female | Indian | No | Yes | No | 70 | 26.67 |
| C | Male | Chinese | No | Yes | No | 66 | 30.86 |
| C | Male | Indian | No | No | No | 61 | 25.36 |
| C | Female | Indian | No | Yes | Yes | 64 | 26.58 |
| C | Male | Chinese | No | Yes | No | 69 | 24.45 |
| C | Male | Chinese | No | No | No | 68 | 16.73 |
| C | Female | Chinese | No | No | No | 61 | 28.69 |
| C | Male | Chinese | No | No | No | 70 | 25.43 |
| C | Female | Chinese | No | No | No | 56 | 25.63 |
| C | Male | Chinese | No | No | No | 60 | 22.15 |
| C | Male | Chinese | No | Yes | No | 64 | 24.13 |
| C | Male | Chinese | No | Yes | No | 78 | 25.76 |
| C | Male | Chinese | No | No | No | 51 | 22.16 |

(continued)

| Sample Type | Gender | Race | Atrial Fibrillation or Flutter | Hypertension | Diabetes | Age | Body Mass Index |
|---|---|---|---|---|---|---|---|
| C | Male | Chinese | No | No | No | 62 | 72.8 |
| C | Female | Chinese | No | Yes | No | 66 | 22.39 |
| C | Female | Chinese | No | No | No | 63 | 22.97 |
| C | Female | Chinese | No | No | No | 71 | 24.85 |
| C | Female | Chinese | No | Yes | No | 64 | 24.78 |
| C | Female | Chinese | No | Yes | Yes | 52 | 32.7 |
| C | Female | Chinese | No | Yes | No | 71 | 17.5 |
| C | Female | Chinese | No | No | No | 70 | 21.62 |
| C | Male | Chinese | No | Yes | No | 62 | 30.4 |
| C | Female | Indian | No | No | Yes | 65 | 27.82 |
| C | Female | Malay | No | No | No | 56 | 22.54 |
| C | Male | Malay | No | No | No | 74 | 26.26 |
| C | Female | Malay | No | Yes | No | 56 | 33.25 |
| C | Female | Malay | No | Yes | No | 79 | 25.22 |
| C | Female | Chinese | No | No | No | 71 | 31.6 |
| C | Female | Malay | No | Yes | No | 71 | 32.37 |
| C | Male | Indian | No | No | No | 68 | 25.51 |
| C | Male | Indian | No | No | No | 54 | 29.12 |
| C | Female | Malay | No | No | No | 64 | 28.22 |
| C | Female | Malay | No | Yes | Yes | 66 | 25.26 |
| C | Male | Malay | No | Yes | No | 58 | 32.44 |
| C | Male | Chinese | No | No | No | 74 | 27.02 |
| C | Female | Chinese | No | Yes | No | 71 | 28.68 |
| C | Male | Malay | No | Yes | No | 53 | 30.91 |
| C | Male | Malay | No | No | No | 60 | 22.38 |
| C | Female | Indian | No | No | No | 58 | 35.81 |
| C | Male | Malay | No | No | No | 69 | 20.96 |
| C | Male | Chinese | No | No | No | 75 | 19.69 |
| C | Female | Chinese | No | Yes | Yes | 75 | 31.03 |
| C | Male | Malay | No | Yes | No | 66 | 25.15 |
| C | Male | Indian | No | No | No | 40 | 23.44 |
| C | Male | Malay | No | Yes | No | 63 | 26.61 |
| C | Female | Chinese | No | Yes | No | 65 | 25.51 |
| C | Male | Chinese | No | Yes | No | 68 | 22.61 |
| C | Female | Chinese | No | No | No | 46 | 19.35 |
| C | Female | Chinese | No | Yes | No | 53 | 23.19 |
| C | Male | Chinese | No | Yes | Yes | 69 | 20.83 |

(continued)

| Sample Type | Gender | Race | Atrial Fibrillation or Flutter | Hypertension | Diabetes | Age | Body Mass Index |
|---|---|---|---|---|---|---|---|
| C | Male | Chinese | No | No | No | 50 | 26.53 |
| C | Female | Chinese | No | No | No | 64 | 22.38 |
| C | Female | Chinese | No | Yes | No | 47 | 23.65 |
| C | Male | Chinese | No | No | No | 54 | 26.33 |
| C | Male | Chinese | No | Yes | No | 65 | 25.38 |
| C | Male | Chinese | No | Yes | No | 67 | 23.95 |
| C | Female | Chinese | No | No | No | 48 | 20.49 |
| C | Female | Chinese | No | No | Yes | 62 | 19.08 |
| C | Male | Chinese | No | Yes | No | 65 | 21.6 |
| C | Female | Malay | No | Yes | No | 73 | 25.7 |
| C | Female | Chinese | No | No | No | 67 | 25.8 |
| C | Male | Malay | No | No | No | 57 | 30.42 |
| C | Male | Chinese | No | Yes | No | 79 | 24.87 |
| C | Male | Chinese | No | Yes | No | 64 | 23.73 |
| C | Female | Chinese | No | No | No | 62 | 21.7 |
| C | Female | Chinese | No | No | No | 51 | 26.31 |
| C | Male | Indian | No | Yes | Yes | 70 | 27.07 |
| C | Female | Malay | No | No | No | 71 | 23.28 |
| C | Male | Chinese | Yes | Yes | No | 81 | 19.47 |
| C | Female | Chinese | No | No | No | 58 | 23.26 |
| C | Female | Chinese | No | No | No | 48 | 21.16 |
| C | Male | Malay | No | No | Yes | 55 | 28.77 |
| C | Female | Chinese | No | No | No | 51 | 28.06 |
| C | Male | Chinese | No | Yes | No | 70 | 32.32 |
| C | Male | Malay | No | No | No | 46 | 21.22 |
| C | Female | Chinese | No | Yes | No | 61 | 24.99 |
| C | Male | Indian | No | Yes | No | 56 | 30.2 |
| C | Female | Indian | No | No | Yes | 62 | 25.85 |
| C | Male | Chinese | No | Yes | No | 59 | 26.43 |
| C | Male | Indian | No | No | Yes | 68 | 32.01 |
| C | Female | Chinese | No | No | No | 55 | 26.85 |
| C | Female | Chinese | No | No | Yes | 63 | 18.72 |
| C | Male | Chinese | No | No | No | 59 | 27.49 |
| C | Female | Chinese | No | No | No | 45 | 21.1 |
| C | Female | Chinese | No | Yes | No | 73 | 24.24 |
| C | Male | Indian | No | No | Yes | 51 | 28.86 |
| C | Female | Chinese | No | Yes | No | 72 | 23.77 |

(continued)

| Sample Type | Gender | Race | Atrial Fibrillation or Flutter | Hypertension | Diabetes | Age | Body Mass Index |
|---|---|---|---|---|---|---|---|
| C | Female | Chinese | No | No | No | 73 | 17.63 |
| C | Male | Chinese | No | No | No | 64 | 23.02 |
| C | Female | Chinese | No | No | No | 59 | 21.63 |
| C | Male | Chinese | No | Yes | No | 67 | 31.2 |
| C | Female | Chinese | No | No | No | 49 | 21.87 |
| C | Male | Malay | No | Yes | No | 48 | 28.36 |
| C | Male | Chinese | No | No | No | 39 | 26.23 |
| C | Female | Chinese | No | No | No | 61 | 27.82 |
| C | Male | Malay | No | No | No | 51 | 21.63 |
| C | Male | Chinese | No | Yes | No | 61 | 23.73 |
| C | Male | Malay | No | No | No | 50 | 29.07 |
| C | Male | Chinese | No | No | No | 59 | 26.45 |
| C | Male | Chinese | No | No | No | 36 | 23.53 |
| C | Female | Chinese | No | No | No | 57 | 24.8 |
| C | Male | Indian | No | Yes | No | 49 | 22.86 |
| C | Female | Malay | No | Yes | No | 61 | 25.48 |
| C | Male | Chinese | No | Yes | No | 54 | 23.47 |
| C | Male | Chinese | No | Yes | No | 57 | 25.96 |
| C | Female | Indian | No | No | No | 62 | 26.56 |
| C | Female | Chinese | No | No | No | 59 | 22.65 |
| C | Female | Chinese | Yes | No | No | 68 | 21.59 |
| C | Male | Chinese | No | Yes | Yes | 59 | 31.04 |
| C | Male | Chinese | No | Yes | No | 73 | 18.89 |
| C | Female | Chinese | No | No | No | 38 | 17.47 |
| C | Male | Chinese | No | Yes | No | 77 | 26.26 |
| C | Female | Malay | No | No | Yes | 59 | 30.18 |
| C | Male | Chinese | No | No | No | 80 | 24.38 |
| C | Female | Chinese | No | Yes | No | 74 | 23.46 |
| C | Female | Chinese | No | No | No | 70 | 23.82 |
| C | Male | Chinese | No | Yes | No | 38 | 31.88 |
| C | Male | Malay | No | No | No | 52 | 32.23 |
| C | Male | Malay | No | No | No | 53 | 24.81 |
| C | Male | Chinese | No | Yes | No | 48 | 33.51 |
| C | Female | Chinese | No | No | No | 55 | 25.13 |
| C | Female | Chinese | No | No | No | 47 | 22.89 |
| C | Female | Chinese | No | No | No | 75 | 26.08 |
| C | Female | Chinese | No | No | No | 52 | 25.94 |

(continued)

| Sample Type | Gender | Race | Atrial Fibrillation or Flutter | Hypertension | Diabetes | Age | Body Mass Index |
|---|---|---|---|---|---|---|---|
| C | Male | Chinese | No | No | No | 59 | 23.95 |
| C | Male | Chinese | No | No | No | 48 | 26.83 |
| C | Female | Chinese | No | No | No | 67 | 22.02 |
| C | Female | Chinese | No | No | No | 59 | 21.3 |
| C | Female | Chinese | No | No | No | 63 | 21.64 |
| C | Male | Malay | No | No | No | 39 | 19.96 |
| C | Female | Chinese | No | No | No | 54 | 21.36 |
| C | Female | Malay | No | No | No | 50 | 29.9 |
| C | Male | Malay | No | No | No | 48 | 30.93 |
| C | Female | Indian | No | No | No | 42 | 24.12 |
| C | Female | Chinese | No | No | No | 47 | 25 |
| C | Male | Chinese | No | No | No | 63 | 26.4 |
| C | Male | Chinese | No | No | No | 61 | 28.3 |
| C | Male | Chinese | No | Yes | Yes | 77 | 22.32 |
| C | Female | Indian | No | Yes | No | 63 | 35.96 |
| C | Female | Indian | No | Yes | Yes | 49 | 25.89 |
| C | Female | Malay | No | No | No | 63 | 22.15 |
| C | Male | Chinese | No | No | No | 49 | 21.42 |
| C | Male | Chinese | No | No | No | 62 | 21.38 |
| C | Female | Chinese | No | Yes | No | 49 | 26.05 |
| C | Female | Malay | No | No | No | 41 | 21.18 |
| C | Male | Indian | No | Yes | No | 53 | 28.8 |
| C | Male | Chinese | No | No | No | 52 | 18.28 |
| C | Female | Chinese | No | No | Yes | 69 | 20.04 |
| C | Female | Chinese | No | No | No | 43 | 26.29 |
| C | Male | Indian | No | No | No | 54 | 24.73 |
| C | Male | Malay | No | Yes | No | 77 | 23.26 |
| C | Male | Malay | No | No | No | 51 | 20.76 |
| C | Female | Chinese | No | No | No | 52 | 25.82 |
| C | Female | Chinese | No | No | No | 72 | 20.58 |
| C | Male | Chinese | No | No | No | 39 | 24.64 |
| C | Female | Indian | No | No | No | 61 | 27.6 |
| C | Female | Chinese | No | No | No | 59 | 27.38 |
| C | Male | Chinese | No | No | No | 71 | 23.41 |
| C | Female | Indian | No | No | No | 59 | 27.41 |
| C | Female | Chinese | No | No | No | 60 | 16.65 |
| C | Female | Chinese | No | Yes | No | 72 | 31.23 |

(continued)

| Sample Type | Gender | Race | Atrial Fibrillation or Flutter | Hypertension | Diabetes | Age | Body Mass Index |
|---|---|---|---|---|---|---|---|
| C | Male | Malay | No | Yes | No | 66 | 27.22 |
| C | Male | Chinese | No | No | No | 41 | 26.42 |
| C | Male | Malay | No | No | No | 52 | 22.77 |
| C | Female | Indian | No | No | No | 54 | 25.19 |
| C | Female | Chinese | No | No | No | 36 | 20.28 |
| C | Male | Chinese | No | No | No | 59 | 28.64 |
| C | Female | Chinese | No | Yes | Yes | 83 | 18.37 |
| C | Male | Malay | No | No | No | 51 | 18.38 |
| C | Male | Chinese | No | No | No | 37 | 28.57 |
| C | Female | Chinese | No | No | No | 71 | 19.05 |
| C | Male | Chinese | No | No | No | 63 | 21.93 |
| C | Female | Chinese | No | No | No | 63 | 19.84 |
| C | Male | Malay | No | No | No | 59 | 30.93 |
| C | Male | Chinese | No | No | No | 64 | 24.61 |
| C | Male | Chinese | No | Yes | No | 72 | 23.58 |
| C | Female | Indian | No | No | No | 48 | 27.67 |
| C | Male | Chinese | No | No | No | 73 | 18.17 |
| C | Female | Chinese | No | No | No | 64 | 17.89 |
| C | Male | Chinese | No | No | No | 72 | 26.52 |
| C | Female | Chinese | No | No | No | 57 | 18.94 |
| C | Female | Chinese | No | No | No | 62 | 25.63 |
| C | Female | Malay | No | Yes | Yes | 48 | 27.1 |
| C | Female | Malay | No | No | No | 39 | 35.91 |
| C | Female | Malay | No | Yes | No | 56 | 26.86 |
| C | Female | Malay | No | No | No | 60 | 32.97 |
| C | Male | Chinese | No | Yes | No | 76 | 27.94 |
| C | Male | Chinese | No | No | No | 43 | 26.68 |
| C | Female | Malay | No | No | No | 53 | 30.99 |
| C | Male | Chinese | No | No | No | 55 | 24.5 |
| C | Male | Malay | No | No | No | 40 | 22.91 |
| C | Female | Chinese | No | Yes | No | 74 | 25.78 |
| C | Male | Chinese | No | Yes | No | 77 | 18.68 |
| C | Male | Chinese | No | No | No | 63 | 30.15 |
| C | Female | Chinese | No | No | No | 55 | 22.18 |
| C | Male | Chinese | No | No | No | 75 | 20.37 |
| C | Male | Indian | No | Yes | No | 51 | 32.14 |
| C | Female | Chinese | No | No | No | 59 | 29.05 |

(continued)

| Sample Type | Gender | Race | Atrial Fibrillation or Flutter | Hypertension | Diabetes | Age | Body Mass Index |
|---|---|---|---|---|---|---|---|
| C | Female | Chinese | No | Yes | No | 59 | 19.96 |
| C | Male | Chinese | No | No | No | 55 | 21.74 |
| C | Male | Chinese | No | No | No | 54 | 23.02 |
| C | Female | Chinese | No | No | No | 73 | 24.09 |
| C | Male | Indian | No | No | No | 48 | 25.28 |
| C | Male | Chinese | No | No | No | 67 | 27.1 |
| C | Male | Malay | No | No | No | 44 | 28.9 |
| C | Male | Chinese | No | No | No | 59 | 22.05 |
| C | Female | Chinese | No | No | No | 49 | 35.67 |
| C | Male | Chinese | No | No | No | 56 | 23.51 |
| PEF | Female | Malay | No | Yes | No | 80 | 26.44 |
| PEF | Female | Chinese | Yes | Yes | No | 72 | 23.33 |
| PEF | Female | Chinese | Yes | Yes | No | 71 | 26.67 |
| PEF | Male | Malay | No | Yes | Yes | 62 | 30.22 |
| PEF | Male | Chinese | No | Yes | Yes | 67 | 24.5 |
| PEF | Male | Chinese | No | Yes | No | 69 | 27.35 |
| PEF | Male | Malay | No | Yes | No | 75 | 26.1 |
| PEF | Female | Malay | Yes | No | No | 76 | 27.61 |
| PEF | Female | Chinese | Yes | Yes | Yes | 72 | 23.52 |
| PEF | Male | Malay | No | No | Yes | 64 | 32.31 |
| PEF | Female | Malay | No | Yes | No | 56 | 34.37 |
| PEF | Female | Indian | No | Yes | Yes | 61 | 26 |
| PEF | Female | Chinese | No | Yes | Yes | 52 | 32.89 |
| PEF | Female | Malay | No | Yes | Yes | 56 | 40.22 |
| PEF | Male | Malay | No | Yes | Yes | 70 | 24 |
| PEF | Male | Indian | No | No | No | 40 | 24.77 |
| PEF | Female | Chinese | No | Yes | No | 71 | 24 |
| PEF | Male | Chinese | No | Yes | Yes | 72 | 25.77 |
| PEF | Female | Malay | No | Yes | Yes | 64 | 25.33 |
| PEF | Female | Chinese | Yes | No | No | 71 | 31.25 |
| PEF | Female | Chinese | Yes | Yes | Yes | 56 | 29.89 |
| PEF | Male | Chinese | No | Yes | Yes | 70 | 26.56 |
| PEF | Male | Indian | Yes | Yes | Yes | 55 | 28.41 |
| PEF | Male | Malay | No | Yes | Yes | 60 | 33.2 |
| PEF | Female | Indian | No | Yes | Yes | 56 | 39.17 |
| PEF | Male | Malay | Yes | No | Yes | 52 | 32.23 |
| PEF | Female | Malay | No | Yes | Yes | 67 | 29.76 |

(continued)

| Sample Type | Gender | Race | Atrial Fibrillation or Flutter | Hypertension | Diabetes | Age | Body Mass Index |
|---|---|---|---|---|---|---|---|
| PEF | Female | Indian | No | Yes | Yes | 65 | 18.29 |
| PEF | Male | Malay | No | Yes | Yes | 53 | 30.75 |
| PEF | Male | Malay | No | Yes | Yes | 60 | 21.16 |
| PEF | Female | Chinese | No | Yes | Yes | 78 | 23.23 |
| PEF | Female | Indian | No | Yes | Yes | 73 | 28.05 |
| PEF | Female | Chinese | No | Yes | Yes | 55 | 24.88 |
| PEF | Female | Malay | No | Yes | Yes | 52 | 27.24 |
| PEF | Female | Chinese | Yes | Yes | No | 74 | 20.81 |
| PEF | Female | Chinese | Yes | No | No | 48 | 33.22 |
| PEF | Male | Indian | No | Yes | Yes | 53 | 43.12 |
| PEF | Female | Malay | No | Yes | Yes | 60 | 27.79 |
| PEF | Female | Chinese | Yes | Yes | Yes | 67 | 22.37 |
| PEF | Male | Chinese | No | Yes | Yes | 60 | 37.04 |
| PEF | Female | Chinese | No | Yes | Yes | 66 | 47.3 |
| PEF | Female | Chinese | Yes | Yes | No | 78 | 29.9 |
| PEF | Female | Chinese | No | Yes | Yes | 76 | 23.59 |
| PEF | Female | Malay | Yes | Yes | Yes | 62 | N.A. |
| PEF | Female | Chinese | Yes | Yes | No | 91 | 21.52 |
| PEF | Female | Chinese | No | No | No | 83 | 23.5 |
| PEF | Female | Chinese | No | Yes | No | 91 | 20.98 |
| PEF | Male | Chinese | No | Yes | No | 59 | 24.57 |
| PEF | Male | Chinese | No | Yes | Yes | 63 | 27.37 |
| PEF | Female | Chinese | Yes | Yes | No | 77 | 25 |
| PEF | Female | Chinese | No | Yes | Yes | 68 | 29.41 |
| PEF | Male | Chinese | Yes | No | No | 60 | 23.14 |
| PEF | Male | Chinese | Yes | Yes | Yes | 51 | 27.4 |
| PEF | Female | Chinese | No | Yes | Yes | 68 | 25.87 |
| PEF | Female | Chinese | No | Yes | Yes | 73 | 27.89 |
| PEF | Female | Chinese | No | Yes | Yes | 89 | 20.45 |
| PEF | Female | Chinese | Yes | Yes | Yes | 87 | 28.31 |
| PEF | Female | Indian | No | Yes | No | 69 | 30 |
| PEF | Female | Chinese | Yes | Yes | No | 74 | 25.54 |
| PEF | Female | Chinese | No | Yes | No | 65 | 39.26 |
| PEF | Female | Malay | Yes | Yes | Yes | 86 | 32.09 |
| PEF | Female | Chinese | Yes | Yes | No | 86 | 26.37 |
| PEF | Female | Malay | Yes | Yes | Yes | 64 | 39.58 |
| PEF | Female | Chinese | No | Yes | Yes | 84 | 22.59 |

(continued)

| Sample Type | Gender | Race | Atrial Fibrillation or Flutter | Hypertension | Diabetes | Age | Body Mass Index |
|---|---|---|---|---|---|---|---|
| PEF | Female | Chinese | Yes | Yes | Yes | 83 | 24.14 |
| PEF | Female | Indian | Yes | Yes | No | 64 | 38.75 |
| PEF | Female | Chinese | No | Yes | Yes | 83 | 19.31 |
| PEF | Male | Chinese | No | Yes | Yes | 66 | 23.66 |
| PEF | Female | Chinese | Yes | Yes | No | 73 | 20.03 |
| PEF | Female | Chinese | No | Yes | No | 81 | 24.03 |
| PEF | Female | Chinese | Yes | Yes | Yes | 52 | 27.64 |
| PEF | Male | Malay | No | Yes | Yes | 82 | N.A. |
| PEF | Female | Chinese | No | Yes | No | 52 | 40.27 |
| PEF | Male | Indian | No | N.A. | Yes | 54 | 30.59 |
| PEF | Male | Malay | No | Yes | Yes | 50 | 31.89 |
| PEF | Male | Chinese | No | Yes | Yes | 62 | 25.15 |
| PEF | Female | Chinese | No | Yes | Yes | 74 | 28.48 |
| PEF | Male | Chinese | No | Yes | No | 78 | 26.67 |
| PEF | Female | Chinese | No | Yes | Yes | 82 | 24.97 |
| PEF | Female | Chinese | Yes | Yes | No | 63 | 42.8 |
| PEF | Male | Chinese | No | Yes | No | 71 | 23.59 |
| PEF | Female | Malay | No | Yes | No | 48 | 25.27 |
| PEF | Female | Chinese | No | Yes | Yes | 79 | 29.22 |
| PEF | Female | Chinese | Yes | No | No | 59 | 27.55 |
| PEF | Male | Chinese | No | Yes | Yes | 57 | 25.8 |
| PEF | Male | Malay | No | Yes | Yes | 83 | N.A. |
| PEF | Female | Malay | No | Yes | Yes | 68 | 38.93 |
| PEF | Male | Chinese | No | Yes | No | 57 | 23.48 |
| PEF | Male | Chinese | No | Yes | Yes | 62 | 20.69 |
| PEF | Male | Indian | Yes | Yes | Yes | 71 | 28.21 |
| PEF | Male | Chinese | Yes | No | No | 36 | 21.6 |
| PEF | Male | Chinese | No | Yes | No | 70 | 26.61 |
| PEF | Male | Chinese | Yes | Yes | Yes | 76 | 24.59 |
| PEF | Male | Malay | No | Yes | Yes | 52 | 28.94 |
| PEF | Male | Malay | No | Yes | Yes | 55 | 26.41 |
| PEF | Female | Malay | No | Yes | Yes | 77 | N.A. |
| PEF | Male | Chinese | No | Yes | No | 61 | 25.31 |
| PEF | Female | Chinese | No | No | No | 78 | 26.9 |
| PEF | Male | Malay | No | No | No | 74 | 27.48 |
| PEF | Female | Chinese | Yes | Yes | No | 77 | 27.62 |
| PEF | Female | Malay | No | Yes | No | 83 | 17.86 |

(continued)

| Sample Type | Gender | Race | Atrial Fibrillation or Flutter | Hypertension | Diabetes | Age | Body Mass Index |
|---|---|---|---|---|---|---|---|
| PEF | Male | Chinese | No | Yes | Yes | 63 | 28.12 |
| PEF | Male | Chinese | No | No | No | 81 | 26.12 |
| PEF | Female | Indian | No | Yes | Yes | 69 | 30.25 |
| PEF | Male | Chinese | No | Yes | No | 84 | 21.91 |
| PEF | Female | Chinese | No | Yes | Yes | 82 | 19.56 |
| PEF | Female | Malay | No | Yes | Yes | 77 | 32.58 |
| PEF | Female | Chinese | Yes | Yes | N.A. | 79 | 22.37 |
| PEF | Male | Indian | No | Yes | Yes | 85 | 20.55 |
| PEF | Female | Chinese | Yes | Yes | No | 74 | 34.27 |
| PEF | Female | Malay | No | Yes | No | 62 | 20.82 |
| PEF | Male | Chinese | No | Yes | Yes | 66 | 26.93 |
| PEF | Female | Indian | No | Yes | Yes | 68 | 26.56 |
| PEF | Male | Chinese | Yes | Yes | Yes | 77 | 24.87 |
| PEF | Male | Malay | Yes | Yes | No | 72 | 24.52 |
| PEF | Female | Chinese | Yes | Yes | No | 72 | 20.4 |
| PEF | Female | Chinese | N.A. | Yes | Yes | 84 | 27.78 |
| PEF | Male | Chinese | No | Yes | No | 65 | 31.63 |
| PEF | Male | Chinese | Yes | No | No | 66 | 31.51 |
| PEF | Female | Malay | No | Yes | Yes | 58 | 30.5 |
| PEF | Male | Chinese | No | Yes | Yes | 62 | 35.25 |
| PEF | Female | Chinese | Yes | Yes | No | 88 | 23.71 |
| PEF | Female | Chinese | No | Yes | Yes | 80 | 24.26 |
| PEF | Female | Chinese | No | Yes | No | 75 | 26.64 |
| PEF | Male | Chinese | Yes | No | Yes | 68 | 0.01 |
| PEF | Male | Malay | No | Yes | Yes | 65 | 29.38 |
| PEF | Female | Chinese | No | No | Yes | 63 | 21.76 |
| PEF | Female | Chinese | Yes | Yes | Yes | 69 | 31.61 |
| PEF | Female | Indian | No | Yes | No | 79 | 28.8 |
| PEF | Female | Chinese | No | No | No | 75 | 23.83 |
| PEF | Female | Chinese | No | Yes | No | 73 | 22.22 |
| PEF | Male | Chinese | Yes | Yes | No | 83 | N.A. |
| PEF | Male | Chinese | No | Yes | Yes | 76 | 25.22 |
| PEF | Female | Chinese | No | Yes | No | 68 | 23.5 |
| PEF | Female | Chinese | Yes | Yes | Yes | 61 | 25.38 |
| PEF | Male | Malay | No | Yes | Yes | 64 | 25.7 |
| PEF | Female | Chinese | No | Yes | No | 77 | 26.43 |
| PEF | Male | Chinese | Yes | Yes | No | 81 | 21.08 |

(continued)

| Sample Type | Gender | Race | Atrial Fibrillation or Flutter | Hypertension | Diabetes | Age | Body Mass Index |
|---|---|---|---|---|---|---|---|
| PEF | Female | Chinese | Yes | Yes | Yes | 78 | 25.63 |
| PEF | Female | Chinese | No | Yes | Yes | 67 | 38.67 |
| PEF | Female | Chinese | No | No | No | 87 | 38.27 |
| PEF | Female | Malay | No | Yes | No | 81 | 32.19 |
| PEF | Male | Chinese | No | Yes | No | 75 | 22.22 |
| PEF | Male | Chinese | Yes | Yes | No | 52 | 28.7 |
| PEF | Male | Chinese | No | Yes | Yes | 75 | 28.98 |
| PEF | Male | Chinese | No | Yes | No | 78 | 22.09 |
| PEF | Male | Chinese | Yes | Yes | Yes | 72 | 32.57 |
| PEF | Male | Chinese | No | Yes | Yes | 47 | 34.01 |
| PEF | Female | Malay | Yes | Yes | No | 72 | 35.25 |
| PEF | Male | Chinese | Yes | Yes | No | 81 | 22.41 |
| PEF | Male | Chinese | No | Yes | Yes | 68 | 25.08 |
| PEF | Female | Chinese | Yes | No | No | 76 | 30.5 |
| PEF | Male | Chinese | Yes | No | Yes | 64 | 25.09 |
| PEF | Male | Chinese | No | Yes | Yes | 53 | 28.72 |
| PEF | Female | Chinese | Yes | Yes | Yes | 78 | 35.18 |
| PEF | Male | Chinese | Yes | Yes | Yes | 65 | 32.42 |
| PEF | Male | Chinese | No | Yes | Yes | 57 | 23.15 |
| PEF | Female | Chinese | Yes | Yes | Yes | 78 | 20.27 |
| REF | Male | Chinese | Yes | Yes | Yes | 71 | 21.38 |
| REF | Male | Indian | No | No | No | 38 | 22.65 |
| REF | Female | Chinese | No | Yes | Yes | 65 | 23.07 |
| REF | Male | Indian | Yes | Yes | Yes | 62 | 20.72 |
| REF | Male | Chinese | No | No | Yes | 77 | 22.63 |
| REF | Female | Indian | No | No | Yes | 68 | 28.74 |
| REF | Male | Chinese | No | Yes | No | 68 | 26.17 |
| REF | Male | Chinese | Yes | Yes | Yes | 62 | 23.26 |
| REF | Male | Chinese | Yes | No | No | 59 | 20.24 |
| REF | Female | Chinese | No | Yes | No | 72 | 31.3 |
| REF | Male | Malay | Yes | No | Yes | 60 | 23.58 |
| REF | Male | Chinese | No | Yes | No | 63 | 20.02 |
| REF | Male | Chinese | No | No | No | 64 | 19.81 |
| REF | Female | Chinese | No | Yes | Yes | 63 | 22 |
| REF | Male | Indian | No | Yes | Yes | 63 | 25.03 |
| REF | Female | Chinese | No | Yes | No | 72 | 22.21 |
| REF | Male | Malay | No | Yes | No | 51 | 21.84 |

(continued)

| Sample Type | Gender | Race | Atrial Fibrillation or Flutter | Hypertension | Diabetes | Age | Body Mass Index |
|---|---|---|---|---|---|---|---|
| REF | Male | Chinese | No | Yes | Yes | 71 | 21.91 |
| REF | Male | Malay | No | Yes | No | 71 | 18.2 |
| REF | Male | Malay | No | Yes | Yes | 76 | 30.16 |
| REF | Male | Malay | No | No | No | 62 | 21.15 |
| REF | Male | Indian | No | No | Yes | 55 | 20.76 |
| REF | Female | Chinese | Yes | Yes | Yes | 66 | 20.08 |
| REF | Male | Chinese | Yes | Yes | No | 69 | 18.47 |
| REF | Female | Malay | No | Yes | Yes | 67 | 23.47 |
| REF | Female | Malay | No | Yes | Yes | 56 | 25.45 |
| REF | Female | Chinese | No | Yes | Yes | 55 | 19.53 |
| REF | Female | Chinese | No | No | No | 76 | 22.52 |
| REF | Male | Malay | No | No | Yes | 64 | 24.22 |
| REF | Male | Malay | No | Yes | Yes | 67 | 24.52 |
| REF | Female | Malay | No | Yes | Yes | 57 | 29.96 |
| REF | Male | Malay | Yes | Yes | No | 60 | 26.76 |
| REF | Female | Chinese | Yes | No | No | 56 | 23.29 |
| REF | Female | Malay | No | Yes | Yes | 72 | 24.89 |
| REF | Female | Chinese | No | No | Yes | 65 | 22.72 |
| REF | Male | Chinese | No | Yes | No | 70 | 16.8 |
| REF | Female | Indian | No | Yes | No | 63 | 19.07 |
| REF | Male | Chinese | No | Yes | Yes | 75 | 23.25 |
| REF | Female | Chinese | No | Yes | Yes | 73 | 26.29 |
| REF | Female | Chinese | Yes | Yes | Yes | 72 | 17.1 |
| REF | Female | Indian | No | Yes | Yes | 74 | 33.19 |
| REF | Female | Chinese | Yes | Yes | Yes | 69 | 27.44 |
| REF | Female | Indian | No | No | Yes | 60 | 17.78 |
| REF | Female | Malay | No | Yes | Yes | 78 | 18.3 |
| REF | Male | Malay | No | Yes | No | 68 | 21.5 |
| REF | Female | Malay | No | Yes | No | 47 | 19.33 |
| REF | Male | Chinese | No | Yes | Yes | 53 | 36.57 |
| REF | Male | Malay | No | Yes | Yes | 62 | 24.24 |
| REF | Female | Chinese | Yes | Yes | No | 70 | 21.33 |
| REF | Male | Indian | No | Yes | Yes | 50 | 22.56 |
| REF | Male | Chinese | No | Yes | No | 60 | 23.57 |
| REF | Male | Chinese | Yes | No | No | 59 | 35.67 |
| REF | Female | Chinese | No | Yes | No | 59 | 23.78 |
| REF | Male | Chinese | No | No | No | 40 | 22.46 |

(continued)

| Sample Type | Gender | Race | Atrial Fibrillation or Flutter | Hypertension | Diabetes | Age | Body Mass Index |
|---|---|---|---|---|---|---|---|
| REF | Male | Chinese | No | Yes | No | 70 | 15.66 |
| REF | Female | Chinese | No | Yes | Yes | 58 | 20.17 |
| REF | Female | Indian | No | No | Yes | 41 | 29.87 |
| REF | Female | Chinese | Yes | Yes | Yes | 62 | 22.27 |
| REF | Female | Chinese | No | Yes | Yes | 79 | 24.7 |
| REF | Female | Chinese | No | No | Yes | 57 | 23.11 |
| REF | Male | Malay | No | No | Yes | 67 | 23.05 |
| REF | Male | Chinese | Yes | No | Yes | 62 | 22.99 |
| REF | Female | Chinese | No | Yes | Yes | 61 | 31.96 |
| REF | Male | Malay | No | Yes | Yes | 56 | 25.89 |
| REF | Male | Malay | Yes | Yes | Yes | 81 | 17.26 |
| REF | Female | Malay | No | Yes | Yes | 64 | 22.06 |
| REF | Male | Indian | No | No | No | 54 | 27.02 |
| REF | Female | Chinese | No | No | No | 57 | 16.14 |
| REF | Female | Indian | No | Yes | Yes | 68 | 29.85 |
| REF | Male | Malay | No | Yes | Yes | 74 | 25.88 |
| REF | Female | Chinese | No | Yes | Yes | 77 | 21.03 |
| REF | Female | Malay | Yes | Yes | Yes | 46 | 23.98 |
| REF | Male | Chinese | No | No | No | 53 | 22.56 |
| REF | Male | Chinese | No | Yes | No | 49 | 32.32 |
| REF | Male | Malay | No | Yes | Yes | 63 | 25.73 |
| REF | Male | Chinese | No | Yes | Yes | 71 | 25.25 |
| REF | Female | Indian | No | No | Yes | 45 | 20.13 |
| REF | Female | Chinese | No | No | No | 59 | 24.67 |
| REF | Female | Malay | No | Yes | Yes | 50 | 25.69 |
| REF | Male | Chinese | No | Yes | No | 46 | 24.22 |
| REF | Male | Chinese | No | Yes | No | 54 | 28.21 |
| REF | Male | Chinese | No | Yes | Yes | 64 | 25.61 |
| REF | Female | Chinese | No | No | No | 81 | 18.63 |
| REF | Male | Chinese | No | No | No | 31 | 26.54 |
| REF | Male | Malay | No | Yes | Yes | 48 | 28.67 |
| REF | Female | Chinese | Yes | Yes | No | 61 | 28.71 |
| REF | Male | Chinese | No | No | Yes | 69 | 22.27 |
| REF | Female | Chinese | No | No | No | 64 | 20.88 |
| REF | Male | Chinese | No | Yes | No | 57 | 17.44 |
| REF | Male | Chinese | No | Yes | Yes | 66 | 23.15 |
| REF | Female | Chinese | No | Yes | Yes | 62 | 24.22 |

(continued)

| Sample Type | Gender | Race | Atrial Fibrillation or Flutter | Hypertension | Diabetes | Age | Body Mass Index |
|---|---|---|---|---|---|---|---|
| REF | Male | Chinese | No | Yes | Yes | 84 | 18.81 |
| REF | Female | Chinese | No | No | No | 34 | 19.83 |
| REF | Male | Malay | No | No | Yes | 38 | 28.86 |
| REF | Female | Malay | No | Yes | Yes | 61 | 34.7 |
| REF | Female | Chinese | Yes | No | No | 76 | 17.5 |
| REF | Male | Chinese | No | Yes | No | 84 | 24.61 |
| REF | Male | Chinese | No | Yes | No | 39 | 33.91 |
| REF | Female | Chinese | No | No | Yes | 45 | 24.17 |
| REF | Male | Chinese | No | No | Yes | 63 | 24.5 |
| REF | Female | Indian | No | No | Yes | 72 | 24.85 |
| REF | Male | Chinese | Yes | No | No | 60 | 30.27 |
| REF | Female | Chinese | No | Yes | Yes | 73 | 29.06 |
| REF | Female | Malay | No | No | No | 37 | 35.84 |
| REF | Male | Chinese | No | No | Yes | 69 | 23.25 |
| REF | Female | Indian | No | Yes | Yes | 45 | 29.91 |
| REF | Male | Indian | No | Yes | No | 71 | 20.65 |
| REF | Male | Malay | No | No | Yes | 52 | 25.21 |
| REF | Male | Chinese | No | No | No | 55 | 31.6 |
| REF | Female | Malay | No | Yes | No | 50 | 29.77 |
| REF | Female | Malay | Yes | Yes | No | 81 | N.A. |
| REF | Male | Malay | No | Yes | Yes | 73 | 26.01 |
| REF | Male | Malay | No | No | Yes | 64 | 28.91 |
| REF | Male | Chinese | No | No | No | 66 | 17.49 |
| REF | Female | Chinese | No | No | No | 49 | 20.48 |
| REF | Male | Indian | No | Yes | Yes | 54 | 20.61 |
| REF | Male | Chinese | No | Yes | No | 73 | 27.34 |
| REF | Female | Chinese | No | Yes | No | 80 | 24.13 |
| REF | Female | Indian | No | Yes | No | 46 | 43.28 |
| REF | Male | Chinese | No | No | Yes | 61 | 22.14 |
| REF | Female | Chinese | No | Yes | Yes | 43 | 30.1 |
| REF | Male | Malay | No | No | No | 50 | 20.76 |
| REF | Male | Chinese | No | Yes | Yes | 54 | 24.13 |
| REF | Female | Malay | No | Yes | Yes | 74 | 17.12 |
| REF | Female | Chinese | No | Yes | Yes | 55 | 23.07 |
| REF | Female | Chinese | No | No | No | 49 | 33.45 |
| REF | Male | Chinese | No | Yes | No | 63 | 21.23 |
| REF | Male | Chinese | No | Yes | Yes | 64 | 28.35 |

(continued)

| Sample Type | Gender | Race | Atrial Fibrillation or Flutter | Hypertension | Diabetes | Age | Body Mass Index |
|---|---|---|---|---|---|---|---|
| REF | Female | Malay | No | Yes | Yes | 58 | 24.43 |
| REF | Male | Chinese | No | No | Yes | 53 | 17.07 |
| REF | Female | Indian | No | Yes | No | 79 | N.A. |
| REF | Male | Chinese | Yes | Yes | Yes | 56 | 24.68 |
| REF | Male | Chinese | No | Yes | Yes | 78 | 24.44 |
| REF | Male | Malay | No | Yes | Yes | 60 | 28.34 |
| REF | Male | Indian | Yes | No | No | 83 | 16.81 |
| REF | Female | Chinese | No | Yes | Yes | 71 | N.A. |
| REF | Female | Chinese | No | No | Yes | 56 | 30.04 |
| REF | Male | Chinese | No | Yes | Yes | 54 | 26.3 |
| REF | Female | Chinese | Yes | No | Yes | 57 | 30.49 |
| REF | Male | Chinese | No | Yes | No | 40 | 25.82 |
| REF | Male | Chinese | Yes | Yes | Yes | 59 | 25.1 |
| REF | Female | Chinese | No | Yes | Yes | 63 | 23.11 |
| REF | Male | Chinese | No | Yes | No | 50 | 26.33 |
| REF | Female | Indian | No | No | No | 50 | 29.24 |
| REF | Female | Malay | No | Yes | Yes | 47 | 26.62 |
| REF | Male | Chinese | No | No | No | 38 | 26.2 |
| REF | Male | Chinese | No | No | No | 42 | 28.74 |
| REF | Male | Malay | No | Yes | No | 47 | 23.94 |
| REF | Male | Indian | No | N.A. | Yes | 57 | 27.28 |
| REF | Female | Malay | No | N.A. | No | 35 | 28.86 |
| REF | Male | Chinese | No | No | Yes | 43 | 28.26 |
| REF | Male | Malay | No | Yes | No | 47 | 27.66 |
| REF | Female | Indian | No | No | Yes | 45 | 27.77 |
| REF | Female | Malay | No | Yes | Yes | 40 | 27.47 |
| REF | Male | Chinese | No | Yes | No | 81 | 32.38 |
| REF | Male | Chinese | No | N.A. | Yes | 81 | 18.09 |
| REF | Male | Chinese | No | No | No | 61 | 21.11 |
| REF | Male | Indian | No | Yes | Yes | 82 | 23.92 |
| REF | Male | Chinese | No | Yes | Yes | 62 | 18.55 |
| REF | Male | Chinese | No | Yes | Yes | 53 | 23.87 |
| REF | Male | Chinese | No | No | No | 61 | 24.62 |
| REF | Male | Chinese | No | Yes | Yes | 58 | 20.76 |
| REF | Male | Malay | No | Yes | Yes | 57 | 23.44 |
| REF | Male | Chinese | No | No | No | 45 | 45.74 |
| REF | Male | Indian | No | Yes | Yes | 56 | 23.44 |

(continued)

| Sample Type | Gender | Race | Atrial Fibrillation or Flutter | Hypertension | Diabetes | Age | Body Mass Index |
|---|---|---|---|---|---|---|---|
| REF | Male | Chinese | No | Yes | Yes | 63 | 24.53 |
| REF | Male | Indian | No | Yes | Yes | 53 | 30.33 |
| REF | Female | Chinese | No | Yes | No | 61 | 29.83 |
| REF | Male | Chinese | Yes | Yes | Yes | 64 | 29.07 |
| REF | Male | Chinese | No | N.A. | No | 76 | 22.49 |
| REF | Female | Chinese | No | Yes | Yes | 71 | 25.44 |
| REF | Male | Chinese | No | No | Yes | 50 | 26 |
| REF | Male | Chinese | Yes | Yes | Yes | 73 | N.A. |
| REF | Male | Chinese | No | No | Yes | 55 | 28.63 |
| REF | Male | Malay | Yes | No | Yes | 65 | 22.68 |
| REF | Male | Chinese | No | Yes | No | 65 | 25.15 |
| REF | Male | Chinese | Yes | N.A. | No | 57 | 19.36 |
| REF | Male | Chinese | Yes | Yes | No | 76 | 31.09 |
| REF | Male | Indian | No | Yes | Yes | 62 | 20.65 |
| REF | Male | Chinese | No | Yes | No | 60 | 19.23 |

Plasma NT-proBNP was measured in all samples by electro-chemiluminescence immunoassay (Elecsys proBNP II assay) on an automated Cobas e411 analyzer according to the manufacturer's instructions (Roche Diagnostics GmbH, Mannheim, Germany). A preliminary examination of the distributions in Control, HFREF and HFPEF groups (Figure 2, A-C) showed that the NT-proBNP levels in all groups were positively skewed (skewness/skewing > 2). Since the statistical methods to be applied require an un-skewed distribution (Student's t distribution or logistic distribution), the natural logarithm was calculated for NT-proBNP to generate a new variable: ln_NT-proBNP for which skewness was close to zero (Figure 2, D-F). The ln_NT-proBNP was used for all analyses involving NT-proBNP.

The characteristics of subject groups are summarized in Table 3.

**Table 3. characteristics of the healthy subjects and heart failure subjects**

| Variables: | C (n=208) | HF (n=338) | p-value (C v.s. HF) | HFREF (n=180) | HFPEF (n=158) | p-value (HFREF v.s. HFPEF) |
|---|---|---|---|---|---|---|
| Left ventricular ejection fraction (100%) | 64.0 ± 3.7 | 42.2 ± 18.7 | - | 25.9 ± 7.7 | 60.7 ± 5.9 | - |
| ln_NT-proBNP (100%) | 4.02 ± 0.92 | 7.44 ± 1.5 | - | 7.95 ± 1.32 | 6.86 ± 1.49 | <0.0001 |
| NT-proBNP | 55.8 | 1704.6 | | 2834.2 | 955.1 | |
| Gender (male) (100%) | 51.0% | 51.8% | 0.85 | 60.0% | 42.4% | 0.0012 |
| Age (100%) | 59.7 ± 10.5 | 64.6 ± 12.0 | <0.0001 | 60.8 ± 11.6 | 68.9 ± 11.0 | <0.0001 |
| Race (100%) | | | 0.66 | | | 0.27 |
| Chinese | 66.8% | 63.6% | | 60.6% | 67.1% | |
| Malay | 20.7% | 24.0% | | 24.4% | 23.4% | |
| Indian | 12.5% | 12.4% | | 15.0% | 9.5% | |

(continued)

| Variables: | C (n=208) | HF (n=338) | p-value (C v.s. HF) | HFREF (n=180) | HFPEF (n=158) | p-value (HFREF v.s. HFPEF) |
|---|---|---|---|---|---|---|
| Body Mass Index (98.4%) | 25.4 ± 5.2 | 26.0 ± 5.5 | 0.22 | 24.7 ± 4.9 | 27.4 ± 5.9 | <0.0001 |
| Arial Fibrillation or Flutter (99.8%) | 0.96% | 24.9% | <0.0001 | 16.7% | 34.4% | 0.00017 |
| Hypertension (99.0%) | 33.7% | 75.9% | <0.0001 | 65.7% | 87.3% | <0.0001 |
| Diabetes (99.8%) | 9.6% | 58.8% | <0.0001 | 58.9% | 58.6% | 0.96 |

Besides demographic variables including age, race and gender, clinical variables critical for HF were recorded including LVEF, ln_NT-proBNP, Body Mass Index (BMI), Atrial Fibrillation or Flutter (AF), hypertension and diabetes. HFPEF patients had similar mean LVEF (60.7 ± 5.9) as healthy control subjects (64.0 ± 3.7) whilst, as expected and as per patient selection and allocation, HFREF patients clearly had lower LVEF (25.9 ± 7.7). Student's t-test was used for the comparisons of numerical variables and the chi-square test was used for the comparisons of categorical variables between control and HF (C vs HF, Table 3) and between HFPEF and HFREF (HFREF vs HFPEF, Table 3). In general, HF patients were older, with higher prevalence of hypertension, AF and diabetes compared to controls. HFREF and HFPEF patients differed with respect to distributions of gender, age, BMI, hypertension and AF. All these differently distributed variables were taken into account in the discovery of miRNA biomarkers for HF detection or for HF subtype categorization by multivariate logistic regression.

Ln_NT-proBNP was lower in HFPEF than HFREF with some results falling below the ESC- promoted NT-proBNP cut-off (<125 pg/ml) for diagnosis of HF in the non-acute setting [57]. The loss of NT-proBNP test performance is pronounced in HFPEF (Figure 3, A). The performance of ln_NT-proBNP as a biomarker for the diagnosis of HF was examined by the ROC analysis. In this study, ln_NT-proBNP had 0.962 AUC (area under the ROC curve) for the diagnosis of HF overall. It performed better for detecting HFREF (AUC=0.985) than HFPEF (AUC=0.935) (Figure 3, B-D). ln_NT-proBNP exhibited an AUC of only 0.706 for categorizing HFREF and HFPEF subtypes (Figure 3, C).

## II. MiRNA measurement

Circulating cell-free miRNAs in the blood originate from various organs and blood cells [58]. Therefore the change in the levels of a miRNA caused by heart failure may be partly obscured by the presence of the same miRNA possibly secreted from other sources due to other stimuli. Thus, determining the differences in expression levels of miRNAs found in heart failure and the control group may be challenging. In addition, most of the cell-free miRNAs are of exceptionally low abundance in blood [59]. Therefore, accurate measurement of multiple miRNA targets from limited volume of serum/plasma is critical and highly challenging. To best facilitate the discovery of significantly altered expressions of miRNAs and the identification of multivariate miRNA biomarker panels for the diagnosis of heart failure, instead of using low sensitivity or semi-quantitative screening methods (microarray, sequencing), the inventors of the present study chose to perform qPCR-based assays with an exceptionally well designed workflow (Figure 4).

All qPCR assays (designed by MiRXES™, Singapore) were performed at least twice in a single-plex for miRNA targets and at least four repeats for synthetic RNA 'spike-in' controls. To ensure the accuracy of the results in a high-throughput qPCR studies, the study designed and established, after much iteration, a robust workflow for the discovery of circulating biomarkers (Refer to the "METHOD" and Figure 4). In this novel workflow, various designed 'spike-in' controls were used to monitor and correct for technical variations in isolation, reverse transcription, augmentation and the qPCR processes. All spike-in controls were non-natural synthetic miRNAs mimics (small single-stranded RNA with length range from 22-24 bases) which were designed *in silica* to have exceptionally low similarity in the sequence to all known human miRNAs, thus minimizing cross-hybridization to the primers used in the assays. In addition, the miRNA assays were deliberately divided into a number of multiplex groups *in silico* to minimize non-specific amplifications and primer-primer interactions. Synthetic miRNAs were used to construct standard curves for the interpolation of absolute copy numbers in all the measurements, thus further correcting for technical variations. Predictably, with this highly robust workflow and multiple levels of controls, the study were able to identify low levels of expression of miRNAs in circulation and the approach of the present study is highly reliable and reproducibility of data is ensured.

Two hundred and three (203) miRNA targets were selected for this study based on the prior-knowledge of highly expressed plasma miRNAs (data not shown) and the expression levels of those miRNAs in all 546 plasma samples (HF and control) were quantitatively measured using highly sensitive qPCR assays (designed by MiRXES™, Singapore).

In the current experimental design, total RNA including miRNAs was extracted from 200 $\mu$l plasma. Extracted RNA was reversed transcribed and augmented by touch-down amplification to increase the amount of cDNA without changing the total miRNA expression levels (Figure 4). The augmented cDNA was then diluted for qPCR measurement. A simple calculation based on the effect of dilution revealed that a miRNA which is expressed at levels $\leq$500 copies/ ml in serum will be quantified at levels close to the detection limit of the single-plex qPCR assay ($\leq$10 copies/well). At such a concentration, measurements will be a significant challenge due to the technical limitations (errors in pipetting and qPCR reactions). Thus, miRNAs expressed at concentration of $\leq$500 copies/ ml were excluded from analyses and considered undetectable.

About 70% (n=137) of the total miRNA assayed were found to be highly expressed across all the samples. These 137 miRNAs were detected in more than 90% of the samples (expression levels $\geq$500 copies/ ml; Table 4). As compare to published data (Table 1), the inventors of the present study detected many more miRNAs not previously reported in heart failure, highlighting the importance of the use of the careful and well-controlled experimental design.

**Table 4. sequence of 137 reliably detected mature miRNA**

| Name | Sequence | SEQ ID NO: |
|---|---|---|
| hsa-miR-125a-5p | UCCCUGAGACCCUUUAACCUGUGA | 1 |
| hsa-miR-134 | UGUGACUGGUUGACCAGAGGGG | 2 |
| hsa-let-7b-3p | CUAUACAACCUACUGCCUUCCC | 3 |
| hsa-miR-34b-3p | CAAUCACUAACUCCACUGCCAU | 4 |
| hsa-miR-101-5p | CAGUUAUCACAGUGCUGAUGCU | 5 |
| hsa-miR-550a-5p | AGUGCCUGAGGGAGUAAGAGCCC | 6 |
| hsa-miR-576-5p | AUUCUAAUUUCUCCACGUCUUU | 7 |
| hsa-miR-181b-5p | AACAUUCAUUGCUGUCGGUGGGU | 8 |
| hsa-miR-197-3p | UUCACCACCUUCUCCACCCAGC | 9 |
| hsa-miR-369-3p | AAUAAUACAUGGUUGAUCUUU | 10 |
| hsa-miR-126-5p | CAUUAUUACUUUUGGUACGCG | 11 |
| hsa-miR-375 | UUUGUUCGUUCGGCUCGCGUGA | 12 |
| hsa-miR-379-5p | UGGUAGACUAUGGAACGUAGG | 13 |
| hsa-miR-579 | UUCAUUUGGUAUAAACCGCGAUU | 14 |
| hsa-miR-106b-3p | CCGCACUGUGGGUACUUGCUGC | 15 |
| hsa-miR-497-5p | CAGCAGCACACUGUGGUUUGU | 16 |
| hsa-miR-199a-5p | CCCAGUGUUCAGACUACCUGUUC | 17 |
| hsa-miR-19b-3p | UGUGCAAAUCCAUGCAAAACUGA | 18 |
| hsa-miR-20a-5p | UAAAGUGCUUAUAGUGCAGGUAG | 19 |
| hsa-miR-424-5p | CAGCAGCAAUUCAUGUUUUGAA | 20 |
| hsa-miR-144-3p | UACAGUAUAGAUGAUGUACU | 21 |
| hsa-miR-154-5p | UAGGUUAUCCGUGUUGCCUUCG | 22 |
| hsa-miR-191-5p | CAACGGAAUCCCAAAAGCAGCUG | 23 |
| hsa-miR-30d-5p | UGUAAACAUCCCCGACUGGAAG | 24 |
| hsa-miR-30e-3p | CUUUCAGUCGGAUGUUUACAGC | 25 |
| hsa-miR-10a-5p | UACCCUGUAGAUCCGAAUUUGUG | 26 |
| hsa-miR-374c-5p | AUAAUACAACCUGCUAAGUGCU | 27 |
| hsa-miR-495 | AAACAAACAUGGUGCACUUCUU | 28 |
| hsa-miR-1275 | GUGGGGGAGAGGCUGUC | 29 |

(continued)

| Name | Sequence | SEQ ID NO: |
|---|---|---|
| hsa-miR-1 | UGGAAUGUAAAGAAGUAUGUAU | 30 |
| hsa-miR-23a-3p | AUCACAUUGCCAGGGAUUUCC | 31 |
| hsa-miR-27a-3p | UUCACAGUGGCUAAGUUCCGC | 32 |
| hsa-miR-122-5p | UGGAGUGUGACAAUGGUGUUUG | 33 |
| hsa-miR-133a | UUUGGUCCCCUUCAACCAGCUG | 34 |
| hsa-miR-146b-5p | UGAGAACUGAAUUCCAUAGGCU | 35 |
| hsa-miR-20b-5p | CAAAGUGCUCAUAGUGCAGGUAG | 36 |
| hsa-miR-27b-3p | UUCACAGUGGCUAAGUUCUGC | 37 |
| hsa-miR-30b-5p | UGUAAACAUCCUACACUCAGCU | 38 |
| hsa-let-7e-3p | CUAUACGGCCUCCUAGCUUUCC | 39 |
| hsa-miR-337-3p | CUCCUAUAUGAUGCCUUUCUUC | 40 |
| hsa-miR-363-3p | AAUUGCACGGUAUCCAUCUGUA | 41 |
| hsa-miR-421 | AUCAACAGACAUUAAUUGGGCGC | 42 |
| hsa-miR-335-5p | UCAAGAGCAAUAACGAAAAAUGU | 43 |
| hsa-miR-518b | CAAAGCGCUCCCCUUUAGAGGU | 44 |
| hsa-miR-103a-3p | AGCAGCAUUGUACAGGGCUAUGA | 45 |
| hsa-miR-660-5p | UACCCAUUGCAUAUCGGAGUUG | 46 |
| hsa-miR-192-5p | CUGACCUAUGAAUUGACAGCC | 47 |
| hsa-miR-199b-5p | CCCAGUGUUUAGACUAUCUGUUC | 48 |
| hsa-miR-19a-3p | UGUGCAAAUCUAUGCAAAACUGA | 49 |
| hsa-miR-493-5p | UUGUACAUGGUAGGCUUUCAUU | 50 |
| hsa-miR-377-3p | AUCACACAAAGGCAACUUUUGU | 51 |
| hsa-miR-500a-5p | UAAUCCUUGCUACCUGGGUGAGA | 52 |
| hsa-miR-125b-5p | UCCCUGAGACCCUAACUUGUGA | 53 |
| hsa-let-7i-5p | UGAGGUAGUAGUUUGUGCUGUU | 54 |
| hsa-miR-299-3p | UAUGUGGGAUGGUAAACCGCUU | 55 |
| hsa-miR-15b-5p | UAGCAGCACAUCAUGGUUUACA | 56 |
| hsa-miR-21-3p | CAACACCAGUCGAUGGGCUGU | 57 |
| hsa-miR-106a-5p | AAAAGUGCUUACAGUGCAGGUAG | 58 |
| hsa-miR-221-3p | AGCUACAUUGUCUGCUGGGUUUC | 59 |
| hsa-miR-22-3p | AAGCUGCCAGUUGAAGAACUGU | 60 |
| hsa-miR-23b-3p | AUCACAUUGCCAGGGAUUACC | 61 |
| hsa-miR-25-3p | CAUUGCACUUGUCUCGGUCUGA | 62 |
| hsa-miR-29b-3p | UAGCACCAUUUGAAAUCAGUGUU | 63 |
| hsa-miR-33a-5p | GUGCAUUGUAGUUGCAUUGCA | 64 |
| hsa-miR-423-5p | UGAGGGGCAGAGAGCGAGACUUU | 65 |
| hsa-miR-124-5p | CGUGUUCACAGCGGACCUUGAU | 66 |
| hsa-miR-532-5p | CAUGCCUUGAGUGUAGGACCGU | 67 |

(continued)

| Name | Sequence | SEQ ID NO: |
|---|---|---|
| hsa-miR-200b-3p | UAAUACUGCCUGGUAAUGAUGA | 68 |
| hsa-miR-222-3p | AGCUACAUCUGGCUACUGGGU | 69 |
| hsa-miR-199a-3p | ACAGUAGUCUGCACAUUGGUUA | 70 |
| hsa-miR-451a | AAACCGUUACCAUUACUGAGUU | 71 |
| hsa-miR-1226-3p | UCACCAGCCCUGUGUUCCCUAG | 72 |
| hsa-miR-127-3p | UCGGAUCCGUCUGAGCUUGGCU | 73 |
| hsa-miR-374b-5p | AUAUAAUACAACCUGCUAAGUG | 74 |
| hsa-miR-4732-3p | GCCCUGACCUGUCCUGUUCUG | 75 |
| hsa-miR-487b | AAUCGUACAGGGUCAUCCACUU | 76 |
| hsa-miR-551b-3p | GCGACCCAUACUUGGUUUCAG | 77 |
| hsa-miR-23c | AUCACAUUGCCAGUGAUUACCC | 78 |
| hsa-miR-183-5p | UAUGGCACUGGUAGAAUUCACU | 79 |
| hsa-miR-29c-3p | UAGCACCAUUUGAAAUCGGUUA | 80 |
| hsa-miR-425-3p | AUCGGGAAUGUCGUGUCCGCCC | 81 |
| hsa-miR-484 | UCAGGCUCAGUCCCUCCCGAU | 82 |
| hsa-miR-485-3p | GUCAUACACGGCUCUCCUCUCU | 83 |
| hsa-miR-93-5p | CAAAGUGCUGUUCGUGCAGGUAG | 84 |
| hsa-miR-92a-3p | UAUUGCACUUGUCCCGGCCUGU | 85 |
| hsa-miR-140-5p | CAGUGGUUUUACCCUAUGGUAG | 86 |
| hsa-miR-15a-5p | UAGCAGCACAUAAUGGUUUGUG | 87 |
| hsa-miR-10b-5p | UACCCUGUAGAACCGAAUUUGUG | 88 |
| hsa-miR-130b-3p | CAGUGCAAUGAUGAAAGGGCAU | 89 |
| hsa-miR-24-3p | UGGCUCAGUUCAGCAGGAACAG | 90 |
| hsa-miR-133b | UUUGGUCCCCUUCAACCAGCUA | 91 |
| hsa-miR-186-5p | CAAAGAAUUCUCCUUUUGGGCU | 92 |
| hsa-miR-193a-5p | UGGGUCUUUGCGGGCGAGAUGA | 93 |
| hsa-miR-23a-5p | GGGGUUCCUGGGGAUGGGAUUU | 94 |
| hsa-miR-454-3p | UAGUGCAAUAUUGCUUAUAGGGU | 95 |
| hsa-miR-501-5p | AAUCCUUUGUCCCUGGGUGAGA | 96 |
| hsa-miR-18b-5p | UAAGGUGCAUCUAGUGCAGUUAG | 97 |
| hsa-miR-223-5p | CGUGUAUUUGACAAGCUGAGUU | 98 |
| hsa-miR-30c-5p | UGUAAACAUCCUACACUCUCAGC | 99 |
| hsa-miR-26a-5p | UUCAAGUAAUCCAGGAUAGGCU | 100 |
| hsa-miR-146a-5p | UGAGAACUGAAUUCCAUGGGUU | 101 |
| hsa-miR-452-5p | AACUGUUUGCAGAGGAAACUGA | 102 |
| hsa-miR-148a-3p | UCAGUGCACUACAGAACUUUGU | 103 |
| hsa-miR-194-5p | UGUAACAGCAACUCCAUGUGGA | 104 |
| hsa-miR-29c-5p | UGACCGAUUUCUCCUGGUGUUC | 105 |

(continued)

| Name | Sequence | SEQ ID NO: |
|---|---|---|
| hsa-miR-196b-5p | UAGGUAGUUUCCUGUUGUUGGG | 106 |
| hsa-miR-345-5p | GCUGACUCCUAGUCCAGGGCUC | 107 |
| hsa-miR-503 | UAGCAGCGGGAACAGUUCUGCAG | 108 |
| hsa-miR-627 | GUGAGUCUCUAAGAAAAGAGGA | 109 |
| hsa-let-7d-3p | CUAUACGACCUGCUGCCUUUCU | 110 |
| hsa-miR-30a-5p | UGUAAACAUCCUCGACUGGAAG | 111 |
| hsa-miR-654-3p | UAUGUCUGCUGACCAUCACCUU | 112 |
| hsa-miR-598 | UACGUCAUCGUUGUCAUCGUCA | 113 |
| hsa-miR-671-3p | UCCGGUUCUCAGGGCUCCACC | 114 |
| hsa-miR-132-3p | UAACAGUCUACAGCCAUGGUCG | 115 |
| hsa-miR-142-5p | CAUAAAGUAGAAAGCACUACU | 116 |
| hsa-let-7b-5p | UGAGGUAGUAGGUUGUGUGGUU | 117 |
| hsa-miR-17-5p | CAAAGUGCUUACAGUGCAGGUAG | 118 |
| hsa-miR-185-5p | UGGAGAGAAAGGCAGUUCCUGA | 119 |
| hsa-miR-486-5p | UCCUGUACUGAGCUGCCCCGAG | 120 |
| hsa-miR-99b-5p | CACCCGUAGAACCGACCUUGCG | 121 |
| hsa-miR-128 | UCACAGUGAACCGGUCUCUUU | 122 |
| hsa-miR-16-5p | UAGCAGCACGUAAAUAUUGGCG | 123 |
| hsa-miR-32-5p | UAUUGCACAUUACUAAGUUGCA | 124 |
| hsa-miR-382-5p | GAAGUUGUUCGUGGUGGAUUCG | 125 |
| hsa-miR-532-3p | CCUCCCACACCCAAGGCUUGCA | 126 |
| hsa-miR-181a-2-3p | ACCACUGACCGUUGACUGUACC | 127 |
| hsa-miR-139-5p | UCUACAGUGCACGUGUCUCCAG | 128 |
| hsa-miR-21-5p | UAGCUUAUCAGACUGAUGUUGA | 129 |
| hsa-miR-1280 | UCCCACCGCUGCCACCC | 130 |
| hsa-miR-331-5p | CUAGGUAUGGUCCCAGGGAUCC | 131 |
| hsa-miR-150-5p | UCUCCCAACCCUUGUACCAGUG | 132 |
| hsa-miR-101-3p | UACAGUACUGUGAUAACUGAA | 133 |
| hsa-miR-200c-3p | UAAUACUGCCGGGUAAUGAUGGA | 134 |
| hsa-miR-205-5p | UCCUUCAUUCCACCGGAGUCUG | 135 |
| hsa-miR-505-3p | CGUCAACACUUGCUGGUUUCCU | 136 |
| hsa-miR-136-5p | ACUCCAUUUGUUUUGAUGAUGGA | 137 |

### III. MiRNA biomarkers

Firstly, all measured miRNAs were examined for targets that were only detectable in heart failure samples but not in control samples. Those miRNAs specifically secreted by heart muscles in heart failure patients would be the ideal biomarker for the detection of the disease. As the miRNAs in the blood circulating system are known to be contributed by various organs and/or type of cells (including heart muscles), it was not surprising that these miRNAs may already been represented in the plasma of normal and heart failure patients. However, the differential expression of these miRNAs in the plasma may still serve as useful biomarker during the development of heart failure.

The global unsupervised analysis (principal component analysis, PCA) was initially performed on the expression levels of all detected plasma miRNAs (137, Table 4) in all 546 samples. The first 15 principal components (PCs) with eigenvalues higher than 0.7 were selected for further analysis, which in total accounted for 85% of the variance (Figure 5, A). To examine the difference between the control and heart failure, the AUCs were calculated for the classification of those two groups at each of the selected PCs (Figure 5, B). Multiple PCs were found to have AUCs significantly higher than 0.5 and the 2nd PC even had an AUC of 0.79 indicating that the differences between those two groups largely contributed to the overall variance of the miRNA expression profile. As the variations between the control and heart failure subjects were found in multiple dimensions (PCs), it was not possible to represent all the information based on single miRNA. Thus a multivariate assay including multiple miRNAs was necessary for optimal classification. Similarly, multiple PCs had AUCs significantly higher than 0.5 for the categorization to either of two heart failure subtypes: HFREF or HFPEF (Figure 5, C) including the 1st PC (AUC = 0.6) although the AUCs were less than those for heart failure detection. Hence, a multivariate assay was necessary to capture the information in multiple dimensions for the classification HFREF and HFPEF as well.

Plotting the two groups of subjects (C and heart failure (HF)) on a space defined by the two major discriminative PCs for HF detection, showed they were separately located (Figure 6, A). Separation of HFREF and HFPEF groups (Figure 6, B) was less distinct. The global analysis revealed that it was possible to separate control, HFREF and HFPEF subjects based their miRNA profiles. However, using only one or two dimensions was not statistically robust for classification.

A pivotal step towards identifying biomarkers is to directly compare the expression levels of each miRNA in normal and disease state as well as between disease subtypes. Student's t-test was used for univariate comparisons to assess the significance of between group differences in individual miRNA and multivariate logistic regression was used to adjust for confounding factors including age, gender, BMI, AF, hypertension and diabetes. All p-values were corrected for false discovery rate (FDR) estimation using Bonferroni-type multiple comparison procedures [60]. MiRNAs with p-values lower than 0.01 were considered significant in this study.

The expressions of the 137 plasma miRNAs were then compared A] Between control (healthy) and heart failure (individual subtypes or both subtypes grouped together), B] Between the two subtypes of heart failure (i.e. HFREF and HFPEF).

## A] Identification of miRNAs differentially expressed between non-HF Control subjects and HF patients

Plasma from patients clinically confirmed to have either subtype of heart failure (HFREF or HFPEF) were grouped together and compared to plasma from healthy non-heart failure donors.

The comparisons were initially carried out using univariate analysis (Student's t-test) where 94 miRNAs were found to be significantly altered in heart failure patients compared to Control (p-value after FDR < 0.01) (Figure 7, A). Further examining the two subtypes separately, 82 and 94 miRNAs were found to be significant altered in HFREF and HFPEF subjects compared to control respectively (Figure 7, A). In total, 101 unique miRNAs were identified by univariate analysis with 75% (n=76) of them significant for both subtypes (Figure 7A).

Since the control subjects were recruited from the community, clinical parameters may not be well matched with the heart failure patients including three risk factors for heart failure: AF, hypertension and diabetes where fewer of the control subjects had such conditions. Also, age differed slightly between the analyzed populations. In order to adjust for those possible confounding factors, multivariate analysis (logistic regression) was performed to test the significance of the miRNAs selected by univariate analysis. In total, 86 out of the 101 miRNAs still differed significantly between test populations after multivariate analysis (Figure 7, B). For the detection of all heart failure compared to control, 75 out of the 94 miRNAs (Table 5) were found to be significant (p-value after FDR< 0.01) in the multivariate analysis; while 52 out of 82 (Table 6) were significant for the detection of HFREF comparing to control and 68 out of 94 (Table 7) were significant for the detection of HFPEF comparing to control (Figure 7B). After multivariate analysis, 36 miRNAs were found to remain significantly different between controls and both heart failure subtypes while 16 differed significantly only between Control and HFREF subtype and 32 differed significantly only between Control and HFPEF subtype (Figure 7, B). In the multivariate analysis, many miRNAs were found to differ between Control and only one of the two heart failure subtypes suggesting genuine differences between the two subtypes in terms of miRNA expression.

**Table 5. miRNAs differentially expressed between control and all heart failure subjects**

| Up-regulated (n=37) | | | | | |
|---|---|---|---|---|---|
| Name | p-value, t-test | p-value, Logistic regression | p-value, ln_BNP | Fold change | AUC |
| hsa-let-7d-3p | 8.9E-23 | 4.1E-09 | 3.8E-05 | 1.32 | 0.78 |

(continued)

| Up-regulated (n=37) | | | | | |
|---|---|---|---|---|---|
| Name | p-value, t-test | p-value, Logistic regression | p-value, ln_BNP | Fold change | AUC |
| hsa-miR-197-3p | 8.9E-23 | 2.7E-08 | 7.9E-05 | 1.27 | 0.77 |
| hsa-miR-24-3p | 2.8E-22 | 5.5E-10 | 6.7E-05 | 1.30 | 0.76 |
| hsa-miR-1280 | 4.3E-16 | 3.3E-06 | 3.0E-04 | 1.41 | 0.74 |
| hsa-miR-221-3p | 5.4E-19 | 4.9E-09 | 6.2E-05 | 1.35 | 0.73 |
| hsa-miR-503 | 1.1E-17 | 1.2E-07 | 9.7E-04 | 1.69 | 0.73 |
| hsa-miR-130b-3p | 1.2E-14 | 3.9E-07 | 1.3E-03 | 1.27 | 0.72 |
| hsa-miR-23b-3p | 1.1E-13 | 2.6E-06 | 8.9E-04 | 1.31 | 0.71 |
| hsa-miR-21-3p | 2.4E-14 | 8.0E-06 | >0.01 | 1.25 | 0.70 |
| hsa-miR-223-5p | 4.4E-13 | 1.6E-06 | 9.2E-04 | 123 | 0.70 |
| hsa-miR-423-5p | 5.6E-14 | 4.9E-09 | 6.1E-04 | 1.27 | 0.70 |
| hsa-miR-34b-3p | 9.5E-14 | 4.6E-04 | >0.01 | 1.84 | 0.69 |
| hsa-miR-22-3p | 1.6E-11 | 1.5E-04 | >0.01 | 1.24 | 0.69 |
| hsa-miR-148a-3p | 2.0E-12 | 2.3E-06 | 1.8E-03 | 1.28 | 0.68 |
| hsa-miR-23a-5p | 6.2E-11 | 4.3E-04 | >0.01 | 1.25 | 0.67 |
| hsa-miR-335-5p | 1.3E-10 | 2.5E-06 | 2.3E-04 | 1.33 | 0.67 |
| hsa-miR-124-5p | 3.8E-09 | 9.8E-04 | >0.01 | 1.54 | 0.66 |
| hsa-miR-382-5p | 6.0E-10 | 1.7E-05 | 7.6E-03 | 1.56 | 0.66 |
| hsa-miR-134 | 6.4E-10 | 2.9E-05 | 6.7E-03 | 1.57 | 0.66 |
| hsa-let-7e-3p | 7.6E-07 | 1.1E-03 | >0.01 | 1.33 | 0.65 |
| hsa-miR-598 | 4.9E-08 | 4.8E-05 | >0.01 | 1.20 | 0.65 |
| hsa-miR-627 | 2.8E-08 | 5.5E-04 | >0.01 | 1.31 | 0.65 |
| hsa-miR-199a-3p | 1.3E-05 | 4.1E-03 | >0.01 | 1.27 | 0.64 |
| hsa-miR-27b-3p | 1.6E-06 | 8.7E-04 | 3.8E-04 | 1.20 | 0.64 |
| hsa-miR-146b-5p | 6.3E-07 | 8.7E-04 | 3.4E-04 | 1.25 | 0.64 |
| hsa-miR-146a-5p | 3.1E-06 | 4.3E-03 | 9.7E-04 | 1.25 | 0.64 |
| hsa-miR-331-5p | 2.7E-07 | 2.7E-03 | >0.01 | 1.13 | 0.64 |
| hsa-miR-654-3p | 7.4E-08 | 2.0E-03 | >0.01 | 1.44 | 0.63 |
| hsa-miR-375 | 1.1E-05 | 7.9E-03 | >0.01 | 1.43 | 0.63 |
| hsa-miR-132-3p | 9.8E-07 | 7.4E-04 | >0.01 | 1.12 | 0.63 |
| hsa-miR-27a-3p | 2.0E-05 | 2.4E-03 | 4.9E-03 | 1.16 | 0.63 |
| hsa-miR-128 | 5.9E-06 | 8.6E-04 | >0.01 | 1.11 | 0.63 |
| hsa-miR-299-3p | 2.9E-06 | 3.3E-03 | >0.01 | 1.43 | 0.62 |
| hsa-miR-424-5p | 4.0E-07 | 1.3E-03 | >0.01 | 1.25 | 0.62 |

(continued)

| Up-regulated (n=37) | | | | | |
|---|---|---|---|---|---|
| **Name** | **p-value, t-test** | **p-value, Logistic regression** | **p-value, ln_BNP** | **Fold change** | **AUC** |
| hsa-miR-154-5p | 5.9E-06 | 1.0E-03 | >0.01 | 1.41 | 0.62 |
| hsa-miR-21-5p | 6.5E-07 | 4.0E-03 | >0.01 | 1.16 | 0.61 |
| hsa-miR-377-3p | 1.3E-05 | 3.9E-03 | >0.01 | 1.37 | 0.60 |
| **Down-regulated n =(38)** | | | | | |
| **Name** | **p-value, t-test** | **p-value, Logistic regression** | **p-value, BNP** | **Fold change** | **AUC** |
| hsa-miR-454-3p | 3.3E-43 | 3.0E-14 | 5.6E-06 | 0.47 | 0.85 |
| hsa-miR-103a-3p | 1.6E-35 | 7.2E-12 | 2.4E-05 | 0.70 | 0.82 |
| hsa-miR-30c-5p | 8.9E-23 | 1.9E-10 | 3.2E-04 | 0.65 | 0.75 |
| hsa-miR-30b-5p | 1.9E-22 | 2.2E-09 | 3.0E-03 | 0.64 | 0.75 |
| hsa-miR-17-5p | 2.4E-19 | 1.4E-06 | 3.4E-04 | 0.73 | 0.74 |
| hsa-miR-196b-5p | 2.2E-15 | 7.8E-06 | 2.0E-04 | 0.79 | 0.73 |
| hsa-miR-500a-5p | 5.4E-19 | 1.1E-07 | 3.8E-04 | 0.68 | 0.73 |
| hsa-miR-106a-5p | 1.1E-16 | 1.3E-06 | 4.7E-05 | 0.76 | 0.72 |
| hsa-miR-20a-5p | 2.6E-17 | 1.4E-06 | 7.9E-05 | 0.74 | 0.72 |
| hsa-miR-451a | 5.4E-19 | 9.8E-08 | 7.9E-05 | 0.54 | 0.72 |
| hsa-miR-29b-3p | 1.5E-16 | 4.6E-08 | 6.7E-05 | 0.76 | 0.71 |
| hsa-miR-374b-5p | 2.4E-16 | 1.1E-07 | 1.8E-03 | 0.69 | 0.71 |
| hsa-miR-20b-5p | 1.5E-16 | 2.3E-06 | 8.1E-05 | 0.60 | 0.71 |
| hsa-miR-501-5p | 2.2E-14 | 3.3E-06 | 1.2E-04 | 0.71 | 0.70 |
| hsa-miR-18b-5p | 4.4E-13 | 3.9E-05 | 4.7E-05 | 0.78 | 0.69 |
| hsa-miR-23c | 3.1E-12 | 1.2E-06 | >0.01 | 0.68 | 0.69 |
| hsa-miR-551b-3p | 3.0E-12 | 3.2E-05 | >0.01 | 0.65 | 0.69 |
| hsa-miR-26a-5p | 4.7E-13 | 3.9E-05 | >0.01 | 0.74 | 0.69 |
| hsa-miR-183-5p | 1.8E-12 | 2.8E-05 | 3.8E-04 | 0.59 | 0.68 |
| hsa-miR-16-5p | 4.2E-12 | 1.9E-05 | 8.4E-04 | 0.71 | 0.68 |
| hsa-miR-191-5p | 1.8E-12 | 9.3E-05 | >0.01 | 0.74 | 0.68 |
| hsa-miR-532-5p | 1.2E-11 | 8.0E-06 | 4.9E-04 | 0.77 | 0.67 |
| hsa-miR-363-3p | 3.9E-11 | 1.7E-04 | 2.7E-03 | 0.70 | 0.67 |
| hsa-miR-374c-5p | 4.5E-10 | 3.7E-04 | >0.01 | 0.71 | 0.67 |
| hsa-let-7b-5p | 3.5E-11 | 3.8E-04 | >0.01 | 0.80 | 0.66 |
| hsa-miR-15a-5p | 3.8E-09 | 9.8E-04 | 4.7E-03 | 0.82 | 0.66 |
| hsa-miR-144-3p | 3.9E-11 | 9.4E-05 | 3.8E-04 | 0.63 | 0.66 |

(continued)

| Down-regulated n =(38) | | | | | |
|---|---|---|---|---|---|
| Name | p-value, t-test | p-value, Logistic regression | p-value, BNP | Fold change | AUC |
| hsa-miR-93-5p | 1.3E-09 | 3.8E-04 | 1.3E-03 | 0.82 | 0.66 |
| hsa-miR-181b-Sp | 3.1E-09 | 1.2E-07 | >0.01 | 0.80 | 0.66 |
| hsa-miR-19b-3p | 2.3E-09 | 3.4E-05 | 8.3E-05 | 0.80 | 0.65 |
| hsa-miR-4732-3p | 2.4E-08 | 4.7E-04 | 3.5E-03 | 0.70 | 0.64 |
| hsa-miR-484 | 5.9E-07 | 9.9E-03 | >0.01 | 0.89 | 0.64 |
| hsa-miR-25-3p | 3.3E-07 | 4.4E-03 | 8.8E-03 | 0.79 | 0.63 |
| hsa-miR-192-5p | 8.9E-06 | 9.9E-04 | >0.01 | 0.76 | 0.63 |
| hsa-miR-205-5p | 3.2E-05 | 2.0E-03 | >0.01 | 0.75 | 0.62 |
| hsa-miR-19a-3p | 2.2E-06 | 1.1E-03 | 6.9E-04 | 0.84 | 0.61 |
| hsa-miR-32-5p | 7.5E-06 | 8.3E-03 | >0.01 | 0.88 | 0.61 |
| hsa-miR-150-5p | 1.2E-05 | 2.9E-05 | >0.01 | 0.79 | 0.60 |

**Table 6. miRNAs differentially expressed between control and REF subjects**

| Up-regulated (n=25) | | | | | |
|---|---|---|---|---|---|
| Name | p-value, t-test | p-value, Logistic regression | p-value, ln_BNP | Fold change | AUC |
| hsa-miR-423-5p | 5.5E-18 | 1.8E-09 | >0.01 | 1.32 | 0.75 |
| hsa-let-7d-3p | 5.0E-15 | 1.2E-06 | >0.01 | 1.26 | 0.75 |
| hsa-miR-24-3p | 5.9E-15 | 5.1E-07 | >0.01 | 1.27 | 0.74 |
| hsa-miR-503 | 5.0E-15 | 1.9E-06 | >0.01 | 1.74 | 0.74 |
| hsa-miR-197-3p | 6.6E-14 | 6.7E-05 | >0.01 | 1.22 | 0.73 |
| hsa-miR-1280 | 4.2E-11 | 1.9E-04 | >0.01 | 1.34 | 0.72 |
| hsa-miR-22-3p | 7.3E-11 | 1.1E-04 | >0.01 | 1.27 | 0.71 |
| hsa-miR-130b-3p | 1.6E-10 | 1.1E-05 | >0.01 | 1.23 | 0.71 |
| hsa-miR-221-3p | 4.4E-12 | 1.2E-06 | >0.01 | 1.31 | 0.71 |
| hsa-miR-34b-3p | 2.4E-12 | 7.2E-04 | >0.01 | 1.90 | 0.70 |
| hsa-miR-30a-5p | 2.0E-10 | 1.1E-03 | >0.01 | 1.39 | 0.70 |
| hsa-miR-21-3p | 1.1E-09 | 3.6E-04 | >0.01 | 1.22 | 0.69 |
| hsa-miR-132-3p | 7.4E-09 | 6.8E-04 | >0.01 | 1.16 | 0.68 |
| hsa-miR-331-5p | 2.7E-09 | 2.2E-03 | >0.01 | 1.18 | 0.68 |
| hsa-miR-124-5p | 3.3E-09 | 5.6E-04 | >0.01 | 1.62 | 0.67 |
| hsa-miR-148a-3p | 1.2E-08 | 2.6E-04 | >0.01 | 1.26 | 0.66 |
| hsa-miR-23b-3p | 3.2E-07 | 2.0E-04 | >0.01 | 1.22 | 0.66 |
| hsa-miR-375 | 1.0E-06 | 3.8E-03 | >0.01 | 1.55 | 0.65 |
| hsa-miR-134 | 2.4E-06 | 4.2E-04 | >0.01 | 1.48 | 0.64 |
| hsa-miR-627 | 1.4E-05 | 2.2E-03 | >0.01 | 1.28 | 0.64 |

(continued)

| Up-regulated (n=25) | | | | | |
|---|---|---|---|---|---|
| Name | p-value, t-test | p-value, Logistic regression | p-value, ln_BNP | Fold change | AUC |
| hsa-miR-382-5p | 6.2E-06 | 6.4E-04 | >0.01 | 1.45 | 0.63 |
| hsa-miR-598 | 6.1E-05 | 2.6E-04 | >0.01 | 1.16 | 0.63 |
| hsa-miR-23a-5p | 1.4E-05 | 3.3E-03 | >0.01 | 1.17 | 0.63 |
| hsa-miR-223-5p | 3.7E-04 | 9.3E-03 | >0.01 | 1.12 | 0.62 |
| hsa-miR-335-5p | 1.6E-04 | 2.6E-04 | >0.01 | 1.19 | 0.61 |
| Down-regulated (n=27) | | | | | |
| Name | p-value, t-test | p-value, Logistic regression | p-value, ln_BNP | Fold change | AUC |
| hsa-miR-454-3p | 2.9E-30 | 2.0E-11 | >0.01 | 0.48 | 0.83 |
| hsa-miR-103a-3p | 8.8E-23 | 3.0E-09 | >0.01 | 0.74 | 0.79 |
| hsa-miR-30b-5p | 3.7E-19 | 3.0E-08 | >0.01 | 0.62 | 0.76 |
| hsa-miR-30c-5p | 3.7E-19 | 1.1E-08 | >0.01 | 0.64 | 0.76 |
| hsa-miR-374b-5p | 1.1E-15 | 5.7E-07 | >0.01 | 0.66 | 0.73 |
| hsa-miR-23c | 1.3E-13 | 2.7E-07 | >0.01 | 0.63 | 0.72 |
| hsa-miR-551b-3p | 6.9E-11 | 1.5E-04 | >0.01 | 0.63 | 0.70 |
| hsa-miR-17-5p | 1.5E-11 | 2.6E-04 | >0.01 | 0.80 | 0.70 |
| hsa-miR-26a-5p | 6.9E-11 | 6.7E-05 | >0.01 | 0.73 | 0.70 |
| hsa-miR-181b-5p | 1.9E-11 | 1.4E-06 | >0.01 | 0.73 | 0.70 |
| hsa-miR-500a-5p | 2.1E-10 | 6.7E-05 | >0.01 | 0.73 | 0.69 |
| hsa-miR-196b-5p | 1.0E-07 | 1.7E-03 | >0.01 | 0.84 | 0.68 |
| hsa-miR-374c-5p | 1.4E-08 | 6.3E-04 | >0.01 | 0.70 | 0.68 |
| hsa-miR-451a | 2.1E-10 | 1.1E-04 | >0.01 | 0.62 | 0.68 |
| hsa-miR-29b-3p | 2.8E-09 | 8.8E-05 | >0.01 | 0.80 | 0.67 |
| hsa-miR-20a-5p | 1.7E-08 | 7.8E-04 | >0.01 | 0.81 | 0.67 |
| hsa-miR-191-5p | 1.7E-08 | 4.1E-04 | >0.01 | 0.77 | 0.66 |
| hsa-miR-106a-5p | 3.0E-07 | 4.7E-03 | >0.01 | 0.85 | 0.65 |
| hsa-miR-181a-2-3p | 1.1E-04 | 5.9E-03 | >0.01 | 0.84 | 0.65 |
| hsa-miR-501-5p | 4.3E-06 | 4.3E-03 | >0.01 | 0.80 | 0.64 |
| hsa-miR-20b-5p | 2.0E-06 | 4.0E-03 | >0.01 | 0.75 | 0.63 |
| hsa-miR-183-5p | 5.8E-06 | 1.4E-03 | >0.01 | 0.69 | 0.63 |
| hsa-miR-16-5p | 1.6E-05 | 3.8E-03 | >0.01 | 0.79 | 0.63 |
| hsa-miR-125a-5p | 7.9E-05 | 6.4E-04 | >0.01 | 0.81 | 0.62 |
| hsa-miR-150-5p | 4.3E-05 | 2.0E-04 | >0.01 | 0.78 | 0.61 |
| hsa-miR-205-5p | 3.3E-04 | 5.9E-03 | >0.01 | 0.76 | 0.61 |
| hsa-miR-532-5p | 2.3E-04 | 9.3E-03 | >0.01 | 0.85 | 0.61 |

**Table 7. MiRNAs differentially expressed between control and HFPEF subjects**

| Up-regulated (n=33) | | | | | |
|---|---|---|---|---|---|
| Name | p-value, t-test | p-value, Logistic regression | p-value, ln_BNP | Fold change | AUC |
| hsa-let-7d-3p | 4.40E-22 | 5.90E-07 | 4.10E-05 | 1.39 | 0.81 |
| hsa-miR-197-3p | 4.10E-24 | 2.10E-07 | 6.40E-05 | 1.34 | 0.81 |
| hsa-miR-223-5p | 6.80E-21 | 2.10E-07 | 7.80E-05 | 1.37 | 0.79 |
| hsa-miR-24-3p | 5.80E-19 | 2.10E-07 | 4.10E-05 | 1.33 | 0.78 |
| hsa-miR-23b-3p | 1.60E-16 | 1.40E-05 | 2.60E-04 | 1.41 | 0.76 |
| hsa-miR-221-3p | 5.90E-17 | 5.90E-07 | 4.10E-05 | 1.4 | 0.76 |
| hsa-miR-1280 | 3.00E-14 | 2.20E-04 | 9.10E-04 | 1.49 | 0.75 |
| hsa-miR-130b-3p | 2.20E-14 | 9.00E-05 | 9.10E-04 | 1.32 | 0.74 |
| hsa-miR-335-5p | 2.40E-14 | 5.30E-06 | 4.10E-05 | 1.5 | 0.73 |
| hsa-miR-503 | 1.40E-11 | 3.80E-05 | 5.80E-04 | 1.64 | 0.72 |
| hsa-miR-23a-5p | 1.90E-12 | 1.40E-03 | 3.60E-03 | 1.34 | 0.72 |
| hsa-miR-21-3p | 8.50E-13 | 8.20E-05 | 3.60E-03 | 1.29 | 0.72 |
| hsa-miR-148a-3p | 4.40E-11 | 1.10E-04 | 2.20E-03 | 1.3 | 0.7 |
| hsa-miR-199a-3p | 2.70E-07 | 8.40E-04 | 2.20E-03 | 1.38 | 0.69 |
| hsa-miR-146a-5p | 3.10E-08 | 2.50E-04 | 1.50E-04 | 1.37 | 0.69 |
| hsa-miR-382-5p | 1.00E-09 | 1.00E-04 | 1.10E-03 | 1.69 | 0.68 |
| hsa-miR-134 | 3.90E-09 | 2.90E-04 | 1.60E-03 | 1.67 | 0.68 |
| hsa-let-7e-3p | 4.00E-08 | 1.30E-03 | 8.50E-03 | 1.43 | 0.68 |
| hsa-miR-146b-5p | 9.90E-08 | 1.60E-04 | 6.40E-05 | 1.33 | 0.67 |
| hsa-miR-27b-3p | 5.10E-07 | 6.70E-04 | 1.50E-04 | 1.26 | 0.67 |
| hsa-miR-598 | 3.10E-08 | 1.20E-03 | >0.01 | 1.25 | 0.67 |
| hsa-miR-27a-3p | 1.60E-06 | 3.40E-04 | 5.80E-04 | 1.21 | 0.67 |
| hsa-miR-128 | 2.60E-07 | 2.90E-04 | 2.50E-03 | 1.15 | 0.67 |
| hsa-miR-627 | 4.20E-07 | 8.70E-03 | >0.01 | 1.36 | 0.66 |
| hsa-miR-299-3p | 2.30E-07 | 2.30E-03 | >0.01 | 1.59 | 0.65 |
| hsa-miR-21-5p | 2.50E-07 | 1.30E-03 | >0.01 | 1.21 | 0.65 |
| hsa-miR-425-3p | 1.30E-04 | 9.30E-04 | 1.60E-03 | 1.15 | 0.65 |
| hsa-miR-154-5p | 1.10E-06 | 9.10E-04 | 3.60E-03 | 1.55 | 0.64 |
| hsa-miR-377-3p | 2.40E-06 | 8.30E-03 | >0.01 | 1.52 | 0.64 |
| hsa-miR-424-5p | 3.50E-06 | 3.00E-03 | >0.01 | 1.28 | 0.63 |
| hsa-miR-423-5p | 2.50E-07 | 1.30E-03 | 4.10E-03 | 1.23 | 0.63 |
| hsa-miR-99b-5p | 8.30E-04 | 9.30E-03 | 4.60E-03 | 1.18 | 0.62 |
| hsa-miR-671-3p | 6.80E-04 | 8.60E-03 | 9.40E-03 | 1.18 | 0.61 |

(continued)

| Down-regulated (n=35) | | | | | |
|---|---|---|---|---|---|
| Name | p-value, t-test | p-value, Logistic regression | p-value, ln_BNP | Fold change | AUC |
| hsa-miR-454-3p | 8.9E-36 | 5.9E-07 | 4.1E-05 | 0.45 | 0.87 |
| hsa-miR-103a-3p | 8.9E-36 | 1.3E-06 | 4.5E-05 | 0.65 | 0.86 |
| hsa-miR-106a-5p | 3.4E-22 | 5.9E-07 | 4.1E-05 | 0.68 | 0.80 |
| hsa-miR-17-5p | 4.0E-21 | 2.5E-05 | 2.9E-04 | 0.67 | 0.80 |
| hsa-miR-20b-5p | 4.1E-24 | 5.0E-07 | 4.1E-05 | 0.47 | 0.79 |
| hsa-miR-20a-5p | 7.0E-21 | 2.1E-06 | 6.4E-05 | 0.66 | 0.79 |
| hsa-miR-196b-5p | 5.6E-18 | 2.8E-05 | 1.8E-04 | 0.75 | 0.78 |
| hsa-miR-451a | 3.3E-21 | 5.9E-07 | 7.0E-05 | 0.46 | 0.78 |
| hsa-miR-18b-5p | 3.0E-17 | 8.2E-05 | 9.2E-05 | 0.69 | 0.77 |
| hsa-miR-500a-5p | 2.1E-19 | 7.0E-06 | 1.6E-04 | 0.62 | 0.77 |
| hsa-miR-29b-3p | 1.0E-18 | 1.3E-06 | 6.2E-05 | 0.72 | 0.76 |
| hsa-miR-501-5p | 5.2E-19 | 2.4E-06 | 4.5E-05 | 0.62 | 0.76 |
| hsa-miR-532-5p | 1.3E-16 | 1.2E-06 | 7.0E-05 | 0.69 | 0.75 |
| hsa-let-7b-5p | 1.1E-16 | 3.8E-05 | 9.0E-04 | 0.71 | 0.74 |
| hsa-miR-30c-5p | 1.3E-15 | 1.0E-04 | 2.1E-03 | 0.67 | 0.74 |
| hsa-miR-183-5p | 2.9E-15 | 3.8E-05 | 3.8E-04 | 0.50 | 0.74 |
| hsa-miR-30b-5p | 3.0E-15 | 5.7E-04 | >0.01 | 0.66 | 0.74 |
| hsa-miR-144-3p | 1.9E-16 | 3.6E-06 | 1.5E-04 | 0.51 | 0.73 |
| hsa-miR-93-5p | 4.6E-15 | 2.5E-05 | 5.0E-04 | 0.74 | 0.73 |
| hsa-miR-16-5p | 6.5E-15 | 4.1E-06 | 2.6E-04 | 0.62 | 0.73 |
| hsa-miR-363-3p | 1.5E-13 | 4.5E-05 | 1.3E-03 | 0.62 | 0.73 |
| hsa-miR-25-3p | 3.3E-12 | 8.2E-05 | 2.5E-03 | 0.68 | 0.71 |
| hsa-miR-4732-3p | 1.1E-13 | 1.1E-05 | 9.1E-04 | 0.57 | 0.71 |
| hsa-miR-192-5p | 2.2E-09 | 4.1E-04 | >0.01 | 0.65 | 0.70 |
| hsa-miR-19b-3p | 1.5E-11 | 1.6E-05 | 1.0E-04 | 0.74 | 0.70 |
| hsa-miR-15a-5p | 3.3E-08 | 3.0E-03 | 3.6E-03 | 0.80 | 0.69 |
| hsa-miR-486-5p | 2.2E-10 | 3.4E-04 | >0.01 | 0.64 | 0.69 |
| hsa-miR-374b-5p | 3.4E-10 | 5.1E-03 | >0.01 | 0.72 | 0.68 |
| hsa-miR-484 | 4.0E-08 | 9.9E-03 | >0.01 | 0.86 | 0.67 |
| hsa-miR-194-5p | 1.0E-06 | 4.6E-03 | >0.01 | 0.71 | 0.67 |
| hsa-miR-101-3p | 2.7E-08 | 5.1E-04 | 6.7E-03 | 0.74 | 0.67 |
| hsa-miR-551b-3p | 3.3E-08 | 8.9E-03 | >0.01 | 0.67 | 0.67 |

(continued)

| Down-regulated (n=35) | | | | | |
|---|---|---|---|---|---|
| Name | p-value, t-test | p-value, Logistic regression | p-value, ln_BNP | Fold change | AUC |
| hsa-miR-185-5p | 1.7E-09 | 1.8E-03 | 4.6E-03 | 0.80 | 0.66 |
| hsa-miR-19a-3p | 3.3E-08 | 1.9E-04 | 9.1E-04 | 0.79 | 0.66 |
| hsa-miR-550a-5p | 3.5E-05 | 3.0E-03 | 5.7E-03 | 0.81 | 0.62 |

A number of miRNAs has previously been reported to be up-regulated and down-regulated in HF (Table 1). Interestingly; the miRNAs found to be differentially expressed in the present study were substantially different from these reports. The significant miRNAs in both univariate and multivariate analysis were listed in Table 5 (C vs heart failure (HF)), Table 6 (C vs HFREF) and Table 7 (C vs HFPEF) where 37, 25 and 33 miRNAs were found to be up-regulated and 38, 27 and 35 miRNAs were found to be down-regulated in the three comparisons, respectively. The number of differentially expressed miRNAs validated by qPCR (101 in univariate analysis and 86 in both univariate analysis and multivariate analysis) was substantially higher than previously reported (Table 8, in total 47). Each miRNA or combinations of from these 86 miRNAs can serve as biomarker or as a component of a panel of biomarkers (multivariate index assays) for the diagnosis of heart failure.

**Table 8. Comparison between the current study and previously published reports**

| Name in miRBase V18 | Name in literature | No in literature | Regulation in literature | Regulation in this study |
|---|---|---|---|---|
| hsa-miR-210 | miR-210 | 2 | Up & Down | N.A. |
| hsa-miR-194-5p | miR-194 | 1 | Up | Down |
| hsa-miR-192-5p | miR-192 | 1 | Up | Down |
| hsa-miR-185-5p | miR-185 | 1 | Up | Down |
| hsa-miR-101-3p | miR-101 | 1 | Up | Down |
| hsa-miR-92b-3p | miR-92b | 1 | Up | N.A. |
| hsa-miR-675-5p | miR-675 | 1 | Up | N.A. |
| hsa-miR-622 | miR-622 | 2 | Up | N.A. |
| hsa-miR-499a-5p | miR-499 | 1 | Up | N.A. |
| hsa-miR-34a-5p | miR-34a | 1 | Up | N.A. |
| hsa-miR-320a | miR-320a | 1 | Up | N.A. |
| hsa-miR-200b-5p | miR-200b* | 1 | Up | N.A. |
| hsa-miR-18b-3p | miR-18b* | 1 | Up | N.A. |
| hsa-miR-17-3p | miR-17* | 1 | Up | N.A. |
| hsa-miR-129-5p | miR-129-5p | 1 | Up | N.A. |
| hsa-miR-1254 | miR-1254 | 1 | Up | N.A. |
| hsa-miR-1228-5p | miR-1228* | 1 | Up | N.A. |
| hsa-miR-92a-3p | miR-92a | 1 | Up | No Change |
| hsa-miR-532-3p | miR-532-3p | 1 | Up | No Change |
| hsa-miR-29c-3p | miR-29c | 1 | Up | No Change |
| hsa-miR-423-5p | miR-423-5p | 2 | Up | Up |
| hsa-miR-30a-5p | miR-30a | 2 | Up | Up |
| hsa-miR-22-3p | miR-22 | 1 | Up | Up |

(continued)

| Name in miRBase V18 | Name in literature | No in literature | Regulation in literature | Regulation in this study |
|---|---|---|---|---|
| hsa-miR-21-5p | miR-21 | 1 | Up | Up |
| hsa-miR-103a-3p | miR-103 | 1 | Down | Down |
| hsa-miR-30b-5p | miR-30b | 1 | Down | Down |
| hsa-miR-191-5p | miR-191 | 2 | Down | Down |
| hsa-miR-150-5p | miR-150 | 1 | Down | Down |
| hsa-miR-28-5p | miR-28-5p | 2 | Down | N.A. |
| hsa-miR-223-3p | miR-223 | 2 | Down | N.A. |
| hsa-miR-142-3p | miR-142-3p | 3 | Down | N.A. |
| hsa-miR-126-3p | miR-126 | 1 | Down | N.A. |
| hsa-miR-342-3p | miR-342-3p | 1 | Down | N.A. |
| hsa-miR-331-3p | miR-331-3p | 2 | Down | N.A. |
| hsa-miR-324-5p | miR-324-5p | 1 | Down | N.A. |
| hsa-miR-574-3p | miR-574-3p | 2 | Down | N.A. |
| hsa-miR-151a-5p | miR-151-5p | 2 | Down | N.A. |
| hsa-miR-744-5p | miR-744 | 2 | Down | N.A. |
| hsa-miR-23a-3p | miR-23a | 1 | Down | No Change |
| hsa-miR-33a-5p | miR-33a | 2 | Down | No Change |
| hsa-miR-199b-5p | miR-199b-5p | 2 | Down | No Change |
| hsa-miR-1 | miR-1 | 1 | Down | No Change |
| hsa-miR-24-3p | miR-24 | 2 | Down | Up |
| hsa-miR-27a-3p | miR-27a | 2 | Down | Up |
| hsa-miR-199a-3p | miR-199a-3p | 1 | Down | Up |
| hsa-miR-27b-3p | miR-27b | 3 | Down | Up |
| hsa-miR-335-5p | miR-335 | 2 | Down | Up |

In total, 47 distinct miRNAs have been reported in the literature (Table 1). Hsa-miR-210 had contradictory observations in the direction of change in the heart failure patients (Table 8). In the present study, 22 of the other 46 reported miRNAs were not measurable or fell below the detection limit (N.A. in Table 8) leaving 24 miRNAs to be used for comparison. Comparing the results (p-value after FDR < 0.01 in univariate analysis) with the 24 reported miRNAs, only 4 of these previously reported miRNAs (hsa-miR-423-5p, hsa-miR-30a-5p, hsa-miR-22-3p, hsa-miR-21-5p) were found to be consistently up-regulated and four (hsa-miR-103a-3p, hsa-miR-30b-5p, hsa-miR-191-5 and hsa-miR-150-5p) were found to be consistently down-regulated in the present study (Table 8). Interestingly, in eight of the dysregulated miRNAs the direction of change was opposite to that previously reported while seven of them remained unchanged (Table 8). Thus, the majority miRNAs previously reported to be differentially regulated in heart failure could NOT be confirmed in the present study. Conversely, the current study identified more than 70 novel miRNAs which could be the potential biomarkers for HF detection not previously reported.
NT-proBNP/BNP is the best studied heart failure biomarker and has exhibited the best clinical performance to date. Thus, the present study aimed to examine whether these significantly regulated miRNAs could provide additional information to NT-proBNP. The enhancement by miRNA of detecting heart failure by NT-proBNP was tested by logistic regression with adjustment for age AF, hypertension and diabetes (p-value, ln_BNP, Table 5-7). Using the p-values after FDR correction lower than 0.01 as the criterion, 55 miRNAs (p-value, ln_BNP, Table 7) were found to have information complementary to ln_NT-proBNP for HFPEF detection but not for HFREF (p-value, ln_BNP, Table 6) . NT-proBNP used alone clearly had better diagnostic performance for detection of HFREF (AUC = 0.985, Figure 3D) than HFPEF (AUC=0.935, Figure 3E). Combining any or multiple of those 55 miRNAs together with ln_NT-proBNP, in multivariate assay, could potentially improve detection of HFPEF.

The AUC values for the most up-regulated (hsa-let-7d-3p, Figure 8, A) and most down-regulated (hsa-miR-454-3p, Figure 8, B) miRNA in heart failure (both subtypes) were 0.78 and 0.85, respectively. Both miRNAs have not previously been reported as useful for detection of heart failure. Although the diagnostic power of single miRNA may not be clinically useful, combining multiple miRNAs in a multivariate manner to may well enhance performance for heart failure diagnosis.

**B] Identification of miRNAs differentially expressed between HFREF and HFPEF**

Univariate analysis (Student's t-test) indicated that 40 miRNAs were significantly altered between HFREF and HFPEF subjects (p-value after FDR < 0.01), with 10 miRNAs having higher expression levels in HFPEF than HFREF and 30 miRNAs higher expressions in HFREF than HFPEF (Table 9).

Background clinical characteristics are expected to differ between the two heart failure subtypes (Table 3). HFPEF patients were more frequently female, had higher BMI, were older and more often had AF or hypertension compared with HFREF patients. On multivariate analysis (logistic regression) with adjustment for these characteristics, only 18 out of the 40 miRNAs remained significant (p-value after FDR < 0.01) (p-value, logistic regression, Table 9). However, since the difference between the two subtypes were due to the natural occurrence and characterization of the disease rather than caused by biased sample selection for the study, all the 40 miRNAs in Table 9 (univariate analysis) could be useful for classifying heart failure subtypes.

The AUC values for discriminating heart failure from Control for the most up-regulated miRNA (hsa-miR-223-5p, Figure 10, A) and most down-regulated miRNA (hsa-miR-185-5p, Figure 10, B) in all heart failure were moderate only at 0.68 and 0.69, respectively. This is the first report of using circulating cell free miRNAs from blood (plasma/ serum) for the classification of heart failure patients into two clinically relevant subtypes. Combining multiple miRNAs in a multivariate index assay provides more diagnostic power for subtype categorization.

Most of the miRNAs differentially expressed between HFREF and HFPEF (38 out of 40 in univariate analysis and 17 out of 18 in multivariate analysis) were also found to differ from Control reflecting the fact that the degree of dysregulation varied between the two heart failure subtypes (Figure 11).To further examine the 38 overlapped miRNAs that were found to be altered in either of the HF subtypes as well as between the two subtypes in the univariate analysis (Figure 9, A), they were classified into 6 groups based on the relationship of their expression levels in three subject groups: control, HFREF and HFPEF (Figure 11). If the p-value (FDR) for the comparison between the two groups was higher than 0.01, the relation was then be defined as equal (indicated as"=") while if the p-value (after FDR correction) was lower than 0.01, the relation was then defined by the direction of the change (indicated as higher ">" or lower "<").

A graded change from control to HFREF to HFPEF was found in most of the miRNAs where 21 miRNAs were gradually decreased (C > HFREF > HFPEF, Figure 11) and 5 miRNAs were gradually increased (C < HFREF < HFPEF, Figure 11). Also, 5 miRNAs were found to be only lower in HFPEF subtype (C = HFREF > HFPEF, Figure 11) and 2 were found to be only higher in HFPEF subtype (C = HFREF < HFPEF, Figure 11) while there was no difference between HFREF and control. Comparing to the control, only 3 miRNAs had more distinct levels in HFREF subtype than in HFPEF (C < HFPEF < HFREF or C = HFPEF > HFREF or C = HFPEF < HFREF, Figure 11). Unlike the LVEF and NT-proBNP, HFPEF had more distinct miRNA profiles than the HFREF subtype compared to the healthy control. This suggested that the miRNAs could complement NT-proBNP to provide better discrimination of HFPEF.

Analyses of all detectable miRNAs revealed a large number to be positively correlated to one another (Pearson correlation coefficient > 0.5, Figure 12) especially between those miRNA both altered in HF patients and differing between the two heart failure subtypes (miRNAs indicated black in the x-axis, towards right hand side of the x-axis, Figure 12). The change of miRNA levels in plasma is due to heart failure (HFREF and/or HFPEF). These observations demonstrate that many pairs of miRNAs were regulated similarly among all subjects. As a result, a panel of miRNAs could be assembled by substituting one or more specific miRNAs with another to systematically optimize diagnostic performance. All the significantly altered miRNAs were critical for the development of a multivariate index diagnostic assay for heart failure detection or heart failure subtype categorization.

**IV. Plasma miRNA as prognostic markers**

At their index admission when recruited to the SHOP cohort study, heart failure patients were sampled after treatment for 3-5 days when symptomatically improved, with resolution of bedside physical signs of HF, and considered fit for discharge. This ensured assessment of marker performance in this study is relevant to the sub-acute or "chronic" phase of HF. The present study assessed the prognostic performance of circulating miRNAs for mortality and heart failure re-hospitalization. 327 of the heart failure patients (176 HFREF and 151 HFPEF) were followed-up for a period of 2 years (Table 10) during which 49 died (15%).

**Table 10. Clinical information of the subjects included in the prognosis study**

| Type | Death or last follow-up (days) | Death | HF related hospitalization or death or last follow-up (days) | HF related hospitalization |
|------|------|------|------|------|
| REF | 650 | Yes | 650 | |
| PEF | 804 | | 804 | Yes |
| PEF | 776 | | 776 | Yes |
| REF | 989 | | 989 | |
| REF | 763 | | 763 | Yes |
| PEF | 664 | | 664 | |
| PEF | 650 | | 650 | Yes |
| REF | 672 | | 672 | Yes |
| REF | 678 | | 678 | |
| REF | 42 | | 42 | |
| REF | 318 | Yes | 318 | |
| REF | 739 | | 739 | Yes |
| REF | 722 | | 722 | Yes |
| REF | 738 | | 738 | |
| REF | 412 | Yes | 412 | Yes |
| REF | 730 | | 730 | |
| REF | 728 | | 728 | Yes |
| PEF | 734 | | 734 | Yes |
| REF | 728 | | 728 | Yes |
| PEF | 372 | | 372 | |
| PEF | 186 | | 186 | |
| REF | 730 | | 730 | Yes |
| REF | 731 | | 731 | |
| REF | 731 | | 731 | |
| REF | 731 | | 731 | Yes |
| REF | 391 | Yes | 391 | Yes |
| REF | 731 | | 731 | Yes |
| REF | 731 | | 731 | |
| REF | 249 | Yes | 249 | Yes |
| REF | 673 | Yes | 673 | Yes |
| REF | 219 | Yes | 219 | |
| REF | 731 | | 731 | Yes |
| REF | 731 | | 731 | |
| REF | 376 | Yes | 376 | Yes |
| REF | 731 | | 731 | |
| REF | 738 | | 738 | Yes |
| REF | 175 | | 175 | |

(continued)

| Type | Death or last follow-up (days) | Death | HF related hospitalization or death or last follow-up (days) | HF related hospitalization |
|---|---|---|---|---|
| PEF | 731 | | 731 | |
| PEF | 731 | | 731 | Yes |
| PEF | 365 | | 365 | Yes |
| PEF | 733 | | 733 | |
| REF | 263 | | 263 | Yes |
| REF | 411 | | 411 | Yes |
| REF | 365 | | 365 | |
| REF | 196 | | 196 | |
| PEF | 717 | | 717 | |
| PEF | 183 | | 183 | |
| PEF | 708 | | 708 | Yes |
| PEF | 706 | | 706 | |
| PEF | 13 | Yes | 13 | |
| PEF | 712 | | 712 | |
| PEF | 736 | | 736 | Yes |
| REF | 731 | | 731 | |
| PEF | 724 | | 724 | Yes |
| PEF | 735 | | 735 | |
| PEF | 404 | | 404 | |
| PEF | 125 | | 125 | |
| PEF | 41 | | 41 | |
| REF | 46 | Yes | 46 | Yes |
| REF | 762 | | 762 | |
| PEF | 713 | | 713 | |
| REF | 441 | Yes | 441 | Yes |
| REF | 715 | | 715 | Yes |
| REF | 52 | Yes | 52 | |
| REF | 773 | | 773 | |
| PEF | 725 | | 725 | Yes |
| REF | 72 | Yes | 72 | |
| REF | 260 | Yes | 260 | |
| REF | 693 | Yes | 693 | Yes |
| PEF | 361 | | 361 | |
| REF | 371 | | 371 | |
| PEF | 361 | | 361 | Yes |
| PEF | 358 | | 358 | |
| REF | 731 | | 731 | |

(continued)

| Type | Death or last follow-up (days) | Death | HF related hospitalization or death or last follow-up (days) | HF related hospitalization |
|------|-------------------------------|-------|------------------------------------------------------------|----------------------------|
| PEF | 742 | | 742 | Yes |
| PEF | 174 | | 174 | |
| PEF | 179 | | 179 | Yes |
| PEF | 731 | | 731 | Yes |
| REF | 434 | Yes | 434 | Yes |
| PEF | 53 | | 53 | Yes |
| PEF | 742 | | 742 | |
| REF | 749 | Yes | 749 | Yes |
| PEF | 733 | | 733 | |
| PEF | 379 | | 379 | Yes |
| PEF | 365 | | 365 | |
| PEF | 1 | | 1 | |
| REF | 353 | | 353 | |
| REF | 731 | | 731 | Yes |
| REF | 365 | | 365 | |
| REF | 768 | | 768 | |
| PEF | 723 | | 723 | |
| REF | 24 | Yes | 24 | |
| PEF | 731 | | 731 | Yes |
| REF | 370 | | 370 | |
| REF | 665 | | 665 | Yes |
| PEF | 468 | | 468 | Yes |
| REF | 40 | | 40 | |
| PEF | 707 | | 707 | |
| PEF | 58 | | 58 | Yes |
| PEF | 723 | | 723 | |
| PEF | 70 | Yes | 70 | |
| REF | 725 | | 725 | |
| REF | 372 | | 372 | |
| PEF | 391 | | 391 | |
| PEF | 734 | | 734 | |
| PEF | 353 | | 353 | Yes |
| REF | 392 | | 392 | |
| PEF | 56 | Yes | 56 | |
| PEF | 739 | | 739 | Yes |
| PEF | 378 | | 378 | |
| PEF | 354 | | 354 | |

(continued)

| Type | Death or last follow-up (days) | Death | HF related hospitalization or death or last follow-up (days) | HF related hospitalization |
|------|-------------------------------|-------|-------------------------------------------------------------|----------------------------|
| REF | 733 | | 733 | |
| REF | 665 | | 665 | |
| REF | 253 | | 253 | |
| REF | 707 | | 707 | Yes |
| REF | 70 | Yes | 70 | Yes |
| PEF | 382 | | 382 | Yes |
| PEF | 365 | | 365 | |
| REF | 2 | | 2 | |
| PEF | 183 | Yes | 183 | |
| REF | 732 | | 732 | |
| PEF | 365 | | 365 | Yes |
| PEF | 721 | | 721 | |
| REF | 740 | | 740 | |
| PEF | 343 | | 343 | Yes |
| REF | 348 | | 348 | |
| REF | 732 | | 732 | Yes |
| PEF | 166 | | 166 | |
| REF | 365 | | 365 | |
| PEF | 200 | Yes | 200 | Yes |
| PEF | 190 | | 190 | |
| PEF | 362 | | 362 | |
| REF | 393 | | 393 | |
| REF | 1 | Yes | 1 | |
| REF | 811 | | 811 | |
| REF | 665 | | 665 | |
| REF | 911 | Yes | 911 | Yes |
| REF | 734 | | 734 | |
| PEF | 750 | | 750 | |
| PEF | 593 | Yes | 593 | |
| PEF | 362 | | 362 | Yes |
| PEF | 506 | Yes | 506 | |
| REF | 348 | | 348 | |
| REF | 734 | | 734 | |
| PEF | 365 | | 365 | Yes |
| PEF | 68 | | 68 | |
| PEF | 370 | | 370 | |
| PEF | 78 | | 78 | |

(continued)

| Type | Death or last follow-up (days) | Death | HF related hospitalization or death or last follow-up (days) | HF related hospitalization |
|------|-------------------------------|-------|-------------------------------------------------------------|----------------------------|
| REF | 752 | | 752 | |
| REF | 378 | | 378 | |
| REF | 53 | | 53 | |
| PEF | 734 | | 734 | |
| REF | 353 | | 353 | Yes |
| REF | 730 | | 730 | |
| REF | 728 | | 728 | |
| PEF | 721 | | 721 | Yes |
| PEF | 394 | | 394 | Yes |
| PEF | 727 | | 727 | Yes |
| PEF | 728 | | 728 | |
| PEF | 920 | | 920 | |
| REF | 732 | | 732 | Yes |
| REF | 748 | | 748 | |
| REF | 672 | | 672 | Yes |
| PEF | 745 | | 745 | |
| REF | 747 | | 747 | |
| PEF | 358 | | 358 | |
| REF | 747 | | 747 | |
| PEF | 97 | | 97 | |
| REF | 4 | | 4 | |
| PEF | 188 | | 188 | |
| PEF | 731 | | 731 | |
| REF | 835 | | 835 | |
| REF | 729 | | 729 | |
| PEF | 729 | | 729 | |
| REF | 674 | | 674 | |
| PEF | 172 | | 172 | |
| PEF | 666 | | 666 | |
| REF | 731 | | 731 | |
| PEF | 274 | Yes | 274 | |
| PEF | 374 | | 374 | |
| REF | 351 | Yes | 351 | Yes |
| REF | 966 | | 966 | |
| PEF | 722 | | 722 | Yes |
| PEF | 730 | | 730 | |
| PEF | 734 | | 734 | Yes |

(continued)

| Type | Death or last follow-up (days) | Death | HF related hospitalization or death or last follow-up (days) | HF related hospitalization |
|------|-------------------------------|-------|-------------------------------------------------------------|----------------------------|
| REF | 686 | | 686 | |
| REF | 595 | Yes | 595 | |
| REF | 368 | | 368 | Yes |
| PEF | 731 | | 731 | |
| PEF | 365 | | 365 | Yes |
| REF | 741 | | 741 | Yes |
| REF | 388 | | 388 | Yes |
| PEF | 49 | | 49 | |
| REF | 365 | | 365 | |
| REF | 385 | | 385 | |
| PEF | 672 | | 672 | |
| PEF | 731 | | 731 | |
| PEF | 741 | | 741 | |
| PEF | 343 | | 343 | |
| REF | 740 | | 740 | |
| REF | 672 | | 672 | |
| PEF | 364 | | 364 | |
| PEF | 743 | | 743 | Yes |
| REF | 398 | | 398 | |
| PEF | 668 | | 668 | |
| REF | 720 | | 720 | |
| PEF | 731 | | 731 | Yes |
| PEF | 193 | Yes | 193 | |
| REF | 365 | | 365 | |
| PEF | 726 | | 726 | |
| PEF | 365 | | 365 | |
| REF | 448 | Yes | 448 | Yes |
| PEF | 123 | Yes | 123 | |
| PEF | 752 | | 752 | Yes |
| PEF | 399 | | 399 | Yes |
| PEF | 657 | | 657 | |
| PEF | 770 | | 770 | |
| PEF | 357 | | 357 | |
| REF | 761 | | 761 | |
| PEF | 372 | | 372 | |
| REF | 366 | | 366 | Yes |
| REF | 730 | | 730 | |

(continued)

| Type | Death or last follow-up (days) | Death | HF related hospitalization or death or last follow-up (days) | HF related hospitalization |
|------|-------------------------------|-------|------------------------------------------------------------|----------------------------|
| REF | 766 | | 766 | |
| REF | 658 | Yes | 658 | Yes |
| REF | 736 | | 736 | |
| REF | 372 | | 372 | Yes |
| PEF | 334 | | 334 | |
| REF | 49 | | 49 | |
| REF | 730 | | 730 | Yes |
| REF | 730 | | 730 | Yes |
| PEF | 360 | | 360 | |
| REF | 672 | | 672 | |
| REF | 338 | | 338 | |
| REF | 196 | | 196 | |
| PEF | 322 | | 322 | Yes |
| PEF | 731 | | 731 | |
| REF | 730 | | 730 | Yes |
| REF | 701 | | 701 | |
| REF | 364 | | 364 | |
| PEF | 742 | | 742 | |
| PEF | 365 | | 365 | |
| REF | 336 | | 336 | |
| REF | 715 | | 715 | Yes |
| REF | 747 | | 747 | |
| REF | 29 | Yes | 29 | |
| REF | 738 | | 738 | |
| REF | 739 | | 739 | Yes |
| REF | 365 | | 365 | |
| REF | 742 | | 742 | |
| PEF | 208 | Yes | 208 | Yes |
| REF | 440 | Yes | 440 | |
| PEF | 731 | | 731 | Yes |
| REF | 636 | Yes | 636 | |
| REF | 741 | | 741 | |
| PEF | 723 | | 723 | Yes |
| PEF | 367 | | 367 | Yes |
| PEF | 735 | | 735 | |
| REF | 730 | | 730 | Yes |
| REF | 366 | | 366 | |

(continued)

| Type | Death or last follow-up (days) | Death | HF related hospitalization or death or last follow-up (days) | HF related hospitalization |
|------|------|------|------|------|
| PEF | 728 | | 728 | |
| REF | 730 | | 730 | Yes |
| REF | 731 | | 731 | Yes |
| REF | 731 | | 731 | Yes |
| REF | 730 | | 730 | |
| REF | 165 | | 165 | Yes |
| PEF | 728 | | 728 | |
| PEF | 939 | | 939 | |
| REF | 674 | | 674 | |
| REF | 379 | | 379 | Yes |
| PEF | 41 | | 41 | |
| REF | 92 | | 92 | |
| REF | 125 | Yes | 125 | Yes |
| REF | 367 | | 367 | |
| PEF | 862 | | 862 | Yes |
| REF | 765 | | 765 | |
| PEF | 732 | | 732 | Yes |
| REF | 393 | | 393 | |
| REF | 731 | | 731 | |
| REF | 753 | | 753 | |
| PEF | 723 | | 723 | |
| REF | 739 | | 739 | |
| PEF | 744 | | 744 | |
| REF | 13 | Yes | 13 | |
| REF | 730 | | 730 | |
| PEF | 379 | | 379 | Yes |
| REF | 715 | | 715 | Yes |
| PEF | 377 | | 377 | |
| REF | 365 | | 365 | |
| PEF | 239 | Yes | 239 | |
| REF | 183 | | 183 | |
| PEF | 25 | Yes | 25 | |
| REF | 714 | | 714 | Yes |
| REF | 729 | | 729 | Yes |
| PEF | 357 | | 357 | |
| REF | 723 | | 723 | Yes |
| PEF | 725 | | 725 | |

(continued)

| Type | Death or last follow-up (days) | Death | HF related hospitalization or death or last follow-up (days) | HF related hospitalization |
|---|---|---|---|---|
| PEF | 335 | | 335 | |
| PEF | 457 | Yes | 457 | |
| PEF | 390 | | 390 | |
| PEF | 726 | | 726 | |
| REF | 404 | | 404 | Yes |
| PEF | 171 | | 171 | |
| REF | 270 | Yes | 270 | Yes |
| REF | 357 | | 357 | |
| REF | 99 | Yes | 99 | |
| REF | 732 | | 732 | Yes |
| REF | 365 | | 365 | |
| REF | 739 | | 739 | Yes |
| PEF | 730 | | 730 | Yes |
| REF | 168 | | 168 | |
| PEF | 367 | | 367 | |
| PEF | 334 | | 334 | |
| PEF | 377 | | 377 | Yes |
| PEF | 361 | Yes | 361 | Yes |
| PEF | 771 | | 771 | |
| REF | 484 | Yes | 484 | Yes |
| REF | 190 | | 190 | |
| REF | 672 | | 672 | Yes |
| PEF | 766 | | 766 | |
| REF | 365 | | 365 | |
| REF | 389 | | 389 | Yes |
| REF | 381 | | 381 | Yes |
| PEF | 357 | | 357 | |
| PEF | 711 | | 711 | Yes |
| PEF | 743 | | 743 | Yes |
| REF | 7 | Yes | 7 | |
| PEF | 34 | Yes | 34 | |

Among all the study cases, 115 were re-hospitalized because of heart failure during the follow-up (Table 10) and 49 died. MiRNAs were assessed as potential markers (ie predictors) of both observed (all-cause survival) OS and event free survival (EFS) the composite of all-cause death and/or recurrent admission for decompensated heart failure.

Anti- heart failure pharmacotherapy prescribed to study participants is summarized in Table 11. Comparing the treatments for HFREF and HFPEF, the frequency of prescription of half the drugs concerned were found to differ (Figure 13). Notably those classes of drugs proven to improve prognosis in HFREF (ACEI's/ARB's, beta blockers and mineralocorticoid antagonists) were more commonly prescribed to HFREF than HFPEF patients. Treatments were according to current clinical practice and were included among clinical variables for the analysis of prognostic markers.

**Table 11. Treatment of subjects included in the prognosis study**

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 1 | Yes | Yes | Yes | Yes | Yes | Yes | | Yes | Yes | | Yes | | | | |
| 2 | | Yes | Yes | Yes | Yes | Yes | | | Yes | Yes | | | | | Yes |
| 3 | Yes | | Yes | Yes | | Yes | Yes | Yes | Yes | | | | | | Yes |
| 4 | Yes | | Yes | Yes | Yes | Yes | | | | | Yes | | Yes | | |
| 5 | | | Yes | Yes | | Yes | Yes | | | | Yes | | | | Yes |
| 6 | | | Yes | Yes | Yes | Yes | | Yes | Yes | | | | Yes | | |
| 7 | Yes | | Yes | Yes | Yes | Yes | Yes | Yes | | | | Yes | Yes | | |
| 8 | Yes | Yes | Yes | Yes | Yes | Yes | Yes | | Yes | | Yes | | Yes | | |
| 9 | Yes | | Yes | Yes | Yes | Yes | Yes | Yes | Yes | | Yes | | Yes | | |
| 10 | | | Yes | Yes | Yes | Yes | Yes | Yes | Yes | | | | Yes | | |
| 11 | Yes | Yes | Yes | Yes | Yes | Yes | Yes | Yes | | | Yes | | | | |
| 12 | Yes | | Yes | Yes | Yes | Yes | Yes | Yes | Yes | | Yes | | Yes | | |
| 13 | Yes | | Yes | Yes | Yes | Yes | Yes | Yes | | | Yes | | | | |
| 14 | | Yes | Yes | Yes | Yes | Yes | Yes | | Yes | | | | | | |
| 15 | Yes | | Yes | Yes | Yes | Yes | Yes | Yes | | | Yes | | Yes | | Yes |
| 16 | Yes | | Yes | Yes | Yes | Yes | Yes | | Yes | | Yes | | | | |
| 17 | Yes | | | Yes | Yes | Yes | Yes | | | | Yes | | | | |
| 18 | | Yes | Yes | Yes | | Yes | | | | Yes | | | | | |
| 19 | | Yes | Yes | Yes | Yes | Yes | Yes | | | Yes | Yes | | Yes | | |
| 20 | | Yes | | Yes | Yes | Yes | | | | | | | | | |
| 21 | Yes | | Yes | Yes | Yes | Yes | | | Yes | | | | | | |
| 22 | Yes | Yes | Yes | Yes | Yes | Yes | | | Yes | | Yes | | Yes | | |
| 23 | | Yes | Yes | Yes | Yes | Yes | | | Yes | Yes | Yes | | | | |
| 24 | Yes | | Yes | Yes | Yes | Yes | | | | Yes | Yes | | | | |
| 25 | Yes | | Yes | Yes | Yes | Yes | | | | Yes | | | Yes | | |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 26 | Yes | | Yes | Yes | Yes | Yes | | | | Yes | Yes | | | | |
| 27 | | | Yes | Yes | Yes | Yes | | | Yes | Yes | Yes | | Yes | | Yes |
| 28 | Yes | | Yes | Yes | Yes | Yes | | | | | Yes | | | | |
| 29 | Yes | | Yes | Yes | Yes | Yes | | | | | Yes | | Yes | | |
| 30 | | | Yes | Yes | Yes | | Yes | | | | | | | | Yes |
| 31 | Yes | | Yes | Yes | Yes | Yes | Yes | | Yes | Yes | Yes | | | | |
| 32 | Yes | | Yes | Yes | Yes | Yes | | | Yes | Yes | Yes | | Yes | Yes | |
| 33 | Yes | | Yes | Yes | Yes | Yes | Yes | | Yes | | Yes | | Yes | | |
| 34 | Yes | | Yes | Yes | Yes | Yes | | | | | Yes | | Yes | | Yes |
| 35 | Yes | | Yes | Yes | Yes | Yes | | | | | | | | | |
| 36 | | | Yes | Yes | Yes | Yes | | | | | Yes | | Yes | | |
| 37 | Yes | | Yes | Yes | Yes | Yes | | | | | | | Yes | | |
| 38 | | | Yes | Yes | | Yes | | | | | | | | | |
| 39 | | Yes | Yes | Yes | Yes | Yes | Yes | Yes | | Yes | | | Yes | | Yes |
| 40 | | | Yes | Yes | Yes | Yes | | | Yes | | | | Yes | | |
| 41 | | Yes | Yes | Yes | Yes | Yes | | Yes | | | | | Yes | | |
| 42 | | Yes | Yes | Yes | | Yes | | Yes | | | Yes | | Yes | | |
| 43 | Yes | Yes | Yes | Yes | Yes | Yes | Yes | | Yes | | Yes | | | | |
| 44 | Yes | | Yes | Yes | Yes | Yes | Yes | | Yes | | Yes | | | | |
| 45 | Yes | Yes | Yes | Yes | Yes | | | | Yes | Yes | Yes | | Yes | | |
| 46 | Yes | | Yes | Yes | Yes | Yes | | | | Yes | | Yes | Yes | | |
| 47 | | | Yes | | Yes | Yes | | | | Yes | | | Yes | Yes | |
| 48 | Yes | | Yes | Yes | | | | | Yes | | | | Yes | Yes | Yes |
| 49 | | | Yes | | | | | | | | | | | | |
| 50 | | | Yes | Yes | | | | | | | | | | Yes | |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 51 | | Yes | | | Yes | | | Yes | | Yes | | | Yes | | |
| 52 | Yes | | Yes | Yes | Yes | Yes | Yes | | Yes | Yes | | | | Yes | Yes |
| 53 | Yes | Yes | | Yes | Yes | Yes | | | | | | | Yes | | Yes |
| 54 | Yes | Yes | Yes | | | Yes | Yes | Yes | Yes | | Yes | | | | |
| 55 | | Yes | Yes | Yes | Yes | Yes | | | Yes | Yes | | | Yes | Yes | |
| 56 | Yes | Yes | Yes | Yes | Yes | Yes | | | | Yes | | | Yes | | Yes |
| 57 | | | Yes | Yes | Yes | | Yes | | Yes | Yes | Yes | | | | |
| 58 | Yes | Yes | Yes | Yes | | Yes | Yes | | | | | | Yes | | |
| 59 | | | Yes | Yes | Yes | Yes | | | | | | | | | |
| 60 | | Yes | Yes | Yes | Yes | Yes | | | | | | | | | |
| 61 | | Yes | Yes | Yes | | Yes | | | | | | | Yes | | |
| 62 | Yes | Yes | Yes | | Yes | Yes | Yes | | Yes | | Yes | | Yes | Yes | Yes |
| 63 | Yes | | Yes | Yes | Yes | Yes | | | Yes | Yes | Yes | | Yes | | |
| 64 | Yes | | Yes | Yes | Yes | Yes | | | Yes | Yes | Yes | | Yes | | Yes |
| 65 | | Yes | Yes | Yes | | Yes | Yes | | | | Yes | | Yes | | Yes |
| 66 | | Yes | Yes | Yes | Yes | Yes | | | Yes | | | Yes | Yes | | |
| 67 | | | Yes | Yes | Yes | Yes | | | Yes | | | | Yes | Yes | |
| 68 | | Yes | Yes | Yes | Yes | Yes | | | | | | Yes | Yes | Yes | Yes |
| 69 | Yes | | Yes | | Yes | Yes | | | Yes | Yes | | | Yes | | Yes |
| 70 | Yes | | Yes | Yes | Yes | Yes | | | Yes | Yes | | | Yes | | |
| 71 | Yes | | | Yes | | Yes | Yes | Yes | Yes | | | | | | |
| 72 | Yes | | Yes | Yes | | Yes | | | | Yes | Yes | Yes | Yes | | Yes |
| 73 | Yes | | Yes | | Yes | Yes | | | Yes | | | | Yes | | |
| 74 | Yes | Yes | Yes | Yes | Yes | Yes | | | | Yes | | | | Yes | |
| 75 | | | Yes | Yes | Yes | Yes | | | | Yes | | | | | |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 76 | Yes | | Yes | | Yes | | | | Yes | | | | Yes | | |
| 77 | | | Yes | Yes | Yes | Yes | | | Yes | Yes | | | Yes | Yes | Yes |
| 78 | Yes | Yes | Yes | Yes | | Yes | | | | | Yes | | Yes | | Yes |
| 79 | Yes | | Yes | Yes | Yes | Yes | Yes | | | | | Yes | Yes | | |
| 80 | Yes | | Yes | Yes | Yes | Yes | Yes | | Yes | | Yes | | Yes | | Yes |
| 81 | Yes | | Yes | Yes | | Yes | Yes | | Yes | Yes | Yes | | | | |
| 82 | Yes | Yes | Yes | Yes | Yes | Yes | | Yes | | | Yes | Yes | Yes | | Yes |
| 83 | | | | | Yes | | Yes | | | | | | | | |
| 84 | Yes | Yes | Yes | Yes | Yes | Yes | | | | Yes | | | Yes | | |
| 85 | | | Yes | Yes | Yes | Yes | | | | | | | Yes | | |
| 86 | Yes | | | Yes | | Yes | | | | | | | Yes | | Yes |
| 87 | Yes | Yes | Yes | Yes | Yes | Yes | | | | | | | | | |
| 88 | | | Yes | Yes | Yes | Yes | Yes | | Yes | | Yes | | Yes | | |
| 89 | Yes | | Yes | Yes | Yes | Yes | | | Yes | | Yes | | | | |
| 90 | Yes | | | Yes | Yes | Yes | | | Yes | | Yes | | Yes | | |
| 91 | Yes | | Yes | Yes | Yes | Yes | | | | | Yes | Yes | Yes | | |
| 92 | Yes | | Yes | Yes | Yes | Yes | | | | | | | Yes | | |
| 93 | Yes | | Yes | Yes | Yes | Yes | | | | | Yes | | Yes | | Yes |
| 94 | Yes | | Yes | Yes | | | | | Yes | | Yes | | Yes | | |
| 95 | Yes | | Yes | | Yes | Yes | | | | | Yes | | | | |
| 96 | Yes | | Yes | Yes | Yes | Yes | | | | | Yes | | | | |
| 97 | | Yes | Yes | Yes | Yes | | | Yes | | | | | Yes | | |
| 98 | | Yes | Yes | Yes | Yes | Yes | | | | Yes | | | Yes | | |
| 99 | | Yes | | Yes | Yes | Yes | Yes | | | Yes | | | | | |
| 100 | | | Yes | Yes | Yes | Yes | Yes | | | | | | | | Yes |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 101 | | | | | | Yes | | Yes | Yes | | | | | | |
| 102 | | Yes | Yes | Yes | Yes | Yes | Yes | | | Yes | | | Yes | | |
| 103 | | Yes | Yes | Yes | Yes | Yes | | | Yes | | Yes | | Yes | | Yes |
| 104 | Yes | Yes | Yes | Yes | | | Yes | | | Yes | Yes | | | | Yes |
| 105 | | Yes | Yes | Yes | Yes | Yes | | | Yes | Yes | | | | | |
| 106 | | Yes | Yes | Yes | Yes | Yes | | | | | | | Yes | | |
| 107 | Yes | | Yes | Yes | Yes | Yes | | | Yes | | Yes | | Yes | | |
| 108 | | Yes | Yes | Yes | Yes | Yes | | | Yes | Yes | | | | | |
| 109 | Yes | | Yes | Yes | Yes | Yes | Yes | Yes | Yes | Yes | | | Yes | Yes | |
| 110 | Yes | | Yes | Yes | | Yes | | Yes | Yes | | | | | | |
| 111 | Yes | | Yes | Yes | Yes | Yes | Yes | Yes | Yes | | | | Yes | | |
| 112 | Yes | | Yes | Yes | | Yes | | Yes | | | Yes | | Yes | | |
| 113 | Yes | | | Yes | Yes | Yes | Yes | Yes | | | | | Yes | | |
| 114 | | | | Yes | Yes | Yes | Yes | | Yes | | | | Yes | | |
| 115 | Yes | | Yes | Yes | Yes | Yes | | Yes | Yes | | Yes | | Yes | | |
| 116 | Yes | | Yes | Yes | | Yes | | | Yes | Yes | | | | | |
| 117 | Yes | | Yes | Yes | Yes | Yes | Yes | | Yes | Yes | Yes | | Yes | | |
| 118 | | Yes | Yes | Yes | Yes | Yes | | | Yes | | | | Yes | | |
| 119 | | | Yes | Yes | Yes | Yes | | | Yes | | Yes | | Yes | | |
| 120 | Yes | | Yes | Yes | Yes | Yes | Yes | Yes | Yes | Yes | | | | | |
| 121 | Yes | | Yes | Yes | | Yes | | | Yes | Yes | | | | | |
| 122 | | | Yes | | | Yes | | | | | Yes | | | Yes | Yes |
| 123 | | Yes | Yes | | | Yes | | | | Yes | | | | | |
| 124 | | Yes | Yes | Yes | | Yes | | | | | Yes | | | | |
| 125 | | | Yes | Yes | Yes | Yes | | | | Yes | | | | | |

88

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 126 | | Yes | Yes | Yes | Yes | Yes | | | Yes | | Yes | | Yes | | Yes |
| 127 | Yes | | Yes | Yes | Yes | Yes | | | Yes | | Yes | Yes | | | |
| 128 | Yes | | Yes | Yes | | Yes | | | | | | | | | |
| 129 | | | Yes | Yes | | | Yes | Yes | | Yes | | | Yes | | Yes |
| 130 | | Yes | | Yes | Yes | Yes | | | Yes | | | | Yes | | Yes |
| 131 | | Yes | Yes | Yes | Yes | | | | | | | | | | |
| 132 | Yes | Yes | Yes | Yes | Yes | Yes | | | | | Yes | | Yes | | Yes |
| 133 | | | Yes | Yes | | | | | | | Yes | | | | |
| 134 | | | | Yes | | | | | | | | | | | |
| 135 | Yes | | Yes | Yes | Yes | Yes | | | | | | | Yes | | |
| 136 | | Yes | | Yes | Yes | Yes | | | Yes | | | Yes | Yes | | |
| 137 | | Yes | Yes | Yes | Yes | Yes | | | Yes | Yes | Yes | | Yes | | Yes |
| 138 | Yes | | Yes | Yes | Yes | Yes | | | | | Yes | | | | |
| 139 | Yes | | Yes | Yes | Yes | Yes | | | Yes | | | | Yes | | |
| 140 | | | Yes | Yes | Yes | Yes | | | Yes | | | | Yes | | |
| 141 | | Yes | Yes | Yes | | | | | | | | | | | |
| 142 | Yes | Yes | Yes | | Yes | Yes | | | Yes | | | | Yes | | Yes |
| 143 | Yes | | Yes | Yes | Yes | Yes | | | | | Yes | | | | |
| 144 | Yes | | Yes | Yes | | Yes | | | | | Yes | | | | |
| 145 | Yes | | Yes | Yes | Yes | Yes | | Yes | Yes | | Yes | | | Yes | Yes |
| 146 | | | Yes | | Yes | Yes | | | | Yes | | | | | |
| 147 | Yes | Yes | Yes | Yes | Yes | Yes | | | | Yes | | | Yes | Yes | Yes |
| 148 | Yes | Yes | Yes | | Yes | Yes | | | | Yes | | | | Yes | |
| 149 | | Yes | Yes | Yes | | Yes | | | | | | | | | |
| 150 | Yes | | Yes | Yes | Yes | Yes | | | | | | | Yes | | |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 151 | Yes | | | Yes | Yes | Yes | | | | | | | | | |
| 152 | Yes | | Yes | Yes | Yes | Yes | | | Yes | | | Yes | Yes | | |
| 153 | | Yes | | Yes | | | Yes | | | | Yes | | | | |
| 154 | Yes | | Yes | Yes | Yes | Yes | | | | | | | Yes | | |
| 155 | Yes | Yes | Yes | Yes | Yes | | Yes | | Yes | Yes | Yes | | | | Yes |
| 156 | Yes | Yes | Yes | Yes | Yes | Yes | | | Yes | Yes | Yes | | Yes | Yes | |
| 157 | | | Yes | Yes | Yes | Yes | | | Yes | | | | Yes | | Yes |
| 158 | | Yes | Yes | Yes | Yes | Yes | | | Yes | Yes | | | | | |
| 159 | Yes | | Yes | Yes | Yes | Yes | | Yes | Yes | Yes | Yes | Yes | Yes | | Yes |
| 160 | | | Yes | Yes | Yes | Yes | | Yes | | | | | | | |
| 161 | Yes | Yes | Yes | Yes | Yes | Yes | Yes | | Yes | | Yes | | Yes | | Yes |
| 162 | | Yes | Yes | Yes | Yes | Yes | | | | | Yes | | | | |
| 163 | Yes | | Yes | Yes | Yes | Yes | Yes | | Yes | Yes | | | Yes | | Yes |
| 164 | | | | Yes | Yes | Yes | Yes | | | | | Yes | | | |
| 165 | | Yes | Yes | Yes | Yes | Yes | Yes | | | | | | | | Yes |
| 166 | Yes | | | | | Yes | | | | Yes | | | | | Yes |
| 167 | Yes | Yes | Yes | Yes | Yes | Yes | | | Yes | | Yes | | Yes | | |
| 168 | | Yes | Yes | | Yes | Yes | | Yes | Yes | | Yes | | Yes | | |
| 169 | Yes | | Yes | Yes | Yes | Yes | Yes | | Yes | Yes | | | | | |
| 170 | | Yes | Yes | Yes | Yes | Yes | | | | | Yes | | | | |
| 171 | Yes | | Yes | Yes | | Yes | Yes | | Yes | | | | | Yes | |
| 172 | Yes | Yes | Yes | Yes | Yes | | | | | | Yes | | | | |
| 173 | Yes | | Yes | | | Yes | | | | | Yes | | Yes | | |
| 174 | | Yes | Yes | Yes | Yes | Yes | | | | Yes | | | Yes | | Yes |
| 175 | Yes | Yes | Yes | Yes | Yes | Yes | | | | Yes | | | Yes | | |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 176 | | | | Yes | | Yes | | | | Yes | | | Yes | | |
| 177 | Yes | | Yes | Yes | Yes | Yes | | | | Yes | Yes | | | | |
| 178 | Yes | | Yes | Yes | Yes | | Yes | | | Yes | | | | | |
| 179 | Yes | Yes | Yes | Yes | Yes | Yes | | | Yes | Yes | | | Yes | Yes | |
| 180 | Yes | | Yes | Yes | Yes | Yes | | | Yes | Yes | Yes | | Yes | | |
| 181 | | | | Yes | Yes | Yes | | | Yes | Yes | | | | Yes | |
| 182 | Yes | | Yes | Yes | | Yes | | Ye s | | | Yes | | Yes | | |
| 183 | Yes | | Yes | Yes | | | Yes | | | | Yes | | | | |
| 184 | | Yes | Yes | Yes | Yes | Yes | | | Yes | Yes | | | | | |
| 185 | | | Yes | Yes | Yes | Yes | | Ye s | Yes | Yes | | | Yes | Yes | Yes |
| 186 | Yes | | Yes | Yes | | | | | | | | | Yes | | |
| 187 | Yes | | Yes | Yes | Yes | Yes | Yes | | Yes | | Yes | | Yes | | |
| 188 | Yes | | Yes | Yes | Yes | Yes | | | | | Yes | | Yes | | |
| 189 | Yes | Yes | Yes | Yes | Yes | Yes | | | | Yes | | Yes | Yes | | |
| 190 | | | | Yes | Yes | Yes | Yes | | Yes | | | | Yes | | Yes |
| 191 | Yes | Yes | Yes | Yes | Yes | Yes | | | | Yes | Yes | | | | |
| 192 | | Yes | Yes | Yes | | Yes | | | | | Yes | | Yes | | |
| 193 | | Yes | Yes | Yes | | Yes | | | | | | | Yes | | Yes |
| 194 | | Yes | Yes | Yes | | Yes | Yes | | Yes | | | | | | |
| 195 | Yes | | Yes | | Yes | | | | Yes | | Yes | Yes | Yes | Yes | Yes |
| 196 | Yes | | | Yes | Yes | Yes | | | | Yes | | | | | Yes |
| 197 | | | Yes | Yes | | Yes | | | | | | | | | |
| 198 | Yes | | Yes | Yes | Yes | Yes | Yes | | Yes | | Yes | | | | Yes |
| 199 | | Yes | Yes | Yes | | Yes | Yes | Ye s | | | | | | | |
| 200 | Yes | | Yes | Yes | Yes | Yes | | Ye s | | | | | Yes | | |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 201 | | Yes | | | Yes | | | | | | Yes | | | | |
| 202 | Yes | | Yes | Yes | Yes | Yes | | | Yes | | | | | Yes | |
| 203 | Yes | | Yes | Yes | Yes | Yes | | Ye s | Yes | Yes | | | Yes | Yes | |
| 204 | Yes | | Yes | Yes | Yes | Yes | | | Yes | | Yes | | Yes | | |
| 205 | Yes | | | Yes | Yes | Yes | Yes | | | | Yes | | | | |
| 206 | Yes | | Yes | Yes | | Yes | | Ye s | | | Yes | | | | |
| 207 | Yes | | Yes | Yes | Yes | Yes | | | Yes | | | | Yes | | |
| 208 | Yes | | Yes | | Yes | Yes | | | | | | | | | |
| 209 | Yes | | Yes | Yes | | Yes | | | | | Yes | | | | |
| 210 | Yes | | Yes | Yes | | | | | | Yes | | | Yes | | Yes |
| 211 | Yes | | | Yes | | Yes | | | | | | | Yes | | Yes |
| 212 | Yes | | Yes | | Yes | Yes | | | Yes | Yes | | | | | |
| 213 | | | Yes | Yes | Yes | Yes | | | | Yes | | | | | |
| 214 | | Yes | Yes | Yes | | Yes | | | Yes | Yes | | | Yes | | |
| 215 | Yes | Yes | | Yes | Yes | Yes | | | Yes | | | | | | |
| 216 | Yes | Yes | Yes | Yes | Yes | Yes | | | Yes | Yes | | | Yes | | Yes |
| 217 | Yes | Yes | Yes | Yes | Yes | Yes | Yes | | | Yes | | | Yes | Yes | Yes |
| 218 | Yes | Yes | Yes | Yes | Yes | Yes | | | Yes | Yes | | | Yes | | Yes |
| 219 | Yes | | Yes | Yes | Yes | Yes | Yes | Ye s | Yes | | | | Yes | | |
| 220 | Yes | | Yes | Yes | | Yes | Yes | Ye s | | | | Yes | Yes | | Yes |
| 221 | | | Yes | Yes | Yes | Yes | Yes | | Yes | | Yes | | Yes | | |
| 222 | Yes | | Yes | Yes | Yes | Yes | | | | | Yes | | | | |
| 223 | | Yes | Yes | Yes | | | | | | | Yes | | | | |
| 224 | | Yes | Yes | Yes | | Yes | | | Yes | Yes | Yes | | Yes | | Yes |
| 225 | Yes | | Yes | Yes | Yes | Yes | | | | | | | | | |

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 226 | | Yes | Yes | Yes | Yes | Yes | | | Yes | | | | | | |
| 227 | | Yes | | Yes | | Yes | | Yes | | | | | | | |
| 228 | Yes | | Yes | | | Yes | | | | | Yes | | | | |
| 229 | Yes | Yes | Yes | Yes | Yes | Yes | Yes | | | | Yes | | Yes | | |
| 230 | | Yes | Yes | Yes | | Yes | Yes | | | Yes | Yes | Yes | Yes | | |
| 231 | | | Yes | Yes | | Yes | Yes | | | | | Yes | | | |
| 232 | Yes | | Yes | Yes | Yes | Yes | Yes | | Yes | | | | | | Yes |
| 233 | | Yes | Yes | Yes | Yes | Yes | | Yes | | | Yes | | Yes | | |
| 234 | | | Yes | | | Yes | | | | | | | | | Yes |
| 235 | | Yes | Yes | Yes | | | | | | Yes | | | | | |
| 236 | Yes | | Yes | Yes | Yes | Yes | | | Yes | | | | | | Yes |
| 237 | Yes | | Yes | Yes | | | Yes | | | | Yes | | | | |
| 238 | Yes | | Yes | Yes | | | | | | | Yes | | | | |
| 239 | Yes | | Yes | Yes | Yes | Yes | | | | | Yes | | | | |
| 240 | Yes | | Yes | Yes | Yes | Yes | | | | | | | | | Yes |
| 241 | | Yes | Yes | Yes | Yes | Yes | | | | Yes | | | Yes | | |
| 242 | Yes | | | Yes | Yes | Yes | | | | | Yes | | | | |
| 243 | Yes | | Yes | Yes | Yes | Yes | | | | Yes | Yes | Yes | | | |
| 244 | Yes | | Yes | Yes | Yes | Yes | Yes | | | | Yes | | Yes | | |
| 245 | Yes | | | Yes | Yes | Yes | Yes | | Yes | | | | | | Yes |
| 246 | Yes | | Yes | Yes | Yes | Yes | Yes | Yes | | | Yes | | Yes | | |
| 247 | Yes | | Yes | Yes | Yes | Yes | Yes | | | | | | Yes | | |
| 248 | Yes | | Yes | Yes | Yes | Yes | | Yes | Yes | | | | Yes | | |
| 249 | Yes | | Yes | Yes | Yes | Yes | | | Yes | | Yes | | | Yes | Yes |

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 250 | | Yes | Yes | Yes | Yes | Yes | | | Yes | Yes | Yes | | | | Yes |
| 251 | | Yes | | Yes | Yes | Yes | | | Yes | | Yes | | Yes | | |
| 252 | | Yes | Yes | | Yes | Yes | Yes | | Yes | Yes | | | | | |
| 253 | Yes | Yes | Yes | Yes | Yes | Yes | Yes | | | | Yes | Yes | Yes | Yes | Yes |
| 254 | Yes | | Yes | Yes | | Yes | Yes | | | | Yes | | Yes | | |
| 255 | | Yes | Yes | Yes | Yes | Yes | | | Yes | Yes | | | Yes | | Yes |
| 256 | | | Yes | Yes | Yes | Yes | | | Yes | Yes | | | | | |
| 257 | | Yes | | Yes | Yes | Yes | Yes | | | Yes | | | Yes | | |
| 258 | Yes | | Yes | Yes | | Yes | | | | | Yes | | | | |
| 259 | Yes | | Yes | Yes | | Yes | | | Yes | | Yes | | | | |
| 260 | Yes | | Yes | Yes | Yes | Yes | | | Yes | Yes | | | Yes | Yes | |
| 261 | | Yes | Yes | Yes | Yes | Yes | Yes | Ye s | | | Yes | | Yes | | |
| 262 | Yes | | Yes | Yes | Yes | Yes | | | Yes | | | | Yes | | |
| 263 | Yes | Yes | Yes | Yes | Yes | Yes | | | | | Yes | | Yes | | |
| 264 | Yes | | Yes | Yes | | | Yes | | Yes | | Yes | | Yes | | Yes |
| 265 | Yes | | Yes | Yes | Yes | Yes | | | Yes | | | | Yes | | |
| 266 | Yes | | Yes | | Yes | Yes | | | | | | | Yes | | |
| 267 | | | Yes | Yes | | Yes | | Ye s | | | | | Yes | | |
| 268 | | Yes | | Yes | | | | | | | | | | | |
| 269 | Yes | | Yes | Yes | Yes | Yes | Yes | | Yes | | Yes | | | | |
| 270 | | Yes | Yes | | Yes | Yes | | | Yes | Yes | | | | Yes | |
| 271 | Yes | | Yes | Yes | Yes | Yes | Yes | | | | | | | | |
| 272 | | Yes | Yes | Yes | Yes | Yes | | | Yes | Yes | | | Yes | | |
| 273 | | Yes | Yes | Yes | | | Yes | | | | Yes | | | | |
| 274 | Yes | | | Yes | Yes | Yes | | | Yes | Yes | | | | | |

(continued)

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 275 | Yes | | Yes | Yes | Yes | Yes | Yes | | | | Yes | | Yes | | Yes |
| 276 | Yes | | Yes | Yes | Yes | Yes | | | Yes | Yes | | | Yes | | |
| 277 | Yes | | Yes | Yes | Yes | Yes | | | Yes | | Yes | | | | |
| 278 | Yes | | Yes | Yes | Yes | Yes | | | | | Yes | | Yes | | |
| 279 | Yes | Yes | Yes | Yes | | Yes | | | | | Yes | | | | |
| 280 | | Yes | Yes | Yes | Yes | Yes | | | | Yes | | | | | Yes |
| 281 | | Yes | Yes | Yes | Yes | Yes | Yes | | Yes | Yes | | | Yes | | Yes |
| 282 | Yes | | Yes | Yes | Yes | Yes | | | | | Yes | | Yes | | Yes |
| 283 | | Yes | Yes | | | Yes | | | | Yes | | | Yes | | |
| 284 | | Yes | Yes | Yes | Yes | Yes | | | | | | | | | |
| 285 | | Yes | Yes | Yes | Yes | Yes | | | Yes | Yes | | | Yes | | Yes |
| 286 | Yes | | Yes | Yes | Yes | Yes | | | Yes | | Yes | | Yes | | Yes |
| 287 | | Yes | Yes | Yes | Yes | Yes | | | Yes | Yes | | | | | |
| 288 | Yes | | Yes | Yes | Yes | Yes | | | Yes | | | | Yes | | |
| 289 | Yes | | Yes | Yes | Yes | Yes | | | Yes | | | | | | |
| 290 | | Yes | Yes | Yes | Yes | Yes | | | Yes | | | | Yes | | |
| 291 | Yes | | Yes | Yes | Yes | Yes | | | | | | | Yes | | |
| 292 | Yes | Yes | Yes | Yes | Yes | Yes | Yes | | | | Yes | | | | |
| 293 | | Yes | Yes | Yes | Yes | Yes | | | Yes | Yes | Yes | | Yes | | |
| 294 | | | Yes | Yes | Yes | Yes | Yes | | | | | | | Yes | |
| 295 | Yes | Yes | Yes | Yes | Yes | Yes | | | | | Yes | | | | |
| 296 | | | Yes | Yes | Yes | Yes | | | | Yes | Yes | | | | |
| 297 | Yes | | Yes | Yes | Yes | Yes | Yes | | | | | | | | |
| 298 | Yes | | | Yes | Yes | | | | | | | | | | |
| 299 | | Yes | Yes | Yes | Yes | Yes | | | | | | | | | Yes |

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 300 | Yes | Yes | Yes | Yes | Yes | Yes | | | Yes | Yes | | | Yes | | Yes |
| 301 | Yes | | Yes | Yes | Yes | Yes | | | | | Yes | | Yes | | |
| 302 | | | Yes | Yes | Yes | Yes | | | | | Yes | | | Yes | |
| 303 | Yes | | Yes | Yes | Yes | Yes | Yes | | Yes | | Yes | | Yes | | |
| 304 | Yes | | Yes | Yes | Yes | Yes | | | Yes | | Yes | | Yes | Yes | |
| 305 | Yes | | Yes | Yes | | Yes | | | Yes | | | | | | |
| 306 | Yes | | Yes | Yes | Yes | Yes | Yes | Ye s | Yes | | Yes | | Yes | | |
| 307 | Yes | | Yes | Yes | Yes | Yes | | | Yes | | Yes | | | | |
| 308 | | Yes | Yes | Yes | Yes | Yes | Yes | | Yes | | | | Yes | | |
| 309 | | Yes | Yes | Yes | Yes | Yes | | Ye s | Yes | Yes | Yes | | Yes | | Yes |
| 310 | | Yes | Yes | Yes | Yes | Yes | | | | | Yes | | Yes | | |
| 311 | Yes | | Yes | Yes | Yes | Yes | | | | Yes | | | Yes | | |
| 312 | Yes | | Yes | Yes | Yes | Yes | | | | Yes | | | | | |
| 313 | | | | Yes | | Yes | | Ye s | | | | | | | |
| 314 | Yes | | Yes | Yes | Yes | Yes | | | | Yes | | | Yes | | Yes |
| 315 | | Yes | Yes | | | Yes | Yes | Ye s | Yes | Yes | | | | | |
| 316 | | | Yes | Yes | Yes | Yes | | | Yes | Yes | | Yes | Yes | Yes | Yes |
| 317 | Yes | | Yes | Yes | Yes | Yes | | | Yes | | Yes | | Yes | | |
| 318 | Yes | | Yes | Yes | Yes | Yes | | Ye s | | Yes | | | Yes | | |
| 319 | Yes | Yes | Yes | Yes | | Yes | | | | Yes | | | Yes | Yes | |
| 320 | Yes | | Yes | Yes | Yes | Yes | | | Yes | | | | Yes | | |
| 321 | Yes | | Yes | Yes | Yes | Yes | Yes | Ye s | | | Yes | | | | |
| 322 | Yes | | Yes | Yes | | Yes | | Ye s | | Yes | Yes | | | | |
| 323 | Yes | | Yes | | Yes | Yes | | | Yes | Yes | | | Yes | | Yes |
| 324 | | Yes | Yes | Yes | Yes | Yes | | | Yes | Yes | | Yes | Yes | | |

| | Me 1 | Me 2 | Me 3 | Me 4 | Me 5 | Me 6 | Me 7 | Me 8 | Me 9 | Me 10 | Me 11 | Me 12 | Me 13 | Me 14 | Me 15 |
|---|---|---|---|---|---|---|---|---|---|---|---|---|---|---|---|
| 325 | | Yes | Yes | Yes | Yes | Yes | | | | Yes | Yes | | Yes | | Yes |
| 326 | Yes | | Yes | Yes | | Yes | Yes | | | Yes | | | | | |
| 327 | | | Yes | | | Yes | Yes | | | Yes | | | Yes | | Yes |

Cox proportional hazards (CoxPH) modeling was used for survival analysis and the explanatory variables were individually (univariate analysis) or simultaneously analyzed in the same model (multivariate analysis). In order to have a better comparison between various hazard ratios (HR), all normally distributed variables including the miRNA expression levels (log2 scale), the clinical variables such as BMI, ln_NT-proBNP, LVEF, age as well as the multivariate scores generated by combining multiple variables were scaled to one standard deviation. The hazard ratio (HR) was then used as the indicator for the prognostic power for those variables. A p-value < 0.05 was considered as statistically significant. Patients were classified as high risk and low risk according to presence or absence of categorical variables and by supra or infra-median levels of normally distributed continuous variables. Kaplan-Meier plots (KM plot) were used to illustrate various risk groups' survival over time with inter-curve comparisons tested by log-rank test. Inter-group survival at 750 days (OS750) and/or EFS at 750 days (EFS750) were also compared.

All the clinical variables were initially assessed for prediction of overall survival (OS). In univariate analyses, five variables (age, hypertension, ln_NT-proBNP, nitrates and hydralazine) were found to be positively associated with risk of death and two variables (BMI and Beta Blockers) were found to be negatively associated with the risk of death (Table 12). Interestingly, overall survival did not differ between HFREF and HFPEF patients. The KM plots of the subject groups defined by those significant parameters are shown in Figure 14, A and the OS750 were shown in Figure 14, B. All the parameters were able to define high and low risk groups with ln_NT-proBNP the most significant (p-value = 7.2E-07, HR =2.36 (95% CI: 1.69-3.30)). Based on the level of ln_NT-proBNP, the low risk group had OS750 of 92.4% while the value for the high risk group was only 66.0%. In the multivariate analysis with all clinical variables included, 6 variables (gender, hypertension, BMI, ln_NT-proBNP, BetaBlockers and Warfarin) were found to be significant. These 6 variables were later combined with each of the 137 miRNAs in the CoxPH model for the identification of prognostic miRNA markers for overall survival.

**Table 12. Analysis of clinical variables of observed survival**

| Variables: | Univariate analysis | | | Multivariate analysis | | |
|---|---|---|---|---|---|---|
| | ln(HR) | SE of ln(HR) | p-value | ln(HR) | SE of ln(HR) | p-value |
| Type (REF/PEF) | -0.50 | 0.30 | 0.10 | 0.03 | 0.98 | 0.98 |
| Gender | 0.01 | 0.29 | 0.97 | **1.03** | **0.50** | **0.040** |
| Atrial Fibrillation/ Flutter | 0.39 | 0.31 | 0.20 | 0.84 | 0.46 | 0.07 |
| Hypertension | **0.81** | **0.41** | **0.048** | **1.24** | **0.49** | **0.0119** |
| Diabetes | 0.28 | 0.30 | 0.36 | 1.01 | 0.53 | 0.06 |
| Smoking History | -0.08 | 0.29 | 0.79 | 0.16 | 0.46 | 0.73 |
| Alcohol History | -0.08 | 0.32 | 0.81 | -0.70 | 0.48 | 0.15 |
| Age | **0.55** | **0.16** | **0.00039** | 0.21 | 0.24 | 0.38 |
| Body Mass Index | **-0.50** | **0.13** | **0.00019** | **-0.55** | **0.25** | **0.026** |
| LVEF | -0.17 | 0.15 | 0.24 | -0.09 | 0.51 | 0.87 |
| ln_NT-proBNP | **0.86** | **0.17** | **7.2E-07** | **0.67** | **0.25** | **0.0078** |
| ACE Inhibitors | -0.01 | 0.29 | 0.96 | 0.07 | 0.39 | 0.86 |
| Angiontensin Receptor Blockers | -0.37 | 0.32 | 0.25 | -0.04 | 0.43 | 0.92 |
| Loop/thiazide Diuretics | 0.01 | 0.52 | 0.99 | 0.71 | 0.79 | 0.37 |
| BetaBlockers | **-0.90** | **0.39** | **0.021** | **-1.72** | **0.57** | **0.0025** |
| Aspirin or Plavix | 0.32 | 0.36 | 0.37 | -0.27 | 0.47 | 0.56 |
| Statins | 0.17 | 0.52 | 0.75 | -0.41 | 0.59 | 0.49 |
| Digoxin | -0.25 | 0.33 | 0.45 | -0.36 | 0.42 | 0.38 |
| Warfarin | -0.83 | 0.52 | 0.11 | **-1.33** | **0.67** | **0.05** |
| Nitrates | **0.60** | **0.29** | **0.039** | 0.09 | 0.41 | 0.82 |
| Calcium Channel Blockers | -0.03 | 0.31 | 0.92 | -0.47 | 0.41 | 0.25 |
| Spironolactone | -0.21 | 0.29 | 0.48 | 0.20 | 0.44 | 0.65 |

(continued)

| Variables: | Univariate analysis | | | Multivariate analysis | | |
|---|---|---|---|---|---|---|
| | ln(HR) | SE of ln(HR) | p-value | ln(HR) | SE of ln(HR) | p-value |
| Fibrate | 0.52 | 0.44 | 0.23 | 0.55 | 0.57 | 0.34 |
| Antidiabetic | -0.10 | 0.29 | 0.73 | -0.78 | 0.52 | 0.13 |
| Hydralazine | **0.78** | **0.37** | **0.036** | 0.11 | 0.52 | 0.83 |
| Iron supplements | 0.43 | 0.30 | 0.15 | -0.07 | 0.39 | 0.85 |

Similar analyses were performed for event free survival (EFS) and seven variables (AF, hypertension, diabetes, age, ln_NT-proBNP, nitrates and hydralazine) were found to be positively correlated with the risk of recurrent admission for decompensated heart failure (Table 13) in univariate analyses. The KM plots of the subject groups defined by those significant parameters were shown in Figure 15, A and the EFS750 were shown in Figure 15, B. Again, there was no difference between HFREF and HFPEF in event free survival and ln_NT-proBNP was the most significant predictor of event free survival (p-value = 1.5E-09, HR =1.79 (95% CI: 1.47-2.17)). Infra-median ln_NT-proBNP was associated with EFS750 of 65.1% and supra-median levels an EFS750 of only 34.1%. By multivariate analysis, only two variables: diabetes and ln_NT-proBNP were found to be significant. These variables were subsequently combined with each of the 137 miRNAs for the identification of prognostic miRNA markers for event free survival.

**Table 13. Analysis of clinical variables for event free survival (EFS)**

| | Univariate analysis | | | Multivariate analysis | | |
|---|---|---|---|---|---|---|
| | ln(HR) | SE of ln(HR) | p-value | ln(HR) | SE of ln(HR) | p-value |
| Type (REF/PEF) | -0.12 | 0.17 | 0.48 | -0.49 | 0.50 | 0.33 |
| Gender | 0.04 | 0.17 | 0.83 | 0.27 | 0.23 | 0.25 |
| Atrial Fibrillation/ Flutter | **0.38** | **0.18** | **0.035** | 0.08 | 0.25 | 0.75 |
| Hypertension | **0.60** | **0.22** | **0.0064** | 0.44 | 0.25 | 0.09 |
| Diabetes | **0.62** | **0.18** | **0.00061** | **1.04** | **0.32** | **0.0011** |
| Smoking History | -0.10 | 0.22 | 0.65 | 0.09 | 0.32 | 0.78 |
| Alcohol History | -0.03 | 0.25 | 0.89 | 0.09 | 0.33 | 0.78 |
| Age | **0.26** | **0.09** | **0.0026** | 0.10 | 0.13 | 0.41 |
| Body Mass Index | -0.13 | 0.09 | 0.14 | 0.05 | 0.12 | 0.66 |
| LVEF | -0.02 | 0.08 | 0.85 | 0.38 | 0.27 | 0.17 |
| ln_NT-proBNP | **0.58** | **0.10** | **1.5E-09** | **0.66** | **0.13** | **9.0E-07** |
| ACE Inhibitors | -0.17 | 0.17 | 0.32 | -0.25 | 0.22 | 0.24 |
| Angiontensin Receptor Blockers | -0.11 | 0.18 | 0.53 | -0.18 | 0.23 | 0.43 |
| Loop/thiazide Diuretics | 0.43 | 0.34 | 0.21 | 0.38 | 0.43 | 0.38 |
| BetaBlockers | -0.11 | 0.29 | 0.71 | -0.49 | 0.35 | 0.17 |
| Aspirin or Plavix | 0.32 | 0.21 | 0.12 | -0.03 | 0.25 | 0.90 |
| Statins | 0.62 | 0.34 | 0.07 | 0.30 | 0.38 | 0.43 |
| Digoxin | 0.12 | 0.18 | 0.51 | 0.15 | 0.23 | 0.50 |
| Warfarin | 0.29 | 0.22 | 0.18 | -0.03 | 0.30 | 0.91 |
| Nitrates | **0.47** | **0.17** | **0.0049** | 0.28 | 0.21 | 0.18 |
| Calcium Channel Blockers | 0.23 | 0.17 | 0.176 | -0.03 | 0.23 | 0.91 |
| Spironolactone | 0.07 | 0.17 | 0.69 | 0.27 | 0.24 | 0.24 |

(continued)

|  | Univariate analysis | | | Multivariate analysis | | |
|---|---|---|---|---|---|---|
|  | ln(HR) | SE of ln(HR) | p-value | ln(HR) | SE of ln(HR) | p-value |
| Fibrate | 0.23 | 0.30 | 0.45 | 0.12 | 0.34 | 0.72 |
| Antidiabetic | 0.29 | 0.17 | 0.09 | -0.53 | 0.29 | 0.07 |
| Hydralazine | **0.49** | **0.25** | **0.048** | -0.03 | 0.29 | 0.93 |
| Iron supplements | 0.30 | 0.18 | 0.09 | -0.04 | 0.22 | 0.87 |

To identify miRNA biomarkers for the prediction of overall survival, each of the 137 miRNAs were tested by the univariate CoxPH model as well as in multivariate CoxPH models including 6 additional predictive clinical variables. In total, 40 miRNAs had p-values less than 0.05. Thirty seven (37) were significant in univariate analyses and 29 were significant in multivariate analyses (Table 14). 11 miRNAs found to be significant in the univariate analysis were not able to improve the prediction performance of clinical parameters (multivariate analysis) and 3 miRNAs were only significant when combined with clinical variables (Figure 16, A). Except for hsa-miR-374b-5p (p-value = 0.25), the 2 miRNAs had p-values less than 0.1 in univariate analysis (Table 14).

The miRNA with the highest hazard ratio (HR) for mortality in both univariate (HR = 1.90 (95% CI: 1.36 - 2.65, p-value = 0.00014)) and multivariate analysis (HR = 1.79 (95% CI: 1.23 - 2.59, p-value = 0.0028)) was hsa-miR-503. Hsa-miR-150-5p had the lowest HR (ie the expression level was negatively correlated with the risk) for both univariate analysis (HR = 0.52 (95% CI: 0.40 - 0.67, p-value = 1.3E-7)) and multivariate analysis (HR = 0.59 (95% CI: 0.45 - 0.78, p-value = 0.00032)) (Table 14). The KM plots for the two miRNAs are shown in Figure 18, A. Good separation between the two risk groups can be observed. Based on a single miRNA, the high risk and low risk group had about 21.3% (hsa-miR-503) or 17.8% (has-miR-150-5p) difference in terms of OS750 (Figure 18, B). With the addition of 6 clinical variables, the combined scores provide better risk predictions where the differences were 25.3% for hsa-miR-503 + 6 clinical variables and 22.4% for has-miR-150-5p + 6 clinical variables (Figure 18, B). Any one or numbers of the 40 miRNAs (Table 14) could be used as the prognostic marker/ panel for risk of death for the chronic HF patients.

For the prediction of event free survival, 13 miRNAs were found significant in the univariate analysis (p-value < 0.05) where 4 were positively correlated and 9 were negative correlated with the risk of recurrent admission for decompensated heart failure after treatment (Table 15). None of the miRNAs were found to be significant for EFS prediction in the multivariate analysis where 2 additional clinical variables were included in the CoxPH model. However, the top positively correlated miRNA (hsa-miR-331-5p, HR = 1.27 (95% CI: 1.09 - 1.49, p-value = 0.0025)) and the top negatively correlated miRNA (hsa-miR-30e-3p, HR = 0.80 (95% CI: 0.69 - 0.94, p-value = 0.0070)) with EFS in the univariate analysis also had certain levels of significance in the multivariate analysis where the p-values were 0.15 and 0.14 respectively (Table 15). The KM plots of the high and low risk groups for EFS defined by either of the miRNAs with and without additional clinical variables were shown in Figure 19, A and their EFS750 were shown in Figure 19, B. Based on a single miRNA (either hsa-miR-331-5p or hsa-miR-30e-3p), the high risk group had EFS750 at about 40% and the low risk group had EFS750 at about 60% while the numbers were 33% and 66% with the addition of 2 clinical variables (Figure 19, B). Any one or numbers of the 13 miRNAs (Table 15) could be used as the prognostic marker/ panel for risk of recurrent admission for decompensated HF for the chronic HF patients.

Fewer miRNA were identified as predictive of event free survival (n=13) than for overall survival (n=43) and only 3 of them overlapped (Figure 16, B). The results suggest differing mechanisms for death and recurrent decompensated heart failure. One important issue to note is that the definition of event free survival in this study involved a less well defined clinical variable - ie hospitalization which could be biased by the patients or the clinicians from case to case. None the less, all the 53 miRNAs could be valuable prognostic markers for chronic heart failure patients.

The 53 prognostic markers were then compared to the 101 markers for HF detection (Figure 17, A) or the 40 markers for heart failure subtype categorization (Figure 17B). Some overlaps were observed but still a large portion of the prognostic markers were not found in the other two lists indicating that a separate set of miRNAs should be used or combined to form the multivariate index assay for the prognosis.

## V. Multivariate biomarker panels for HF detection

As discussed above, panels consisting of combinations of multiple miRNAs might serve to provide better diagnostic power than the use of a single miRNA.

An important criterion to assemble such multivariate panel was to include at least one miRNA from the specific list for each subtype of heart failure to ensure all heart failure subgroups were covered. However, the miRNAs defining the two subtypes of heart failure overlapped (Figure 7). At the same time, large numbers of heart failure related or non-related miRNAs were found to be positively correlated (Figure 12) which makes the choice of the best miRNA combinations for heart failure diagnosis challenging.

In view of the complexity of the task, the inventors of the present study decided to identify panels of miRNA with the highest AUC using sequence forward floating search algorithm [53]. The state-of-the art linear support vector machine, a well utilized and recognized modeling tool for the construction of panels of variables, was also used to aid in the selection of the combinations of miRNAs [54]. The model yields a score based on a linear formula accounting for the expression level of each member and their weightages. These linear models could be readily applied in the clinical practice.

A critical requirement for the success of such process is the availability of high quality data. The quantitative data of all the detected miRNAs in a large number of well-defined clinical samples not only improves the accuracy as well as precision of the result but also ensures the consistency of the identified biomarker panels for further clinical application using qPCR.

To ensure the veracity of the result, multiple (>80) times of hold-out validation (two fold cross validation) were carried out to test the performance of the identified biomarker panel based on the discovery set (half of the samples at each fold) in an independent set of validation samples (the remaining half of the samples at each fold). With the large number of clinical samples (546) the issue of over-fitting of data in modeling was minimized as there were only 137 candidate features to be selected from while at each fold 273 samples was used as the discovery set and the sample to feature ratio is more than two. During the cross validation process, the samples were matched for subtype, gender and race. And the process was carried out to optimize the biomarker panel with 3, 4, 5, 6, 7, 8, 9 or 10 miRNAs separately.

The boxplots representative of the results (the AUC of the biomarker panel in both discovery phase and validation phase) were shown in Figure 20, A. The AUC values were quite close in the various discovery sets (box size < 0.01) and they approached unity (AUC=1.0) with increasing number of miRNAs in the panel. With 4 or more miRNAs, the size of the box in the validation phase, indicative of a spread of values, was quite small (≤0.01 AUC values) as well. As predicted, there was a decrease in AUC values with the validation set for each search (0.02-0.05 AUC).

A more quantitative representation of the results was shown in Figure 20, B. Although there was always a gradual increase of the AUC in the discovery phase when increasing the number of miRNA in the biomarker panel, there were no further significant improvements in the AUC values in the validation phase when the numbers of the miRNAs were greater than 8. Although the difference between 6 miRNA and 8 miRNA biomarker panels was statistically significant, the improvement was less than 0.01 in AUC values. Thus, a biomarker panel with 6 or more miRNAs giving AUC value around 0.93 should be useful for heart failure detection.

To examine the composition of multivariate biomarker panels, the present study counted the occurrence of miRNAs in all the panels containing 6-10 miRNAs, where the panels with the top 10% and bottom 10% AUC were excluded. This was carried out to avoid counting of falsely discovered biomarkers due to fitting of inaccurate data from subpopulations generated by the randomization process in cross-validation analysis. Excluding these miRNAs chosen in less than 2% of the panels, a total of 51 miRNA were selected in the discovery process (Table 16) where the expression of 42 of these were also found to be significantly altered in HF (Table 5-7). The inclusion of 9 others, although not altered in heart failure, were found to significantly improve the AUC values as 39% of the panels included at least one of these miRNA from the list and the most frequently selected miRNA (hsa-miR-10b-5p) presented in 35% of the panels. Without a direct and quantitative measurement of all miRNA targets, these miRNAs would never have been selected in high-through put screening studies (microarray, sequencing) and would have been excluded for further qPCR validation.

When comparing the identities of the chosen miRNAs for multivariate panels and single miRNA as diagnostic markers, they were not necessarily the same. For example, the top up - regulated (hsa-let-7d-3p) miRNA was not present in the list while the top down-regulated (hsa-miR-454-3p) was only used in 24.2% of the panels. Hence, it was not possible merely to combine the best single miRNA identified to form the optimal biomarker panel but rather a panel of miRNAs providing complementary information gave the best result.

All those miRNAs were not randomly selected as 7 of them presented in more than 30% of the panels but it was also difficult to find miRNAs to be critical for a good biomarkers panel as the two most frequently selected miRNAs hsa-miR-551b-3p and hsa-miR-24-3p were only found in 59.7% and 57.3% of the panels respectively. As discussed, a lot of those miRNAs were correlated (Figure 11) which could serve as replacement or substitutes for each other in the biomarker panels. In conclusion, a biomarker panel with at least 6 miRNAs from the frequently selected list (Table 16) should be used for the detection of heart failure.

To compare the miRNA biomarkers and NT-proBNP, one of the six miRNA biomarker panel was selected to calculate the combined miRNA scores for all subjects, which were plotted against the NT-proBNP levels from the same subjects (Figure 21, A). In general, the inventors of the present study observed a positive correlation where the Pearson correlation coefficient between the miRNA score and ln_NT-proBNP was 0.61 (p-value = 8.2E-56). Applying the suggested cut-off for NT-proBNP (125 pg/mL, dashed line), 35 of the healthy subjects were falsely classified as heart failure patient (false positive, FP, NT-proBNP>125) and 23 heart failure patients had NT-proBNP levels lower than the cut-off (false negative, FN). Predictably, most of the false negative (FN) were HFPEF subjects (n=20). Those false positive (FP) and false negative (FN) subjects with respect to NT-proBNP were selected and results plotted against the miRNA score (Figure 21, B). Based on the separate plot, most of the false positive (FP) and false negative (FN) subjects could be correctly reclassified by the miRNA score with zero as the cut-off (dashed line). The results validated the hypothesis that the miRNA biomarkers carry different information than NT-proBNP. The next step was to explore a multivariate biomarker panel including both miRNA and NT-proBNP.

The same biomarker identification process (multiple times of two fold cross validation) was performed where NT-proBNP was pre-fixed as one of the predictive variables and the level of ln _NT-proBNP together with the miRNA expression levels (log2 scale) were used to build the classifier using the support-vector-machine. Since there was no significant increase of AUC when more than 8 miRNAs were used to predict heart failure (Figure 20), the process was carried out to optimize the biomarker panel with 2, 3, 4, 5, 6, 7 or 8 miRNAs (together with NT-proBNP).

The classifier built in the discovery phase approached perfect separation (AUC = 1.00) with increasing numbers of miRNAs. Performance decreased somewhat in the validation phase (Figure 22, A). Nevertheless, the AUC of the panels containing NT-proBNP in the validation phase (mean AUC > 0.96) were always higher than those biomarker panels including only miRNA (mean AUC < 0.94). The quantitative results (Figure 22, B) showed that there were no further significant improvements in the AUC values in the validation phase when the numbers of the miRNAs were greater than 4 and there was only a tiny increase (0.001 AUC) between 4 and 5 miRNA biomarker panels. Thus, when combining with NT-proBNP, a biomarker panel with 4 or more miRNAs giving AUC value around 0.98 can be used for heart failure detection. By combining miRNA and NT-proBNP, the classification efficiency was significantly improved over NT-proBNP (AUC = 0.962, Figure 22, B).

Excluding the panels with the top 10% and bottom 10% AUC, the composition of multivariate biomarker panels containing 3-8 miRNAs was examined (Table 17). A total number of 49 miRNA were selected in the discovery process with 14 of them having prevalence higher than 10% (Table 17). Forty two (42) of them also carried information additional to NT-proBNP (p-value after FDR lower than 0.01 in the logistic regression). Again, 46% of the panels included at least one of the 13 miRNAs found to be insignificant in addition to NT-proBNP.

Although more than half of the significant miRNAs (Table 17, significant list) were also frequently selected when searching for miRNA only biomarker panels (Table 16, significant list) (Figure 23, A), the ranking of the prevalence was different. Some of the highly selected miRNA in conjunction with NT-proBNP (hsa-miR-17-5p (11.6%) and hsa-miR-25-3p (11.0%)) were even not chosen when searching for miRNA based biomarker panels (without NT-proBNP). Also there were only two miRNAs overlapped between the insignificant lists (Table 16 and Table 17, insignificant list) (Figure 23, B). Together, the evidences suggested that a different list of miRNAs should be used together with NT-proBNP compared to the list used for the construction of miRNA only biomarker panels.

### VI. Multivariate biomarker panels for HF subtype categorization

The next attempt was made to identify multivariate biomarker panels for distinguishing between HFREF and HFPEF. Again, all the quantitative data for 137 miRNAs on the 338 heart failure patients were used. Due to the constraints of sample size, multiple (>50) times of four fold cross validation were carried out where all the subjects were randomly divided into four even groups and three of the groups were used (discovery group) to build the classifier to predict the last group (validation group) in turn. In this way, 253-254 subjects will be used in the discovery phase ensuring the same size in each subgroup (HFREF or HFPEF) similar to the number of candidate features (137) to be selected to minimize the over-fitting. Again the process was performed to optimize 3, 4, 5, 6, 7, 8, 9 or 10 miRNA biomarker panels and 2, 3, 4, 5, 6, 7 or 8 miRNA plus NT-proBNP biomarker panels separately.

The quantitative results showed that there were no improvements in AUC values when the miRNA-only biomarker panel contained more than 5 miRNAs (Figure 24, A). About 0.76 AUC could be achieved with miRNA biomarker panels which is better than NT-proBNP (AUC = 0.706). Counting all the 6-10 miRNA panels (excluding the top 10% and bottom 10% in terms of AUC), 46 miRNAs were frequently selected (in > 2% of the panel) where 22 were found to be significant in the t-test comparing HFREF and HFPEF while 24 were not (Table 18). The panels for heart failure subtype categorization were less diversified than those for heart failure detection as two of the miRNAs presented in more than 80% of the panels (hsa-miR-30a-5p (94.6%) and hsa-miR-181a-2-3p (83.7%), Table 18).

For the biomarker panels consisting both miRNA and NT-proBNP, fewer miRNAs were needed when compared to the miRNA-only panels as there were no improvements on the AUC values beyond inclusion of 4 miRNAs (Figure 24, B). Even clearer classification could be achieved (AUC~0.82) when compared to miRNA-only panels. Again, miRNAs and NT-proBNP may carry complementary information for heart failure subtype categorization. Examining the composition of the 5-8 miRNA plus NT-proBNP panels, 31 miRNAs were frequently selected (in >2% of the panels) where 14 were found to be significant in the logistic regressions together with ln _NT-proBNP while 17 were not (Table 19). Two different miRNAs were found in more than 80% of the panels: hsa-miR-199b-5p (91.5%) and hsa-miR-191-5p (74.9%). Although, the most frequently selected insignificant miRNA for both the miRNA only and miRNA plus NT-proBNP panel were the same (hsa-miR-199b-5p), remarkable differences could be found between the rest of the significant and insignificant lists in terms of identities and rankings.

**REFERENCE**

1. Organization, W.H., Annex Table 2: Deaths by cause, sex and mortality stratum in WHO regions, estimates for 2002. The world health report 2004 - changing history, 2004.

2. Alla, F., F. Zannad, and G. Filippatos, Epidemiology of acute heart failure syndromes. Heart Fail Rev, 2007. 12(2): p. 91-5.

3. Stewart, S., et al., More 'malignant' than cancer? Five-year survival following a first admission for heart failure. Eur J Heart Fail, 2001. 3(3): p. 315-22.

4. Pritchard, C.C., et al., Blood cell origin of circulating microRNAs: a cautionary note for cancer biomarker studies. Cancer Prev Res (Phila), 2012. 5(3): p. 492-7.

5. McDonald, J.S., et al., Analysis of circulating microRNA: preanalytical and analytical challenges. Clin Chem, 2011. 57(6): p. 833-40.

6. Nishimura, J. and Y. Kanakura, [Paroxysmal nocturnal hemoglobinuria (PNH)]. Nihon Rinsho, 2008. 66(3): p. 490-6.

7. Owan, T.E., et al., Trends in prevalence and outcome of heart failure with preserved ejection fraction. N Engl J Med, 2006. 355(3): p. 251-9.

8. Bhatia, R.S., et al., Outcome of heart failure with preserved ejection fraction in a population-based study. N Engl J Med, 2006. 355(3): p. 260-9.

9. Yancy, C.W., et al., Clinical presentation, management, and in-hospital outcomes of patients admitted with acute decompensated heart failure with preserved systolic function: a report from the Acute Decompensated Heart Failure National Registry (ADHERE) Database. J Am Coll Cardiol, 2006. 47(1): p. 76-84.

10. Borlaug, B.A. and M.M. Redfield, Diastolic and systolic heart failure are distinct phenotypes within the heart failure spectrum. Circulation, 2011. 123(18): p. 2006-13; discussion 2014.

11. Hogg, K., K. Swedberg, and J. McMurray, Heart failure with preserved left ventricular systolic function; epidemiology, clinical characteristics, and prognosis. J Am Coll Cardiol, 2004. 43(3): p. 317-27.

12. Yusuf, S., et al., Effects of candesartan in patients with chronic heart failure and preserved left-ventricular ejection fraction: the CHARM-Preserved Trial. Lancet, 2003. 362(9386): p. 777-81.

13. Massie, B.M., et al., Irbesartan in patients with heart failure and preserved ejection fraction. N Engl J Med, 2008. 359(23): p. 2456-67.

14. Writing Committee, M., et al., 2013 ACCF/AHA guideline for the management of heart failure: a report of the American College of Cardiology Foundation/American Heart Association Task Force on practice guidelines. Circulation, 2013. 128(16): p. e240-327.

15. Troughton, R.W., et al., Effect of B-type natriuretic peptide-guided treatment of chronic heart failure on total mortality and hospitalization: an individual patient meta-analysis. Eur Heart J, 2014. 35(23): p. 1559-67.

16. Christenson, R.H., et al., Impact of increased body mass index on accuracy of B-type natriuretic peptide (BNP) and N-terminal proBNP for diagnosis of decompensated heart failure and prediction of all-cause mortality. Clin Chem, 2010. 56(4): p. 633-41.

17. Richards, M., et al., Atrial fibrillation impairs the diagnostic performance of cardiac natriuretic peptides in dyspneic patients: results from the BACH Study (Biomarkers in ACute Heart Failure). JACC Heart Fail, 2013. 1(3): p. 192-9.

18. Masson, S., et al., Direct comparison of B-type natriuretic peptide (BNP) and amino-terminal proBNP in a large population of patients with chronic and symptomatic heart failure: the Valsartan Heart Failure (Val-HeFT) data. Clin Chem, 2006. 52(8): p. 1528-38.

19. Komajda, M., et al., Factors associated with outcome in heart failure with preserved ejection fraction: findings from the Irbesartan in Heart Failure with Preserved Ejection Fraction Study (I-PRESERVE). Circ Heart Fail, 2011. 4(1): p. 27-35.

20. Kraigher-Krainer, E., et al., Impaired systolic function by strain imaging in heart failure with preserved ejection fraction. J Am Coll Cardiol, 2014. 63(5): p. 447-56.

21. Richards, A.M., J.L. Januzzi, Jr., and R.W. Troughton, Natriuretic Peptides in Heart Failure with Preserved Ejection Fraction. Heart Fail Clin, 2014. 10(3): p. 453-470.

22. Liang, H., et al., The origin, function, and diagnostic potential of extracellular microRNAs in human body fluids. Wiley Interdiscip Rev RNA, 2014. 5(2): p. 285-300.

23. Cortez, M.A., et al., MicroRNAs in body fluids--the mix of hormones and biomarkers. Nat Rev Clin Oncol, 2011. 8(8): p. 467-77.

24. Bronze-da-Rocha, E., MicroRNAs Expression Profiles in Cardiovascular Diseases. Biomed Res Int, 2014. 2014: p. 985408.

25. Kim, K.M. and S.G. Kim, Autophagy and microRNA dysregulation in liver diseases. Arch Pharm Res, 2014.

26. Tan, L., J.T. Yu, and L. Tan, Causes and Consequences of MicroRNA Dysregulation in Neurodegenerative Diseases. Mol Neurobiol, 2014.

27. Lee, R.C., R.L. Feinbaum, and V. Ambros, The C. elegans heterochronic gene lin-4 encodes small RNAs with antisense complementarity to lin-14. Cell, 1993. 75(5): p. 843-54.

28. Friedman, R.C., et al., Most mammalian mRNAs are conserved targets of microRNAs. Genome Res, 2009. 19(1): p. 92-105.

29. Hayashita, Y., et al., A polycistronic microRNA cluster, miR-17-92, is overexpressed in human lung cancers and enhances cell proliferation. Cancer Res, 2005. 65(21): p. 9628-32.

30. Jovanovic, M. and M.O. Hengartner, miRNAs and apoptosis: RNAs to die for. Oncogene, 2006. 25(46): p. 6176-87.

31. Grasso, M., et al., Circulating miRNAs as biomarkers for neurodegenerative disorders. Molecules, 2014. 19(5): p. 6891-910.

32. Sayed, A.S., et al., Diagnosis, Prognosis and Therapeutic Role of Circulating miRNAs in Cardiovascular Diseases. Heart Lung Circ, 2014. 23(6): p. 503-510.

33. de Candia, P., et al., Serum microRNAs as Biomarkers of Human Lymphocyte Activation in Health and Disease. Front Immunol, 2014. 5: p. 43.

34. Sheinerman, K.S. and S.R. Umansky, Circulating cell-free microRNA as biomarkers for screening, diagnosis and monitoring of neurodegenerative diseases and other neurologic pathologies. Front Cell Neurosci, 2013. 7: p. 150.

35. Dorval, V., P.T. Nelson, and S.S. Hebert, Circulating microRNAs in Alzheimer's disease: the search for novel biomarkers. Front Mol Neurosci, 2013. 6: p. 24.

36. Vogel, B., et al., Multivariate miRNA signatures as biomarkers for non-ischaemic systolic heart failure. Eur Heart J, 2013. 34(36): p. 2812-22.

37. Endo, K., et al., MicroRNA 210 as a biomarker for congestive heart failure. Biol Pharm Bull, 2013. 36(1): p. 48-54.

38. Zhang, R., et al., Elevated plasma microRNA-1 predicts heart failure after acute myocardial infarction. Int J Cardiol, 2013. 166(1): p. 259-60.

39. Fukushima, Y., et al., Assessment of plasma miRNAs in congestive heart failure. Circ J, 2011. 75(2): p. 336-40.

40. Corsten, M.F., et al., Circulating MicroRNA-208b and MicroRNA-499 reflect myocardial damage in cardiovascular disease. Circ Cardiovasc Genet, 2010. 3(6): p. 499-506.

41. Matsumoto, S., et al., Circulating p53-responsive microRNAs are predictive indicators of heart failure after acute myocardial infarction. Circ Res, 2013. 113(3): p. 322-6.

42. Goren, Y., et al., Serum levels of microRNAs in patients with heart failure. Eur J Heart Fail, 2012. 14(2): p. 147-54.

43. Xiao, J., et al., MicroRNA-134 as a potential plasma biomarker for the diagnosis of acute pulmonary embolism. J Transl Med, 2011. 9: p. 159.

44. Tijsen, A.J., et al., MiR423-5p as a circulating biomarker for heart failure. Circ Res, 2010. 106(6): p. 1035-9.

45. Zhao, D.S., et al., Serum miR-210 and miR-30a expressions tend to revert to fetal levels in Chinese adult patients with chronic heart failure. Cardiovasc Pathol, 2013. 22(6): p. 444-50.

46. Goren, Y., et al., Relation of reduced expression of MiR-150 in platelets to atrial fibrillation in patients with chronic systolic heart failure. Am J Cardiol, 2014. 113(6): p. 976-81.

47. Ellis, K.L., et al., Circulating microRNAs as candidate markers to distinguish heart failure in breathless patients. Eur J Heart Fail, 2013. 15(10): p. 1138-47.

48. Redova, M., J. Sana, and O. Slaby, Circulating miRNAs as new blood-based biomarkers for solid cancers. Future Oncol, 2013. 9(3): p. 387-402.

49. Mestdagh, P., et al., Evaluation of quantitative miRNA expression platforms in the microRNA quality control (miRQC) study. Nat Methods, 2014.

50. Cissell, K.A. and S.K. Deo, Trends in microRNA detection. Anal Bioanal Chem, 2009. 394(4): p. 1109-16.

51. Tsongalis, G.J., et al., MicroRNA analysis: is it ready for prime time? Clin Chem, 2013. 59(2): p. 343-7.

52. Hindson, B.J., et al., High-throughput droplet digital PCR system for absolute quantitation of DNA copy number. Anal Chem, 2011. 83(22): p. 8604-10.

53. Xiong, M., X. Fang, and J. Zhao, Biomarker identification by feature wrappers. Genome Res, 2001. 11(11): p. 1878-87.

54. Saeys, Y., I. Inza, and P. Larranaga, A review of feature selection techniques in bioinformatics. Bioinformatics, 2007. 23(19): p. 2507-17.

55. Santhanakrishnan, R., et al., The Singapore Heart Failure Outcomes and Phenotypes (SHOP) study and Prospective Evaluation of Outcome in Patients with Heart Failure with Preserved Left Ventricular Ejection Fraction (PEOPLE) study: rationale and design. J Card Fail, 2013. 19(3): p. 156-62.

56. Santhanakrishnan, R., et al., Growth differentiation factor 15, ST2, high-sensitivity troponin T, and N-terminal pro brain natriuretic peptide in heart failure with preserved vs. reduced ejection fraction. Eur J Heart Fail, 2012. 14(12): p. 1338-47.

57. McMurray, J.J., et al., ESC Guidelines for the diagnosis and treatment of acute and chronic heart failure 2012: The Task Force for the Diagnosis and Treatment of Acute and Chronic Heart Failure 2012 of the European Society of Cardiology. Developed in collaboration with the Heart Failure Association (HFA) of the ESC. Eur Heart J, 2012. 33(14): p. 1787-847.

58. Etheridge, A., et al., Extracellular microRNA: a new source of biomarkers. Mutat Res, 2011. 717(1-2): p. 85-90.

59. Li, Y. and K.V. Kowdley, Method for microRNA isolation from clinical serum samples. Anal Biochem, 2012. 431(1): p. 69-75.

60. Benjamini, Y., and Hochberg, Y., Controlling the false discovery rate: A practical and powerful approach to multiple testing. ournal of the Royal Statistical Society, 1995. 57(289-300).

**Claims**

1. A method of determining whether a subject suffers from heart failure, or is at risk of developing heart failure, the method comprising the steps of

   (a) measuring the level of at least hsa-miR-454-3p in a bodily fluid sample obtained from the subject, and
   (b) determining whether it is different as compared to a control,

   wherein a decreased level of hsa-miR-454-3p indicates that the subject has heart failure or is at a risk of developing heart failure.

2. A method according to claim 1 further comprising measuring the levels of at least hsa-miR-24-3p and hsa-miR-551b-3p in a bodily fluid sample obtained from the subject wherein an increased level of miR-24-3p and decreased levels of hsa-miR-454-3p and 551b-3p indicates that the subject has heart failure or is at a risk of developing heart failure.

3. A method of determining whether a subject suffers from heart failure, or is at risk of developing heart failure, the method comprising the steps of

   (a) measuring the levels of at least hsa-miR-454-3p, hsa-miR-24-3p, and hsa-miR-551b-3p in a bodily fluid sample obtained from the subject; and
   (b) using a score based on the levels of the miRNAs measured in step (a) to predict the likelihood of the subject to develop or to have heart failure.

4. A method according to claim 3 wherein an increased level of miR-24-3p and decreased levels of hsa-miR-454-3p and 551b-3p indicates that the subject has heart failure or is at a risk of developing heart failure.

5. The method according to any one of the preceding claims wherein step (a) further comprises measuring the levels of at least one further miRNA listed in Table 16 or 23 in a bodily fluid sample obtained from the subject.

6. The method of claim 1, 2 or 4, wherein the control is a sample obtained from a non-heart failure subject (normal).

7. The method of any one of the preceding claims, wherein the subject is of Asian ethnicity.

**Patentansprüche**

1. Verfahren zum Bestimmen, ob ein Individuum an Herzversagen leidet oder ein Risiko zur Entwicklung von Herzversagen aufweist, wobei das Verfahren folgende Schritte umfasst:

   (a) Messen des Spiegels von zumindest hsa-miR-454-3p in einer Körperflüssigkeitsprobe, die von dem Individuum erhalten wurde, und
   (b) Bestimmen, ob er verglichen mit einer Kontrolle abweicht,

wobei ein verringerter Spiegel von hsa-miR-454-3p angibt, dass das Individuum an Herzversagen leidet oder ein Risiko zur Entwicklung von Herzversagen aufweist.

2. Verfahren nach Anspruch 1, das weiters das Messen der Spiegel von zumindest hsa-miR-24-3p und hsa-miR-551b-3p in einer Körperflüssigkeitsprobe umfasst, die von dem Individuum erhalten wurde, wobei ein erhöhter Spiegel von miR-24-3p und verringerte Spiegel von hsa-miR-454-3p und 551b-3p angeben, dass das Individuum an Herzversagen leidet oder ein Risiko zur Entwicklung von Herzversagen aufweist.

3. Verfahren zum Bestimmen, ob ein Individuum an Herzversagen leidet oder ein Risiko zur Entwicklung von Herzversagen aufweist, wobei das Verfahren folgende Schritte umfasst:

(a) Messen der Spiegel von zumindest hsa-miR-454-3p, hsa-miR-24-3p und hsa-miR-551b-3p in einer Körperflüssigkeitsprobe, die von dem Individuum erhalten wurde; und
(b) Verwenden einer Bewertung basierend auf den Spiegeln der in Schritt (a) gemessenen miRNAs, um die Wahrscheinlichkeit vorherzusagen, dass das Individuum Herzversagen entwickelt oder aufweist.

4. Verfahren nach Anspruch 3, wobei ein erhöhter Spiegel von miR-24-3p und verringerte Spiegel von hsa-miR-454-3p und 551b-3p angeben, dass das Individuum an Herzversagen leidet oder ein Risiko zur Entwicklung von Herzversagen aufweist.

5. Verfahren nach einem der vorangegangenen Ansprüche, wobei Schritt (a) weiters das Messen der Spiegel von zumindest einer weiteren miRNA, die in Tabelle 16 oder 23 aufgeführt ist, in einer Körperflüssigkeitsprobe umfasst, die von dem Individuum erhalten wurde.

6. Verfahren nach Anspruch 1, 2 oder 4, wobei die Kontrolle eine Probe ist, die von einem Individuum ohne Herzversagen (normal) erhalten wurde.

7. Verfahren nach einem der vorangegangenen Ansprüche, wobei das Individuum asiatischer Ethnie angehört.


## Revendications

1. Procédé pour déterminer si un sujet souffre d'insuffisance cardiaque, ou présente un risque de développer une insuffisance cardiaque, le procédé comprenant les étapes consistant à

(a) mesurer le niveau d'au moins hsa-miR-454-3p dans un échantillon de fluide corporel obtenu auprès du sujet, et
(b) déterminer s'il est différent par comparaison à un témoin,

dans lequel un niveau diminué de hsa-miR-454-3p indique que le sujet souffre d'insuffisance cardiaque ou présente un risque de développer une insuffisance cardiaque.

2. Procédé selon la revendication 1, comprenant en outre la mesure des niveaux d'au moins hsa-miR-24-3p et hsa-miR-551b-3p dans un échantillon de fluide corporel obtenu auprès du sujet, dans lequel un niveau accru de miR-24-3p et des niveaux diminués de hsa-miR-454-3p et 551b-3p indiquent que le sujet souffre d'insuffisance cardiaque ou présente un risque de développer une insuffisance cardiaque.

3. Procédé pour déterminer si un sujet souffre d'insuffisance cardiaque, ou présente un risque de développer une insuffisance cardiaque, le procédé comprenant les étapes consistant à

(a) mesurer les niveaux d'au moins hsa-miR-454-3p, hsa-miR-24-3p et hsa-miR-551b-3p dans un échantillon de fluide corporel obtenu auprès du sujet ; et
(b) utiliser un score basé sur les niveaux des ARNmi mesurés à l'étape (a) pour prédire la probabilité que le sujet développe ou présente une insuffisance cardiaque.

4. Procédé selon la revendication 3, dans lequel un niveau accru de miR-24-3p et des niveaux diminués de hsa-miR-454-3p et 551b-3p indiquent que le sujet souffre d'insuffisance cardiaque ou présente un risque de développer une insuffisance cardiaque.

**5.** Procédé selon l'une quelconque des revendications précédentes, dans lequel l'étape (a) comprend en outre la mesure des niveaux d'au moins un autre ARNmi répertorié dans le Tableau 16 ou 23 dans un échantillon de fluide corporel obtenu auprès du sujet.

**6.** Procédé selon la revendication 1, 2 ou 4, dans lequel le témoin est un échantillon obtenu auprès d'un sujet sans insuffisance cardiaque (normal).

**7.** Procédé selon l'une quelconque des revendications précédentes, dans lequel le sujet est d'origine ethnique asiatique.

Figure 1

Figure 2

Figure 3

Figure 4

## (A): eigenvalues

PC 1-15

## (B): AUC of C v.s. HF

PC 1-15

## (C): AUC of REF v.s. PEF

PC 1-15

Figure 5

## (A): C vs HF

## (B): REF vs PEF

Figure 6

**(A): Significant in univariate analysis**
adjusted p-value < 0.01

**(B): Significant in both univariate**
**and multivariate analysis**
adjusted p-value < 0.01

Figure 7

EP 3 875 605 B1

Figure 8

**(A): Significant in univariate analysis**
adjusted p-value < 0.01

C vs HF
C vs REF                    REF vs PEF
C vs PEF                    (40)
(101)

**(B): Significant in both univariate**
**and multivariate analysis**
adjusted p-value < 0.01

C vs HF
C vs REF                    REF vs PEF
C vs PEF                    (18)
(86)

Figure 9

Figure 10

**C > REF > PEF**

(n=21)

| hsa-miR-532-5p | hsa-miR-19b-3p |
| hsa-miR-451a | hsa-miR-20a-5p |
| hsa-miR-4732-3p | hsa-miR-144-3p |
| hsa-miR-183-5p | hsa-miR-20b-5p |
| hsa-miR-93-5p | hsa-miR-363-3p |
| hsa-miR-501-5p | hsa-miR-103a-3p |
| hsa-miR-18b-5p | hsa-miR-19a-3p |
| hsa-miR-196b-5p | hsa-miR-500a-5p |
| hsa-let-7b-5p | hsa-let-7i-5p |
| hsa-miR-17-5p | hsa-miR-106a-5p |
| hsa-miR-16-5p | |

**C < REF < PEF**

(n=5)

hsa-miR-335-5p
hsa-miR-23b-3p
hsa-miR-23a-5p
hsa-miR-223-5p
hsa-miR-452-5p

**C < PEF < REF**

(n=1)

hsa-miR-181b-5p

**C = PEF > REF**

(n=1)

hsa-miR-181a-2-3p

**C = PEF < REF**

(n=1)

hsa-miR-30a-5p

**C = REF > PEF**

(n=7)

hsa-miR-550a-5p
hsa-miR-106b-3p
hsa-miR-192-5p
hsa-miR-25-3p
hsa-miR-194-5p
hsa-miR-185-5p
hsa-miR-486-5p

**C = REF < PEF**

(n=2)

hsa-miR-126-5p
hsa-miR-146a-5p

Figure 11

**(A): Correlation efficiency > 0.5**

**(B): Correlation efficiency < -0.5**

Figure 12

Figure 13

# (A) Kaplan-Meier plot

Figure 14A

## (B) EFS at 750 days

Figure 14B

## (A) Kaplan-Meier plot

Figure 15A

Figure 15B

Figure 16

**(A): Compare prognosis and
HF detection miRNAs**

Prognosis (50)      HF detection (101)

16    34    67

**(B): Compare Prognosis and
subtype categorization miRNAs**

Prognosis (50)      Subtype
categorization (40)

41    9    31

Figure 17

# (A) Kaplan-Meier plot

Figure 18A

Figure 18B

## (A) Kaplan-Meier plot

Figure 19A

Figure 19B

Figure 20A

Figure 20B

Figure 21

Figure 22

## (A): Significant miRNA

miRNA panel (42)

miRNA + NT-proBNP panel(36)

## (B): Insignificant miRNA

miRNA panel (9)

miRNA + NT-proBNP panel(13)

Figure 23

Figure 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014187884 A2
- DE 102012101557 A1
- WO 2011133036 A2

**Non-patent literature cited in the description**

- **ORGANIZATION, W.H.** Annex Table 2: Deaths by cause, sex and mortality stratum in WHO regions. *The world health report 2004 - changing history, 2004.,* 2002
- **ALLA, F. ; F. ZANNAD ; G. FILIPPATOS.** Epidemiology of acute heart failure syndromes. *Heart Fail Rev,* 2007, vol. 12 (2), 91-5
- **STEWART, S et al.** More 'malignant' than cancer? Five-year survival following a first admission for heart failure. *Eur J Heart Fail,* 2001, vol. 3 (3), 315-22
- **PRITCHARD, C.C. et al.** Blood cell origin of circulating microRNAs: a cautionary note for cancer biomarker studies. *Cancer Prev Res (Phila),* 2012, vol. 5 (3), 492-7
- **MCDONALD, J.S. et al.** Analysis of circulating microRNA: preanalytical and analytical challenges. *Clin Chem,* 2011, vol. 57 (6), 833-40
- **NISHIMURA, J. ; Y. KANAKURA.** Paroxysmal nocturnal hemoglobinuria (PNH)]. *Nihon Rinsho,* 2008, vol. 66 (3), 490-6
- **OWAN, T.E. et al.** Trends in prevalence and outcome of heart failure with preserved ejection fraction. *N Engl J Med,* 2006, vol. 355 (3), 251-9
- **BHATIA, R.S. et al.** Outcome of heart failure with preserved ejection fraction in a population-based study. *N Engl J Med,* 2006, vol. 355 (3), 260-9
- **YANCY, C.W. et al.** Clinical presentation, management, and in-hospital outcomes of patients admitted with acute decompensated heart failure with preserved systolic function: a report from the Acute Decompensated Heart Failure National Registry (ADHERE) Database. *J Am Coll Cardiol,* 2006, vol. 47 (1), 76-84
- **BORLAUG, B.A. ; M.M. REDFIELD.** Diastolic and systolic heart failure are distinct phenotypes within the heart failure spectrum. *Circulation,* 2011, vol. 123 (18), 2006-13
- **HOGG, K. ; K. SWEDBERG ; J. MCMURRAY.** Heart failure with preserved left ventricular systolic function; epidemiology, clinical characteristics, and prognosis. *J Am Coll Cardiol,* 2004, vol. 43 (3), 317-27
- **YUSUF, S. et al.** Effects of candesartan in patients with chronic heart failure and preserved left-ventricular ejection fraction: the CHARM-Preserved Trial. *Lancet,* 2003, vol. 362 (9386), 777-81
- **MASSIE, B.M. et al.** Irbesartan in patients with heart failure and preserved ejection fraction. *N Engl J Med,* 2008, vol. 359 (23), 2456-67
- **WRITING COMMITTEE, M. et al.** ACCF/AHA guideline for the management of heart failure: a report of the American College of Cardiology Foundation/American Heart Association Task Force on practice guidelines. *Circulation,* 2013, vol. 128 (16), e240-327
- **TROUGHTON, R.W. et al.** Effect of B-type natriuretic peptide-guided treatment of chronic heart failure on total mortality and hospitalization: an individual patient meta-analysis. *Eur Heart J,* 2014, vol. 35 (23), 1559-67
- **CHRISTENSON, R.H. et al.** Impact of increased body mass index on accuracy of B-type natriuretic peptide (BNP) and N-terminal proBNP for diagnosis of decompensated heart failure and prediction of all-cause mortality. *Clin Chem,* 2010, vol. 56 (4), 633-41
- **RICHARDS, M. et al.** Atrial fibrillation impairs the diagnostic performance of cardiac natriuretic peptides in dyspneic patients: results from the BACH Study (Biomarkers in ACute Heart Failure). *JACC Heart Fail,* 2013, vol. 1 (3), 192-9
- **MASSON, S. et al.** Direct comparison of B-type natriuretic peptide (BNP) and amino-terminal proBNP in a large population of patients with chronic and symptomatic heart failure: the Valsartan Heart Failure (Val-HeFT) data. *Clin Chem,* 2006, vol. 52 (8), 1528-38
- **KOMAJDA, M. et al.** Factors associated with outcome in heart failure with preserved ejection fraction: findings from the Irbesartan in Heart Failure with Preserved Ejection Fraction Study (I-PRESERVE). *Circ Heart Fail,* 2011, vol. 4 (1), 27-35

- **KRAIGHER-KRAINER, E. et al.** Impaired systolic function by strain imaging in heart failure with preserved ejection fraction. *J Am Coll Cardiol,* 2014, vol. 63 (5), 447-56
- **RICHARDS, A.M. ; J.L. JANUZZI, JR. ; R.W. TROUGHTON.** Natriuretic Peptides in Heart Failure with Preserved Ejection Fraction. *Heart Fail Clin,* 2014, vol. 10 (3), 453-470
- **LIANG, H. et al.** The origin, function, and diagnostic potential of extracellular microRNAs in human body fluids. *Wiley Interdiscip Rev RNA,* 2014, vol. 5 (2), 285-300
- **CORTEZ, M.A. et al.** MicroRNAs in body fluids--the mix of hormones and biomarkers. *Nat Rev Clin Oncol,* 2011, vol. 8 (8), 467-77
- **BRONZE-DA-ROCHA, E.** MicroRNAs Expression Profiles in Cardiovascular Diseases. *Biomed Res Int,* 2014, 985408
- **KIM, K.M. ; S.G. KIM.** Autophagy and microRNA dysregulation in liver diseases. *Arch Pharm Res,* 2014
- **TAN, L. ; J.T. YU ; L. TAN.** Causes and Consequences of MicroRNA Dysregulation in Neurodegenerative Diseases. *Mol Neurobiol,* 2014
- **LEE, R.C. ; R.L. FEINBAUM ; V. AMBROS.** The C. elegans heterochronic gene lin-4 encodes small RNAs with antisense complementarity to lin-14. *Cell,* 1993, vol. 75 (5), 843-54
- **FRIEDMAN, R.C. et al.** Most mammalian mRNAs are conserved targets of microRNAs. *Genome Res,* 2009, vol. 19 (1), 92-105
- **HAYASHITA, Y. et al.** A polycistronic microRNA cluster, miR-17-92, is overexpressed in human lung cancers and enhances cell proliferation. *Cancer Res,* 2005, vol. 65 (21), 9628-32
- **JOVANOVIC, M. ; M.O. HENGARTNER.** miRNAs and apoptosis: RNAs to die for. *Oncogene,* 2006, vol. 25 (46), 6176-87
- **GRASSO, M. et al.** Circulating miRNAs as biomarkers for neurodegenerative disorders. *Molecules,* 2014, vol. 19 (5), 6891-910
- **SAYED, A.S. et al.** Diagnosis, Prognosis and Therapeutic Role of Circulating miRNAs in Cardiovascular Diseases. *Heart Lung Circ,* 2014, vol. 23 (6), 503-510
- **DE CANDIA, P. et al.** Serum microRNAs as Biomarkers of Human Lymphocyte Activation in Health and Disease. *Front Immunol,* 2014, vol. 5, 43
- **SHEINERMAN, K.S. ; S.R. UMANSKY.** Circulating cell-free microRNA as biomarkers for screening, diagnosis and monitoring of neurodegenerative diseases and other neurologic pathologies. *Front Cell Neurosci,* 2013, vol. 7, 150
- **DORVAL, V. ; P.T. NELSON ; S.S. HEBERT.** Circulating microRNAs in Alzheimer's disease: the search for novel biomarkers. *Front Mol Neurosci,* 2013, vol. 6, 24
- **VOGEL, B et al.** Multivariate miRNA signatures as biomarkers for non-ischaemic systolic heart failure. *Eur Heart J,* 2013, vol. 34 (36), 2812-22
- **ENDO, K. et al.** MicroRNA 210 as a biomarker for congestive heart failure. *Biol Pharm Bull,* 2013, vol. 36 (1), 48-54
- **ZHANG, R. et al.** Elevated plasma microRNA-1 predicts heart failure after acute myocardial infarction. *Int J Cardiol,* 2013, vol. 166 (1), 259-60
- **FUKUSHIMA, Y. et al.** Assessment of plasma miRNAs in congestive heart failure. *Circ J,* 2011, vol. 75 (2), 336-40
- **CORSTEN, M.F. et al.** Circulating MicroRNA-208b and MicroRNA-499 reflect myocardial damage in cardiovascular disease. *Circ Cardiovasc Genet,* 2010, vol. 3 (6), 499-506
- **MATSUMOTO, S. et al.** Circulating p53-responsive microRNAs are predictive indicators of heart failure after acute myocardial infarction. *Circ Res,* 2013, vol. 113 (3), 322-6
- **GOREN, Y. et al.** Serum levels of microRNAs in patients with heart failure. *Eur J Heart Fail,* 2012, vol. 14 (2), 147-54
- **XIAO, J et al.** MicroRNA-134 as a potential plasma biomarker for the diagnosis of acute pulmonary embolism. *J Transl Med,* 2011, vol. 9, 159
- **TIJSEN, A.J. et al.** MiR423-5p as a circulating biomarker for heart failure. *Circ Res,* 2010, vol. 106 (6), 1035-9
- **ZHAO, D.S. et al.** Serum miR-210 and miR-30a expressions tend to revert to fetal levels in Chinese adult patients with chronic heart failure. *Cardiovasc Pathol,* 2013, vol. 22 (6), 444-50
- **GOREN, Y. et al.** Relation of reduced expression of MiR-150 in platelets to atrial fibrillation in patients with chronic systolic heart failure. *Am J Cardiol,* 2014, vol. 113 (6), 976-81
- **ELLIS, K.L. et al.** Circulating microRNAs as candidate markers to distinguish heart failure in breathless patients. *Eur J Heart Fail,* 2013, vol. 15 (10), 1138-47
- **REDOVA, M. ; J. SANA ; O. SLABY.** Circulating miRNAs as new blood-based biomarkers for solid cancers. *Future Oncol,* 2013, vol. 9 (3), 387-402
- **MESTDAGH, P. et al.** Evaluation of quantitative miRNA expression platforms in the microRNA quality control (miRQC) study. *Nat Methods,* 2014
- **CISSELL, K.A. ; S.K. DEO.** Trends in microRNA detection. *Anal Bioanal Chem,* 2009, vol. 394 (4), 1109-16
- **TSONGALIS, G.J. et al.** MicroRNA analysis: is it ready for prime time?. *Clin Chem,* 2013, vol. 59 (2), 343-7
- **HINDSON, B.J. et al.** High-throughput droplet digital PCR system for absolute quantitation of DNA copy number. *Anal Chem,* 2011, vol. 83 (22), 8604-10

- **XIONG, M. ; X. FANG ; J. ZHAO.** Biomarker identification by feature wrappers. *Genome Res,* 2001, vol. 11 (11), 1878-87
- **SAEYS, Y. ; I. INZA ; P. LARRANAGA.** A review of feature selection techniques in bioinformatics. *Bioinformatics,* 2007, vol. 23 (19), 2507-17
- **SANTHANAKRISHNAN, R. et al.** The Singapore Heart Failure Outcomes and Phenotypes (SHOP) study and Prospective Evaluation of Outcome in Patients with Heart Failure with Preserved Left Ventricular Ejection Fraction (PEOPLE) study: rationale and design. *J Card Fail,* 2013, vol. 19 (3), 156-62
- **SANTHANAKRISHNAN, R. et al.** Growth differentiation factor 15, ST2, high-sensitivity troponin T, and N-terminal pro brain natriuretic peptide in heart failure with preserved vs. reduced ejection fraction. *Eur J Heart Fail,* 2012, vol. 14 (12), 1338-47

- **MCMURRAY, J.J. et al.** ESC Guidelines for the diagnosis and treatment of acute and chronic heart failure 2012: The Task Force for the Diagnosis and Treatment of Acute and Chronic Heart Failure 2012 of the European Society of Cardiology. Developed in collaboration with the Heart Failure Association (HFA) of the ESC. *Eur Heart J,* 2012, vol. 33 (14), 1787-847
- **ETHERIDGE, A. et al.** Extracellular microRNA: a new source of biomarkers. *Mutat Res,* 2011, vol. 717 (1-2), 85-90
- **LI, Y. ; K.V. KOWDLEY.** Method for microRNA isolation from clinical serum samples. *Anal Biochem,* 2012, vol. 431 (1), 69-75
- **BENJAMINI, Y. ; HOCHBERG, Y.** Controlling the false discovery rate: A practical and powerful approach to multiple testing. *ournal of the Royal Statistical Society,* 1995, vol. 57, 289-300